(19) Europäisches Patentamt
European Patent Office
Office européen des brevets

(11) EP 4 578 948 A1

(12) EUROPEAN PATENT APPLICATION
published in accordance with Art. 153(4) EPC

(43) Date of publication:
**02.07.2025 Bulletin 2025/27**

(21) Application number: **24866584.6**

(22) Date of filing: **18.03.2024**

(51) International Patent Classification (IPC):
***C12N 15/113*** *(2010.01)* ***A61K 31/713*** *(2006.01)*
***A61P 3/06*** *(2006.01)* ***A61P 9/10*** *(2006.01)*
***A61P 9/00*** *(2006.01)*

(86) International application number:
**PCT/CN2024/082133**

(87) International publication number:
**WO 2025/097633 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.11.2023 CN 202311463375**

(71) Applicant: **Hangzhou Tianlong Pharmaceutical Co., Ltd.**
**Hangzhou, Zhejiang 310009 (CN)**

(72) Inventors:
- **SONG, Gengshen**
  **Beijing 100176 (CN)**
- **HUANG, Zeao**
  **Beijing 100176 (CN)**
- **YANG, Shuo**
  **Beijing 100176 (CN)**
- **TIAN, Zhikang**
  **Beijing 100176 (CN)**
- **WU, Yucheng**
  **Beijing 100176 (CN)**
- **YAO, Peng**
  **Beijing 100176 (CN)**
- **YU, Xiaowen**
  **Beijing 100176 (CN)**
- **HUANG, Dawei**
  **Beijing 100176 (CN)**
- **PANG, Xue**
  **Beijing 100176 (CN)**
- **LIN, Fang**
  **Beijing 100176 (CN)**

(74) Representative: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

Remarks:
The complete document including Reference Table(s) and the Sequence Listing(s) can be downloaded from the EPO website

# (54) SIRNA FOR TARGETED REGULATION OF PCSK9 GENE EXPRESSION, AND USE THEREOF

(57) The present disclosure relates to siRNAs that target and regulate PCSK9 gene expression and use thereof. Experimental results show that some alternately modified and specific template-modified oligonucleotide sequences can significantly inhibit PCSK9 expression and can be used to develop drugs to treat PCSK9-related diseases.



Processed by Luminess, 75001 PARIS (FR)

## Description

This application claims the priority of Chinese Patent Application No. 202311463375.7, filed with the China National Intellectual Property Administration on November 6, 2023, and titled with "SIRNA TARGETING AND REGULATING PCSK9 GENE EXPRESSION AND USE THEREOF", which is hereby incorporated by reference in its entirety.

## TECHNICAL FIELD

The present disclosure relates to the technical field of nucleic acid modification, and specifically relates to a small interfering ribonucleic acid (siRNA) modified by multiple chemical methods and use thereof in the manufacture of a medicament for a disease related to PCSK9 gene expression.

## BACKGROUND

Nucleic acid drugs, particularly oligonucleotide drugs, are widely employed due to their straightforward synthesis and high activity. Oligonucleotide drugs typically encompass antisense oligonucleotides (ASOs), small interfering RNA (siRNA), microRNA (miRNA), and nucleic acid aptamers.

Oligonucleotides are a class of short DNA or RNA molecules or oligomers that readily bind to their respective complementary oligonucleotides, DNA or RNA in a sequence-specific manner to form duplexes, or less commonly, higher order hybrids. This fundamental property renders oligonucleotides a valuable tool in genetic testing, research and medicine. In their natural state, oligonucleotides are typically small RNA molecules that regulate gene expression or intermediates derived from the decomposition of larger nucleic acid molecules.

RNA interference (RNAi) is a natural defense mechanism against foreign genes. siRNA can knock out target genes by recognizing specific sequences and degrading target mRNA.

Effective molecules of classic RNAi are composed of the iconic 19 + 2 nucleotide polymer structure, which is a double helix consisting of a 21-nucleotide RNA molecule and a nucleotide molecule corresponding to 19 nucleobases, including a 2-nucleotide 3'-end overhang. One strand of siRNA (the guide strand or antisense strand) is complementary to the target gene transcript mRNA, and the other strand is labeled as the passenger strand (or sense strand). The antisense strand of siRNA guides Argonaute protein (AGO2) to complement the target transcript and is incorporated into the RNA-induced silencing complex (RISC). The complete complementarity between the siRNA (the antisense strand) and the target leads to the cleavage of the target transcript at the 10 to 11 position opposite the guide strand (antisense strand) under the catalysis of the AGO2 protein.

siRNA has innate advantages over small molecules and antibody drugs because siRNA executes its function by complete Watson-Crick base pairing with mRNA, whereas small molecules and monoclonal antibody drugs need to recognize the complicated spatial conformation of certain proteins. As a result, there are many diseases are not treatable by small molecules and monoclonal antibody drugs since their target molecules have high activity, and the molecular structures with which they have affinity and binding specificity cannot be recognized. The mechanism of action of siRNA drugs enables them to regulate the expression of target proteins at the genetic level, and has targeting specificity compared to small molecules or antibody drugs. Its mechanism based on the principle of complementary base pairing also confers the siRNA with a wider therapeutic area, simpler design and a shorter research and development span.

Oligonucleotides are capable of binding complementary RNA chains in a sequence-specific manner and can induce RNase H to cleave target RNA after hybridization. In natural oligonucleotides, nucleotides are linked by phosphodiester bonds, which are particularly vulnerable to nucleases under physiological conditions. Therefore, natural, structurally unmodified, and unmodified oligonucleotide drugs, which are susceptible to rapid degradation by nucleases in vivo, exhibit low activity and poor drugability. Chemical modification of the oligonucleotide structure is an effective method of increasing its activity, which can improve its stability to nucleases and its affinity to RNA, and facilitate cellular endocytosis and tissue targeting, thereby effectively regulating the expression of the target gene.

Four types of chemical modifications can be performed on oligonucleotides according to their basic structure including a base, a sugar ring, a phosphate backbone, and ends.

1) Modification of base: It is mainly divided into three forms: purine modification, pyrimidine modification and base substitution. Purine modification includes N6-methyladenosine, N1-methyladenosine, and 7-methylguanylate. Pyrimidine modification includes 3-methyluridine, 5-methyluridine, 5-methylcytosine, N4-acetylcytidine, pseudouridine, thiouridine, propyne uridine and dihydrouridine.

2) Modification of sugar ring: It is mainly divided into sugar ring modification and substitution. Sugar ring modification includes 2'-modification, 4'-modification, 5'-modification, isomeric modification, and combinations thereof. The most common 2'-modifications of siRNA are 2'-OMe (2'-methoxy) and 2'-F (2'-fluoro) modifications. siRNA modified with

both 2'-OMe and 2'-F has a higher Tm value, enhanced serum stability and better activity than natural siRNA.

3) Modification of phosphate backbone: It mainly includes modification with phosphorothioate; modification with methyl phosphonate, selenophosphate, methylborylphosphate or dithiophosphate, and replacement of the bridging oxygen atom in the phosphodiester linkage region with a sulfur atom; replacement of the entire phosphate group between nucleosides with a group that does not contain a phosphorus atom, such as replacement of the P atom with a C, S, or N atom to form a guanidine group, S-methylthiourea, and the like.

4) Modification of end: It includes covalent conjugation of special groups at the 5'-end and/or 3'-end of the sense chain and phosphorylation modification of the 5'-end of the antisense chain.

All oligonucleotide drugs that have been marketed have undergone chemical modification. Since the first nucleic acid drug, Fomivirsen (Vitravene), was approved for marketing in 1998, the chemical modification technology of nucleic acid drugs has been continuously upgraded. So far, 18 nucleic acid drugs have been approved for marketing worldwide:

Table 1 Nucleic acid drugs approved for marketing worldwide

| No. | Generic name (Trade name) | Company | Classification | Indications | Approval Date |
|---|---|---|---|---|---|
| 1 | Fomivirsen (Vitravene) | "Ionis/Novartis" Novartis | Antisense Oligonucleotides | Cytomegalovirus (CMV) retinitis | 1998-08-01 |
| 2 | Pegaptanib (Macugen) | NeXstar/Eyetech | Aptamers | Neovascular age-related macular degeneration | 2004-12-01 |
| 3 | Mipomersen (Kynamro) | Ionis/Genzyme/Kastle | Antisense Oligonucleotides | Homozygous familial hypercholesterolemia | 2013-01-01 |
| 4 | Defibrotide (Defitelio) | Jazz | Oligonucleotide mixture | Hepatic veno-occlusive disease | 2016-03-01 |
| 5 | Eteplirsen (Exondys 51) | Sarepta | Antisense Oligonucleotides | Duchenne muscular dystrophy | 2016-09-01 |
| 6 | Nusinersen (Spinraza) | Ionis/Biogen | Antisense Oligonucleotides | Spinal muscular atrophy | 2016-12-01 |
| 7 | Patisiran (Onpattro) | Alnylam | siRNA | Hereditary transthyretin amyloidosis, polyneuropathy | 2018-08-01 |
| 8 | Inotersen (Tegsedi) | Ionis/Akcea | Antisense Oligonucleotides | Hereditary transthyretin amyloidosis, polyneuropathy | 2018-10-01 |
| 9 | Volanesorsen (Waylivra) | Ionis | Antisense Oligonucleotides | Familial Chylomicronemia Syndrome (FCS) | 2019-05-03 |
| 10 | Givosiran (Givlaari) | Alnylam | siRNA | Hepatic porphyria | 2019-11-01 |
| 11 | Golodirsen (Vyondys 53) | Sareta | Antisense Oligonucleotides | Duchenne muscular dystrophy | 2019-12-01 |
| 12 | Viltolarsen (Viltepso) | Nippon Shinyaku | Antisense Oligonucleotides | Pseudohypertrophic muscular dystrophy | 2020-03-25 |
| 13 | Lumasiran (Oxlumo) | Alnylam Pharmaceuticals, Dicerna Pharmaceuticals (Novo Nordisk) | siRNA | Hyperoxaluria | 2020-11-24 |
| 14 | Casimersen (Amondys 45) | Sarepta Therapeutics, University of Western Australia | Antisense Oligonucleotides | Pseudohypertrophic muscular dystrophy | 2021-02-25 |

(continued)

| No. | Generic name (Trade name) | Company | Classification | Indications | Approval Date |
|---|---|---|---|---|---|
| 15 | Inclisiran (Leqvio) | Alnylam Pharmaceuticals, The Medicines Company (Novartis) | siRNA | Atherosclerosis, hypercholesterolemia, combined hyperlipidemia | 2021-12-22 |
| 16 | Amvuttra (vutrisiran) | Alnylam | siRNA | Amyloid polyneuropathy | 2022-06-13 |
| 17 | Qalsody (tofersen) | Biogen | Antisense Oligonucleotides | Amyotrophic lateral sclerosis (ALS) | 2023-04-25 |
| 18 | Avaccincaptad Pegol (Izervay) | Iveric Bio | Aptamers | Geographic atrophy (GA) secondary to age-related macular degeneration (AMD) | 2023-08-04 |

The treatment strategy for hypercholesterolemia is primarily focused on the reduction of low-density lipoprotein cholesterol (LDL-C), which causes atherosclerosis, and the elevation of high-density lipoprotein cholesterol (HDL-C), which has a potential cardioprotective effect. Clinical studies involving nearly 170,000 subjects demonstrated that a 1.0 mmol/L reduction in LDL-C levels is associated with a 20% reduction in the average annual incidence rate of major cardiovascular disease events. The drugs recommended in the 2019 version of the "Guidelines for Primary Diagnosis and Treatment of Dyslipidemia" for the reduction of LDL-C encompass a range of options, including statins, cholesterol absorption inhibitors, Robutecol, PCSK9 inhibitors, and others, among which statins are identified as the preferred initial choice. The 2018 version of the "Chinese Expert Consensus on Screening, Diagnosis and Treatment of Familial Hypercholesterolemia" recommends statins and combined treatment with ezetimibe and PCSK9 inhibitors.

Proprotein convertase subtilisin/kexin type 9 (PCSK9) is a serine protease encoded by the PCSK9 gene, with the majority of its production occurring in the liver. PCSK9 binds to the LDL receptor (LDL-R) on the surface of liver cells, leading to the degradation of LDL-R. Given that LDL-R is responsible for binding to LDL-C in plasma and subsequently transporting LDL-C into the liver, which processes the blood lipids in it and excretes it through bile, the degradation of LDL-R will result in an elevation of LDL-C levels in plasma. Inhibitors of PCSK9 reduce the expression level of PCSK9, increase the level of LDL-R on the surface of liver cells, and thus reduce the level of LDL-C in plasma, achieving the objective of lowering blood lipids.

In order to effectively treat hypercholesterolemia and dyslipidemia, there is a need in the art to further develop drugs that regulate PCSK9 gene expression.

**SUMMARY**

In the present disclosure, a series of unique siRNA sequences are designed by targeting the PCSK9 mRNA sequence and subjected to alternate modifications and specific template modifications.

It is common practice to modify siRNA with 2'-methoxy (2'-OMe) and 2'-fluoro (2'-F) for the monomer. However, even if only the combination of the above two monomer modifications is considered, for siRNA, which has a total of 44 bases on the sense strand and antisense strand, there are $2^{44}$ potential combinations. Furthermore, with different terminal thiolation modifications, the number of possible modification schemes is significantly increased.

Identical siRNA sequences undergoing different modification methods display markedly disparate activities. Similarly, siRNA sequences that have been modified using the same modification method exhibit considerable variation in their activities. Despite certain principles for the design of modification of siRNAs, previous studies have demonstrated that the activity cannot be accurately inferred based on the modification method employed. This indicates that there is no definitive correlation between the modification method and the activity. Consequently, it is exceedingly challenging to identify modification schemes with high activity from the multitude of potential combinations.

In the present disclosure, chemical modifications are introduced to the designed siRNA sequences, and some sequences with alternate modifications and special modifications that exhibit a notable inhibitory effect on PCSK9 gene expression are screened out.

In an aspect, the present disclosure provides a double-stranded RNAi agent for reducing PCSK9 expression, comprising an oligonucleotide duplex consisting of paired sense strand and antisense strand, wherein

(1) the sense strand comprises a sequence set forth in SEQ ID NO: 1 or a fragment thereof, or a modified sequence of

the sequence or the fragment thereof; and the antisense strand comprises a sequence set forth in SEQ ID NO: 13 or a fragment thereof, or a modified sequence of the sequence or the fragment thereof;

(2) the sense strand comprises a sequence set forth in SEQ ID NO: 2 or a fragment thereof, or a modified sequence of the sequence or the fragment thereof; and the antisense strand comprises a sequence set forth in SEQ ID NO: 14 or a fragment thereof, or a modified sequence of the sequence or the fragment thereof;

(3) the sense strand comprises a sequence set forth in SEQ ID NO: 3 or a fragment thereof, or a modified sequence of the sequence or the fragment thereof; and the antisense strand comprises a sequence set forth in SEQ ID NO: 15 or a fragment thereof, or a modified sequence of the sequence or the fragment thereof;

(4) the sense strand comprises a sequence set forth in SEQ ID NO: 4 or a fragment thereof, or a modified sequence of the sequence or the fragment thereof; and the antisense strand comprises a sequence set forth in SEQ ID NO: 16 or a fragment thereof, or a modified sequence of the sequence or the fragment thereof;

(5) the sense strand comprises a sequence set forth in SEQ ID NO: 5 or a fragment thereof, or a modified sequence of the sequence or the fragment thereof; and the antisense strand comprises a sequence set forth in SEQ ID NO: 17 or a fragment thereof, or a modified sequence of the sequence or the fragment thereof;

(6) the sense strand comprises a sequence set forth in SEQ ID NO: 6 or a fragment thereof, or a modified sequence of the sequence or the fragment thereof; and the antisense strand comprises a sequence set forth in SEQ ID NO: 18 or a fragment thereof, or a modified sequence of the sequence or the fragment thereof;

(7) the sense strand comprises a sequence set forth in SEQ ID NO: 7 or a fragment thereof, or a modified sequence of the sequence or the fragment thereof; and the antisense strand comprises a sequence set forth in SEQ ID NO: 19 or a fragment thereof, or a modified sequence of the sequence or the fragment thereof;

(8) the sense strand comprises a sequence set forth in SEQ ID NO: 8 or a fragment thereof, or a modified sequence of the sequence or the fragment thereof; and the antisense strand comprises a sequence set forth in SEQ ID NO: 20 or a fragment thereof, or a modified sequence of the sequence or the fragment thereof;

(9) the sense strand comprises a sequence set forth in SEQ ID NO: 9 or a fragment thereof, or a modified sequence of the sequence or the fragment thereof; and the antisense strand comprises a sequence set forth in SEQ ID NO: 21 or a fragment thereof, or a modified sequence of the sequence or the fragment thereof;

(10) the sense strand comprises a sequence set forth in SEQ ID NO: 10 or a fragment thereof, or a modified sequence of the sequence or the fragment thereof; and the antisense strand comprises a sequence set forth in SEQ ID NO: 22 or a fragment thereof, or a modified sequence of the sequence or the fragment thereof;

(11) the sense strand comprises a sequence set forth in SEQ ID NO: 11 or a fragment thereof, or a modified sequence of the sequence or the fragment thereof; and the antisense strand comprises a sequence set forth in SEQ ID NO: 23 or a fragment thereof, or a modified sequence of the sequence or the fragment thereof; or

(12) the sense strand comprises a sequence set forth in SEQ ID NO: 12 or a fragment thereof, or a modified sequence of the sequence or the fragment thereof; and the antisense strand comprises a sequence set forth in SEQ ID NO: 24 or a fragment thereof, or a modified sequence of the sequence or the fragment thereof.

In another aspect, the present disclosure further provides a conjugate for reducing PCSK9 expression, comprising the double-stranded RNAi agent and a ligand conjugated thereto.

In another aspect, the present disclosure further provides a pharmaceutical composition, comprising the double-stranded RNAi agent or the conjugate, and a pharmaceutically acceptable carrier.

In another aspect, the present disclosure further provides use of the aforementioned double-stranded RNAi agent, the conjugate or the pharmaceutical composition in the manufacture of a medicament for treating a PCSK9-related disease.

In another aspect, the present disclosure further provides a method for treating or preventing a diseases or condition that can be modulated by downregulating PCSK9 gene expression.

The beneficial effects achieved by the present disclosure are at least as follows:

(1) Unmodified sequences, including basic sequences 5, 51, 81, 82, 84, 3, 8, 9, 21, 31, 73 and 83, all exhibit a significant inhibitory effect on PCSK9, with the highest inhibition rate exceeding 60%.

(2) The inhibition rate of sequences with multiple modifications reaches up to 90% or more. Moreover, the modified sequence conjugated with a GalNAc compound can be efficiently delivered to the animal liver, markedly inhibiting PCSK9 gene expression, substantially reducing low-density lipoprotein cholesterol (LDL-C) levels in serum, and significantly reducing total cholesterol (TC) levels in serum.

(3) Each sequence modified by alternate modifications and template modifications of the present disclosure has significantly improved inhibitory activity against PCSK9, exhibiting up to 40% improvement compared with the unmodified sequences which are either identical to or have very little difference from the sequences disclosed in the prior art.

(4) The present disclosure finds that siRNAs with similar sequences exhibit markedly disparate activities, irrespective of whether they are unmodified or alternately modified. For example, the inhibition rate of the unmodified basic sequence 5 is 51.2% higher than that of the basic sequence 4, and the inhibition rate of the alternately modified sequence P92-si5 is 61.8% higher than that of P92-si4, which represents a significant improvement.

(5) The present disclosure also reveals that different sequences exhibit disparate effects on activity after alternate modifications. Some inhibition rates demonstrate a notable enhancement, exemplified by basic sequence 5, whose alternately modified sequence exhibits an elevated inhibition rate of 12.4% relative to its unmodified sequence. Some inhibition rates exhibit a less pronounced alteration, for example, as observed in basic sequences 4, 22, and 52, the inhibition rates of alternately modified and unmodified sequences exhibit minimal deviation.

## BRIEF DESCRIPTION OF DRAWINGS

In order to more clearly illustrate the technical solutions of the embodiments of the present disclosure, the drawings of the embodiments will be briefly introduced below. Obviously, the drawings in the following description only relate to some embodiments of the present disclosure and do not limit the present disclosure.

FIG. 1 shows alternately modified sequences that have a significant inhibitory effect on the PCSK9 gene, with an inhibition rate higher than 50%.

FIG. 2 shows alternately modified sequences with an inhibition rate against the PCSK9 gene between 30% and 50%.

FIG. 3 shows alternately modified sequences with an inhibition rate against the PCSK9 gene less than 30%.

FIG. 4 shows alternately modified sequences with an inhibition rate against the PCSK9 gene less than 30%.

FIG. 5 shows unmodified sequences that have a significant inhibitory effect on the PCSK9 gene, with an inhibition rate higher than 50%.

FIG. 6 shows unmodified sequences with an inhibition rate against the PCSK9 gene less than 40%.

FIG. 7 shows the inhibition rates against the PCSK9 gene by basic sequence 5 modified with different template modifications.

FIG. 8 shows the inhibition rates against the PCSK9 gene by basic sequences 5, 51, 81 and 84 modified with template modification, anti-off-target or/and 5'-E-VP modification.

FIG. 9 shows the inhibition rates of the PCSK9 protein in animal serum by basic sequences 5, 51, 81, 82 and 84 modified with different schemes and conjugated with a GalNAc compound.

FIG. 10 shows the reduction levels of low-density lipoprotein cholesterol (LDL-C) in animal serum by basic sequences 5, 51, 81, 82 and 84 modified with different schemes and conjugation with a GalNAc compound.

FIG. 11 shows the reduction levels of total cholesterol (TC) in animal serum by basic sequences 5, 51, 81, 82 and 84 modified with different schemes and conjugated with a GalNAc compound.

## DETAILED DESCRIPTION

In order to make the present disclosure more easily understood, certain terms are first defined. In addition, it should be noted that whenever a value or a range of values for a parameter is listed, it is intended to indicate that the intermediate values and ranges of these cited values are also intended to be part of the present disclosure.

As used herein, the articles "a" and "an" refer to one or more than one (i.e., at least one) of the grammatical object of the article. By way of example, "an element" refers to one element or more than one element, such as a plurality of elements.

The term "including" is used herein to mean, and is used interchangeably with, the phrase "including, but not limited to."

The term "or" is used herein to mean, and is used interchangeably with, the term "and/or," unless the context clearly dictates otherwise.

As used herein, the term "about" or "approximately" as applied to one or more target values refers to a value similar to the reference value. In certain embodiments, unless otherwise specified or in addition apparent from the context, the term "about" or "approximately" refers to a range of values falling into 20%, 19%, 18%, 17%, 16%, 15%, 14%, 13%, 12%, 11%, 10%, 9%, 8%, 7%, 6%, 5%, 4%, 3%, 2%, 1% or less in either direction (greater than or less than) of the reference value (unless such numeral will exceed 100% of a possible value).

As used herein, "PCSK9" refers to the proprotein convertase subtilisin Kexin 9 gene or protein.

"G", "C", "A" and "U" each generally represent nucleotides comprising guanine, cytosine, adenine and uracil as bases, respectively. "T" and "dT" are used interchangeably herein and refer to deoxyribonucleotides in which the nucleobase is thymine, such as deoxyribothymine, 2'-deoxythymidine or thymidine. However, it should be understood that the term "ribonucleotide" or "nucleotide" or "deoxyribonucleotide" may also refer to a modified nucleotide (as further described below) or an alternative replacement moiety. The skilled person will be well aware that guanine, cytosine, adenine and uracil may be replaced by other moieties without substantially changing the base pairing properties of an oligonucleotide (including a nucleotide having such a replacement moiety). For example, without limitation, a nucleotide comprising inosine as its base may base pair with a nucleotide comprising adenine, cytosine or uracil. Thus, a nucleotide comprising uracil, guanine or adenine may be replaced in the nucleotide sequences of the present disclosure by a nucleotide comprising, for example, inosine. Sequences comprising such replacement moieties are embodiments of the present disclosure.

The terms "RNAi agent", "iRNA", "iRNA agent", and "RNA interference agent" are used interchangeably herein and refer to RNA agents as defined herein, which mediate targeted cleavage of RNA transcripts through the RNA induced silencing complex (RISC) pathway. RNAi directs sequence-specific degradation of mRNA through a process known as RNA interference (RNAi). RNAi regulates, e.g., inhibits, expression of PCSK9 in cells such as cells in a subject (e.g., a mammalian subject). RNAi molecules include single-stranded RNAi molecules and double-stranded siRNAs, as well as short hairpin RNAs (shRNAs).

The term "small interfering ribonucleic acid" or "siRNA" refers to a small interfering ribonucleic acid RNAi molecule. It is a class of double-stranded RNA molecules, also referred to in the art as short interfering RNA or silencing RNA. siRNAs typically comprise a sense strand (also referred to as a passenger strand) and an antisense strand (also referred to as a guide strand), and each strand is 17 to 30 nucleotides in length, typically 19 to 25 nucleosides in length. The antisense strand is complementary (such as at least 95% complementary, such as fully complementary) to the target nucleic acid (suitably a mature mRNA sequence), and the sense strand is complementary to the antisense strand, such that the sense strand and the antisense strand form a duplex or duplex region. The siRNA strand may form a blunt-ended duplex, or preferably, the 3'-ends of the sense and antisense strands may form a 3' overhang of, for example, 1, 2 or 3 nucleosides, similar to the product produced by Dicer, which may form a RISC substrate in vivo. Effective extended forms of Dicer substrates have been described in US 8,349,809 and US 8,513,207, which are incorporated herein by reference. In some embodiments, both the sense and antisense strands have 2 nt 3' overhangs. Thus, the length of the duplex region may be, for example, 17 to 25 nucleotides, such as 21 to 23 nucleotides in length.

The term "antisense strand" refers to a strand of RNAi (e.g., dsRNA) that includes a region that is substantially complementary to a target sequence. As used herein, the term "complementarity region" refers to a region on the antisense strand that is substantially complementary to the sequence as defined herein (e.g., a target sequence). When the complementary region is not completely complementary to the target sequence, mispairing may be in the interior or terminal regions of the molecule. Typically, the most tolerated mispairing is in the terminal regions, e.g., within 5, 4, 3, or 2 nucleotides at the 5' and/or 3'-ends.

The term "sense strand" as used herein refers to the strand of RNAi that includes a region that is substantially complementary to a region of the antisense strand (as that term is defined herein).

As used herein, the term "inhibit" may be used interchangeably with "reduce," "silence," "downregulate," "suppress," and other similar terms, and includes any level of inhibition.

As used herein, the phrase "inhibiting PCSK9 expression" includes inhibiting the expression of any PCSK9 gene

(such as, for example, a mouse PCSK9 gene, a rat PCSK9 gene, a monkey PCSK9 gene, or a human PCSK9 gene) and a variant (such as a naturally occurring variant) or mutant of a PCSK9 gene. Thus, the PCSK9 gene can be a wild-type PCSK9 gene, a mutant PCSK9 gene, or a transgenic PCSK9 gene in the context of a genetically manipulated cell, cell group, or organism.

"Inhibiting PCSK9 gene expression" includes any level of inhibition against the PCSK9 gene, such as at least partial inhibition of the expression of the PCSK9 gene, such as inhibition of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

Based on any variable level associated with PCSK9 gene expression, such as PCSK9 mRNA level, PCSK9 protein level, or serum lipid level, the expression of PCSK9 gene can be assessed. Inhibition can be assessed by the reduction of one or more of these variables in absolute or relative levels compared to a control level. A control level can be any type of control level utilized in the art, such as a baseline level before administration or a level determined from a similar untreated or control (e.g., only a buffer control or an inert agent control) treated subject, cell, or sample.

As used herein, "patient" or "subject" is intended to include humans or non-human animals, preferably mammals, such as monkeys. Most preferably, the subject or patient is a human.

As used herein, "PCSK9-related diseases" are intended to include any disease associated with the PCSK9 gene or protein. Such diseases may be caused, for example, by overproduction of PCSK9 protein, by PCSK9 gene mutations, by abnormal cleavage of PCSK9 protein, by abnormal interactions between PCSK9 and other proteins or other endogenous or exogenous substances. Exemplary PCSK9-related diseases include lipidemias, such as hyperlipidemia, and other forms of lipid imbalances, such as hypercholesterolemia, hypertriglyceridemia, and pathological conditions associated with these disorders, such as heart and circulatory system diseases.

As used herein, "therapeutically effective amount" is intended to include an amount of an RNAi agent sufficient to achieve treatment of a PCSK9-related disease (e.g., by attenuating, ameliorating, or maintaining the existing disease or one or more symptoms of the disease) when administered to a patient. The "therapeutically effective amount" may vary depending on the RNAi agent, how the agent is administered, the disease and its severity, medical history, age, weight, family history, genetic makeup, the stage of the pathological process mediated by PCSK9 expression, the type of previous or concomitant therapy (if any), and other individual characteristics of the patient to be treated.

As used herein, a "prophylactically effective amount" is intended to include an amount of an RNAi agent sufficient to prevent or ameliorate a PCSK9-related disease or one or more symptoms of the disease when administered to a subject who has not yet experienced or shown symptoms of the disease but may be susceptible to the disease. Ameliorating the disease includes slowing the progression of the disease or reducing the severity of a subsequent disease. The "prophylactically effective amount" may vary depending on the RNAi agent, how the agent is administered, the degree of risk of the disease, medical history, age, weight, family history, genetic makeup, the type of previous or concomitant therapy (if any), and other individual characteristics of the patient to be treated.

"Therapeutically effective amount" or "prophylactically effective amount" also includes an amount of an RNAi agent that produces a certain desired local or systemic effect at a reasonable benefit/risk ratio applicable to any treatment. The RNAi agent used in the method of the present disclosure can be administered in an amount sufficient to produce a reasonable benefit/risk ratio applicable to such treatment.

As used herein, the term "sample" includes similar fluids, cells or tissues separated from a subject, and a collection of fluids, cells or tissues present in a subject. Examples of biological fluids include blood, serum and serosal fluid, plasma, cerebrospinal fluid, ocular fluid, lymph, urine, saliva, etc. Tissue samples may include samples from tissues, organs or local areas. For example, a sample may be derived from a specific organ, an organ part or a fluid or cell from these organs. In certain embodiments, a sample may be derived from liver (for example, the whole liver or some segments of liver, or some types of cells in the liver, for example, hepatocytes). In a preferred embodiment, "sample derived from a subject" refers to blood or plasma extracted from the subject. In some other embodiments, "sample derived from a subject" refers to liver tissue (or its subcomponent) derived from the subject.

In an aspect, the present disclosure provides a double-stranded RNAi agent for reducing PCSK9 expression, comprising an oligonucleotide duplex selected from any one of the following paired sense strand and antisense strand, wherein

(1) the sense strand has a sequence set forth in SEQ ID NO: 1 or a fragment thereof, or a modified sequence of the sequence or the fragment thereof; and the antisense strand has a sequence set forth in SEQ ID NO: 13 or a fragment thereof, or a modified sequence of the sequence or the fragment thereof;

(2) the sense strand has a sequence set forth in SEQ ID NO: 2 or a fragment thereof, or a modified sequence of the

sequence or the fragment thereof; and the antisense strand has a sequence set forth in SEQ ID NO: 14 or a fragment thereof, or a modified sequence of the sequence or the fragment thereof;

(3) the sense strand has a sequence set forth in SEQ ID NO: 3 or a fragment thereof, or a modified sequence of the sequence or the fragment thereof; and the antisense strand has a sequence set forth in SEQ ID NO: 15 or a fragment thereof, or a modified sequence of the sequence or the fragment thereof;

(4) the sense strand has a sequence set forth in SEQ ID NO: 4 or a fragment thereof, or a modified sequence of the sequence or the fragment thereof; and the antisense strand has a sequence set forth in SEQ ID NO: 16 or a fragment thereof, or a modified sequence of the sequence or the fragment thereof;

(5) the sense strand has a sequence set forth in SEQ ID NO: 5 or a fragment thereof, or a modified sequence of the sequence or the fragment thereof; and the antisense strand has a sequence set forth in SEQ ID NO: 17 or a fragment thereof, or a modified sequence of the sequence or the fragment thereof;

(6) the sense strand has a sequence set forth in SEQ ID NO: 6 or a fragment thereof, or a modified sequence of the sequence or the fragment thereof; and the antisense strand has a sequence set forth in SEQ ID NO: 18 or a fragment thereof, or a modified sequence of the sequence or the fragment thereof;

(7) the sense strand has a sequence set forth in SEQ ID NO: 7 or a fragment thereof, or a modified sequence of the sequence or the fragment thereof; and the antisense strand has a sequence set forth in SEQ ID NO: 19 or a fragment thereof, or a modified sequence of the sequence or the fragment thereof;

(8) the sense strand has a sequence set forth in SEQ ID NO: 8 or a fragment thereof, or a modified sequence of the sequence or the fragment thereof; and the antisense strand has a sequence set forth in SEQ ID NO: 20 or a fragment thereof, or a modified sequence of the sequence or the fragment thereof;

(9) the sense strand has a sequence set forth in SEQ ID NO: 9 or a fragment thereof, or a modified sequence of the sequence or the fragment thereof; and the antisense strand has a sequence set forth in SEQ ID NO: 21 or a fragment thereof, or a modified sequence of the sequence or the fragment thereof;

(10) the sense strand has a sequence set forth in SEQ ID NO: 10 or a fragment thereof, or a modified sequence of the sequence or the fragment thereof; and the antisense strand has a sequence set forth in SEQ ID NO: 22 or a fragment thereof, or a modified sequence of the sequence or the fragment thereof;

(11) the sense strand has a sequence set forth in SEQ ID NO: 11 or a fragment thereof, or a modified sequence of the sequence or the fragment thereof; and the antisense strand has a sequence set forth in SEQ ID NO: 23 or a fragment thereof, or a modified sequence of the sequence or the fragment thereof; or

(12) the sense strand has a sequence set forth in SEQ ID NO: 12 or a fragment thereof, or a modified sequence of the sequence or the fragment thereof; and the antisense strand has a sequence set forth in SEQ ID NO: 24 or a fragment thereof, or a modified sequence of the sequence or the fragment thereof.

In some embodiments, all nucleotides in the sense strand and the antisense strand are modified nucleotides.

In some embodiments, the double-stranded RNAi agent is an RNAi agent for inhibiting PCSK9 gene expression.

In some embodiments, the sense strand differs from any one of SEQ ID NOs. 1 to 12 by 1 to 3 nucleotides.

In some embodiments, the antisense strand differs from any one of SEQ ID NOs. 13 to 24 by 1 to 3 nucleotides.

In some embodiments, at least one modified nucleotide is selected from the group consisting of: a deoxynucleotide, a 3'-end deoxy-thymine (dT) nucleotide, a 2'-O-methyl modified nucleotide, a 2'-fluoro modified nucleotide, a 2'-deoxy-modified nucleotide, a locked nucleotide, a non-locked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising phosphorothioate, a nucleotide comprising methylphosphonate, a nucleotide comprising a 5'-phosphate, and a nucleotide comprising a 5'-phosphate mimic.

In some embodiments, at least one strand comprises a 3' overhang of at least 1 nucleotide.

In some embodiments, at least one strand comprises a 3' overhang of at least 2 nucleotides.

In some embodiments, the double-stranded region is from 15 to 30 nucleotide pairs in length.

In some embodiments, the double-stranded region is from 17 to 25 nucleotide pairs in length.

In some embodiments, the double-stranded region is from 19 to 23 nucleotide pairs in length.

In some embodiments, the double-stranded region is from 21 nucleotide pairs in length.

In some embodiments, each strand has 15 to 30 nucleotides.

In some embodiments, each strand has 19 to 25 nucleotides.

In some embodiments, the sense strand has 21 nucleotides and the antisense strand has 23 nucleotides.

In some embodiments, all nucleotides in the sense strand and the antisense strand are modified with a chemical modification on the 2' position of the ribose of the nucleotide.

In some embodiments, the chemical modification on the 2' position of the ribose of the nucleotide is selected from the group consisting of 2'-methoxy, 2'-methoxyethyl, 2'-fluoro, 2'-benzyloxy, 2'-methylcarbonylamino, 2'-pyridylmethoxy, and a combination thereof.

In some embodiments, the chemical modification on the 2' position of the ribose of the nucleotide is a combination of 2'-methoxy and 2'-fluoro.

In some embodiments, the chemical modification on the 2' position of the ribose of the nucleotide is alternate 2'-methoxy and 2'-fluoro.

In some embodiments, for the chemical modification on the 2' position of the ribose of the nucleotide, the nucleotides in the sense strand are modified with 2'-fluoro in odd-numbered positions and with 2'-methoxy in even-numbered positions, and the nucleotides in the antisense strand are modified with 2'-methoxy in odd-numbered positions and with 2'-fluoro in even-numbered positions.

In some embodiments, nucleotide monomers are connected by a 3',5'-phosphodiester bond.

In some embodiments, nucleotide monomers are connected by a 3',5'-phosphodiester bond with thio-modification.

In some embodiments, the aforementioned oligonucleotides comprises the following modifications:

the antisense strand is modified with modifications selected from the group consisting of A, B and C:

| No. | Antisense strand (AS) 5'-3' | | | | | | | | | | | | | | | | | | | | | | |
|---|---|---|---|---|---|---|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** |
| **A** | 2'-OMe+PS | **2'-F+PS** | 2'-OMe | 2'-OMe | 2'-OMe | **2'-F** | 2'-OMe | 2'-OMe | 2'-OMe | 2'-OMe | 2'-OMe | 2'-OMe | 2'-OMe | **2'-F** | 2'-OMe | **2'-F** | 2'-OMe | 2'-OMe | 2'-OMe | 2'-OMe | 2'-OMe+PS | 2'-OMe+PS | 2'-OMe |
| **B** | 2'-OMe+PS | **2'-F+PS** | **2'-F** | **2'-F** | 2'-OMe | **2'-F** | 2'-OMe | 2'-OMe | 2'-OMe | 2'-OMe | 2'-OMe | 2'-OMe | 2'-OMe | **2'-F** | 2'-OMe | **2'-F** | 2'-OMe | 2'-OMe | 2'-OMe | 2'-OMe | 2'-OMe+PS | 2'-OMe+PS | 2'-OMe |
| **C** | 2'-OMe+PS | **2'-F+PS** | 2'-OMe | **2'-F** | **2'-F** | **2'-F** | 2'-OMe | 2'-OMe | 2'-OMe | 2'-OMe | 2'-OMe | 2'-OMe | 2'-OMe | **2'-F** | 2'-OMe | **2'-F** | 2'-OMe | 2'-OMe | 2'-OMe | 2'-OMe | 2'-OMe+PS | 2'-OMe+PS | 2'-OMe |

the sense strand is modified with modifications selected from the group consisting of a and b:

| No. | Sense strand (SS) 5’-3’ | | | | | | | | | | | | | | | | | | | | |
|---|---|---|---|---|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** | **17** | **18** | **19** | **20** | **21** |
| **a** | 2’-OMe+PS | 2’-OMe+PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | 2’-OMe | **2’-F** | 2’-OMe | 2’-OMe | 2’-OMe | 2’-OMe |
| **b** | 2’-OMe+PS | 2’-OMe+PS | 2’-OMe | 2’-OMe | 2’-OMe | 2’-OMe | **2’-F** | 2’-OMe | **2’-F** | 2’-OMe | **2’-F** | 2’-OMe | 2’-OMe | 2’-OMe | 2’-OMe | 2’-OMe | **2’-F** | 2’-OMe | 2’-OMe | 2’-OMe | 2’-OMe |

in the above tables, 2’-OMe represents 2’-methoxy, 2’-F represents 2’-fluoro, and PS represents a thiophosphate backbone;

wherein the antisense strand is modified with modification A, and the sense strand is modified with modification a;

the antisense strand is modified with modification B, and the sense strand is modified with modification a;

the antisense strand is modified with modification C, and the sense strand is modified with modification a;

the antisense strand is modified with modification B, and the sense strand is modified with modification b; or

the antisense strand is modified with modification C, and the sense strand is modified with modification b.

In some embodiments, the antisense strand is modified with a modification group in the 2-8 positions from the 5’-end, wherein the modification group is selected from the group consisting of UNA, GNA, and DNA, wherein UNA and GNA have the structures of:

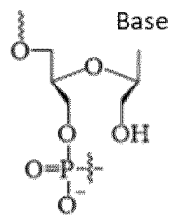

Unlocked Nucleic Acids (UNA)

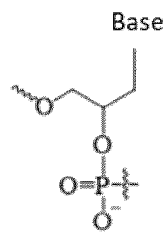

Glycol Nucleic Acids (GNA)

wherein the base is selected from the group consisting of adenine, guanine, cytosine, thymine and uracil.

In some embodiments, the 5’ carbon atom of the glycoside of the 5’-end nucleotide in the modified antisense strand is phosphorylated by a 5’ phosphorylation group selected from the group consisting of 5’-(E)-vinyl phosphonate (5’-E-VP), 5’-methyl phosphonate (5’-MP), 5’-C-methyl phosphate, 5’-thiophosphate (5’-PS), and 5’-phosphate (5’-P), with structures of

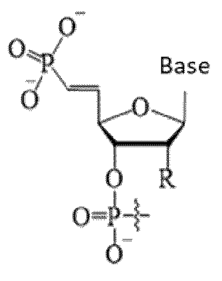

5’-(E)-vinyl phosphonate (5’-E-VP)

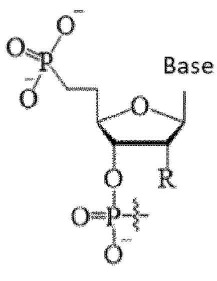

5’-methyl phosphonate (5’-MP)

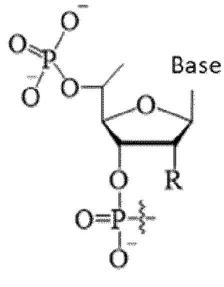

5’-C-methyl phosphate

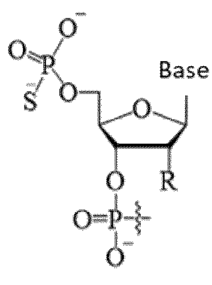

5’-thiophosphate (5’-PS)

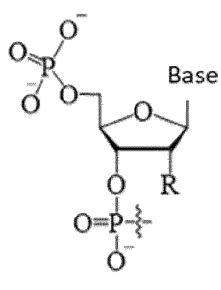

5’-thiophosphate (5’-P)

wherein, R is selected from the group consisting of hydrogen, hydroxyl, amine, $C_{1-4}$ alkyl, aryl, $C_{1-4}$ alkoxy, $C_{1-4}$ alkylcarbonylamino, and halogen; and

the base is selected from the group consisting of adenine, guanine, cytosine, thymine and uracil.

**In** some embodiments, a 3',5'-phosphodiester bond connecting nucleotide monomers at an end of the strand has thio-modification and form a chirally pure 3',5'-thiophosphodiester bond, wherein the 5'-ends of the sense strand and the antisense strand comprise 1-3 thio-modifications as linkages, and the 3'-end of the antisense strand comprises 1-3 thio-modifications as linkages.

The double-stranded RNA (dsRNA) agent of the present disclosure may optionally be conjugated with one or more ligands. The ligand may be conjugated to the sense strand, antisense strand, or both strands at the 3'-end, 5'-end, or both ends. For example, the ligand may be conjugated to the sense strand. In a preferred embodiment, the ligand is conjugated to the 3'-end of the sense strand. In a preferred embodiment, the ligand is a GalNAc ligand.

In another aspect, the present disclosure provides a conjugate for reducing PCSK9 expression, comprising the double-stranded RNAi agent and a ligand conjugated thereto.

In some embodiments, the ligand is conjugated to the 3'-end or the 5'-end of the sense strand of the oligonucleotide duplex.

In some embodiments, the ligand is one or more GalNAc derivatives attached via a divalent or trivalent branched linker.

In some embodiments, the ligand is:

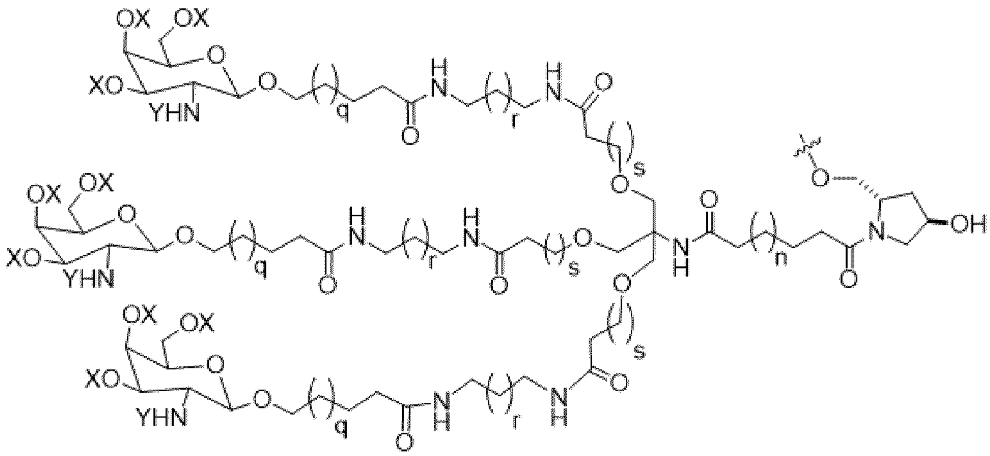

,

wherein X is hydrogen or a hydroxyl protecting group or H, wherein the hydroxyl protecting group is selected from the group consisting of acetyl, benzoyl and isobutyryl; Y is an amine protecting group or H, wherein the amine protecting group is selected from the group consisting of formyl, acetyl, propionyl, n-butyryl and isobutyryl; n is an integer selected from 0-20; and q, r and s are independently integers selected from 1 to 7.

In some embodiments, the ligand is:

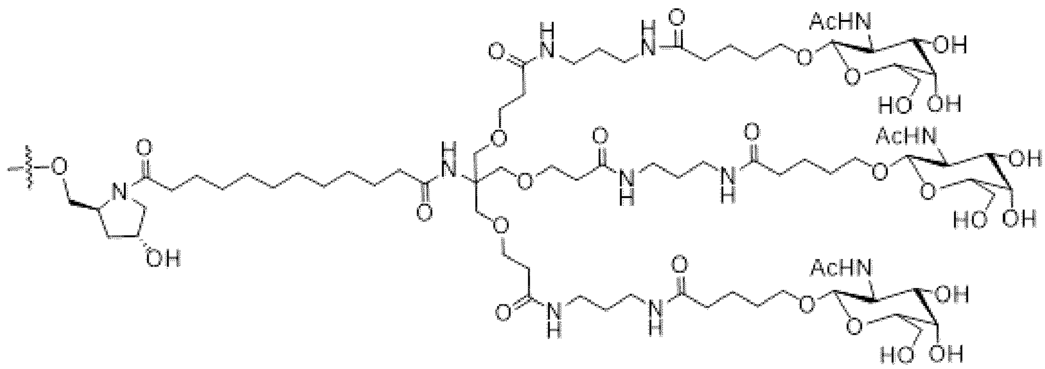

.

In some embodiments, the ligand is:

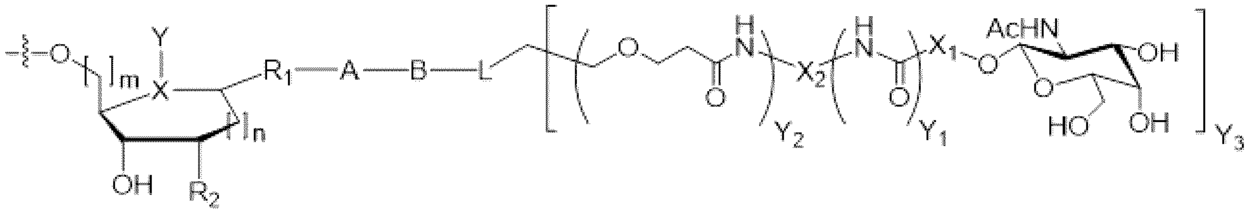

wherein, X is selected from the group consisting of oxygen, nitrogen and sulfur;

Y is alkyl or aryl;

$R_1$ is oxygen or sulfur;

$R_2$ is selected from the group consisting of hydrogen, amine, $C_{1-4}$ alkyl, aryl, $C_{1-4}$ alkoxy and halogen;

A is selected from the group consisting of $-(CH_2)_a-$, $-(CH_2CH_2O)_b-$, $-((CH_2)_cNHCO)_d-$, and $-((CH_2)_cCONH)_d-$, wherein a is an integer selected from 1 to 15, b is an integer selected from 1 to 7, c is an integer selected from 1 to 7, and d is an integer selected from 1 to 5;

B is $-(CH_2)_e-$, wherein e is an integer selected from 0 to 7;

L is-CONH- or -NHCO-;

$X_1$ is $-(CH_2)_f-$ or $-(CH_2CH_2O)_fCH_2-$, wherein f is an integer selected from 1 to 5;

$X_2$ is $-(CH_2)_g-$, wherein g is an integer selected from 1 to 6;

$Y_1$ is 0 or 1;

$Y_2$ is 0, 1 or 2;

$Y_3$ is 1, 2 or 3;

m is an integer selected from 0 to 4; and

n is an integer selected from 0 to 4.

In some embodiments, the ligand is G4, G5, G6 or G7:

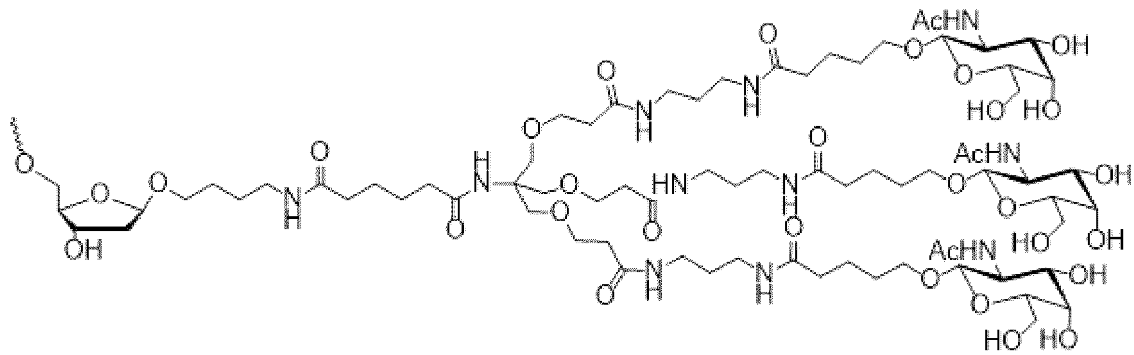

G4

,

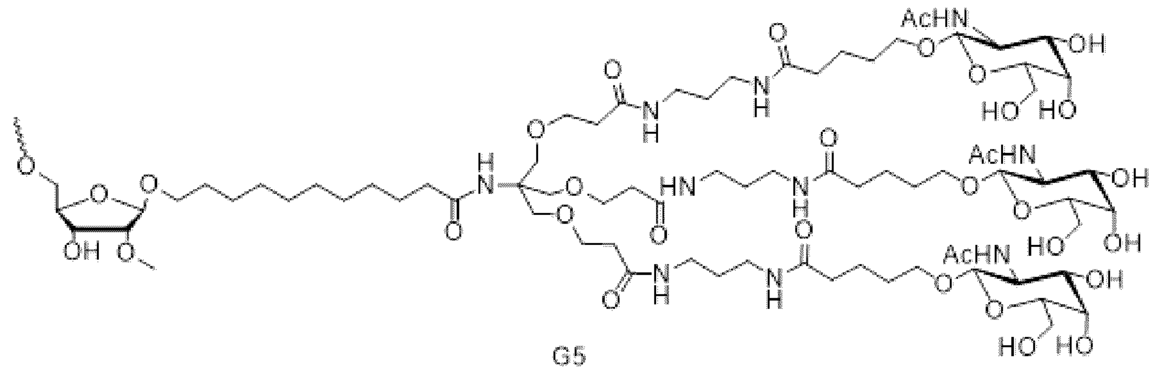

G5

,

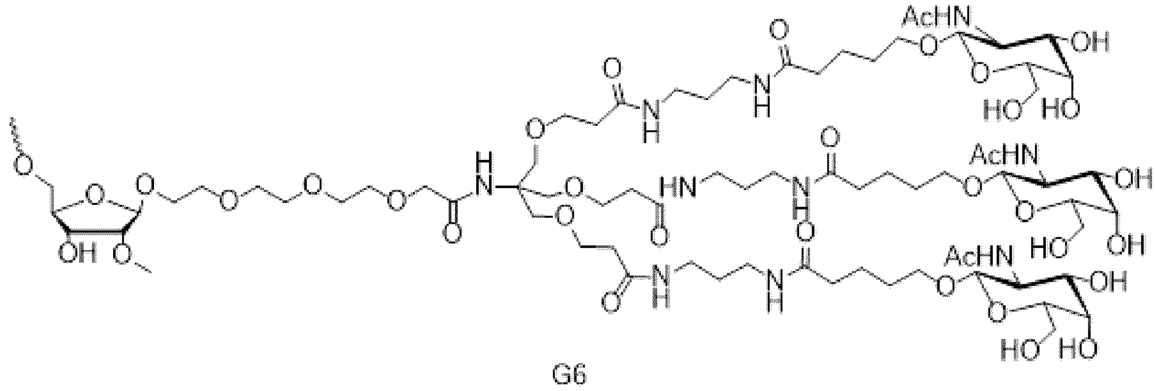

G6

,

or

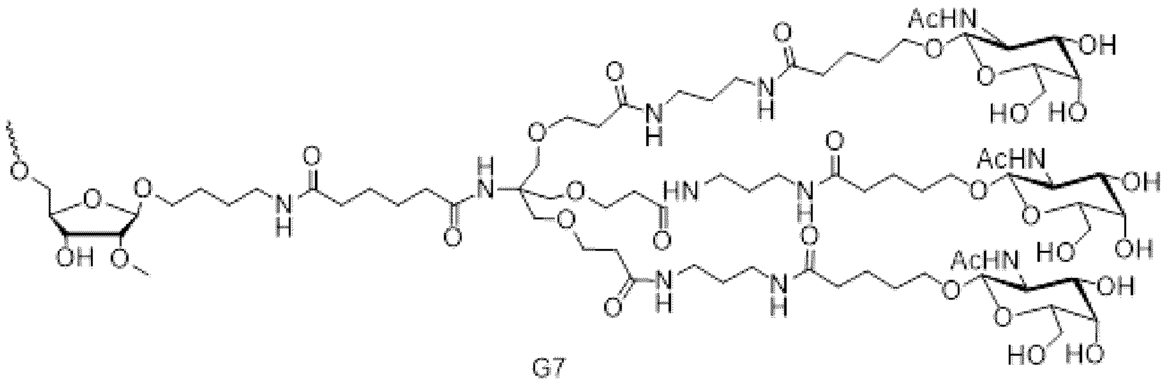

G7

.

In some embodiments, the conjugate has a structure selected from the group consisting of:

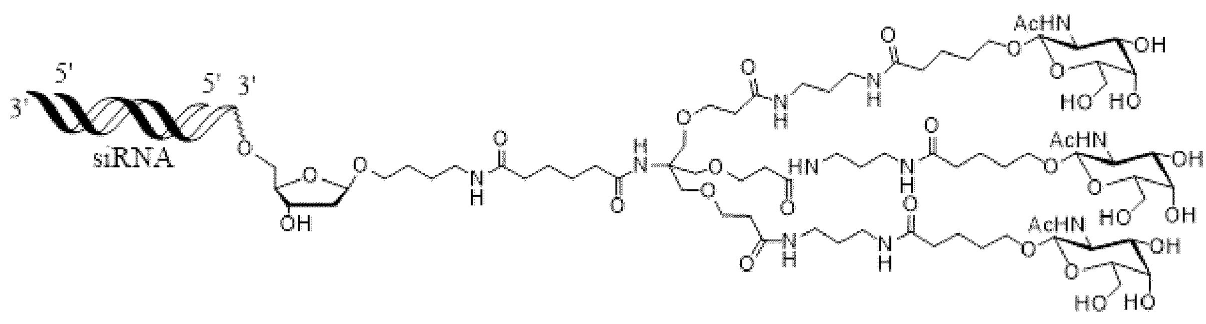

,

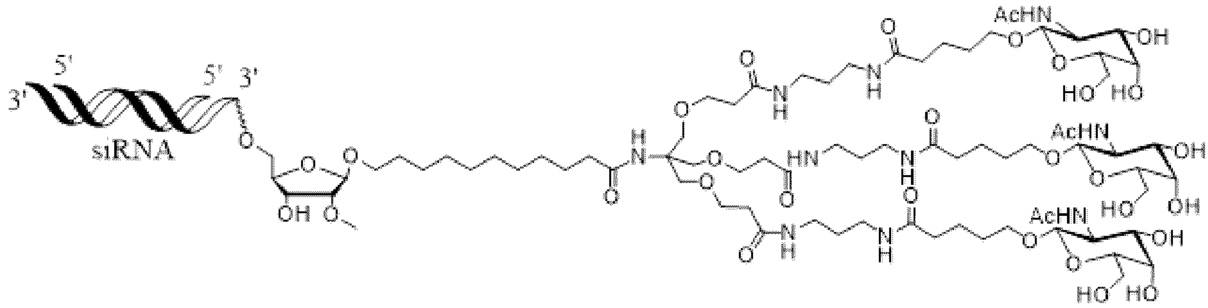

,

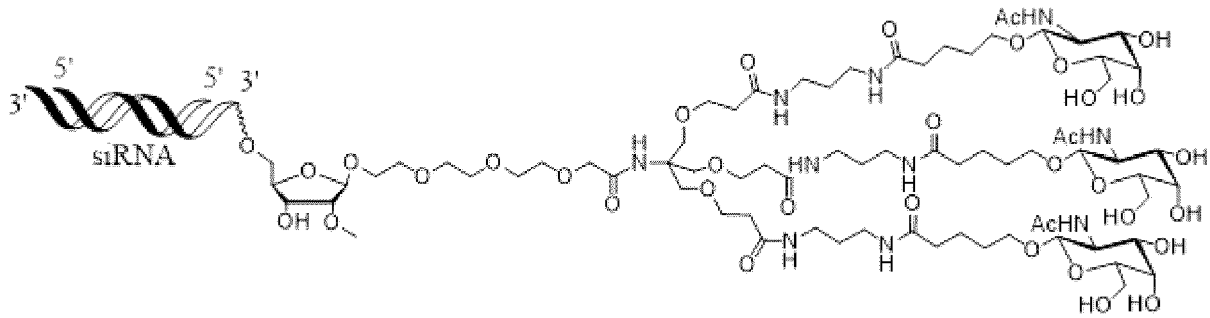

,

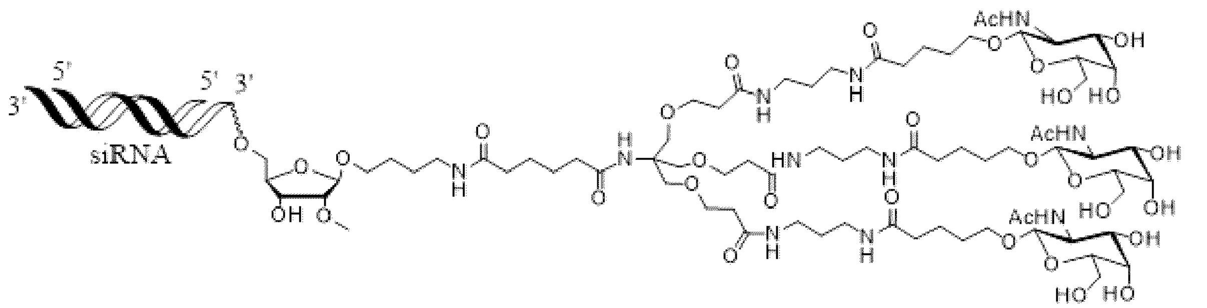

,

or

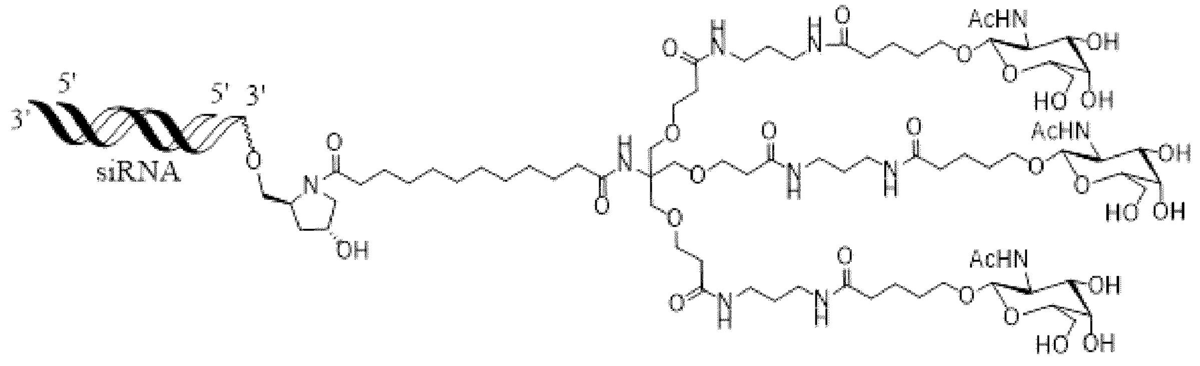

.

In some embodiments, the double-stranded RNAi agent comprises an oligonucleotide duplex consisting of paired sense strand and antisense strand, wherein

(1) the sense strand has a sequence set forth in SEQ ID NO: 337; and the antisense strand has a sequence set forth in SEQ ID NO: 427;

(2) the sense strand has a sequence set forth in SEQ ID NO: 337; and the antisense strand has a sequence set forth in SEQ ID NO: 428;

(3) the sense strand has a sequence set forth in SEQ ID NO: 342; and the antisense strand has a sequence set forth in SEQ ID NO: 381;

(4) the sense strand has a sequence set forth in SEQ ID NO: 342; and the antisense strand has a sequence set forth in SEQ ID NO: 430;

(5) the sense strand has a sequence set forth in SEQ ID NO: 342; and the antisense strand has a sequence set forth in SEQ ID NO: 431;

(6) the sense strand has a sequence set forth in SEQ ID NO: 347; and the antisense strand has a sequence set forth in SEQ ID NO: 384;

(7) the sense strand has a sequence set forth in SEQ ID NO: 347; and the antisense strand has a sequence set forth in SEQ ID NO: 437;

(8) the sense strand has a sequence set forth in SEQ ID NO: 347; and the antisense strand has a sequence set forth in SEQ ID NO: 438;

(9) the sense strand has a sequence set forth in SEQ ID NO: 348; and the antisense strand has a sequence set forth in SEQ ID NO: 385;

(10) the sense strand has a sequence set forth in SEQ ID NO: 352; and the antisense strand has a sequence set forth in SEQ ID NO: 448;

(11) the sense strand has a sequence set forth in SEQ ID NO: 353; and the antisense strand has a sequence set forth in SEQ ID NO: 390;

(12) the sense strand has a sequence set forth in SEQ ID NO: 353; and the antisense strand has a sequence set forth in SEQ ID NO: 448;

(14) the sense strand has a sequence set forth in SEQ ID NO: 357; and the antisense strand has a sequence set forth in SEQ ID NO: 393;

(15) the sense strand has a sequence set forth in SEQ ID NO: 357; and the antisense strand has a sequence set forth in SEQ ID NO: 446; or

(16) the sense strand has a sequence set forth in SEQ ID NO: 357; and the antisense strand has a sequence set forth in SEQ ID NO: 447;

wherein, the oligonucleotide duplex is conjugated with ligand G4, G5, G6 or G7.

In some embodiments, the double-stranded RNAi agent comprises an oligonucleotide duplex consisting of paired sense strand and antisense strand, wherein

(1) the sense strand has a sequence set forth in SEQ ID NO: 352; and the antisense strand has a sequence set forth in SEQ ID NO: 448;

(2) the sense strand has a sequence set forth in SEQ ID NO: 353; and the antisense strand has a sequence set forth in SEQ ID NO: 390; or

(3) the sense strand has a sequence set forth in SEQ ID NO: 353; and the antisense strand has a sequence set forth in SEQ ID NO: 448;

wherein, the oligonucleotide duplex is conjugated with ligand G4, G5, G6 or G7.

In some embodiments, the double-stranded RNAi agent comprises an oligonucleotide duplex consisting of paired sense strand and antisense strand, wherein the sense strand has a sequence set forth in SEQ ID NO: 353, the antisense strand has a sequence set forth in SEQ ID NO: 448, and the oligonucleotide duplex is conjugated with ligand G5.

In another aspect, the present disclosure further provides a pharmaceutical composition, comprising the double-stranded RNAi agent or the conjugate, and a pharmaceutically acceptable carrier.

In certain embodiments, provided herein is a pharmaceutical composition comprising RNAi as described herein and a pharmaceutically acceptable carrier. The pharmaceutical compositions comprising RNAi is capable of treating a disease or condition associated with the expression or activity of the PCSK9 gene, such as lipid disorders. Such pharmaceutical compositions are formulated based on delivery models. An example is a composition formulated so as to be delivered parenterally, such as by intravenous (IV) delivery for systemic administration. Another example is a composition formulated for direct delivery to the brain parenchyma, such as by infusion to the brain, such as by continuous pump infusion.

The pharmaceutical composition comprising the RNAi agent of the present disclosure may be, for example, a solution with or without a buffer or a composition comprising a pharmaceutically acceptable carrier. Such compositions include, for example, aqueous or crystalline compositions, liposome formulations, micellar formulations, emulsions, and gene therapy vectors.

In the method of the present disclosure, the RNAi agent may be administered in a solution. A free RNAi agent may be administered in a non-buffered solution, for example, in physiological saline or in water. Alternatively, the free siRNA may also be administered in a suitable buffer solution. The buffer solution may include acetate, citrate, prolamin, carbonate or phosphate, or any combination thereof. In a preferred embodiment, the buffer solution is phosphate buffered saline (PBS). The pH and volume molar osmotic pressure concentration of the buffer containing the RNAi agent may be adjusted so that it is suitable for administering to a subject.

In some embodiments, the buffer solution further comprises an agent for controlling the osmolality of the solution so that the osmolality is maintained at a desired value, such as the physiological value of human plasma. Solutes that can be added to the buffer solution to control the osmolality include, but are not limited to, proteins, peptides, amino acids, non-metabolizable polymers, vitamins, ions, sugars, metabolites, organic acids, lipids, or salts. In some embodiments, the agent for controlling the osmolality of the solution is a salt. In certain embodiments, the agent for controlling the osmolality of the solution is sodium chloride or potassium chloride.

The pharmaceutical composition of the present disclosure may be administered at an amount sufficient to inhibit the expression of the PCSK9 gene. Typically, a suitable amount of the RNAi of the present disclosure is in the range of about 0.001 to about 200.0 mg per kg body weight of the subject per day, typically in the range of about 1 to 50 mg per kg body weight per day. For example, the RNAi agent (e.g., dsRNA) may be administered at about 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 31, 32, 33, 34, 34, 35, 36, 37, 38, 39, 40, 41, 42, 43, 44, 45, 46, 47, 48, 49, or about 50 mg/kg.

The pharmaceutical composition may be given once a day, or the RNAi may be given twice, three times or more subdoses at appropriate intervals in one day, or even may be given by continuous infusion or delivery through a controlled release formulation. In this case, the RNAi contained in each subdose must be correspondingly less, so as to achieve a daily total dose. The dosage unit may also be compounded for delivery over several days, for example using a conventional sustained release formulation that provides sustained RNAi release over a period of several days. Sustained release formulations are well known in the art and are particularly useful for delivering agents at specific sites, and thus can be used with agents of the present disclosure. In this embodiment, the dosage unit comprises a plurality of corresponding daily doses.

In some other embodiments, a single dose of the pharmaceutical composition may be long-lasting, so that subsequent doses are administered at intervals of no more than 3, 4, or 5 days or at intervals of no more than 1, 2, 3, or 4 weeks. In some embodiments of the present disclosure, a single dose of the pharmaceutical composition of the present disclosure is administered once a week. In some other embodiments of the present disclosure, a single dose of the pharmaceutical composition of the present disclosure is administered once every two months.

Those skilled in the art will appreciate that certain factors may affect the dosage and timing required to effectively treat a subject, including, but not limited to, the severity of the disease or condition, previous treatment, the subject's overall health and/or age, and other existing diseases. In addition, treating a subject with a therapeutically effective amount of a composition may include a single treatment or a series of treatments. As described elsewhere herein, the effective amount and in vivo half-life of each RNAi encompassed by the present disclosure may be estimated using conventional methods or based on in vivo testing using appropriate animal models.

The pharmaceutical compositions of the present disclosure may be administered in a number of ways, depending on whether local or systemic treatment is desired and on the area to be treated. Administration may be topical (e.g., by a skin patch); pulmonary; for example, by inhalation or insufflation of a powder or aerosol, including by a nebulizer; intratracheal; intranasal; epidermal and transdermal, oral or parenteral. Parenteral administration includes intravenous, intraarterial, subcutaneous, intraperitoneal or intramuscular injection or infusion; subdermal, for example, via an implant device; or intracranial, for example, intraparenchymal, intrathecal or intraventricular administration.

RNAi for use in the compositions and methods of the present disclosure may be formulated for delivery in a membrane molecular assembly such as liposomes or micelles. As used herein, the term "liposome" refers to a vesicle composed of amphiphilic lipids arranged in at least one bilayer (e.g., one or more bilayers). Liposomes include unilayer or multilayer vesicles having a membrane formed from a lipophilic material and an aqueous interior. The aqueous portion contains the RNAi composition. The lipophilic material separates the aqueous interior from the aqueous exterior that does not typically include (although in some instances, it may include) the RNAi composition. Liposomes are useful for transferring active ingredients and delivering them to the site of action. Because the liposome membrane is structurally similar to a biological membrane, when the liposome is applied to a tissue, the liposome bilayer fuses with the bilayer of the cell membrane. As the fusion of the liposome and the cell proceeds, the internal aqueous content comprising the RNAi is delivered to the cell, wherein the RNAi can specifically bind to a target RNA and can mediate RNAi. In some cases, the liposomes are also specifically targeting, for example to direct the RNAi to a particular cell type.

Liposomes comprising RNAi agents may be prepared by a variety of methods. In an example, the lipid component of the liposome is dissolved in a detergent so that micelles are formed with the lipid component. For example, the lipid component may be an amphipathic cationic lipid or a lipid conjugate. The detergent may have a high critical micelle concentration and may be nonionic. Exemplary detergents include cholate, CHAPS, octyl glucoside, deoxycholate, and lauroyl sarcosine. The RNAi agent formulation is then added to the micelle comprising the lipid component. The cationic group on the lipid interacts with the RNAi agent and condenses around the RNAi agent to form a liposome. After condensation, the detergent is removed, for example, by dialysis to obtain a liposome formulation of the RNAi agent.

RNAi, such as dsRNA of the present disclosure, may be fully encapsulated in a lipid formulation (e.g., LNP or other nucleic acid-lipid particle).

As used herein, the term "LNP" refers to a stable nucleic acid-lipid particle. LNP contains a cationic lipid, a non-cationic lipid and a lipid that prevents the particle from gathering (e.g., a PEG-lipid conjugate). LNP is extremely useful for synthetic applications because they demonstrate a prolonged circulation life after intravenous (i.v.) injection and accumulate at distal sites (e.g., at a site physically separated from the site of administration).

In an embodiment, the ratio of lipid to drug (mass/mass ratio) (e.g., lipid to dsRNA ratio) will be in the range of from about 1:1 to about 50:1, from about 1:1 to about 25:1, from about 3:1 to about 15:1, from about 4:1 to about 10:1, from about 5:1 to about 9:1, or from about 6:1 to about 9:1.

In some preferred embodiments, the lipid nanoparticles include cationic lipids, neutral lipids, structural lipids, and polymer-conjugated lipids.

In some preferred embodiments, the cationic lipid is a compound represented by formula (I), or an N-oxide, solvate, pharmaceutically acceptable salt or stereoisomer thereof, wherein $G_1$ is $C_{1-6}$ alkylene; $G_2$ is $C_{2-8}$ alkylene; G3 is $C_{1-3}$ alkylene; $L_1$ is $C_{6-15}$ linear alkyl; $L_2$ is $C_{12-25}$ branched alkyl. For example, YK-009 of formula (I-I) (see patent CN114044741B).

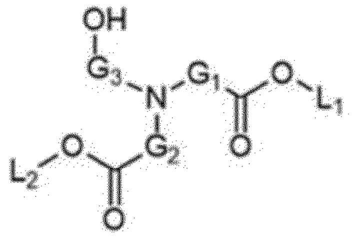

(I)

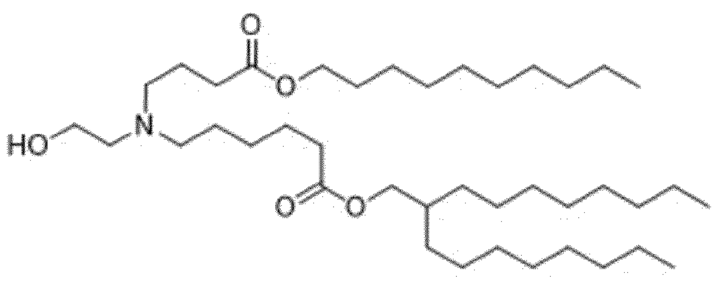

YK-009

(I-I)

In some preferred embodiments, the cationic lipid is a compound represented by formula (II), or an N-oxide, solvate, pharmaceutically acceptable salt or stereoisomer thereof, wherein: G1 is $C_{2-8}$ alkylene; $G_2$ is $C_{2-8}$ alkylene; $L_1$ is-C(O)O- or -OC(O)-; $L_2$ is-C(O)O- or -OC(O)-; $R_1$ is $C_{6-25}$ linear or branched alkyl; $R_2$ is $C_{6-25}$ linear or branched alkyl; $G_3$ is

$HO(CH_2)_2$- or $HO(CH_2)_3$-; $G_4$ is $HO(CH_2)_2$- or $HO(CH_2)_3$-; L is $(CH_2)_2$- or $-(CH_2)_3$- or $-(CH_2)_4$-. For example, YK-401 of formula (II-I) and YK-402 of formula (II-II) (see patent CN115784921B).

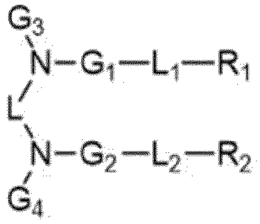

(II)

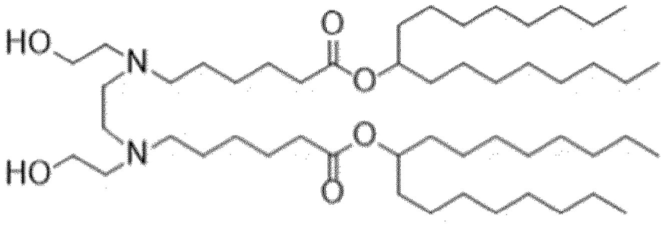

YK-401

(II-I)

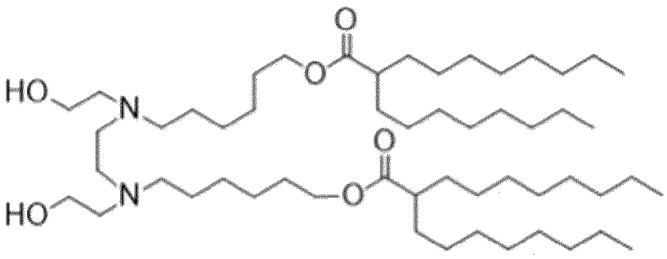

YK-402

(II-II)

In some preferred embodiments, the cationic lipid is a compound represented by formula (III), or an N-oxide, solvate, pharmaceutically acceptable salt or stereoisomer thereof, wherein: $G_1$ is $C_{1-6}$ alkylene; $G_2$ is $C_{2-8}$ alkylene; $R_1$ is $C_{6-20}$ linear or branched alkyl; $R_2$ is $C_{12-25}$ branched alkyl; $G_3$ is selected from the group consisting of $HO(CH_2)_2N(CH_3)(CH_2)_2$-, $HO(CH_2)_2N(CH_2CH_3)(CH_2)_2$-, $(HO(CH_2)_2)_2N(CH_2)_2$-, $CH_3O(CH_2)_2N(CH_3)(CH_2)_2$-, $(CH_3)_2N(CH_2)_3SC(O)O(CH_2)_2$-, $(CH_3)_2N(CH_2)_3SC(O)$-, $CH_3NH(CH_2)_2N(CH_3)(CH_2)_2$- and $CH_3CH_2NH(CH_2)_2$-. For example, YK-201 of formula (III-I) and YK-202 of formula (III-II) (see patent CN115677518B).

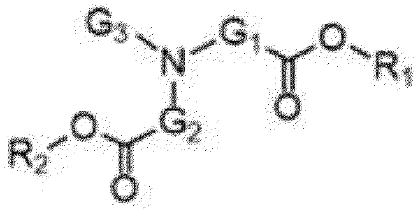

(III)

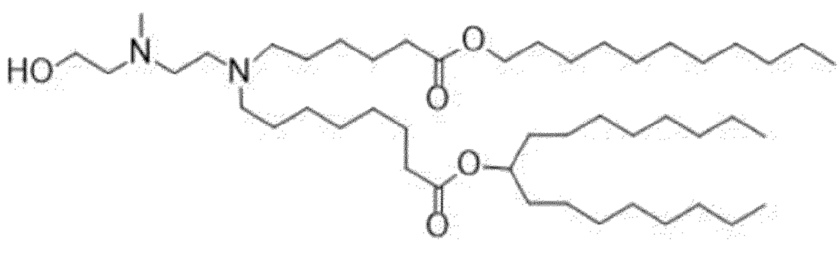

YK-201

(III-I)

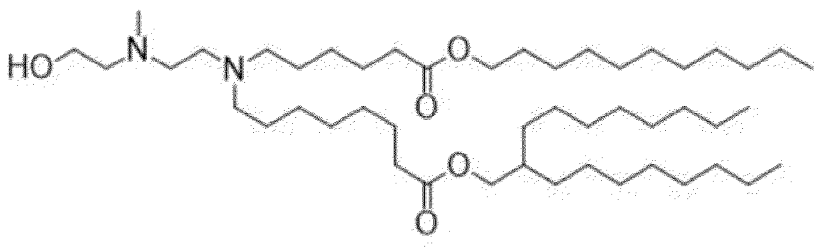

YK-202

(III-II)

In some preferred embodiments, the cationic lipid is a compound represented by formula (IV), or an N-oxide, solvate, pharmaceutically acceptable salt or stereoisomer thereof, wherein $G_1$ is $C_{1-8}$ alkylene; $G_2$ is $C_{2-8}$ alkylene; $R_1$ is $C_{6-25}$ linear or branched alkyl; $R_2$ is $C_{12-25}$ linear or branched alkyl; $G_3$ is $HO(CH_2)_2N(R_3)CH_2CH(OH)CH_2$-, wherein $R_3$ is $-CH_3$, $-CH_2CH_3$ or $-CH_2CH_2OH$. For example, YK-305 of formula (IV-I) and YK-310 of formula (IV-II) (see patent CN115745820B).

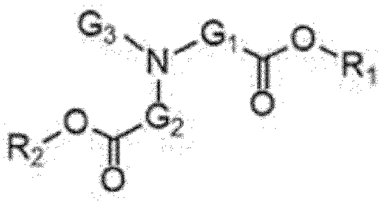

(IV)

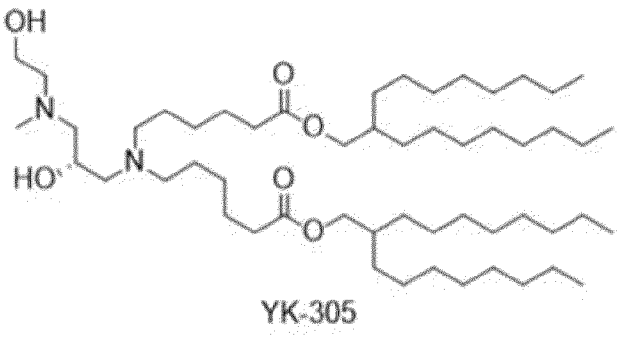

YK-305

(IV-I)

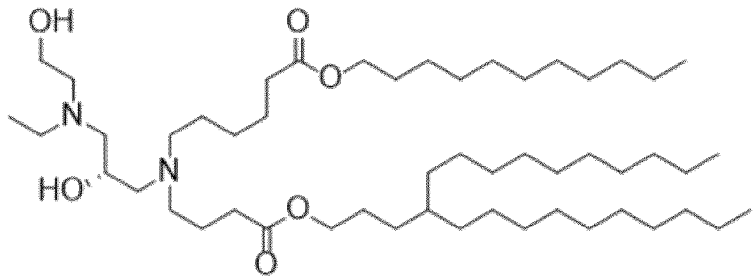

YK-310

(IV-II)

In some preferred embodiments, the cationic lipid is a compound represented by formula (V), or an N-oxide, solvate, pharmaceutically acceptable salt or stereoisomer thereof, wherein $G^1$ and $G^2$ are each independently unsubstituted $C_6$-$C_{10}$ alkylene; $G^3$ is unsubstituted $C_1$-$C_{12}$ alkylene; $R^1$ and $R^2$ are each independently $C_6$-$C_{24}$ alkyl or $C_6$-$C_{24}$ alkenyl; $R^3$ is selected from the group consisting of $OR^5$, N, $-C(=O)OR^4$, $-OC(=O)R^4$ and $-NR^5C(=O)R^4$; $R^4$ is a $C_1$-$C_{12}$ hydrocarbon group; and $R^5$ is H or a $C_1$-$C_6$ hydrocarbon group; for example, ALC0315 of formula (V-I) (see patent CN108368028B);

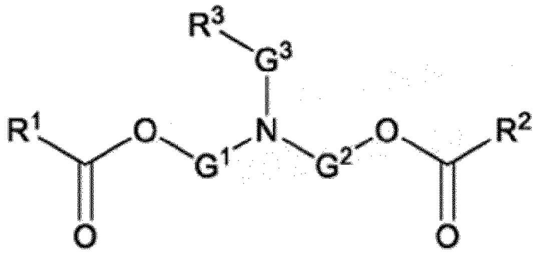

(V)

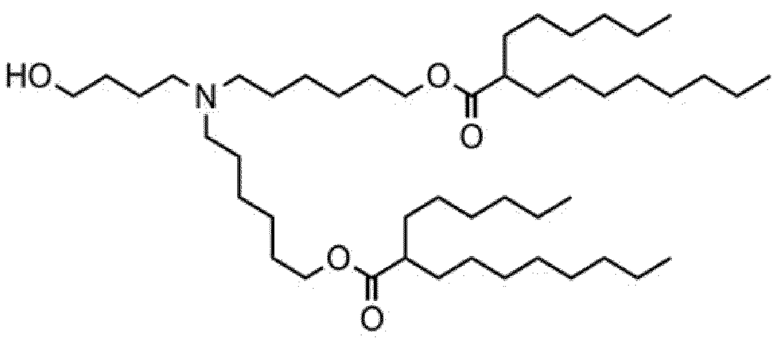

(V-I)

In some preferred embodiments, the cationic lipid is a compound represented by formula (VI), or an N-oxide, solvate, pharmaceutically acceptable salt or stereoisomer thereof, wherein $R_4$ is $-(CH_2)_nQ$ or $-(CH_2)_nCHQR$; Q is selected from the group consisting of: -OR, -OH, $-O(CH_2)_nN(R)_2$, -OC(O)R, $-CX_3$, -CN, -N(R)C(O)R, -N(H)C(O)R, $-N(R)S(O)_2R$, $-N(H)S(O)_2R$, $-N(R)C(O)N(R)_2$, $-N(H)C(O)N(R)_2$, -N(H)C(O)N(H)(R), $-N(R)C(S)N(R)_2$, $-N(H)C(S)N(R)_2$, -N(H)C(S)N(H)(R), $-N(R)S(O)_2R_8$ and heterocycle; n is 1, 2 or 3; each R is independently selected from the group consisting of: $C_{1-3}$ alkyl, $C_{2-3}$ alkenyl, $(CH_2)_qOR^*$ and H, and each q is independently selected from 1, 2 and 3, each $R^*$ is independently selected from the group consisting of: $C_{1-12}$ alkyl and $C_{2-12}$ alkenyl; each X is independently selected from the group consisting of: F, Cl, Br and I; for example, SM102 of formula (VI-I) (see patent CN110520409A).

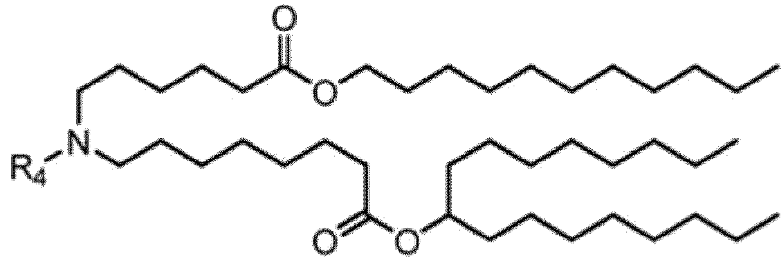

(VI)

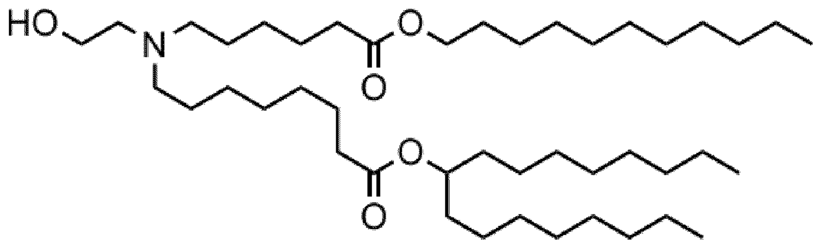

(VI-I)

In some preferred embodiments, the cationic lipid is a compound represented by formula (VII), or an N-oxide, solvate, pharmaceutically acceptable salt or stereoisomer thereof (see CN102625696B, DLIN-MC3-DMA),

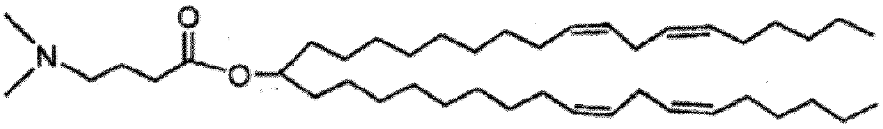

(VII).

In some more preferred embodiments, the cationic lipid includes YK-009, YK-401, YK-305, ALC0315, SM102, and DLIN-MC3-DMA.

In some preferred embodiments, the molar ratio of the cationic lipid to the neutral lipid is 1:1 to 10:1.

In some preferred embodiments, the molar ratio of the cationic lipid to the structural lipid is 1:1 to 5:1.

In some preferred embodiments, the molar ratio of the cationic lipid, the neutral lipid, the structural lipid, and the polymer conjugated lipid is (25-65):(5-25):(25-70):(0.5-5).

In some preferred embodiments, the molar ratio of the cationic lipid, the neutral lipid, the structural lipid, and the polymer conjugated lipid is (25-65):(5-25):(25-45):(0.5-5).

In some more preferred embodiments, the molar ratio of the cationic lipid, the neutral lipid, the structural lipid, and the polymer-conjugated lipid is 50:10:38.5:1.5 or 49:10:39.5:1.5.

In some preferred embodiments, the neutral lipid is selected from the group consisting of phosphatidylcholine, phosphatidylethanolamine, sphingomyelin, ceramide, sterol and derivatives thereof.

In some more preferred embodiments, the neutral lipid is selected from the group consisting of: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and mixtures thereof.

In some more preferred embodiments, the neutral lipid is DOPE and/or DSPC.

In some preferred embodiments, the structural lipid is selected from the group consisting of: cholesterol, non-sterol, sitosterol, ergosterol, campesterol, stigmasterol, brassicasterol, tomatine, ursolic acid, $\alpha$-tocopherol, corticosteroid, and a mixture thereof.

In some more preferred embodiments, the structural lipid is cholesterol.

In some preferred embodiments, the polymer-conjugated lipid is selected from the group consisting of: PEG-modified phosphatidylethanolamine, PEG-modified phosphatidic acid, PEG-modified ceramide, PEG-modified dialkylamine, PEG-modified diacylglycerol, PEG-modified dialkylglycerol, and a mixture thereof.

In some more preferred embodiments, the polymer conjugated lipid is selected from the group consisting of: distearoylphosphatidylethanolamine polyethylene glycol 2000 (DSPE-PEG2000), dimyristoylglycerol-3-methoxy polyethylene glycol 2000 (DMG-PEG2000) and methoxy polyethylene glycol ditetradecanoyl acetamide (ALC-0159).

The pharmaceutical compositions of the present disclosure include, but are not limited to, solutions, emulsions, and liposome-containing formulations. These compositions may be produced from a variety of components, including, but not limited to, preformed liquids, self-emulsifying solids, and self-emulsifying semisolids. Particularly preferred are formulations that target the liver when treating liver disorders (e.g., liver cancer).

The pharmaceutical formulations of the present disclosure, which can conveniently be in unit dosage form, may be prepared according to conventional techniques well known in the pharmaceutical industry. Such techniques include the steps of combining the active ingredients with the pharmaceutical carrier(s) or excipient(s). Generally speaking, the formulations are prepared by uniformly and finely combining the active ingredients with liquid carriers or finely dispersed solid carriers or both, and, if desired, shaping the product.

The compositions of the present disclosure can be formulated into any one of many possible dosage forms, such as but not limited to tablets, capsules, gel capsules, liquid syrups, soft capsules, suppositories and enemas. The compositions of the present disclosure can also be formulated into suspensions in aqueous, non-aqueous media or mixed media. Aqueous suspensions can further include materials that increase the viscosity of the suspension, and such materials include, for example, sodium carboxymethylcellulose, sorbitol and/or dextran. The suspension may also include a stabilizer.

Certain compositions of the present disclosure also incorporate carrier compounds into the formulation. As used herein, a "carrier compound" or "carrier" may refer to a nucleic acid or its analog, which is inert (i.e., not biologically active per se), but is considered to be a nucleic acid in an in vivo process, such as by degrading biologically active nucleic acids or promoting their removal from circulation to reduce the bioavailability of biologically active nucleic acids. Co-administration of nucleic acids and carrier compounds (generally an excess of the latter substance) can result in a significant reduction in the amount of nucleic acids recovered in the liver, kidneys, or other external circulation reservoirs, assuming that competition for common receptors between the carrier compound and the nucleic acid is due. For example, the recovery of partially phosphorothioated dsRNA in liver tissue can be reduced when it is co-administered with polyinosinic acid, dextran sulfate, polycytidylic acid, or 4-acetamido-4'stilbene isothiocyanate-2,2'-disulfonic acid.

In contrast to carrier compounds, a "pharmaceutical carrier" or "excipient" is a pharmaceutically acceptable solvent, suspending agent, or any other pharmaceutically inert vehicle for delivering one or more nucleic acids to an animal. The excipient may be liquid or solid, and when combined with the nucleic acid and other components of a particular pharmaceutical composition, the excipient is selected to provide the desired volume, thickness, etc., with reference to the intended mode of administration. Typical pharmaceutical carriers include, but are not limited to, binders (e.g., pregelatinized corn starch, polyvinyl pyrrolidone, or hydroxypropyl methylcellulose); fillers (e.g., lactose and other sugars, microcrystalline cellulose, pectin, gelatin, calcium sulfate, ethyl cellulose, polyacrylates, or calcium hydrogen phosphate); lubricants (e.g., magnesium stearate, talc, silicon dioxide, colloidal silicon dioxide, stearic acid, metal stearates, hydrogenated vegetable oils, corn starch, polyethylene glycol, sodium benzoate, sodium acetate); disintegrants (e.g., starch, sodium starch glycolate); and wetting agents (e.g., sodium lauryl sulfate).

Pharmaceutically acceptable organic or inorganic excipients suitable for non-parenteral administration, which do not react toxically with nucleic acids, can also be used to prepare the compositions of the present disclosure. Suitable pharmaceutically acceptable carriers include, but are not limited to, water, saline solution, alcohol, polyethylene glycol, gelatin, lactose, amylose, magnesium stearate, talc, silicic acid, viscous paraffin, hydroxymethylcellulose, or polyvinyl pyrrolidone.

The formulation for local administration of nucleic acid can include aseptic or non-sterile aqueous solution, non-aqueous solution in common solvent such as alcohol, or nucleic acid solution in liquid or solid oil matrix. These solutions can also include buffer, diluent and other suitable additives. Pharmaceutically acceptable organic or inorganic excipients suitable for non-parenteral administration, which do not react toxically with nucleic acids, can be used.

Suitable pharmaceutically acceptable excipients include, but are not limited to, water, saline solution, alcohol, polyethylene glycol, gelatin, lactose, amylose, magnesium stearate, talc, silicic acid, viscous paraffin, hydroxymethylcellulose, or polyvinyl pyrrolidone.

The dosage form, carrier compound, drug carrier, excipient, etc. of the above-mentioned composition are

described in US Pat. No.10125369B2, which is incorporated herein by reference.

The present disclosure further provides a method for treating or preventing diseases and conditions that can be regulated by down-regulating PCSK9 gene expression. For example, the RNAi agents described herein can be used to treat lipidemia, such as hyperlipidemia, and other forms of lipid imbalance, such as hypercholesterolemia, hypertriglyceridemia, and pathological conditions associated with these disorders, such as heart and circulatory system diseases. Other diseases and conditions that can be regulated by down-regulating PCSK9 gene expression include lysosomal storage diseases, including but not limited to Niemann-Pick disease, Tay-Sachs disease, lysosomal acid lipase deficiency, and Gaucher disease. The RNAi agents described herein can be used to treat cardiovascular diseases such as coronary heart disease (CHD), cerebrovascular disease (CVD), aortic valve stenosis, peripheral vascular disease, atherosclerosis, arteriosclerosis, myocardial infarction (heart attack), cerebrovascular disease (stroke), transient ischemic attack (TIA), angina (stable and unstable), atrial fibrillation, arrhythmia, valvular disease and/or congestive heart failure or any other trait. The method comprises administering to the subject a therapeutically effective amount or a prophylactically effective amount of the RNAi agent, conjugate or composition of the present disclosure. In some embodiments, the method comprises administering a therapeutic amount of PCSK9 siRNA to a patient having a heterozygous LDLR genotype.

The RNAi agents of the present disclosure can be administered to a subject through any mode of administration known in the art, including, but not limited to, subcutaneous, intravenous, intramuscular, intraocular, intrabronchial, intrapleural, intraperitoneal, intraarterial, lymphatic, cerebrospinal administration, and any combination thereof. In a preferred embodiment, the agents are administered subcutaneously.

In some other embodiments, the siRNA is administered in combination with an additional therapeutic agent. The siRNA and the additional therapeutic agent can be administered in combination in the same composition, e.g., parenterally, or the additional therapeutic agent can be administered as part of a separate composition or by another method described herein.

Examples of additional therapeutic agents include drugs known to treat lipid disorders such as hypercholesterolemia, atherosclerosis, dyslipidemia or cardiovascular and cerebrovascular diseases. For example, additional therapeutic agents for treating hyperlipidemia are selected from fibrates, statins, bile acid sequestering agents and nicotinic acids. Additional therapeutic agents for treating cardiovascular and cerebrovascular diseases are selected from angiotensin converting enzyme inhibitors (e.g., captopril, enalapril, benazepril, or perindopril), angiotensin II receptor antagonists (e.g., losartan, losartan hydrochlorothiazide, valsartan, valsartan hydrochlorothiazide, telmisartan, telmisartan hydrochlorothiazide, or olmesartan medoxomil), and beta-blockers (e.g., propranolol, bisoprolol, metoprolol tartrate, or metoprolol succinate).

In some embodiments, the RNAi agent is administered to a patient and subsequently an additional therapeutic agent is administered to the patient (or vice versa). In some other embodiments, the RNAi agent and the additional therapeutic agent are administered simultaneously.

In another aspect, the present disclosure provides use of the aforementioned double-stranded RNAi agent, conjugate or composition in the manufacture of a medicament for treating a PCSK9-related disease.

In another aspect, the present disclosure provides use of the aforementioned double-stranded RNAi agent, conjugate or composition in the manufacture of a medicament for treating hypercholesterolemia, atherosclerosis, dyslipidemia or cardiovascular and cerebrovascular diseases.

In some embodiments, the PCSK9-related disease is selected from the group consisting of hypercholesterolemia, atherosclerosis, dyslipidemia, and cardiovascular and cerebrovascular diseases

The following examples are used to illustrate the present disclosure, but are not intended to limit the scope of the present disclosure. Unless otherwise specified, the technical means used in the examples are conventional means well known to those skilled in the art, and the raw materials used are all commercially available products.

The nucleotide abbreviations herein are as follows:

A = Adenosine-3'-phosphate

Am = 2'-O-methoxyadenosine-3'-phosphate

Ams = 2'-O-methoxyadenosine-3'-phosphorothioate

Af = 2'-fluoroadenosine-3'-phosphate

Afs = 2'-fluoroadenosine-3'-phosphorothioate

G = Guanosine-3'-phosphate

Gm = 2'-O-methoxyguanosine-3'-phosphate

Gms = 2'-O-methoxyguanosine-3'-phosphorothioate

Gf = 2'-fluoroguanosine-3'-phosphate

Gfs = 2'-fluoroguanosine-3'-phosphorothioate

C = Cytidine-3'-phosphate

Cm = 2'-O-methoxycytidine-3'-phosphate

Cms = 2'-O-methoxycytidine-3'-phosphorothioate

Cf = 2'-fluorocytidine-3'-phosphate

Cfs = 2'-fluorocytidine-3'-phosphorothioate

U = Uridine-3'-phosphate

Um = 2'-O-methoxyuridine-3'-phosphate

Ums = 2'-O-methoxyuridine-3'-phosphorothioate

Uf = 2'-fluorouridine-3'-phosphate

Ufs = 2'-fluorouridine-3'-phosphorothioate

AmsEVP = 5'-vinyl-(E)-phosphonate-2'-methoxyadenosine-3'-phosphorothioate

UmsEVP = 5'-vinyl-(E)-phosphonate-2'-methoxyuridine-3'-phosphorothioate

Agna = adenosine-diol nucleic acid

Cgna = cytidine-diol nucleic acid

Ggna = guanosine-diol nucleic acid

Tgna = thymidine-diol nucleic acid

Ugna = uridine-diol nucleic acid

**Examples**

**Example 1: Synthesis of alternately modified small interfering oligonucleotides**

84 siRNA basic sequences were designed based on the PCSK9 mRNA sequence. To improve the inhibition efficiency and stability of the sequence, the basic sequence was modified with 2'-methoxy (2'-OMe) and 2'-fluoro (2'-F) alternately, and the terminal was thio-modified. The nucleotides in the sense strand were modified with 2'-F in odd-numbered positions and with 2'-OMe in even-numbered positions, and the nucleotides in the antisense strand were modified with 2'-OMe in odd-numbered positions and with 2'-F in even-numbered positions. In addition, there were two thio-modifications at the 5'-end of the sense strand, and two thio-modifications at each of the 5'- and 3'-ends of the antisense strand. The alternately modified siRNA sequences are shown in Table 2.

**1. Synthesis of the alternately modified sequence P92-si5**

The basic sequence of the small interfering RNA with sequence number P92-si5 in Table 2 is:

Sense strand: 5'-AAGAUCCUGCAUGUCUUCCAU-3' (SEQ ID NO: 1)

Antisense strand: 5'-AUGGAAGACAUGCAGGAUCUUGG-3' (SEQ ID NO: 13)

The nucleotides in odd-numbered positions in the sense strand and the nucleotides in even-numbered positions in the antisense strand were modified with 2'-F, and the nucleotides in other positions were modified with 2'-OMe. In addition, there were two thio-modifications at the 5'-end of the sense strand, and two thio-modifications at the 5'- and 3'-ends of the antisense strand.

Instruments and reagents: Tsingke's DNA/RNA automatic synthesizer, model 192 P, and the solid phase carrier was a universal carrier of cross-linked polystyrene beads, model Primer support 5G Unylinker 350 (cytiva manufacturer).

Preparation method:

According to the monomer concentration of 0.15 M, the following nucleotide monomer solutions were prepared with acetonitrile: DMT-A-OMe phosphoramidite monomer (Formula 1), DMT-C-OMe phosphoramidite monomer (Formula 2), DMT-G-OMe phosphoramidite monomer (Formula 3), DMT-U-OMe phosphoramidite monomer (Formula 4), DMT-A-F phosphoramidite monomer (Formula 5), DMT-C-F phosphoramidite monomer (Formula 6), DMT-G-F phosphoramidite monomer (Formula 7) and DMT-U-F phosphoramidite monomer (Formula 8).

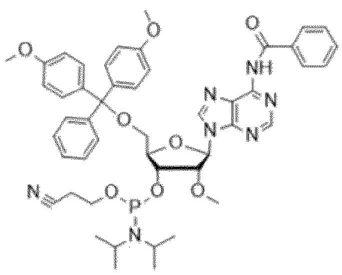

Formula 1

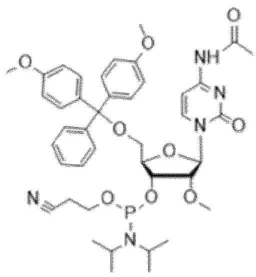

Formula 2

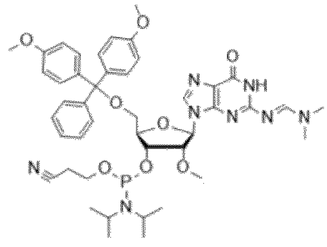

Formula 3

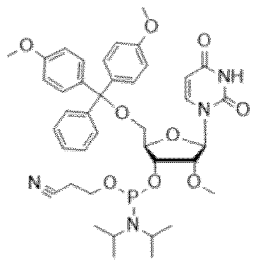

Formula 4

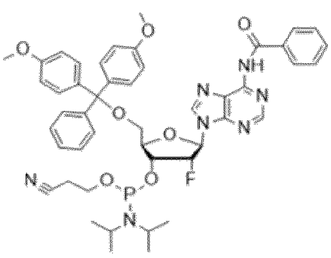

Formula 5

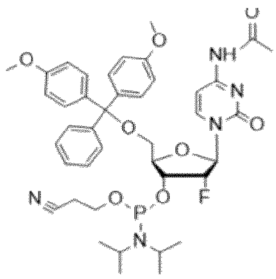

Formula 6

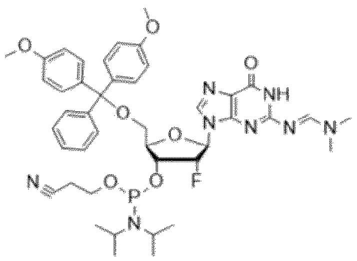

Formula 7

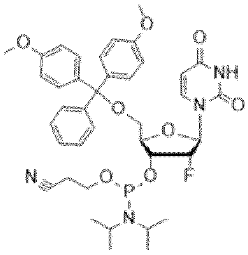

Formula 8

The following steps were performed for the preparation:

(1) Deprotection

The DMT protecting group was removed with a 3% dichloroacetic acid toluene solution as a deprotection agent, followed by washing with acetonitrile.

(2) Conjugation

Each nucleotide monomer was conjugated in acetonitrile using 0.25 M 5-ethylthiotetrazolyl as an activating agent, followed by rinsing with acetonitrile.

(3) Oxidation/sulfurization

Oxidation: Oxidation was performed using 0.05 M iodine in pyridine/water (90/10) solution as an oxidant, followed by rinsing with acetonitrile.

Sulfurization: Sulfurization was performed using 3% hydrogenated xanthan gum in pyridine as a sulfurizing agent, followed by rinsing with acetonitrile.

(4) Hydroxyl protection

Hydroxyl protection was performed using 10% acetic anhydride tetrahydrofuran solution (CAP A) and tetrahydrofuran/pyridine/nitromethylimidazole 74/10/16 (v/v/v) (CAP B) as hydroxyl protecting reagents, followed by rinsing with acetonitrile.

The above steps were repeated and cycled according to the set sequence to obtain a fully protected product.

(5) The DMT protecting group of the last nucleotide was removed using 3% dichloroacetic acid toluene solution as a deprotection reagent, followed by rinsing with acetonitrile.

(6) Aminolysis and purification

The solid phase carrier was transferred to a reactor, and concentrated ammonia water (25-28%) was added. After the aminolysis was maintained at 60°C for 12 h, the system was cooled to room temperature. The mixture was then transferred to a filter press and eluted with a mixed solution of purified water and ethanol. The filtrate was combined, passed through a chromatography column, concentrated, and freeze-dried to obtain the product.

(7) Annealing

The purified sense strand and antisense strand were mixed in a 1:1 ratio, heated to 95°C and maintained for 3 min, and then slowly cooled to room temperature to form a double strand.

P92-si5 purity 97.4%; measured molecular weight: 14493.52

**2. Synthesis of other sequences**

The other sequences listed in Table 2 were synthesized according to the above method.

Table 2 Alternately modified small interfering RNA sequences

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si5 | Afs-Ams-Gf-Am-Uf-Cm-Cf-Um-Gf-Cm-Af-Um-Gf-Um-Cf-Um-Uf-Cm-Cf-Am-Uf | 169/1 | Ams-Ufs-Gm-Gf-Am-Af-Gm-Af-Cm-Af-Um-Gf-Cm-Af-Gm-Gf-Am-Uf-Cm-Uf-Ums-Gfs-Gm | 181/13 |
| P92-si51 | Ufs-Gms-Gf-Am-Gf-Gm-Cf-Um-Uf-Am-Gf-Cm-Uf-Um-Uf-Cm-Uf-Gm-Gf-Am-Uf | 170/2 | Ams-Ufs-Cm-Cf-Am-Gf-Am-Af-Am-Gf-Cm-Uf-Am-Af-Gm-Cf-Cm-Uf-Cm-Cf-Ams-Ufs-Um | 182/14 |
| P92-si81 | Cfs-Ums-Uf-Um-Uf-Gm-Uf-Am-Af-Cm-Uf-Um-Gf-Am-Af-Gm-Af-Um-Af-Um-Uf | 171/3 | Ams-Afs-Um-Af-Um-Cf-Um-Uf-Cm-Af-Am-Gf-Um-Uf-Am-Cf-Am-Af-Am-Af-Gms-Cfs-Am | 183/15 |
| P92-si82 | Gfs-Ams-Af-Gm-Af-Um-Af-Um-Uf-Um-Af-Um-Uf-Cm-Uf-Gm-Gf-Gm-Uf-Um-Uf | 172/4 | Ams-Afs-Am-Cf-Cm-Cf-Am-Gf-Am-Af-Um-Af-Am-Af-Um-Af-Um-Cf-Um-Uf-Cms-Afs-Am | 184/16 |
| P92-si84 | Gfs-Ums-Uf-Um-Uf-Gm-Uf-Am-Gf-Cm-Af-Um-Uf-Um-Uf-Um-Af-Um-Uf-Am-Af | 173/5 | Ums-Ufs-Am-Af-Um-Af-Am-Af-Am-Af-Um-Gf-Cm-Uf-Am-Cf-Am-Af-Am-Af-Cms-Cfs-Cm | 185/17 |
| P92-si3 | Cfs-Ams-Cf-Cm-Af-Am-Gf-Am-Uf-Cm-Cf-Um-Gf-Cm-Af-Um-Gf-Um-Cf-Um-Uf | 174/6 | Ams-Afs-Gm-Af-Cm-Af-Um-Gf-Cm-Af-Gm-Gf-Am-Uf-Cm-Uf-Um-Gf-Gm-Uf-Gms-Afs-Gm | 186/18 |
| P92-si8 | Cfs-Ums-Cf-Am-Uf-Am-Gf-Gm-Cf-Cm-Uf-Gm-Gf-Am-Gf-Um-Uf-Um-Af-Um-Uf | 175/7 | Ams-Afs-Um-Af-Am-Af-Cm-Uf-Cm-Cf-Am-Gf-Gm-Cf-Cm-Uf-Am-Uf-Gm-Af-Gms-Gfs-Gm | 187/19 |
| P92-si9 | Gfs-Gms-Cf-Cm-Uf-Gm-Gf-Am-Gf-Um-Uf-Um-Af-Um-Uf-Cm-Gf-Gm-Af-Am-Af | 176/8 | Ums-Ufs-Um-Cf-Cm-Gf-Am-Af-Um-Af-Am-Af-Cm-Uf-Cm-Cf-Am-Gf-Gm-Cf-Cms-Ufs-Am | 188/20 |
| P92-si21 | Cfs-Ams-Gf-Cm-Af-Cm-Cf-Um-Gf-Cm-Uf-Um-Uf-Gm-Uf-Gm-Uf-Cm-Af-Cm-Af | 177/9 | Ums-Gfs-Um-Gf-Am-Cf-Am-Cf-Am-Af-Am-Gf-Cm-Af-Gm-Gf-Um-Gf-Cm-Uf-Gms-Cfs-Am | 189/21 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si31 | Gfs-Gms-Uf-Cm-Uf-Gm-Gf-Am-Af-Um-Gf-Cm-Af-Am-Af-Gm-Uf-Cm-Af-Am-Gf | 178/10 | Cms-Ufs-Um-Gf-Am-Cf-Um-Uf-Um-Gf-Cm-Af-Um-Uf-Cm-Cf-Am-Gf-Am-Cf-Cms-Ufs-Gm | 190/22 |
| P92-si73 | Gfs-Cms-Gf-Gm-Af-Gm-Af-Um-Gf-Cm-Uf-Um-Cf-Um-Af-Am-Gf-Gm-Cf-Am-Uf | 179/11 | Ams-Ufs-Gm-Cf-Cm-Uf-Um-Af-Gm-Af-Am-Gf-Cm-Af-Um-Cf-Um-Cf-Cm-Gf-Cms-Cfs-Am | 191/23 |
| P92-si83 | Gfs-Gms-Gf-Um-Uf-Um-Uf-Gm-Uf-Am-Gf-Cm-Af-Um-Uf-Um-Uf-Um-Af-Um-Uf | 180/12 | Ams-Afs-Um-Af-Am-Af-Am-Af-Um-Gf-Cm-Uf-Am-Cf-Am-Af-Am-Af-Cm-Cf-Cms-Afs-Gm | 192/24 |
| P92-si1 | Gfs-Gms-Gf-Am-Uf-Am-Cf-Cm-Uf-Cm-Af-Cm-Cf-Am-Af-Gm-Af-Um-Cf-Cm-Uf | 193/25 | Ams-Gfs-Gm-Af-Um-Cf-Um-Uf-Gm-Gf-Um-Gf-Am-Gf-Gm-Uf-Am-Uf-Cm-Cf-Cms-Cfs-Gm | 265/97 |
| P92-si2 | Afs-Ums-Af-Cm-Cf-Um-Cf-Am-Cf-Cm-Af-Am-Gf-Am-Uf-Cm-Cf-Um-Gf-Cm-Af | 194/26 | Ums-Gfs-Cm-Af-Gm-Gf-Am-Uf-Cm-Uf-Um-Gf-Gm-Uf-Gm-Af-Gm-Gf-Um-Af-Ums-Cfs-Cm | 266/98 |
| P92-si4 | Cfs-Ams-Af-Gm-Af-Um-Cf-Cm-Uf-Gm-Cf-Am-Uf-Gm-Uf-Cm-Uf-Um-Cf-Cm-Af | 195/27 | Ums-Gfs-Gm-Af-Am-Gf-Am-Cf-Am-Uf-Gm-Cf-Am-Gf-Gm-Af-Um-Cf-Um-Uf-Gms-Gfs-Um | 267/99 |
| P92-si6 | Gfs-Gms-Cf-Um-Uf-Cm-Cf-Um-Gf-Gm-Uf-Gm-Af-Am-Gf-Am-Uf-Gm-Af-Gm-Uf | 196/28 | Ams-Cfs-Um-Cf-Am-Uf-Cm-Uf-Um-Cf-Am-Cf-Cm-Af-Gm-Gf-Am-Af-Gm-Cf-Cms-Afs-Gm | 268/10 0 |
| P92-si7 | Gfs-Cms-Uf-Gm-Gf-Am-Gf-Cm-Uf-Gm-Gf-Cm-Cf-Um-Uf-Gm-Af-Am-Gf-Um-Uf | 197/29 | Ams-Afs-Cm-Uf-Um-Cf-Am-Af-Gm-Gf-Cm-Cf-Am-Gf-Cm-Uf-Cm-Cf-Am-Gf-Cms-Afs-Gm | 269/10 1 |
| P92-si10 | Cfs-Cms-Uf-Gm-Gf-Am-Gf-Um-Uf-Um-Af-Um-Uf-Cm-Gf-Gm-Af-Am-Af-Am-Gf | 198/30 | Cms-Ufs-Um-Uf-Um-Cf-Cm-Gf-Am-Af-Um-Af-Am-Af-Cm-Uf-Cm-Cf-Am-Gf-Gms-Cfs-Cm | 270/10 2 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si11 | Gfs-Cms-Cf-Am-Gf-Cm-Uf-Gm-Gf-Um-Cf-Cm-Af-Gm-Cf-Cm-Uf-Gm-Uf-Gm-Gf | 199/31 | Cms-Cfs-Am-Cf-Am-Gf-Gm-Cf-Um-Gf-Gm-Af-Cm-Cf-Am-Gf-Cm-Uf-Gm-Gf-Cms-Ufs-Um | 271/10 3 |
| P92-si12 | Afs-Cms-Uf-Gm-Gf-Um-Gf-Gm-Uf-Gm-Cf-Um-Gf-Cm-Uf-Gm-Cf-Cm-Cf-Cm-Uf | 200/32 | Ams-Gfs-Gm-Gf-Gm-Cf-Am-Gf-Cm-Af-Gm-Cf-Am-Cf-Cm-Af-Cm-Cf-Am-Gf-Ums-Gfs-Gm | 272/10 4 |
| P92-si13 | Ufs-Gms-Cf-Cm-Cf-Cm-Uf-Gm-Gf-Cm-Gf-Gm-Gf-Um-Gf-Gm-Gf-Um-Af-Cm-Af | 201/33 | Ums-Gfs-Um-Af-Cm-Cf-Cm-Af-Cm-Cf-Cm-Gf-Cm-Cf-Am-Gf-Gm-Gf-Gm-Cf-Ams-Gfs-Cm | 273/10 5 |
| P92-si14 | Ufs-Gms-Gf-Gm-Uf-Am-Cf-Am-Gf-Cm-Cf-Gm-Cf-Gm-Uf-Cm-Cf-Um-Cf-Am-Af | 202/34 | Ums-Ufs-Gm-Af-Gm-Gf-Am-Cf-Gm-Cf-Gm-Gf-Cm-Uf-Gm-Uf-Am-Cf-Cm-Cf-Ams-Cfs-Cm | 274/10 6 |
| P92-si15 | Cfs-Gms-Uf-Cm-Cf-Um-Cf-Am-Af-Cm-Gf-Cm-Cf-Gm-Cf-Cm-Uf-Gm-Cf-Cm-Af | 203/35 | Ums-Gfs-Gm-Cf-Am-Gf-Gm-Cf-Gm-Gf-Cm-Gf-Um-Uf-Gm-Af-Gm-Gf-Am-Cf-Gms-Cfs-Gm | 275/10 7 |
| P92-si16 | Gfs-Gms-Uf-Cm-Gf-Um-Gf-Cm-Uf-Gm-Gf-Um-Cf-Am-Cf-Cm-Gf-Cm-Uf-Gm-Cf | 204/36 | Gms-Cfs-Am-Gf-Cm-Gf-Gm-Uf-Gm-Af-Cm-Cf-Am-Gf-Cm-Af-Cm-Gf-Am-Cf-Cms-Cfs-Cm | 276/10 8 |
| P92-si17 | Afs-Gms-Gf-Am-Cf-Am-Uf-Cm-Af-Um-Uf-Gm-Gf-Um-Gf-Cm-Cf-Um-Cf-Cm-Af | 205/37 | Ums-Gfs-Gm-Af-Gm-Gf-Cm-Af-Cm-Cf-Am-Af-Um-Gf-Am-Uf-Gm-Uf-Cm-Cf-Ums-Cfs-Cm | 277/10 9 |
| P92-si18 | Ufs-Cms-Af-Um-Uf-Gm-Gf-Um-Gf-Cm-Cf-Um-Cf-Cm-Af-Gm-Cf-Gm-Af-Cm-Uf | 206/38 | Ams-Gfs-Um-Cf-Gm-Cf-Um-Gf-Gm-Af-Gm-Gf-Cm-Af-Cm-Cf-Am-Af-Um-Gf-Ams-Ufs-Gm | 278/11 0 |
| P92-si19 | Gfs-Ums-Gf-Cm-Cf-Um-Cf-Cm-Af-Gm-Cf-Gm-Af-Cm-Uf-Gm-Cf-Am-Gf-Cm-Af | 207/39 | Ums-Gfs-Cm-Uf-Gm-Cf-Am-Gf-Um-Cf-Gm-Cf-Um-Gf-Gm-Af-Gm-Gf-Cm-Af-Cms-Cfs-Am | 279/11 1 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si20 | Afs-Gms-Cf-Gm-Af-Cm-Uf-Gm-Cf-Am-Gf-Cm-Af-Cm-Cf-Um-Gf-Cm-Uf-Um-Uf | 208/40 | Ams-Afs-Am-Gf-Cm-Af-Gm-Gf-Um-Gf-Cm-Uf-Gm-Cf-Am-Gf-Um-Cf-Gm-Cf-Ums-Gfs-Gm | 280/11 2 |
| P92-si22 | Gfs-Cms-Af-Cm-Cf-Um-Gf-Cm-Uf-Um-Uf-Gm-Uf-Gm-Uf-Cm-Af-Cm-Af-Gm-Af | 209/41 | Ums-Cfs-Um-Gf-Um-Gf-Am-Cf-Am-Cf-Am-Af-Am-Gf-Cm-Af-Gm-Gf-Um-Gf-Cms-Ufs-Gm | 281/11 3 |
| P92-si23 | Cfs-Ums-Uf-Um-Gf-Um-Gf-Um-Cf-Am-Cf-Am-Gf-Am-Gf-Um-Gf-Gm-Gf-Am-Cf | 210/42 | Gms-Ufs-Cm-Cf-Cm-Af-Cm-Uf-Cm-Uf-Gm-Uf-Gm-Af-Cm-Af-Cm-Af-Am-Af-Gms-Cfs-Am | 282/11 4 |
| P92-si24 | Afs-Gms-Uf-Gm-Gf-Gm-Af-Cm-Af-Um-Cf-Am-Cf-Am-Gf-Gm-Cf-Um-Gf-Cm-Uf | 211/43 | Ams-Gfs-Cm-Af-Gm-Cf-Cm-Uf-Gm-Uf-Gm-Af-Um-Gf-Um-Cf-Cm-Cf-Am-Cf-Ums-Cfs-Um | 283/11 5 |
| P92-si25 | Afs-Ums-Cf-Am-Cf-Am-Gf-Gm-Cf-Um-Gf-Cm-Uf-Gm-Cf-Cm-Cf-Am-Cf-Gm-Uf | 212/44 | Ams-Cfs-Gm-Uf-Gm-Gf-Gm-Cf-Am-Gf-Cm-Af-Gm-Cf-Cm-Uf-Gm-Uf-Gm-Af-Ums-Gfs-Um | 284/11 6 |
| P92-si26 | Ufs-Gms-Uf-Cm-Uf-Gm-Cf-Cm-Gf-Am-Gf-Cm-Cf-Gm-Gf-Am-Gf-Cm-Uf-Cm-Af | 213/45 | Ums-Gfs-Am-Gf-Cm-Uf-Cm-Cf-Gm-Gf-Cm-Uf-Cm-Gf-Gm-Cf-Am-Gf-Am-Cf-Ams-Gfs-Cm | 285/11 7 |
| P92-si27 | Gfs-Gms-Cf-Cm-Gf-Am-Gf-Um-Uf-Gm-Af-Gm-Gf-Cm-Af-Gm-Af-Gm-Af-Cm-Uf | 214/46 | Ams-Gfs-Um-Cf-Um-Cf-Um-Gf-Cm-Cf-Um-Cf-Am-Af-Cm-Uf-Cm-Gf-Gm-Cf-Cms-Afs-Gm | 286/11 8 |
| P92-si28 | Cfs-Ams-Uf-Cm-Cf-Am-Cf-Gm-Cf-Um-Uf-Cm-Cf-Um-Gf-Cm-Uf-Gm-Cf-Cm-Af | 215/47 | Ums-Gfs-Gm-Cf-Am-Gf-Cm-Af-Gm-Gf-Am-Af-Gm-Cf-Gm-Uf-Gm-Gf-Am-Uf-Gms-Cfs-Um | 287/11 9 |
| P92-si29 | Cfs-Cms-Cf-Am-Gf-Gm-Uf-Cm-Uf-Gm-Gf-Am-Af-Um-Gf-Cm-Af-Am-Af-Gm-Uf | 216/48 | Ams-Cfs-Um-Uf-Um-Gf-Cm-Af-Um-Uf-Cm-Cf-Am-Gf-Am-Cf-Cm-Uf-Gm-Gf-Gms-Gfs-Cm | 288/12 0 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si30 | Afs-Gms-Gf-Um-Cf-Um-Gf-Gm-Af-Am-Uf-Gm-Cf-Am-Af-Am-Gf-Um-Cf-Am-Af | 217/49 | Ums-Ufs-Gm-Af-Cm-Uf-Um-Uf-Gm-Cf-Am-Uf-Um-Cf-Cm-Af-Gm-Af-Cm-Cf-Ums-Gfs-Gm | 289/12 1 |
| P92-si32 | Gfs-Ams-Af-Um-Gf-Cm-Af-Am-Af-Gm-Uf-Cm-Af-Am-Gf-Gm-Af-Gm-Cf-Am-Uf | 218/50 | Ams-Ufs-Gm-Cf-Um-Cf-Cm-Uf-Um-Gf-Am-Cf-Um-Uf-Um-Gf-Cm-Af-Um-Uf-Cms-Cfs-Am | 290/12 2 |
| P92-si33 | Cfs-Ams-Af-Am-Gf-Um-Cf-Am-Af-Gm-Gf-Am-Gf-Cm-Af-Um-Gf-Gm-Af-Am-Uf | 219/51 | Ams-Ufs-Um-Cf-Cm-Af-Um-Gf-Cm-Uf-Cm-Cf-Um-Uf-Gm-Af-Cm-Uf-Um-Uf-Gms-Cfs-Am | 291/12 3 |
| P92-si34 | Cfs-Cms-Cf-Um-Cf-Am-Gf-Gm-Af-Gm-Cf-Am-Gf-Gm-Uf-Gm-Af-Cm-Cf-Gm-Uf | 220/52 | Ams-Cfs-Gm-Gf-Um-Cf-Am-Cf-Cm-Uf-Gm-Cf-Um-Cf-Cm-Uf-Gm-Af-Gm-Gf-Gms-Gfs-Cm | 292/12 4 |
| P92-si35 | Gfs-Ams-Gf-Gm-Af-Gm-Gf-Gm-Cf-Um-Gf-Gm-Af-Cm-Cf-Cm-Uf-Gm-Af-Cm-Uf | 221/53 | Ams-Gfs-Um-Cf-Am-Gf-Gm-Gf-Um-Cf-Cm-Af-Gm-Cf-Cm-Cf-Um-Cf-Cm-Uf-Cms-Gfs-Cm | 293/12 5 |
| P92-si36 | Gfs-Cms-Uf-Gm-Cf-Am-Gf-Um-Gf-Cm-Cf-Cm-Uf-Cm-Cf-Cm-Uf-Gm-Gf-Gm-Af | 222/54 | Ums-Cfs-Cm-Cf-Am-Gf-Gm-Gf-Am-Gf-Gm-Gf-Cm-Af-Cm-Uf-Gm-Cf-Am-Gf-Cms-Cfs-Am | 294/12 6 |
| P92-si37 | Cfs-Cms-Uf-Cm-Cf-Cm-Uf-Gm-Gf-Gm-Af-Cm-Cf-Um-Cf-Cm-Cf-Am-Cf-Gm-Uf | 223/55 | Ams-Cfs-Gm-Uf-Gm-Gf-Gm-Af-Gm-Gf-Um-Cf-Cm-Cf-Am-Gf-Gm-Gf-Am-Gf-Gms-Gfs-Cm | 295/12 7 |
| P92-si38 | Cfs-Cms-Cf-Um-Gf-Gm-Gf-Am-Cf-Cm-Uf-Cm-Cf-Cm-Af-Cm-Gf-Um-Cf-Cm-Uf | 224/56 | Ams-Gfs-Gm-Af-Cm-Gf-Um-Gf-Gm-Gf-Am-Gf-Gm-Uf-Cm-Cf-Cm-Af-Gm-Gf-Gms-Afs-Gm | 296/12 8 |
| P92-si39 | Gfs-Gms-Gf-Cm-Cf-Um-Af-Cm-Gf-Cm-Cf-Gm-Uf-Am-Gf-Am-Cf-Am-Af-Cm-Af | 225/57 | Ums-Gfs-Um-Uf-Gm-Uf-Cm-Uf-Am-Cf-Gm-Gf-Cm-Gf-Um-Af-Gm-Gf-Cm-Cf-Cms-Cfs-Cm | 297/12 9 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si40 | Gfs-Cms-Cf-Gm-Uf-Am-Gf-Am-Cf-Am-Af-Cm-Af-Cm-Gf-Um-Gf-Um-Gf-Um-Af | 226/58 | Ums-Afs-Cm-Af-Cm-Af-Cm-Gf-Um-Gf-Um-Uf-Gm-Uf-Cm-Uf-Am-Cf-Gm-Gf-Cms-Gfs-Um | 298/13 0 |
| P92-si41 | Cfs-Cms-Gf-Um-Af-Gm-Af-Cm-Af-Am-Cf-Am-Cf-Gm-Uf-Gm-Uf-Gm-Uf-Am-Gf | 227/59 | Cms-Ufs-Am-Cf-Am-Cf-Am-Cf-Gm-Uf-Gm-Uf-Um-Gf-Um-Cf-Um-Af-Cm-Gf-Gms-Cfs-Gm | 299/13 1 |
| P92-si42 | Ufs-Ams-Gf-Am-Cf-Am-Af-Cm-Af-Cm-Gf-Um-Gf-Um-Gf-Um-Af-Gm-Uf-Cm-Af | 228/60 | Ums-Gfs-Am-Cf-Um-Af-Cm-Af-Cm-Af-Cm-Gf-Um-Gf-Um-Uf-Gm-Uf-Cm-Uf-Ams-Cfs-Gm | 300/13 2 |
| P92-si43 | Gfs-Ums-Gf-Um-Gf-Um-Af-Gm-Uf-Cm-Af-Gm-Gf-Am-Gf-Cm-Cf-Gm-Gf-Gm-Af | 229/61 | Ums-Cfs-Cm-Cf-Gm-Gf-Cm-Uf-Cm-Cf-Um-Gf-Am-Cf-Um-Af-Cm-Af-Cm-Af-Cms-Gfs-Um | 301/13 3 |
| P92-si44 | Gfs-Ams-Gf-Cm-Cf-Gm-Gf-Gm-Af-Cm-Gf-Um-Cf-Am-Gf-Cm-Af-Cm-Uf-Am-Cf | 230/62 | Gms-Ufs-Am-Gf-Um-Gf-Cm-Uf-Gm-Af-Cm-Gf-Um-Cf-Cm-Cf-Gm-Gf-Cm-Uf-Cms-Cfs-Um | 302/13 4 |
| P92-si45 | Ufs-Cms-Af-Gm-Cf-Am-Cf-Um-Af-Cm-Af-Gm-Gf-Cm-Af-Gm-Cf-Am-Cf-Cm-Af | 231/63 | Ums-Gfs-Gm-Uf-Gm-Cf-Um-Gf-Cm-Cf-Um-Gf-Um-Af-Gm-Uf-Gm-Cf-Um-Gf-Ams-Cfs-Gm | 303/13 5 |
| P92-si46 | Ufs-Cms-Uf-Cm-Cf-Am-Gf-Cm-Uf-Am-Af-Cm-Uf-Gm-Uf-Gm-Gf-Am-Gf-Am-Af | 232/64 | Ums-Ufs-Cm-Uf-Cm-Cf-Am-Cf-Am-Gf-Um-Uf-Am-Gf-Cm-Uf-Gm-Gf-Am-Gf-Ams-Ufs-Gm | 304/13 6 |
| P92-si47 | Gfs-Cms-Uf-Cm-Cf-Cm-Uf-Gm-Af-Um-Uf-Am-Af-Um-Gf-Gm-Af-Gm-Gf-Cm-Uf | 233/65 | Ams-Gfs-Cm-Cf-Um-Cf-Cm-Af-Um-Uf-Am-Af-Um-Cf-Am-Gf-Gm-Gf-Am-Gf-Cms-Cfs-Cm | 305/13 7 |
| P92-si48 | Ufs-Cms-Cf-Cm-Uf-Gm-Af-Um-Uf-Am-Af-Um-Gf-Gm-Af-Gm-Gf-Cm-Uf-Um-Af | 234/66 | Ums-Afs-Am-Gf-Cm-Cf-Um-Cf-Cm-Af-Um-Uf-Am-Af-Um-Cf-Am-Gf-Gm-Gf-Ams-Gfs-Cm | 306/13 8 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si49 | Cfs-Cms-Uf-Gm-Af-Um-Uf-Am-Af-Um-Gf-Gm-Af-Gm-Gf-Cm-Uf-Um-Af-Gm-Cf | 235/67 | Gms-Cfs-Um-Af-Am-Gf-Cm-Cf-Um-Cf-Cm-Af-Um-Uf-Am-Af-Um-Cf-Am-Gf-Gms-Gfs-Am | 307/13 9 |
| P92-si50 | Gfs-Ams-Uf-Um-Af-Am-Uf-Gm-Gf-Am-Gf-Gm-Cf-Um-Uf-Am-Gf-Cm-Uf-Um-Uf | 236/68 | Ams-Afs-Am-Gf-Cm-Uf-Am-Af-Gm-Cf-Cm-Uf-Cm-Cf-Am-Uf-Um-Af-Am-Uf-Cms-Afs-Gm | 308/14 0 |
| P92-si52 | Gfs-Gms-Uf-Gm-Gf-Um-Cf-Am-Cf-Am-Gf-Gm-Cf-Um-Gf-Um-Gf-Cm-Cf-Um-Uf | 237/69 | Ams-Afs-Gm-Gf-Cm-Af-Cm-Af-Gm-Cf-Cm-Uf-Gm-Uf-Gm-Af-Cm-Cf-Am-Cf-Cms-Afs-Gm | 309/14 1 |
| P92-si53 | Ufs-Cms-Af-Cm-Af-Gm-Gf-Cm-Uf-Gm-Uf-Gm-Cf-Cm-Uf-Um-Gf-Gm-Uf-Um-Uf | 238/70 | Ams-Afs-Am-Cf-Cm-Af-Am-Gf-Gm-Cf-Am-Cf-Am-Gf-Cm-Cf-Um-Gf-Um-Gf-Ams-Cfs-Cm | 310/14 2 |
| P92-si54 | Gfs-Ums-Gf-Cm-Cf-Um-Uf-Gm-Gf-Um-Uf-Um-Cf-Cm-Uf-Gm-Af-Gm-Cf-Cm-Af | 239/71 | Ums-Gfs-Gm-Cf-Um-Cf-Am-Gf-Gm-Af-Am-Af-Cm-Cf-Am-Af-Gm-Gf-Cm-Af-Cms-Afs-Gm | 311/14 3 |
| P92-si55 | Afs-Gms-Cf-Cm-Af-Cm-Cf-Um-Uf-Um-Af-Cm-Uf-Cm-Uf-Gm-Cf-Um-Cf-Um-Af | 240/72 | Ums-Afs-Gm-Af-Gm-Cf-Am-Gf-Am-Gf-Um-Af-Am-Af-Gm-Gf-Um-Gf-Gm-Cf-Ums-Cfs-Am | 312/14 4 |
| P92-si56 | Cfs-Cms-Af-Cm-Cf-Um-Uf-Um-Af-Cm-Uf-Cm-Uf-Gm-Cf-Um-Cf-Um-Af-Um-Gf | 241/73 | Cms-Afs-Um-Af-Gm-Af-Gm-Cf-Am-Gf-Am-Gf-Um-Af-Am-Af-Gm-Gf-Um-Gf-Gms-Cfs-Um | 313/14 5 |
| P92-si57 | Cfs-Ums-Uf-Um-Af-Cm-Uf-Cm-Uf-Gm-Cf-Um-Cf-Um-Af-Um-Gf-Cm-Cf-Am-Gf | 242/74 | Cms-Ufs-Gm-Gf-Cm-Af-Um-Af-Gm-Af-Gm-Cf-Am-Gf-Am-Gf-Um-Af-Am-Af-Gms-Gfs-Um | 314/14 6 |
| P92-si58 | Gfs-Cms-Uf-Gm-Uf-Gm-Cf-Um-Af-Gm-Cf-Am-Af-Cm-Af-Cm-Cf-Cm-Af-Am-Af | 243/75 | Ums-Ufs-Um-Gf-Gm-Gf-Um-Gf-Um-Uf-Gm-Cf-Um-Af-Gm-Cf-Am-Cf-Am-Gf-Cms-Cfs-Um | 315/14 7 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si59 | Ufs-Cms-Af-Cm-Cf-Um-Af-Gm-Gf-Am-Cf-Um-Gf-Am-Cf-Um-Cf-Gm-Gf-Cm-Af | 244/76 | Ums-Gfs-Cm-Cf-Gm-Af-Gm-Uf-Cm-Af-Gm-Uf-Cm-Cf-Um-Af-Gm-Gf-Um-Gf-Ams-Ufs-Gm | 316/14 8 |
| P92-si60 | Afs-Cms-Uf-Gm-Af-Cm-Uf-Cm-Gf-Gm-Cf-Am-Gf-Um-Gf-Um-Gf-Cm-Af-Gm-Uf | 245/77 | Ams-Cfs-Um-Gf-Cm-Af-Cm-Af-Cm-Uf-Gm-Cf-Cm-Gf-Am-Gf-Um-Cf-Am-Gf-Ums-Cfs-Cm | 317/14 9 |
| P92-si61 | Ufs-Cms-Gf-Gm-Cf-Am-Gf-Um-Gf-Um-Gf-Cm-Af-Gm-Uf-Gm-Gf-Um-Gf-Cm-Af | 246/78 | Ums-Gfs-Cm-Af-Cm-Cf-Am-Cf-Um-Gf-Cm-Af-Cm-Af-Cm-Uf-Gm-Cf-Cm-Gf-Ams-Gfs-Um | 318/15 0 |
| P92-si62 | Gfs-Gms-Cf-Am-Gf-Um-Gf-Um-Gf-Cm-Af-Gm-Uf-Gm-Gf-Um-Gf-Cm-Af-Um-Gf | 247/79 | Cms-Afs-Um-Gf-Cm-Af-Cm-Cf-Am-Cf-Um-Gf-Cm-Af-Cm-Af-Cm-Uf-Gm-Cf-Cms-Gfs-Am | 319/15 1 |
| P92-si63 | Gfs-Cms-Af-Gm-Uf-Gm-Gf-Um-Gf-Cm-Af-Um-Gf-Cm-Af-Cm-Uf-Gm-Uf-Cm-Uf | 248/80 | Ams-Gfs-Am-Cf-Am-Gf-Um-Gf-Cm-Af-Um-Gf-Cm-Af-Cm-Cf-Am-Cf-Um-Gf-Cms-Afs-Cm | 320/15 2 |
| P92-si64 | Gfs-Ums-Gf-Cm-Af-Um-Gf-Cm-Af-Cm-Uf-Gm-Uf-Cm-Uf-Cm-Af-Gm-Cf-Cm-Af | 249/81 | Ums-Gfs-Gm-Cf-Um-Gf-Am-Gf-Am-Cf-Am-Gf-Um-Gf-Cm-Af-Um-Gf-Cm-Af-Cms-Cfs-Am | 321/15 3 |
| P92-si65 | Afs-Ams-Cf-Cm-Cf-Gm-Cf-Um-Cf-Cm-Af-Cm-Uf-Am-Cf-Cm-Cf-Gm-Gf-Cm-Af | 250/82 | Ums-Gfs-Cm-Cf-Gm-Gf-Gm-Uf-Am-Gf-Um-Gf-Gm-Af-Gm-Cf-Gm-Gf-Gm-Uf-Ums-Gfs-Gm | 322/15 4 |
| P92-si66 | Afs-Cms-Uf-Am-Cf-Cm-Cf-Gm-Gf-Cm-Af-Gm-Gf-Gm-Uf-Am-Cf-Am-Cf-Am-Uf | 251/83 | Ams-Ufs-Gm-Uf-Gm-Uf-Am-Cf-Cm-Cf-Um-Gf-Cm-Cf-Gm-Gf-Gm-Uf-Am-Gf-Ums-Gfs-Gm | 323/15 5 |
| P92-si67 | Cfs-Cms-Uf-Am-Cf-Um-Uf-Cm-Af-Cm-Af-Gm-Af-Gm-Gf-Am-Af-Gm-Af-Am-Af | 252/84 | Ums-Ufs-Um-Cf-Um-Uf-Cm-Cf-Um-Cf-Um-Gf-Um-Gf-Am-Af-Gm-Uf-Am-Gf-Gms-Gfs-Gm | 324/15 6 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si68 | Cfs-Cms-Af-Gm-Gf-Am-Af-Gm-Cf-Um-Cf-Gm-Gf-Um-Gf-Am-Gf-Um-Gf-Am-Uf | 253/85 | Ams-Ufs-Cm-Af-Cm-Uf-Cm-Af-Cm-Cf-Gm-Af-Gm-Cf-Um-Uf-Cm-Cf-Um-Gf-Gms-Ufs-Cm | 325/15 7 |
| P92-si69 | Afs-Gms-Af-Am-Cf-Gm-Af-Um-Gf-Cm-Cf-Um-Gf-Cm-Af-Gm-Gf-Cm-Af-Um-Gf | 254/86 | Cms-Afs-Um-Gf-Cm-Cf-Um-Gf-Cm-Af-Gm-Gf-Cm-Af-Um-Cf-Gm-Uf-Um-Cf-Ums-Gfs-Cm | 326/15 8 |
| P92-si70 | Ufs-Gms-Cf-Cm-Uf-Gm-Cf-Am-Gf-Gm-Cf-Am-Uf-Gm-Gf-Am-Af-Cm-Uf-Um-Uf | 255/87 | Ams-Afs-Am-Gf-Um-Uf-Cm-Cf-Am-Uf-Gm-Cf-Cm-Uf-Gm-Cf-Am-Gf-Gm-Cf-Ams-Ufs-Cm | 327/15 9 |
| P92-si71 | Cfs-Gms-Uf-Um-Af-Um-Cf-Am-Cf-Cm-Cf-Am-Gf-Gm-Cf-Cm-Uf-Gm-Af-Um-Uf | 256/88 | Ams-Afs-Um-Cf-Am-Gf-Gm-Cf-Cm-Uf-Gm-Gf-Gm-Uf-Gm-Af-Um-Af-Am-Cf-Gms-Gfs-Am | 328/16 0 |
| P92-si72 | Cfs-Ams-Cf-Cm-Cf-Am-Gf-Gm-Cf-Cm-Uf-Gm-Af-Um-Uf-Cm-Af-Cm-Uf-Gm-Gf | 257/89 | Cms-Cfs-Am-Gf-Um-Gf-Am-Af-Um-Cf-Am-Gf-Gm-Cf-Cm-Uf-Gm-Gf-Gm-Uf-Gms-Afs-Um | 329/16 1 |
| P92-si74 | Cfs-Gms-Gf-Am-Gf-Am-Uf-Gm-Cf-Um-Uf-Cm-Uf-Am-Af-Gm-Gf-Cm-Af-Um-Gf | 258/90 | Cms-Afs-Um-Gf-Cm-Cf-Um-Uf-Am-Gf-Am-Af-Gm-Cf-Am-Uf-Cm-Uf-Cm-Cf-Gms-Cfs-Cm | 330/16 2 |
| P92-si75 | Afs-Gms-Af-Um-Gf-Cm-Uf-Um-Cf-Um-Af-Am-Gf-Gm-Cf-Am-Uf-Gm-Gf-Um-Cf | 259/91 | Gms-Afs-Cm-Cf-Am-Uf-Gm-Cf-Cm-Uf-Um-Af-Gm-Af-Am-Gf-Cm-Af-Um-Cf-Ums-Cfs-Cm | 331/16 3 |
| P92-si76 | Afs-Cms-Af-Am-Cf-Um-Gf-Um-Cf-Cm-Cf-Um-Cf-Cm-Uf-Um-Gf-Am-Gf-Cm-Af | 260/92 | Ums-Gfs-Cm-Uf-Cm-Af-Am-Gf-Gm-Af-Gm-Gf-Gm-Af-Cm-Af-Gm-Uf-Um-Gf-Ums-Ufs-Gm | 332/16 4 |
| P92-si77 | Cfs-Ams-Cf-Cm-Cf-Am-Af-Gm-Cf-Am-Af-Gm-Cf-Am-Gf-Am-Cf-Am-Uf-Um-Uf | 261/93 | Ams-Afs-Am-Uf-Gm-Uf-Cm-Uf-Gm-Cf-Um-Uf-Gm-Cf-Um-Uf-Gm-Gf-Gm-Uf-Gms-Gfs-Gm | 333/16 5 |

(continued)

| Sequenc e No. | Sense strand sequence 5'-3' | SEQ ID NO:/Co rrespon ding basic sequenc e SEQ ID NO: | Antisense strand sequence 5'-3' | SEQ ID NO:/C orresp onding basic sequen ce SEQ ID NO: |
|---|---|---|---|---|
| P92-si78 | Cfs-Ams-Af-Gm-Cf-Am-Af-Gm-Cf-Am-Gf-Am-Cf-Am-Uf-Um-Uf-Am-Uf-Cm-Uf | 262/94 | Ams-Gfs-Am-Uf-Am-Af-Am-Uf-Gm-Uf-Cm-Uf-Gm-Cf-Um-Uf-Gm-Cf-Um-Uf-Gms-Gfs-Gm | 334/16 6 |
| P92-si79 | Ufs-Ums-Uf-Gm-Gf-Gm-Uf-Cm-Uf-Gm-Uf-Cm-Cf-Um-Cf-Um-Cf-Um-Gf-Um-Uf | 263/95 | Ams-Afs-Cm-Af-Gm-Af-Gm-Af-Gm-Gf-Am-Cf-Am-Gf-Am-Cf-Cm-Cf-Am-Af-Ams-Afs-Gm | 335/16 7 |
| P92-si80 | Gfs-Ums-Cf-Um-Gf-Um-Cf-Cm-Uf-Cm-Uf-Cm-Uf-Gm-Uf-Um-Gf-Cm-Cf-Um-Uf | 264/96 | Ams-Afs-Gm-Gf-Cm-Af-Am-Cf-Am-Gf-Am-Gf-Am-Gf-Gm-Af-Cm-Af-Gm-Af-Cms-Cfs-Cm | 336/16 8 |

**Example 2: Inhibitory effect of alternately modified sequences on the PCSK9 gene**

2'-OMe and 2'-F alternately modified siRNA sequences synthesized in Example 1 were transfected into HELA cells via lipid nanoparticles (LNP). The inhibitory effect of each sequence on the PCSK9 gene was detected using qPCR technology.

**1. Experimental Materials**

Test sample: the alternately modified small interfering RNA sequence P92-si1-84 listed in Table 2 (synthesized in Example 1).

Cell type: HeLa cell line HELA cells

Drug solvent: DNase/RNase-free water, Gibco Opti-MEM.

**2. Experimental methods**

The inhibitory effect of the samples on the mRNA expression of the PCSK9 gene was detected in HELA cell lines using qRT-PCR.

**2.1 Cell culture**

The passaged HELA cell line cells were used. The logarithmically growing cells were cultured with 10% fetal bovine serum RPMI 1640 medium (supplemented with 100× penicillin and streptomycin 10 μL/mL) in a cell culture incubator at 37°C containing 5% $CO_2$, and the medium was changed once a day. The cells were digested with 0.25% trypsin and centrifuged at 1000 r/min for 5 min. The supernatant was discarded, and fresh culture medium was added for subculture.

**2.2 Cell transfection**

Preparation of a transfection mixture: Lipofectamine RNAiMAX and Opti-MEM were mixed in a ratio of 1.5:98.5 and then mixed well by vortex.

Preparation of transfection reagent: 60 μL of siRNA solution diluted in Opti-MEM was added to 60 μL of the transfection mixture at a ratio of 1:1 (v/v), mixed well by vortex and left to stand at room temperature for 15 min to obtain lipid nanoparticles (LNPs), 12.5 μL of which was used for detection of encapsulation efficiency.

Transfection reagent for blank control group: 60 μL of the prepared transfection mixture was added to 60 μL of Opti-MEM, mixed well by vortex and left to stand at room temperature for 15 min.

The prepared transfection reagent was added to a 24-well cell culture plate (100 μL per well) to a final siRNA concentration of 1 nM per well. 500 μL of cell suspension ($6\times10^4$ cells per ml) was added and mixed well using the cross method. The plate was placed in a 37°C, 5% $CO_2$ cell culture incubator for 40 h.

**2.3 PCSK9 mRNA detection**

1) RNA extraction

a. The culture medium was removed from the 12-well plate. 0.5 mL of 1×PBS was added to each well to wash the cells, and the PBS was removed. 0.5 mL TRIzol reagent was added to the wells. The cells were pipetted up and down to ensure complete lysis, then transferred to a 1.5 mL RNase-free EP tube, and left to stand at room temperature for 5 min.

b. Each tube was added with 0.1 mL of chloroform, shaken vigorously for 15 sec, and left to stand at room temperature for 5 min. After centrifugation at 4°C, 12,000×g for 15 min, 200 μL of the supernatant was collected into a new EP tube.

c. An equal volume of isopropanol was added. The liquid in the tube was gently mixed by inverting the tube upside

down, and the tube was left to stand at -20°C for 10 min. After centrifugation at 4°C, 12,000×g for 15 min, the supernatant was discarded.

d. 0.5 mL of 75% ethanol was added to gently wash the RNA precipitate. After centrifugation at 4°C, 12,000 × g for 5 min, the supernatant was removed. The washing was repeated once. After centrifugation at 4°C 12,000×g for 1 min, the residual ethanol was removed with a micropipette tip.

e. The residual ethanol was dried off at room temperature for 2-3 min. 40 μL of RNase-free ddH$_2$O was added for dissolution.

2) RNA concentration detection

The RNA concentration was detected using nanodrop. 2 μL of RNase-free ddH$_2$O was used as a blank control, and 2 μL of RNA sample was used for detection each time. The sample concentration was recorded.

3) PCSK9 mRNA quantitative detection

In a 15 mL centrifuge tube, the remaining components except primers and template were added in 7.5 × 84 = 630 portions, marked as A.

84 EP tubes of 1.5 mL were marked. 77 μL of A and 420 ng of total RNA were added to each tube and mixed well for later use (marked as B).

The upstream and downstream primers of the internal reference gene GAPDH and the target gene PCSK9 were mixed well for later use (marked as C).

11 μL of B + 1 μL of C were added to each well of a PCR plate, which was covered with a sealing film, centrifuged at 3000 rpm for 1 min, and transferred to the instrument.

*This step was performed on ice to maintain low temperature conditions.

The plate was placed into the qPCR instrument and subjected to the following program:

Reverse transcription: 55°C, 15 min;

Pre-denaturation: 95°C, 30 sec;

Cyclic reaction: 95°C, 10 sec; 60°C, 35 sec; 40 cycles;

Melting curve: 95°C, 15 sec; 60°C, 60 sec; 95°C, 15 sec.

The running time was about 2 h. The experimental results were analyzed and 2-ΔΔCt was calculated.

**2.4 Data processing**

The PCSK9 mRNA expression rate (%) was calculated as follows:

Expression rate = (PCSK9 mRNA expression level/PCSK9 mRNA expression level in blank control group) × 100%;

$$\text{PCSK9 gene expression inhibition rate} = 1\text{-expression rate } (\%).$$

**3. Experimental Results**

The inhibition rate in this example was the average value of 4 experiments. The inhibition rate of each sequence on the expression of PCSK9 mRNA in HELA cells is shown in Tables 4-6.

Experimental results show that some sequences alternately modified with 2'-OMe and 2'-F exhibited a significant inhibitory effect on the expression of PCSK9 mRNA in HELA cells with the inhibition rates exceeding 50%, including P92-si5, P92-si51, P92-si81, P92-si82, P92-si84, P92-si3, P92-si8, P92-si9, P92-si21, P92-si31, P92-si73 and P92-si83 (see Table 3 for specific sequences), among which the inhibition rate of P92-si5, P92-si81, P92-si82, P92-si3, P92-si8 and P92-si31 exceeded 70%.

The other sequences had a relatively poor inhibitory effect on PCSK9 mRNA expression, and the inhibition rates were all lower than 50%.

Table 3 siRNA sequences with significant inhibitory effect on PCSK9 gene

| Se qu en ce No . | Sense strand sequence 5'-3' | SEQ ID NO:/Correspo nding basic sequence SEQ ID NO: | Antisense strand sequence 5'-3' | SEQ ID NO:/Correspo nding basic sequence SEQ ID NO: |
|---|---|---|---|---|
| P9 2-s i5 | Afs-Ams-Gf-Am-Uf-Cm-Cf-Um-Gf-Cm-Af-Um-Gf-Um-Cf-Um-Uf-Cm-Cf-Am-Uf | 169/1 | Ams-Ufs-Gm-Gf-Am-Af-Gm-Af-Cm-Af-Um-Gf-Cm-Af-Gm-Gf-Am-Uf-Cm-Uf-Ums-Gfs-Gm | 181/13 |
| P9 2-s i51 | Ufs-Gms-Gf-Am-Gf-Gm-Cf-Um-Uf-Am-Gf-Cm-Uf-Um-Uf-Cm-Uf-Gm-Gf-Am-Uf | 170/2 | Ams-Ufs-Cm-Cf-Am-Gf-Am-Af-Am-Gf-Cm-Uf-Am-Af-Gm-Cf-Cm-Uf-Cm-Cf-Ams-Ufs-Um | 182/14 |
| P9 2-s i81 | Cfs-Ums-Uf-Um-Uf-Gm-Uf-Am-Af-Cm-Uf-Um-Gf-Am-Af-Gm-Af-Um-Af-Um-Uf | 171/3 | Ams-Afs-Um-Af-Um-Cf-Um-Uf-Cm-Af-Am-Gf-Um-Uf-Am-Cf-Am-Af-Am-Af-Gms-Cfs-Am | 183/15 |
| P9 2-s i82 | Gfs-Ams-Af-Gm-Af-Um-Af-Um-Uf-Um-Af-Um-Uf-Cm-Uf-Gm-Gf-Gm-Uf-Um-Uf | 172/4 | Ams-Afs-Am-Cf-Cm-Cf-Am-Gf-Am-Af-Um-Af-Am-Af-Um-Af-Um-Cf-Um-Uf-Cms-Afs-Am | 184/16 |
| P9 2-s i84 | Gfs-Ums-Uf-Um-Uf-Gm-Uf-Am-Gf-Cm-Af-Um-Uf-Um-Uf-Um-Af-Um-Uf-Am-Af | 173/5 | Ums-Ufs-Am-Af-Um-Af-Am-Af-Am-Af-Um-Gf-Cm-Uf-Am-Cf-Am-Af-Am-Af-Cms-Cfs-Cm | 185/17 |
| P9 2-s i3 | Cfs-Ams-Cf-Cm-Af-Am-Gf-Am-Uf-Cm-Cf-Um-Gf-Cm-Af-Um-Gf-Um-Cf-Um-Uf | 174/6 | Ams-Afs-Gm-Af-Cm-Af-Um-Gf-Cm-Af-Gm-Gf-Am-Uf-Cm-Uf-Um-Gf-Gm-Uf-Gms-Afs-Gm | 186/18 |
| P9 2-s i8 | Cfs-Ums-Cf-Am-Uf-Am-Gf-Gm-Cf-Cm-Uf-Gm-Gf-Am-Gf-Um-Uf-Um-Af-Um-Uf | 175/7 | Ams-Afs-Um-Af-Am-Af-Cm-Uf-Cm-Cf-Am-Gf-Gm-Cf-Cm-Uf-Am-Uf-Gm-Af-Gms-Gfs-Gm | 187/19 |
| P9 2-s i9 | Gfs-Gms-Cf-Cm-Uf-Gm-Gf-Am-Gf-Um-Uf-Um-Af-Um-Uf-Cm-Gf-Gm-Af-Am-Af | 176/8 | Ums-Ufs-Um-Cf-Cm-Gf-Am-Af-Um-Af-Am-Af-Cm-Uf-Cm-Cf-Am-Gf-Gm-Cf-Cms-Ufs-Am | 188/20 |
| P9 2-s i21 | Cfs-Ams-Gf-Cm-Af-Cm-Cf-Um-Gf-Cm-Uf-Um-Uf-Gm-Uf-Gm-Uf-Cm-Af-Cm-Af | 177/9 | Ums-Gfs-Um-Gf-Am-Cf-Am-Cf-Am-Af-Am-Gf-Cm-Af-Gm-Gf-Um-Gf-Cm-Uf-Gms-Cfs-Am | 189/21 |

(continued)

| Se qu en ce No . | Sense strand sequence 5'-3' | SEQ ID NO:/Correspo nding basic sequence SEQ ID NO: | Antisense strand sequence 5'-3' | SEQ ID NO:/Correspo nding basic sequence SEQ ID NO: |
|---|---|---|---|---|
| P9 2-s i31 | Gfs-Gms-Uf-Cm-Uf-Gm-Gf-Am-Af-Um-Gf-Cm-Af-Am-Af-Gm-Uf-Cm-Af-Am-Gf | 178/10 | Cms-Ufs-Um-Gf-Am-Cf-Um-Uf-Um-Gf-Cm-Af-Um-Uf-Cm-Cf-Am-Gf-Am-Cf-Cms-Ufs-Gm | 190/22 |
| P9 | Gfs-Cms-Gf-Gm-Af-Gm-Af- | 179/11 | Ams-Ufs-Gm-Cf-Cm-Uf-Um-Af- | 191/23 |
| 2-s i73 | Um-Gf-Cm-Uf-Um-Cf-Um-Af-Am-Gf-Gm-Cf-Am-Uf | | Gm-Af-Am-Gf-Cm-Af-Um-Cf-Um-Cf-Cm-Gf-Cms-Cfs-Am | |
| P9 2-s i83 | Gfs-Gms-Gf-Um-Uf-Um-Uf-Gm-Uf-Am-Gf-Cm-Af-Um-Uf-Um-Uf-Um-Af-Um-Uf | 180/12 | Ams-Afs-Um-Af-Am-Af-Am-Af-Um-Gf-Cm-Uf-Am-Cf-Am-Af-Am-Af-Cm-Cf-Cms-Afs-Gm | 192/24 |

The inhibition rates of PCSK9 mRNA expression in HELA cells by each sequence are shown in Tables 4-6.

**(1) Among the 84 designed sequences, 12 sequences exhibited a significant inhibitory effect on the PCSK9 gene, with inhibition rates exceeding 50%, including P92-si5, P92-si51, P92-si81, P92-si82, and P92-si84, P92-si3, P92-si8, P92-si9, P92-si21, P92-si31, P92-si73 and P92-si83, among which the inhibition rate of P92-si5, P92-si81, P92-si82, P92-si3, P92-si8 and P92-si31 exceeded 70%.**

Table 4 Alternately modified sequences with a significant inhibitory effect (inhibition rate exceeding 50%) on the PCSK9 gene

| Basic sequence NO. | Sequence No. | Inhibition rate (%) |
|---|---|---|
| 5 | P92-si5 | 72.7 |
| 51 | P92-si51 | 61.4 |
| 81 | P92-si81 | 71.8 |
| 82 | P92-si82 | 75.3 |
| 84 | P92-si84 | 66.2 |
| 3 | P92-si3 | 70.9 |
| 8 | P92-si8 | 72.0 |
| 9 | P92-si9 | 63.9 |
| 21 | P92-si21 | 62.8 |
| 31 | P92-si31 | 70.4 |
| 73 | P92-si73 | 60.6 |
| 83 | P92-si83 | 58.8 |

As can be seen from Table 4, the alternately modified sequences P92-si5, P92-si51, P92-si81, P92-si82, P92-si84, P92-si3, P92-si8, P92-si9, P92-si21, P92-si31, P92-si73 and P92-si83 (see Table 3 for specific sequences), a total of 12 sequences, exhibited a significant inhibitory effect on the expression of PCSK9 mRNA, with the inhibition rates all above 50%, among which the inhibition rate of P92-si5, P92-si81, P92-si82, P92-si3, P92-si8 and P92-si31 exceeded 70% (FIG. 1). These 12 sequences can be used as candidate sequences.

**(2) The inhibition rates of 26 sequences on the PCSK9 gene ranged from 30% to 50%. For example, the inhibition rates of P92-si6 and P92-si7 were 38.3% and 35.7%, respectively.**

Table 5 Alternately modified sequences with an inhibition rate of 30%-50% on the PCSK9 gene

| Basic sequence NO. | Sequence No. | Inhibition rate (%) |
|---|---|---|
| 6 | P92-si6 | 38.3 |
| 7 | P92-si7 | 35.7 |
| 11 | P92-si11 | 33.2 |
| 15 | P92-si15 | 31.6 |
| 17 | P92-si17 | 32.2 |
| 18 | P92-si18 | 48.2 |
| 19 | P92-si19 | 35.1 |
| 25 | P92-si25 | 48.1 |
| 30 | P92-si30 | 31.0 |
| 32 | P92-si32 | 30.1 |
| 33 | P92-si33 | 47.4 |
| 37 | P92-si37 | 37.0 |

(continued)

| Basic sequence NO. | Sequence No. | Inhibition rate (%) |
|---|---|---|
| 40 | P92-si40 | 46.0 |
| 47 | P92-si47 | 31.7 |
| 48 | P92-si48 | 40.9 |
| 56 | P92-si56 | 30.3 |
| 59 | P92-si59 | 39.8 |
| 61 | P92-si61 | 37.4 |
| 63 | P92-si63 | 33.6 |
| 65 | P92-si65 | 38.2 |
| 67 | P92-si67 | 35.2 |
| 70 | P92-si70 | 46.4 |
| 71 | P92-si71 | 32.9 |
| 75 | P92-si75 | 40.7 |
| 78 | P92-si78 | 34.1 |
| 80 | P92-si80 | 42.1 |

The 26 sequences listed in Table 5 exhibited a relatively poor inhibitory effect on the PCSK9 gene, with inhibition rates below 50% and ranging from 30% to 50%. For example, the inhibition rates of P92-si6 and P92-si7 were 38.3% and 35.7%, respectively (FIG. 2).

**(3) The inhibition rates of 46 sequences on the PCSK9 gene were below 30%. For example, the inhibition rates of P92-si1 and P92-si20 were only 9.2% and 6.0%, respectively.**

Table 6 Alternately modified sequences with an inhibition rate below 30% on the PCSK9 gene

| Basic sequence NO. | Sequence No. | Inhibition rate (%) |
|---|---|---|
| 1 | P92-si1 | 9.2 |
| 2 | P92-si2 | 25.0 |
| 4 | P92-si4 | 10.9 |
| 10 | P92-si10 | 10.8 |
| 12 | P92-si12 | -5.7 |
| 13 | P92-si13 | 19.4 |
| 14 | P92-si14 | 17.1 |
| 16 | P92-si16 | -4.6 |
| 20 | P92-si20 | 6.0 |
| 22 | P92-si22 | -2.8 |
| 23 | P92-si23 | 26.1 |
| 24 | P92-si24 | 2.9 |
| 26 | P92-si26 | -2.5 |
| 27 | P92-si27 | 21.6 |
| 28 | P92-si28 | 11.1 |
| 29 | P92-si29 | -6.0 |
| 34 | P92-si34 | -5.4 |
| 35 | P92-si35 | 15.2 |

(continued)

| Basic sequence NO. | Sequence No. | Inhibition rate (%) |
|---|---|---|
| 36 | P92-si36 | -3.1 |
| 38 | P92-si38 | 5.5 |
| 39 | P92-si39 | 26.8 |
| 41 | P92-si41 | 10.7 |
| 42 | P92-si42 | 29.7 |
| 43 | P92-si43 | 20.4 |
| 44 | P92-si44 | 15.7 |
| 45 | P92-si45 | 29.7 |
| 46 | P92-si46 | -6.4 |
| 49 | P92-si49 | 29.0 |
| 50 | P92-si50 | 14.6 |
| 52 | P92-si52 | -1.2 |
| 53 | P92-si53 | 28.0 |
| 54 | P92-si54 | 7.3 |
| 55 | P92-si55 | 14.1 |
| 57 | P92-si57 | 18.2 |
| 58 | P92-si58 | 25.4 |
| 60 | P92-si60 | -4.6 |
| 62 | P92-si62 | 4.7 |
| 64 | P92-si64 | -7.9 |
| 66 | P92-si66 | 22.9 |
| 68 | P92-si68 | 11.8 |
| 69 | P92-si69 | -6.0 |
| 72 | P92-si72 | -3.9 |
| 74 | P92-si74 | -1.6 |
| 76 | P92-si76 | 5.2 |
| 77 | P92-si77 | 5.7 |
| 79 | P92-si79 | 25.4 |

The 46 sequences listed in Table 6 exhibited a very poor inhibitory effect on the expression of PCSK9 mRNA, with the inhibition rate all less than 30%. For example, the inhibition rates of P92-si1 and P92-si20 were only 9.2% and 6.0%, respectively (FIGs. 3 and 4).

(4) siRNAs with similar sequences had very different activities. For example, the inhibition rate of P92-si5 was 61.8% higher than that of P92-si4, indicating a significant improvement.

The inhibitory effect of some siRNAs with similar sequences on the PCSK9 mRNA is shown in Table 7.

Table 7 Comparison of the inhibition rate on the PCSK9 gene expression by siRNAs with similar sequences

| Si mil ar seq uen ce gro up | Ba sic seq ue nce N O. | Seq uen ce No. | Corresponding basic sequence sense strand | Corres pondin g basic sequenc e SEQ ID NO: | Corresponding basic sequence antisense strand | Corres pondin g basic sequen ce SEQ ID NO: | Inhi biti on rate (%) |
|---|---|---|---|---|---|---|---|
| A | 4 | P92-si4 | C**AAGAUCCUGCAUGU CUUCCA** | 27 | **UGGAAGACAUGCAGGA UCUUGG**U | 99 | 10.9 |
| | 5 | P92-si5 | **AAGAUCCUGCAUGUC UUCCA**U | 1 | A**UGGAAGACAUGCAGG AUCUUGG** | 13 | 72.7 |
| B | 50 | P92-si50 | GAUUAA**UGGAGGCUU AGCUUU** | 68 | **AAAGCUAAGCCUCCAUU** AAUCAG | 140 | 14.6 |
| | 51 | P92-si51 | **UGGAGGCUUAGCUUU** CUGGAU | 2 | AUCCAG**AAAGCUAAGCC UCCAUU** | 14 | 61.4 |
| C | 2 | P92-si2 | AUACCU**CACCAAGAU CCUGCA** | 26 | **UGCAGGAUCUUGGUGA G**GUAUCC | 98 | 25.0 |
| | 3 | P92-si3 | **CACCAAGAUCCUGCA** UGUCUU | 6 | AAGACA**UGCAGGAUCUU GGUGAG** | 18 | 70.9 |
| D | 9 | P92-si9 | GG**CCUGGAGUUUAUU CGGAAA** | 8 | **UUUCCGAAUAAACUCCA GGCC**UA | 20 | 63.9 |
| | 10 | P92-si10 | **CCUGGAGUUUAUUCG GAAA**AG | 30 | CU**UUUCCGAAUAAACUC CAGGCC** | 102 | 10.8 |
| E | 21 | P92-si21 | CA**GCACCUGCUUUGU GUCACA** | 9 | **UGUGACACAAAGCAGG UGCUG**CA | 21 | 62.8 |
| | 22 | P92-si22 | **GCACCUGCUUUGUGU** | 41 | UC**UGUGACACAAAGCAG** | 113 | -2.8 |
| | | | **CACA**GA | | **GUGCUG** | | |

(continued)

| Similar sequence group | Basic sequence NO. | Sequence No. | Corresponding basic sequence sense strand | Corresponding basic sequence SEQ ID NO: | Corresponding basic sequence antisense strand | Corresponding basic sequence SEQ ID NO: | Inhibition rate (%) |
|---|---|---|---|---|---|---|---|
| F | 30 | P92-si30 | AGGUCUGGAAUGCAAAGUCAA | 49 | UUGACUUUGCAUUCCAGACCUGG | 121 | 31.0 |
| | 31 | P92-si31 | GGUCUGGAAUGCAAAGUCAAG | 10 | CUUGACUUUGCAUUCCAGACCUG | 22 | 70.4 |
| G | 73 | P92-si73 | GCGGAGAUGCUUCUAAGGCAU | 11 | AUGCCUUAGAAGCAUCUCCGCCA | 23 | 60.6 |
| | 74 | P92-si74 | CGGAGAUGCUUCUAAGGCAUG | 90 | CAUGCCUUAGAAGCAUCUCCGCC | 162 | -1.6 |

It can be seen from Table 7 that some siRNAs with similar sequences exhibited very different inhibitory effects on the expression of PCSK9 mRNA.

Basic sequence 5 differs from basic sequence 4 only in the terminal 1 base. For the sense strand, there is one more C at the 5'-end of basic sequence 4 and one more U at the 3'-end of basic sequence 5, and the remaining bases are identical. For the antisense strand, there is one more U at the 3'-end of basic sequence 4 and one more A at the 5'-end of basic sequence 5, and the remaining bases are identical. However, the inhibition rate of the alternately modified sequence P92-si5 was 61.8% higher than that of P92-si4, indicating a significant improvement.

Basic sequence 51 differs from basic sequence 50 only in the terminal 6 bases. For the sense strand, the 5'-end of the basic sequence 50 is GAUUAA, the 3'-end of the sequence 51 is CUGGAU, and the remaining bases are identical. For the antisense strand, the 3'-end of the basic sequence 50 is AAUCAG, the 5'-end of the basic sequence 51 is AUCCAG, and the remaining bases are identical. However, the inhibition rate of the alternately modified sequence P92-si51 was 46.8% higher than that of P92-si50, indicating a significant improvement.

Basic sequence 3 differs from basic sequence 2 only in the terminal 6 bases. For the sense strand, the 5'-end of basic sequence 2 is AUACCU, the 3'-end of basic sequence 3 is UGUCUU, and the remaining bases are identical. For the antisense strand, the 3'-end of basic sequence 2 is GUAUCC, the 5'-end of basic sequence 3 is AAGACA, and the remaining bases are identical. However, the inhibition rate of the alternately modified sequence P92-si3 was 45.9% higher than that of P92-si2, indicating a significant improvement.

Basic sequence 10 differs from basic sequence 9 only in the terminal 2 bases. For the sense strand, the 5'-end of basic sequence 9 is GG, the 3'-end of basic sequence 10 is AG, and the remaining bases are identical. For the antisense strand, the 3'-end of basic sequence 9 is UA, the 5'-end of basic sequence 10 is CU, and the remaining bases are identical. However, the inhibition rate of the alternately modified sequence P92-si9 was 53.1% higher than that of the sequence P92-si10, indicating a significant improvement.

Basic sequence 22 differs from basic sequence 21 only in the terminal 2 bases. For the sense strand, the 5'-end of basic sequence 21 is CA, the 3'-end of basic sequence 22 is GA, and the remaining bases are identical. For the antisense strand, the 3'-end of basic sequence 21 is CA, the 5'-end of basic sequence 22 is UC, and the remaining bases are identical. However, the inhibition rate of the alternately modified sequence P92-si21 was 65.6% higher than that of P92-si22, indicating a significant improvement.

Basic sequence 30 differs from basic sequence 31 only in the terminal 1 base. For the sense strand, the 5'-end of basic sequence 30 is A, the 3'-end of basic sequence 31 is G, and the remaining bases are identical. For the antisense strand, the 3'-end of basic sequence 30 is G, the 5'-end of basic sequence 31 is C, and the remaining bases are identical. However, the inhibition rate of the alternately modified sequence P92-si31 was 39.4% higher than that of P92-si30, indicating a significant improvement.

Basic sequence 73 differs from basic sequence 74 only in the terminal 1 base. For the sense strand, the 5'-end of the basic sequence 73 is G, the 3'-end of the basic sequence 74 is G, and the remaining bases are identical. For the antisense strand, the 3'-end of the basic sequence 73 is A, the 5'-end of the basic sequence 74 is C, and the remaining bases are identical. However, the inhibition rate of the alternately modified sequence P92-si73 was 62.2% higher than that of P92-si74, indicating a significant improvement.

Therefore, it is not easy to screen out sequences with significant inhibitory activity from a very large number of oligonucleotide sequences designed against the PCSK9 mRNA sequence, and a lot of inventive work is required.

**Summary:**

(1) Among the 84 designed sequences, 12 sequences exhibited significant inhibitory effects on the PCSK9 gene, with inhibition rates exceeding 50%, including P92-si5, P92-si51, P92-si81, P92-si82, P92-si84, P92-si3, P92-si8, P92-si9, P92-si21, P92-si31, P92-si73 and P92-si83, among which the inhibition rates of P92-si5, P92-si81, P92-si82, P92-si3, P92-si8 and P92-si31 exceeded 70%.

(2) The inhibition rates of 26 sequences on the PCSK9 gene were 30%-50%. For example, the inhibition rates of P92-si6 and P92-si7 were 38.3% and 35.7%, respectively.

(3) The inhibition rates of 46 sequences on the PCSK9 gene were less than 30%. For example, the inhibition rates of P92-si1 and P92-si20 were only 9.2% and 6.0%, respectively.

(4) siRNAs with similar sequences had very different activities. For example, the inhibition rate of P92-si5 was 61.8% higher than that of P92-si4, indicating a significant improvement. Therefore, it is not easy to screen out sequences with significant inhibitory activity from a very large number of oligonucleotide sequences designed against the PCSK9

mRNA sequence, and a lot of inventive work is required.

**Example 3: Inhibitory effect of unmodified sequence on PCSK9 gene**

In this example, some unmodified sequences corresponding to the modified sequences in Example 2 were synthesized and transfected into HELA cells via lipid nanoparticles (LNPs). The inhibitory effect of each unmodified sequence on the PCSK9 gene was detected using qPCR technology, and unmodified siRNA sequences with good inhibitory effects were screened out.

**1. Experimental Materials**

Test samples:

The unmodified siRNA sequences are listed in Table 8. All sequences were synthesized according to the method in Example 1.

Table 8 Unmodified siRNA sequences

| Sequenc e No. | Sense strand sequence (Basic sequence) 5'-3' | SEQ ID NO: | Antisense strand sequence (Basic sequence) 5'-3' | SEQ ID NO: |
|---|---|---|---|---|
| si5 | AAGAUCCUGCAUGUCUUCC AU | 1 | AUGGAAGACAUGCAGGAUCU UGG | 13 |
| si51 | UGGAGGCUUAGCUUUCUGG AU | 2 | AUCCAGAAAGCUAAGCCUCCA UU | 14 |
| si81 | CUUUUGUAACUUGAAGAU AUU | 3 | AAUAUCUUCAAGUUACAAAA GCA | 15 |
| si82 | GAAGAUAUUUAUUCUGGG UUU | 4 | AAACCCAGAAUAAAUAUCUU CAA | 16 |
| si84 | GUUUUGUAGCAUUUUUAU UAA | 5 | UUAAUAAAAAUGCUACAAAA CCC | 17 |
| si3 | CACCAAGAUCCUGCAUGUC UU | 6 | AAGACAUGCAGGAUCUUGGU GAG | 18 |
| si8 | CUCAUAGGCCUGGAGUUUA UU | 7 | AAUAAACUCCAGGCCUAUGA GGG | 19 |
| si9 | GGCCUGGAGUUUAUUCGGA AA | 8 | UUUCCGAAUAAACUCCAGGCC UA | 20 |
| si21 | CAGCACCUGCUUUGUGUCA CA | 9 | UGUGACACAAAGCAGGUGCU GCA | 21 |
| si31 | GGUCUGGAAUGCAAAGUCA AG | 10 | CUUGACUUUGCAUUCCAGACC UG | 22 |
| si73 | GCGGAGAUGCUUCUAAGGC AU | 11 | AUGCCUUAGAAGCAUCUCCGC CA | 23 |
| si83 | GGGUUUUGUAGCAUUUUU AUU | 12 | AAUAAAAAUGCUACAAAACC CAG | 24 |
| si1 | GGGAUACCUCACCAAGAUC CU | 25 | AGGAUCUUGGUGAGGUAUCC CCG | 97 |

(continued)

| Sequenc e No. | Sense strand sequence (Basic sequence) 5'-3' | SEQ ID NO: | Antisense strand sequence (Basic sequence) 5'-3' | SEQ ID NO: |
|---|---|---|---|---|
| si2 | AUACCUCACCAAGAUCCUG CA | 26 | UGCAGGAUCUUGGUGAGGUA UCC | 98 |
| si4 | CAAGAUCCUGCAUGUCUUC CA | 27 | UGGAAGACAUGCAGGAUCUU GGU | 99 |
| si6 | GGCUUCCUGGUGAAGAUGA GU | 28 | ACUCAUCUUCACCAGGAAGCC AG | 100 |
| si7 | GCUGGAGCUGGCCUUGAAG UU | 29 | AACUUCAAGGCCAGCUCCAGC AG | 101 |
| si10 | CCUGGAGUUUAUUCGGAAA AG | 30 | CUUUUCCGAAUAAACUCCAGG CC | 102 |
| si11 | GCCAGCUGGUCCAGCCUGU GG | 31 | CCACAGGCUGGACCAGCUGGC UU | 103 |
| si12 | ACUGGUGGUGCUGCUGCCC CU | 32 | AGGGGCAGCAGCACCACCAGU GG | 104 |
| si13 | UGCCCCUGGCGGGUGGGUA CA | 33 | UGUACCCACCCGCCAGGGGCA GC | 105 |
| si14 | UGGGUACAGCCGCGUCCUC AA | 34 | UUGAGGACGCGGCUGUACCCA CC | 106 |
| si15 | CGUCCUCAACGCCGCCUGC CA | 35 | UGGCAGGCGGCGUUGAGGAC GCG | 107 |
| si16 | GGUCGUGCUGGUCACCGCU GC | 36 | GCAGCGGUGACCAGCACGACC CC | 108 |
| si17 | AGGACAUCAUUGGUGCCUC CA | 37 | UGGAGGCACCAAUGAUGUCC UCC | 109 |
| si18 | UCAUUGGUGCCUCCAGCGA CU | 38 | AGUCGCUGGAGGCACCAAUG AUG | 110 |
| si19 | GUGCCUCCAGCGACUGCAG CA | 39 | UGCUGCAGUCGCUGGAGGCAC CA | 111 |
| si20 | AGCGACUGCAGCACCUGCU UU | 40 | AAAGCAGGUGCUGCAGUCGC UGG | 112 |
| si22 | GCACCUGCUUUGUGUCACA GA | 41 | UCUGUGACACAAAGCAGGUG CUG | 113 |
| si23 | CUUUGUGUCACAGAGUGGG AC | 42 | GUCCCACUCUGUGACACAAAG CA | 114 |
| si24 | AGUGGGACAUCACAGGCUG CU | 43 | AGCAGCCUGUGAUGUCCCACU CU | 115 |
| si25 | AUCACAGGCUGCUGCCCAC GU | 44 | ACGUGGGCAGCAGCCUGUGA UGU | 116 |

(continued)

| Sequenc e No. | Sense strand sequence (Basic sequence) 5'-3' | SEQ ID NO: | Antisense strand sequence (Basic sequence) 5'-3' | SEQ ID NO: |
|---|---|---|---|---|
| si26 | UGUCUGCCGAGCCGGAGCU CA | 45 | UGAGCUCCGGCUCGGCAGACA GC | 117 |
| si27 | GGCCGAGUUGAGGCAGAGA CU | 46 | AGUCUCUGCCUCAACUCGGCC AG | 118 |
| si28 | CAUCCACGCUUCCUGCUGC CA | 47 | UGGCAGCAGGAAGCGUGGAU GCU | 119 |
| si29 | CCCAGGUCUGGAAUGCAAA GU | 48 | ACUUUGCAUUCCAGACCUGGG GC | 120 |
| si30 | AGGUCUGGAAUGCAAAGUC AA | 49 | UUGACUUUGCAUUCCAGACCU GG | 121 |
| si32 | GAAUGCAAAGUCAAGGAGC AU | 50 | AUGCUCCUUGACUUUGCAUUC CA | 122 |
| si33 | CAAAGUCAAGGAGCAUGGA AU | 51 | AUUCCAUGCUCCUUGACUUUG CA | 123 |
| si34 | CCCUCAGGAGCAGGUGACC GU | 52 | ACGGUCACCUGCUCCUGAGGG GC | 124 |
| si35 | GAGGAGGGCUGGACCCUGA CU | 53 | AGUCAGGGUCCAGCCCUCCUC GC | 125 |
| si36 | GCUGCAGUGCCCUCCCUGG GA | 54 | UCCCAGGGAGGGCACUGCAGC CA | 126 |
| si37 | CCUCCCUGGGACCUCCCAC GU | 55 | ACGUGGGAGGUCCCAGGGAG GGC | 127 |
| si38 | CCCUGGGACCUCCCACGUC CU | 56 | AGGACGUGGGAGGUCCCAGG GAG | 128 |
| si39 | GGGCCUACGCCGUAGACAA CA | 57 | UGUUGUCUACGGCGUAGGCCC CC | 129 |
| si40 | GCCGUAGACAACACGUGUG UA | 58 | UACACACGUGUUGUCUACGGC GU | 130 |
| si41 | CCGUAGACAACACGUGUGU AG | 59 | CUACACACGUGUUGUCUACGG CG | 131 |
| si42 | UAGACAACACGUGUGUAGU CA | 60 | UGACUACACACGUGUUGUCU ACG | 132 |
| si43 | GUGUGUAGUCAGGAGCCGG GA | 61 | UCCCGGCUCCUGACUACACAC GU | 133 |
| si44 | GAGCCGGGACGUCAGCACU AC | 62 | GUAGUGCUGACGUCCCGGCUC CU | 134 |
| si45 | UCAGCACUACAGGCAGCAC CA | 63 | UGGUGCUGCCUGUAGUGCUG ACG | 135 |

(continued)

| Sequenc e No. | Sense strand sequence (Basic sequence) 5'-3' | SEQ ID NO: | Antisense strand sequence (Basic sequence) 5'-3' | SEQ ID NO: |
|---|---|---|---|---|
| si46 | UCUCCAGCUAACUGUGGAG AA | 64 | UUCUCCACAGUUAGCUGGAG AUG | 136 |
| si47 | GCUCCCUGAUUAAUGGAGG CU | 65 | AGCCUCCAUUAAUCAGGGAGC CC | 137 |
| si48 | UCCCUGAUUAAUGGAGGCU UA | 66 | UAAGCCUCCAUUAAUCAGGG AGC | 138 |
| si49 | CCUGAUUAAUGGAGGCUUA GC | 67 | GCUAAGCCUCCAUUAAUCAGG GA | 139 |
| si50 | GAUUAAUGGAGGCUUAGC UUU | 68 | AAAGCUAAGCCUCCAUUAAUC AG | 140 |
| si52 | GGUGGUCACAGGCUGUGCC UU | 69 | AAGGCACAGCCUGUGACCACC AG | 141 |
| si53 | UCACAGGCUGUGCCUUGGU UU | 70 | AAACCAAGGCACAGCCUGUGA CC | 142 |
| si54 | GUGCCUUGGUUUCCUGAGC CA | 71 | UGGCUCAGGAAACCAAGGCAC AG | 143 |
| si55 | AGCCACCUUUACUCUGCUC UA | 72 | UAGAGCAGAGUAAAGGUGGC UCA | 144 |
| si56 | CCACCUUUACUCUGCUCUA UG | 73 | CAUAGAGCAGAGUAAAGGUG GCU | 145 |
| si57 | CUUUACUCUGCUCUAUGCC AG | 74 | CUGGCAUAGAGCAGAGUAAA GGU | 146 |
| si58 | GCUGUGCUAGCAACACCCA AA | 75 | UUUGGGUGUUGCUAGCACAG CCU | 147 |
| si59 | UCACCUAGGACUGACUCGG CA | 76 | UGCCGAGUCAGUCCUAGGUG AUG | 148 |
| si60 | ACUGACUCGGCAGUGUGCA GU | 77 | ACUGCACACUGCCGAGUCAGU CC | 149 |
| si61 | UCGGCAGUGUGCAGUGGUG CA | 78 | UGCACCACUGCACACUGCCGA GU | 150 |
| si62 | GGCAGUGUGCAGUGGUGCA UG | 79 | CAUGCACCACUGCACACUGCC GA | 151 |
| si63 | GCAGUGGUGCAUGCACUGU CU | 80 | AGACAGUGCAUGCACCACUGC AC | 152 |
| si64 | GUGCAUGCACUGUCUCAGC CA | 81 | UGGCUGAGACAGUGCAUGCA CCA | 153 |
| si65 | AACCCGCUCCACUACCCGG CA | 82 | UGCCGGGUAGUGGAGCGGGU UGG | 154 |

(continued)

| Sequenc e No. | Sense strand sequence (Basic sequence) 5'-3' | SEQ ID NO: | Antisense strand sequence (Basic sequence) 5'-3' | SEQ ID NO: |
|---|---|---|---|---|
| si66 | ACUACCCGGCAGGGUACAC AU | 83 | AUGUGUACCCUGCCGGGUAG UGG | 155 |
| si67 | CCUACUUCACAGAGGAAGA AA | 84 | UUUCUUCCUCUGUGAAGUAG GGG | 156 |
| si68 | CCAGGAAGCUCGGUGAGUG AU | 85 | AUCACUCACCGAGCUUCCUGG UC | 157 |
| si69 | AGAACGAUGCCUGCAGGCA UG | 86 | CAUGCCUGCAGGCAUCGUUCU GC | 158 |
| si70 | UGCCUGCAGGCAUGGAACU UU | 87 | AAAGUUCCAUGCCUGCAGGCA UC | 159 |
| si71 | CGUUAUCACCCAGGCCUGA UU | 88 | AAUCAGGCCUGGGUGAUAAC GGA | 160 |
| si72 | CACCCAGGCCUGAUUCACU GG | 89 | CCAGUGAAUCAGGCCUGGGU GAU | 161 |
| si74 | CGGAGAUGCUUCUAAGGCA UG | 90 | CAUGCCUUAGAAGCAUCUCCG CC | 162 |
| si75 | AGAUGCUUCUAAGGCAUGG UC | 91 | GACCAUGCCUUAGAAGCAUCU CC | 163 |
| si76 | ACAACUGUCCCUCCUUGAG CA | 92 | UGCUCAAGGAGGGACAGUUG UUG | 164 |
| si77 | CACCCAAGCAAGCAGACAU UU | 93 | AAAUGUCUGCUUGCUUGGGU GGG | 165 |
| si78 | CAAGCAAGCAGACAUUUAU CU | 94 | AGAUAAAUGUCUGCUUGCUU GGG | 166 |
| si79 | UUUGGGUCUGUCCUCUCUG UU | 95 | AACAGAGAGGACAGACCCAA AAG | 167 |
| si80 | GUCUGUCCUCUCUGUUGCC UU | 96 | AAGGCAACAGAGAGGACAGA CCC | 168 |

Cell type: HeLa cell line HELA cells
Drug solvent: DNase/RNase-free water, Gibco Opti-MEM.

**2. Experimental methods**

Refer to Example 2.

**3. Experimental results**

The inhibition rates of PCSK9 mRNA expression by each unmodified sequence are shown in Tables 9 and 10.

The unmodified sequences si5, si51, si81, si82, si84, si3, si8, si9, si21, si31, si73 and si83 exhibited a significant inhibitory effect on the expression of PCSK9 mRNA in HELA cells, with inhibition rates all above 50%, among which the inhibition rates of si5, si81, si82, si3 and si8 exceeded 60%.

**(1) 12 unmodified sequences exhibited a significant inhibitory effect on the PCSK9 gene, with inhibition rates exceeding 50%, including si5, si51, si81, si82, si84, si3, si8, si9, si21, si31, si73 and si83, among which the inhibition rates of si5, si81, si82, si3 and si8 exceeded 60%.**

Table 9 Unmodified sequences with a significant inhibitory effect (inhibition rate higher than 50%) on the PCSK9 gene

| Basic sequence NO. | Sequence No. | Inhibition rate (%) |
|---|---|---|
| 5 | si5 | 60.3 |
| 51 | si51 | 53.4 |
| 81 | si81 | 62.0 |
| 82 | si82 | 63.1 |
| 84 | si84 | 55.7 |
| 3 | si3 | 62.3 |
| 8 | si8 | 60.6 |
| 9 | si9 | 52.6 |
| 21 | si21 | 51.2 |
| 31 | si31 | 59.2 |
| 73 | si73 | 52.4 |
| 83 | si83 | 50.1 |

The unmodified sequences listed in Table 9, including si5, si51, si81, si82, si84, si3, si8, si9, si21, si31, si73 and si83, exhibited a significant inhibitory effect on the expression of PCSK9 mRNA, with an inhibition rate all above 50%, among which the inhibition rate of si5, si81, si82, si3 and si8 exceeded 60% (FIG. 5). These 12 sequences can be used as candidate sequences.

**(2) The inhibition rates of other unmodified sequences on the PCSK9 gene were less than 40%. For example, the inhibition rates of si52 and si74 were only 0.5% and 2.2%, respectively.**

Table 10 Unmodified sequences with an inhibition rate lower than 40% on the PCSK9 gene

| Basic sequence NO. | Sequence No. | Inhibition rate (%) |
|---|---|---|
| 2 | si2 | 19.6 |
| 4 | si4 | 9.1 |
| 6 | si6 | 31.5 |
| 7 | si7 | 31.1 |
| 10 | si10 | 11.4 |
| 20 | si20 | -3.0 |
| 30 | si30 | 26.7 |
| 32 | si32 | 27.3 |
| 50 | si50 | 10.8 |
| 52 | si52 | 0.5 |
| 22 | si22 | -2.0 |
| 72 | si72 | 1.0 |
| 74 | si74 | 2.2 |
| 80 | si80 | 38.5 |

Each sequence in Table 10 exhibited a very poor inhibitory effect on the expression of PCSK9 mRNA, with an

inhibition rate less than 40%. For example, the inhibition rates of si52 and si74 were only 0.5% and 2.2%, respectively (FIG. 6).

**(3) siRNAs with similar sequences had very different activities. For example, the inhibition rate of basic sequence 5 was 51.2% higher than that of basic sequence 4, indicating a significant improvement.**

A comparison of the inhibitory effects of some siRNAs with similar sequences on PCSK9 mRNA is shown in Table 11.

Table 11 Comparison of the inhibition rate on the PCSK9 gene expression by unmodified sequences with similar sequences

| Similar sequence group | Basic sequence NO. | Sequence No. | Sense strand | Basic sequence SEQ ID NO: | Antisense strand | Basic sequence SEQ ID NO: | Inhibition rate (%) |
|---|---|---|---|---|---|---|---|
| A | 4 | si4 | C**AAGAUCCUGCAUGUCUUCCA** | 27 | **UGGAAGACAUGCAGGAUCUUGG**U | 99 | 9.1 |
| | 5 | si5 | **AAGAUCCUGCAUGUCUUCCA**U | 1 | A**UGGAAGACAUGCAGGAUCUUGG** | 13 | 60. 3 |
| B | 50 | si5 0 | GAUUAA**UGGAGGCUUAGCUUU** | 68 | **AAAGCUAAGCCUCCAUU**AAUCAG | 140 | 10. 8 |
| | 51 | si5 1 | **UGGAGGCUUAGCUUU**CUGGAU | 2 | AUCCAG**AAAGCUAAGCCUCCAUU** | 14 | 53. 4 |
| C | 2 | si2 | AUACCU**CACCAAGAUCCUGCA** | 26 | **UGCAGGAUCUUGGUGAG**GUAUCC | 98 | 19. 6 |
| | 3 | si3 | **CACCAAGAUCCUGCA**UGUCUU | 6 | AAGACA**UGCAGGAUCUUGGUGAG** | 18 | 62. 3 |
| D | 9 | si9 | GG**CCUGGAGUUUAUUCGGAAA** | 8 | **UUUCCGAAUAAACUCCAGGCC**UA | 20 | 52. 6 |
| | 10 | si1 0 | **CCUGGAGUUUAUUCGGAAA**AG | 30 | CU**UUUCCGAAUAAACUCCAGGCC** | 102 | 11. 4 |
| E | 21 | 21 | CA**GCACCUGCUUUGUGUCACA** | 9 | **UGUGACACAAAGCAGGUGCUG**CA | 21 | 51. 2 |
| | 22 | 22 | **GCACCUGCUUUGUGUCACA**GA | 41 | UC**UGUGACACAAAGCAGGUGCUG** | 113 | -2. 0 |
| F | 30 | 30 | A**GGUCUGGAAUGCAAAGUCAA** | 49 | **UUGACUUUGCAUUCCAGACCUG**G | 121 | 26. 7 |
| | 31 | 31 | **GGUCUGGAAUGCAAAGUCAA**G | 10 | C**UUGACUUUGCAUUCCAGACCUG** | 22 | 59. 2 |

(continued)

| Similar sequence group | Basic sequence NO. | Sequence No. | Sense strand | Basic sequence SEQ ID NO: | Antisense strand | Basic sequence SEQ ID NO: | Inhibition rate (%) |
|---|---|---|---|---|---|---|---|
| G | 73 | 73 | GCGGAGAUGCUUCUAAGGCAU | 11 | AUGCCUUAGAAGCAUCUCCGCCA | 23 | 52.4 |
| | 74 | 74 | CGGAGAUGCUUCUAAGGCAUG | 90 | CAUGCCUUAGAAGCAUCUCCGCC | 162 | 2.2 |

It can be seen from Table 11 that some siRNAs with similar sequences exhibited very different inhibitory effects on PCSK9 mRNA expression.

Basic sequence 5 differs from basic sequence 4 only in the terminal 1 base. For the sense strand, there is one more C at the 5'-end of basic sequence 4 and one more U at the 3'-end of basic sequence 5, and the remaining bases are identical. For the antisense strand, there is one more U at the 3'-end of basic sequence 4 and one more A at the 5'-end of the basic sequence 5, and the remaining bases are identical. However, the inhibition rate of basic sequence 5 is 51.2% higher than that of basic sequence 4, indicating a significant improvement.

Basic sequence 51 differs from basic sequence 50 only in the terminal 6 bases. For the sense strand, the 5'-end of the basic sequence 50 is GAUUAA, the 3'-end of the basic sequence 51 is CUGGAU, and the remaining bases are identical. For the antisense strand, the 3'-end of the basic sequence 50 is AAUCAG, the 5'-end of the basic sequence 51 is AUCCAG, and the remaining bases are identical. However, the inhibition rate of basic sequence 51 is 42.6% higher than that of basic sequence 50, indicating a significant improvement.

Basic sequence 3 differs from basic sequence 2 only in the terminal 6 bases. For the sense strand, the 5'-end of basic sequence 2 is AUACCU, the 3'-end of basic sequence 3 is UGUCUU, and the remaining bases are identical. For the antisense strand, the 3'-end of basic sequence 2 is GUAUCC, the 5'-end of basic sequence 3 is AAGACA, and the remaining bases are identical. However, the inhibition rate of basic sequence 3 is 42.7% higher than that of basic sequence 2, indicating a significant improvement.

Basic sequence 10 differs from basic sequence 9 only in the terminal 2 bases. For the sense strand, the 5'-end of basic sequence 9 is GG, the 3'-end of basic sequence 10 is AG, and the remaining bases are identical. For the antisense strand, the 3'-end of basic sequence 9 is UA, the 5'-end of basic sequence 10 is CU, and the remaining bases are identical. However, the inhibition rate of basic sequence 9 is 41.2% higher than that of basic sequence 10, indicating a significant improvement.

Basic sequence 22 differs from basic sequence 21 only in the terminal two bases. For the sense strand, the 5'-end of basic sequence 21 is CA, the 3'-end of basic sequence 22 is GA, and the remaining bases are identical. For the antisense strand, the 3'-end of basic sequence 21 is CA, the 5'-end of basic sequence 22 is UC, and the remaining bases are identical. However, the inhibition rate of basic sequence 21 is 53.2% higher than that of basic sequence 22, indicating a significant improvement.

Basic sequence 30 differs from basic sequence 31 only in the terminal 1 base. For the sense strand, the 5'-end of basic sequence 30 is A, the 3'-end of basic sequence 31 is G, and the remaining bases are identical. For the antisense strand, the 3'-end of basic sequence 30 is G, the 5'-end of basic sequence 31 is C, and the remaining bases are identical. However, the inhibition rate of basic sequence 31 is 32.5% higher than that of basic sequence 30, indicating a significant improvement.

Basic sequence 73 differs from basic sequence 74 only in the terminal 1 base. For the sense strand, the 5'-end of the basic sequence 73 is G, the 3'-end of the basic sequence 74 is G, and the remaining bases are identical. For the antisense strand, the 3'-end of the basic sequence 73 is A, the 5'-end of the basic sequence 74 is C, and the remaining bases are identical. However, the inhibition rate of basic sequence 73 is 50.2% higher than that of basic sequence 74, indicating a significant improvement.

Therefore, it is not easy to screen out sequences with significant inhibitory activity from a very large number of oligonucleotide sequences designed against the PCSK9 mRNA sequence, and a lot of inventive work is required.

**(4) Different sequences exhibited disparate effects on activity after alternate modifications. Some inhibition rates demonstrated a notable enhancement, exemplified by basic sequence 5, whose alternately modified sequence exhibited an elevated inhibition rate of 12.4% relative to its unmodified sequence. Conversely, some inhibition rates exhibited a less pronounced alteration, as observed in basic sequences 4, 22, and 52, where the inhibition rates of alternately modified and unmodified sequences exhibited minimal deviation.**

Table 12 Comparison of the inhibition rate on the PCSK9 gene between alternately modified sequences and unmodified sequences

| Basic sequence NO. | Sequence No. | Inhibition rate of the unmodified sequence (%) | Inhibition rate of the alternately modified sequence (%) | Increase in inhibition rate (%) |
|---|---|---|---|---|
| 5 | si5 | 60.3 | 72.7 | 12.4 |
| 51 | si51 | 53.4 | 61.4 | 8.0 |
| 81 | si81 | 62.0 | 71.8 | 9.8 |
| 82 | si82 | 63.1 | 75.3 | 12.2 |
| 84 | si84 | 55.7 | 66.2 | 10.5 |

(continued)

| Basic sequence NO. | Sequence No. | Inhibition rate of the unmodified sequence (%) | Inhibition rate of the alternately modified sequence (%) | Increase in inhibition rate (%) |
|---|---|---|---|---|
| 3 | si3 | 62.3 | 70.9 | 8.6 |
| 8 | si8 | 60.6 | 72.0 | 11.4 |
| 9 | si9 | 52.6 | 63.9 | 11.3 |
| 21 | si21 | 51.2 | 62.8 | 11.6 |
| 31 | si31 | 59.2 | 70.4 | 11.2 |
| 73 | si73 | 52.4 | 60.6 | 8.2 |
| 83 | si83 | 50.1 | 58.8 | 8.7 |
| 2 | si2 | 19.6 | 25.0 | 5.4 |
| 4 | si4 | 9.1 | 10.9 | 1.8 |
| 6 | si6 | 31.5 | 38.3 | 6.8 |
| 7 | si7 | 31.1 | 35.7 | 4.6 |
| 10 | si10 | 11.4 | 10.8 | -0.6 |
| 20 | si20 | -3.0 | 6.0 | 9.0 |
| 22 | si22 | -2.0 | -2.8 | -0.8 |
| 30 | si30 | 26.7 | 31.0 | 4.3 |
| 32 | si32 | 27.3 | 30.1 | 2.8 |
| 50 | si50 | 10.8 | 14.6 | 3.8 |
| 52 | si52 | 0.5 | -1.2 | -1.7 |
| 72 | si72 | 1.0 | -3.9 | -4.9 |
| 74 | si74 | 2.2 | -1.6 | -3.8 |
| 80 | si80 | 38.5 | 42.1 | 3.6 |

It can be seen from Table 12 that different alternately modified sequences exhibited inconsistent effects on the inhibition rate of PCSK9 mRNA. Some were significantly improved; for example, the inhibition rate of basic sequence 5 with alternate modifications was 12.4% higher than that of the unmodified sequence, and the inhibition rate of basic sequence 82 with alternate modifications was 12.2% higher than that of the unmodified sequence. Some were not obvious; for example, for basic sequences 4, 22 and 52, the inhibition rates of alternately modified sequences and unmodified sequences were basically unchanged.

Therefore, not all unmodified sequences can improve their activity through alternate modifications, and alternate modifications have inconsistent effects on the activity of different sequences.

**Summary:**

(1) 12 unmodified sequences exhibited a significant inhibitory effect on the PCSK9 gene, with inhibition rates exceeding 50%, including si5, si51, si81, si82, si84, si3, si8, si9, si21, si31, si73 and si83, among which the inhibition rates of si5, si81, si82, si3 and si8 exceeded 60%.

(2) The inhibition rates of other unmodified sequences on the PCSK9 gene were less than 40%. For example, the inhibition rates of si52 and si74 were only 0.5% and 2.2%, respectively.

(3) siRNAs with similar sequences had very different activities. For example, the inhibition rate of basic sequence 5 was 51.2% higher than that of basic sequence 4, indicating a significant improvement.

(4) Different sequences exhibited disparate effects on activity after alternate modifications. Some inhibition rates demonstrated a notable enhancement, exemplified by basic sequence 5, whose alternately modified sequence exhibited an elevated inhibition rate of 12.4% relative to its unmodified sequence. Conversely, some inhibition rates exhibited a less pronounced alteration, as observed in basic sequences 4, 22, and 52, where the inhibition rates of alternately modified and unmodified sequences exhibited minimal deviation.

**Example 4: Inhibitory effect of template-modified sequences on PCSK9 gene**

In this example, the sequences screened out in Example 3, that is, the unmodified sequences si5, si51, si81, si82, si84, si3, si8, si9, si21, si31, si73 and si83, a total of 12 sequences, were modified with templates. DV25, DV26, DV27, DV28, DV29, DV30 and DV310 are new modification templates designed in the present disclosure, and DV21 and DV22 are disclosed Advanced ESC modification templates.

**1. Experimental Materials**

Test samples:

Table 13 lists the sequences si5, si51, si81, si82, si84, si3, si8, si9, si21, si31, si73 and si83 in Example 3 modified with different templates (DV25, DV26, DV27, DV28, DV29, DV30, DV31, DV21 and DV22), and the corresponding alternately modified sequences P92-si5, P92-si51, P92-si81, P92-si82, P92-si84, P92-si3, P92-si8, P92-si9, P92-si21, P92-si31, P92-si73 and P92-si83 (synthesized in Example 1).

The modification principles of the modification templates of the present disclosure are as follows:

**DV25-29:**

The antisense strand was modified with modifications selected from the group consisting of A, B and C:

| No. | Antisense strand (AS) 5’-3’ | | | | | | | | | | | | | | | | | | | | | | |
|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 |
| A | 2’-OMe+PS | **2’-F+PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |
| B | 2’-OMe+PS | **2’-F+PS** | **2’-F** | **2’-F** | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |
| C | 2’-OMe+PS | **2’-F+PS** | 2’-OMe | **2’-F** | **2’-F** | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |

The sense strand was modified with modifications selected from the group consisting of a and b:

| No. | Sense strand (SS) 5’-3’ | | | | | | | | | | | | | | | | | | | | |
|---|---|---|---|---|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** | **17** | **18** | **19** | **20** | **21** |
| **a** | 2’-OMe+PS | 2’-OMe+PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | 2’-OMe | **2’-F** | 2’-OMe | 2’-OMe | 2’-OMe | 2’-OMe |
| **b** | 2’-OMe+PS | 2’-OMe+PS | 2’-OMe | 2’-OMe | 2’-OMe | 2’-OMe | **2’-F** | 2’-OMe | **2’-F** | 2’-OMe | **2’-F** | 2’-OMe | 2’-OMe | 2’-OMe | 2’-OMe | 2’-OMe | **2’-F** | 2’-OMe | 2’-OMe | 2’-OMe | 2’-OMe |

In the above table, PS represents a thiophosphate backbone.

The siRNA modification template with modification A for the antisense strand and modification a for the sense strand was named DV25;

The siRNA modification template with modification B for the antisense strand and modification a for the sense strand was named DV26;

The siRNA modification template with modification C for the antisense strand and modification a for the sense strand was named DV27;

The siRNA modification template with modification B for the antisense strand and modification b for the sense strand was named DV28;

The siRNA modification template with modification C for the antisense strand and modification b for the sense strand was named DV29.

**DV30:**

Antisense strand:

| Antisense strand (AS) 5’-3’ | | | | | | | | | | | | | | | | | | | | | | |
|---|---|---|---|---|---|---|---|---|---|---|---|---|---|---|---|---|---|---|---|---|---|---|
| **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** |
| 2’-OMe+PS | **2’-F+PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | 2’-OMe | 2’-OMe | 2’-OMe | 2’-OMe | 2’-OMe | 2’-OMe+PS | 2’-OMe+PS | 2’-OMe |

Sense strand:

| Sense strand (SS) 5’-3’ | | | | | | | | | | | | | | | | | | | | |
|---|---|---|---|---|---|---|---|---|---|---|---|---|---|---|---|---|---|---|---|---|
| **1** | **2** | **3** | **4** | **5** | **6** | **7** | **8** | **9** | **10** | **11** | **12** | **13** | **14** | **15** | **16** | **17** | **18** | **19** | **20** | **21** |
| 2’-OMe+PS | 2’-OMe+PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | 2’-OMe | 2’-OMe | 2’-OMe | 2’-OMe | 2’-OMe | 2’-OMe |

In the above table, PS represents a thiophosphate backbone.

**DV31:**

Antisense strand:

| Antisense strand (AS) 5’-3’ | | | | | | | | | | | | | | | | | | | | | | |
|---|---|---|---|---|---|---|---|---|---|---|---|---|---|---|---|---|---|---|---|---|---|---|
| **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** |
| 2’-OMe+PS | **2’-F+PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |

Sense strand:

| Sense strand (SS) 5’-3’ | | | | | | | | | | | | | | | | | | | | |
|---|---|---|---|---|---|---|---|---|---|---|---|---|---|---|---|---|---|---|---|---|
| **1** | **2** | **3** | **4** | **5** | **6** | **7** | **8** | **9** | **10** | **11** | **12** | **13** | **14** | **15** | **16** | **17** | **18** | **19** | **20** | **21** |
| 2’-OMe+PS | 2’-OMe+PS | 2’-OMe | 2’-OMe | 2’-OMe | 2’-OMe | **2’-F** | 2’-OMe | **2’-F** | **2’-F** | 2’-OMe | 2’-OMe | 2’-OMe | 2’-OMe | 2’-OMe | 2’-OMe | 2’-OMe | 2’-OMe | 2’-OMe | 2’-OMe | 2’-OMe |

In the above table, PS represents a thiophosphate backbone.

**DV21:**

Antisense strand:

| Antisense strand (AS) 5’-3’ | | | | | | | | | | | | | | | | | | | | | | |
|---|---|---|---|---|---|---|---|---|---|---|---|---|---|---|---|---|---|---|---|---|---|---|
| **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** |
| 2’-OMe+PS | **2’-F+PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |

Sense strand:

| Sense strand (SS) 5’-3’ | | | | | | | | | | | | | | | | | | | | |
|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 |
| 2’-OMe +PS | 2’-OMe +PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | **2’-F** | **2’-F** | 2’-OMe | 2’-OMe | 2’-OMe | 2’-OMe |

In the above table, PS represents a thiophosphate backbone.

**DV22:**

Antisense strand:

| Antisense strand (AS) 5’-3’ | | | | | | | | | | | | | | | | | | | | | | |
|---|---|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 |
| 2’-OMe +PS | **2’-F +PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe +PS | 2’-OMe +PS | 2’-OMe |

Sense strand:

| Sense strand (SS) 5’-3’ | | | | | | | | | | | | | | | | | | | | |
|---|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 | 21 |
| 2’-Ome +PS | 2’-Ome +PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | 2’-OMe | 2’-OMe | 2’-OMe | 2’-OMe | 2’-OMe | 2’-OMe |

In the above table, PS represents a thiophosphate backbone.

The sequences modified with each modification template are detailed in Table 13, and the synthesis methods of each sequence are the same as in Example 1.

Table 13 Sequences modified with different modification templates

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| 5 | P92-si5-DV25 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |
| | P92-si5-DV26 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gf-Gf-Am-Af-Gm-Am-Cm-Am-Um-Gm-Cm-Af-Gm-Gf-Am-Um-Cm-Um-Ums-Gms-Gm | 377/13 |
| | P92-si5-DV27 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gf-Af-Af-Gm-Am-Cm-Am-Um-Gm-Cm-Af-Gm-Gf-Am-Um-Cm-Um-Ums-Gms-Gm | 378/13 |
| | P92-si5-DV28 | Ams-Ams-Gm-Am-Um-Cm-Cf-Um-Gf-Cm-Af-Um-Gm-Um-Cm-Um-Uf-Cm-Cm-Am-Um | 338/1 | Ams-Ufs-Gf-Gf-Am-Af-Gm-Am-Cm-Am-Um-Gm-Cm-Af-Gm-Gf-Am-Um-Cm-Um-Ums-Gms-Gm | 377/13 |
| | P92-si5-DV29 | Ams-Ams-Gm-Am-Um-Cm-Cf-Um-Gf-Cm-Af-Um-Gm-Um-Cm-Um-Uf-Cm-Cm-Am-Um | 338/1 | Ams-Ufs-Gm-Gf-Af-Af-Gm-Am-Cm-Am-Um-Gm-Cm-Af-Gm-Gf-Am-Um-Cm-Um-Ums-Gms-Gm | 378/13 |
| | P92-si5-DV30 | Ams-Ams-Gm-Am-Um-Cm-Cf-Um-Gf-Cf-Af-Um-Gm-Um-Cm-Um-Um-Cm-Cm-Am-Um | 339/1 | Ams-Ufs-Gm-Gm-Am-Af-Gm-Am-Cm-Am-Um-Gm-Cm-Af-Gm-Gm-Am-Um-Cm-Um-Ums-Gms-Gm | 379/13 |
| | P92-si5-DV31 | Ams-Ams-Gm-Am-Um-Cm-Cf-Um-Gf-Cf-Am-Um-Gm-Um-Cm-Um-Um-Cm-Cm-Am-Um | 340/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si5-DV21 | Ams-Ams-Gm-Am-Um-Cm-Cf-Um-Gf-Cf-Af-Um-Gm-Um-Cm-Uf-Uf-Cm-Cm-Am-Um | 341/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |
| | P92-si5-DV22 | Ams-Ams-Gm-Am-Um-Cm-Cf-Um-Gf-Cf-Af-Um-Gm-Um-Cm-Um-Um-Cm-Cm-Am-Um | 339/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |
| 51 | P92-si51-DV25 | Ums-Gms-Gm-Am-Gm-Gm-Cf-Um-Uf-Af-Gf-Cm-Um-Um-Um-Cm-Uf-Gm-Gm-Am-Um | 342/2 | Ams-Ufs-Cm-Cm-Am-Gf-Am-Am-Am-Gm-Cm-Um-Am-Af-Gm-Cf-Cm-Um-Cm-Cm-Ams-Ums-Um | 380/14 |
| | P92-si51-DV26 | Ums-Gms-Gm-Am-Gm-Gm-Cf-Um-Uf-Af-Gf-Cm-Um-Um-Um-Cm-Uf-Gm-Gm-Am-Um | 342/2 | Ams-Ufs-Cf-Cf-Am-Gf-Am-Am-Am-Gm-Cm-Um-Am-Af-Gm-Cf-Cm-Um-Cm-Cm-Ams-Ums-Um | 381/14 |
| | P92-si51-DV27 | Ums-Gms-Gm-Am-Gm-Gm-Cf-Um-Uf-Af-Gf-Cm-Um-Um-Um-Cm-Uf-Gm-Gm-Am-Um | 342/2 | Ams-Ufs-Cm-Cf-Af-Gf-Am-Am-Am-Gm-Cm-Um-Am-Af-Gm-Cf-Cm-Um-Cm-Cm-Ams-Ums-Um | 382/14 |
| | P92-si51-DV28 | Ums-Gms-Gm-Am-Gm-Gm-Cf-Um-Uf-Am-Gf-Cm-Um-Um-Um-Cm-Uf-Gm-Gm-Am-Um | 343/2 | Ams-Ufs-Cf-Cf-Am-Gf-Am-Am-Am-Gm-Cm-Um-Am-Af-Gm-Cf-Cm-Um-Cm-Cm-Ams-Ums-Um | 381/14 |
| | P92-si51-DV29 | Ums-Gms-Gm-Am-Gm-Gm-Cf-Um-Uf-Am-Gf-Cm-Um-Um-Um-Cm-Uf-Gm-Gm-Am-Um | 343/2 | Ams-Ufs-Cm-Cf-Af-Gf-Am-Am-Am-Gm-Cm-Um-Am-Af-Gm-Cf-Cm-Um-Cm-Cm-Ams-Ums-Um | 382/14 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si51-DV30 | Ums-Gms-Gm-Am-Gm-Gm-Cf-Um-Uf-Af-Gf-Cm-Um-Um-Um-Cm-Um-Gm-Gm-Am-Um | 344/2 | Ams-Ufs-Cm-Cm-Am-Gf-Am-Am-Am-Gm-Cm-Um-Am-Af-Gm-Cm-Cm-Um-Cm-Cm-Ams-Ums-Um | 383/14 |
| | P92-si51-DV31 | Ums-Gms-Gm-Am-Gm-Gm-Cf-Um-Uf-Af-Gm-Cm-Um-Um-Um-Cm-Um-Gm-Gm-Am-Um | 345/2 | Ams-Ufs-Cm-Cm-Am-Gf-Am-Am-Am-Gm-Cm-Um-Am-Af-Gm-Cf-Cm-Um-Cm-Cm-Ams-Ums-Um | 380/14 |
| | P92-si51-DV21 | Ums-Gms-Gm-Am-Gm-Gm-Cf-Um-Uf-Af-Gf-Cm-Um-Um-Um-Cf-Uf-Gm-Gm-Am-Um | 346/2 | Ams-Ufs-Cm-Cm-Am-Gf-Am-Am-Am-Gm-Cm-Um-Am-Af-Gm-Cf-Cm-Um-Cm-Cm-Ams-Ums-Um | 380/14 |
| | P92-si51-DV22 | Ums-Gms-Gm-Am-Gm-Gm-Cf-Um-Uf-Af-Gf-Cm-Um-Um-Um-Cm-Um-Gm-Gm-Am-Um | 344/2 | Ams-Ufs-Cm-Cm-Am-Gf-Am-Am-Am-Gm-Cm-Um-Am-Af-Gm-Cf-Cm-Um-Cm-Cm-Ams-Ums-Um | 380/14 |
| 81 | P92-si81-DV25 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Am-Um-Cf-Um-Um-Cm-Am-Am-Gm-Um-Uf-Am-Cf-Am-Am-Am-Am-Gms-Cms-Am | 384/15 |
| | P92-si81-DV26 | Cms-Ums-Um-Um-Um-Gm-Uf-Am-Af-Cf-Uf-Um-Gm-Am-Am-Gm-Af-Um-Am-Um-Um | 347/3 | Ams-Afs-Uf-Af-Um-Cf-Um-Um-Cm-Am-Am-Gm-Um-Uf-Am-Cf-Am-Am-Am-Am-Gms-Cms-Am | 385/15 |
| | P92-si81-DV27 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Af-Uf-Cf-Um-Um-Cm-Am-Am-Gm-Um-Uf-Am-Cf-Am-Am-Am-Am-Gms-Cms-Am | 386/15 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si81-DV28 | Cms-Ums-Um-Um-Um-Gm-Uf-Am-Af-Cm-Uf-Um-Gm-Am-Am-Gm-Af-Um-Am-Um-Um | 348/3 | Ams-Afs-Uf-Af-Um-Cf-Um-Um-Cm-Am-Am-Gm-Um-Uf-Am-Cf-Am-Am-Am-Am-Gms-Cms-Am | 385/15 |
| | P92-si81-DV29 | Cms-Ums-Um-Um-Um-Gm-Uf-Am-Af-Cm-Uf-Um-Gm-Am-Am-Gm-Af-Um-Am-Um-Um | 348/3 | Ams-Afs-Um-Af-Uf-Cf-Um-Um-Cm-Am-Am-Gm-Um-Uf-Am-Cf-Am-Am-Am-Am-Gms-Cms-Am | 386/15 |
| | P92-si81-DV30 | Cms-Ums-Um-Um-Um-Gm-Uf-Am-Af-Cf-Uf-Um-Gm-Am-Am-Gm-Am-Um-Am-Um-Um | 349/3 | Ams-Afs-Um-Am-Um-Cf-Um-Um-Cm-Am-Am-Gm-Um-Uf-Am-Cm-Am-Am-Am-Am-Gms-Cms-Am | 387/15 |
| | P92-si81-DV31 | Cms-Ums-Um-Um-Um-Gm-Uf-Am-Af-Cf-Um-Um-Gm-Am-Am-Gm-Am-Um-Am-Um-Um | 350/3 | Ams-Afs-Um-Am-Um-Cf-Um-Um-Cm-Am-Am-Gm-Um-Uf-Am-Cf-Am-Am-Am-Am-Gms-Cms-Am | 384/15 |
| | P92-si81-DV21 | Cms-Ums-Um-Um-Um-Gm-Uf-Am-Af-Cf-Uf-Um-Gm-Am-Am-Gf-Af-Um-Am-Um-Um | 351/3 | Ams-Afs-Um-Am-Um-Cf-Um-Um-Cm-Am-Am-Gm-Um-Uf-Am-Cf-Am-Am-Am-Am-Gms-Cms-Am | 384/15 |
| | P92-si81-DV22 | Cms-Ums-Um-Um-Um-Gm-Uf-Am-Af-Cf-Uf-Um-Gm-Am-Am-Gm-Am-Um-Am-Um-Um | 349/3 | Ams-Afs-Um-Am-Um-Cf-Um-Um-Cm-Am-Am-Gm-Um-Uf-Am-Cf-Am-Am-Am-Am-Gms-Cms-Am | 384/15 |
| | P92-si82-DV25 | Gms-Ams-Am-Gm-Am-Um-Af-Um-Uf-Uf-Af-Um-Um-Cm-Um-Gm-Gf-Gm-Um-Um-Um | 352/4 | Ams-Afs-Am-Cm-Cm-Cf-Am-Gm-Am-Am-Um-Am-Am-Af-Um-Af-Um-Cm-Um-Um-Cms-Ams-Am | 388/16 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| 82 | P92-si82-DV26 | Gms-Ams-Am-Gm-Am-Um-Af-Um-Uf-Uf-Af-Um-Um-Cm-Um-Gm-Gf-Gm-Um-Um-Um | 352/4 | Ams-Afs-Af-Cf-Cm-Cf-Am-Gm-Am-Am-Um-Am-Am-Af-Um-Af-Um-Cm-Um-Um-Cms-Ams-Am | 389/16 |
| | P92-si82-DV27 | Gms-Ams-Am-Gm-Am-Um-Af-Um-Uf-Uf-Af-Um-Um-Cm-Um-Gm-Gf-Gm-Um-Um-Um | 352/4 | Ams-Afs-Am-Cf-Cf-Cf-Am-Gm-Am-Am-Um-Am-Am-Af-Um-Af-Um-Cm-Um-Um-Cms-Ams-Am | 390/16 |
| | P92-si82-DV28 | Gms-Ams-Am-Gm-Am-Um-Af-Um-Uf-Um-Af-Um-Um-Cm-Um-Gm-Gf-Gm-Um-Um-Um | 353/4 | Ams-Afs-Af-Cf-Cm-Cf-Am-Gm-Am-Am-Um-Am-Am-Af-Um-Af-Um-Cm-Um-Um-Cms-Ams-Am | 389/16 |
| | P92-si82-DV29 | Gms-Ams-Am-Gm-Am-Um-Af-Um-Uf-Um-Af-Um-Um-Cm-Um-Gm-Gf-Gm-Um-Um-Um | 353/4 | Ams-Afs-Am-Cf-Cf-Cf-Am-Gm-Am-Am-Um-Am-Am-Af-Um-Af-Um-Cm-Um-Um-Cms-Ams-Am | 390/16 |
| | P92-si82-DV30 | Gms-Ams-Am-Gm-Am-Um-Af-Um-Uf-Uf-Af-Um-Um-Cm-Um-Gm-Gm-Gm-Um-Um-Um | 354/4 | Ams-Afs-Am-Cm-Cm-Cf-Am-Gm-Am-Am-Um-Am-Am-Af-Um-Am-Um-Cm-Um-Um-Cms-Ams-Am | 391/16 |
| | P92-si82-DV31 | Gms-Ams-Am-Gm-Am-Um-Af-Um-Uf-Uf-Am-Um-Um-Cm-Um-Gm-Gm-Gm-Um-Um-Um | 355/4 | Ams-Afs-Am-Cm-Cm-Cf-Am-Gm-Am-Am-Um-Am-Am-Af-Um-Af-Um-Cm-Um-Um-Cms-Ams-Am | 388/16 |
| | P92-si82-DV21 | Gms-Ams-Am-Gm-Am-Um-Af-Um-Uf-Uf-Af-Um-Um-Cm-Um-Gf-Gf-Gm-Um-Um-Um | 356/4 | Ams-Afs-Am-Cm-Cm-Cf-Am-Gm-Am-Am-Um-Am-Am-Af-Um-Af-Um-Cm-Um-Um-Cms-Ams-Am | 388/16 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si82-DV22 | Gms-Ams-Am-Gm-Am-Um-Af-Um-Uf-Uf-Af-Um-Um-Cm-Um-Gm-Gm-Gm-Um-Um-Um | 354/4 | Ams-Afs-Am-Cm-Cm-Cf-Am-Gm-Am-Am-Um-Am-Am-Af-Um-Af-Um-Cm-Um-Um-Cms-Ams-Am | 388/16 |
| 84 | P92-si84-DV25 | Gms-Ums-Um-Um-Um-Gm-Uf-Am-Gf-Cf-Af-Um-Um-Um-Um-Um-Af-Um-Um-Am-Am | 357/5 | Ums-Ufs-Am-Am-Um-Af-Am-Am-Am-Am-Um-Gm-Cm-Uf-Am-Cf-Am-Am-Am-Am-Cms-Cms-Cm | 392/17 |
| | P92-si84-DV26 | Gms-Ums-Um-Um-Um-Gm-Uf-Am-Gf-Cf-Af-Um-Um-Um-Um-Um-Af-Um-Um-Am-Am | 357/5 | Ums-Ufs-Af-Af-Um-Af-Am-Am-Am-Am-Um-Gm-Cm-Uf-Am-Cf-Am-Am-Am-Am-Cms-Cms-Cm | 393/17 |
| | P92-si84-DV27 | Gms-Ums-Um-Um-Um-Gm-Uf-Am-Gf-Cf-Af-Um-Um-Um-Um-Um-Af-Um-Um-Am-Am | 357/5 | Ums-Ufs-Am-Af-Uf-Af-Am-Am-Am-Am-Um-Gm-Cm-Uf-Am-Cf-Am-Am-Am-Am-Cms-Cms-Cm | 394/17 |
| | P92-si84-DV28 | Gms-Ums-Um-Um-Um-Gm-Uf-Am-Gf-Cm-Af-Um-Um-Um-Um-Um-Af-Um-Um-Am-Am | 358/5 | Ums-Ufs-Af-Af-Um-Af-Am-Am-Am-Am-Um-Gm-Cm-Uf-Am-Cf-Am-Am-Am-Am-Cms-Cms-Cm | 393/17 |
| | P92-si84-DV29 | Gms-Ums-Um-Um-Um-Gm-Uf-Am-Gf-Cm-Af-Um-Um-Um-Um-Um-Af-Um-Um-Am-Am | 358/5 | Ums-Ufs-Am-Af-Uf-Af-Am-Am-Am-Am-Um-Gm-Cm-Uf-Am-Cf-Am-Am-Am-Am-Cms-Cms-Cm | 394/17 |
| | P92-si84-DV30 | Gms-Ums-Um-Um-Um-Gm-Uf-Am-Gf-Cf-Af-Um-Um-Um-Um-Um-Am-Um-Um-Am-Am | 359/5 | Ums-Ufs-Am-Am-Um-Af-Am-Am-Am-Am-Um-Gm-Cm-Uf-Am-Cm-Am-Am-Am-Am-Cms-Cms-Cm | 395/17 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si84-DV31 | Gms-Ums-Um-Um-Um-Gm-Uf-Am-Gf-Cf-Am-Um-Um-Um-Um-Um-Am-Um-Um-Am-Am | 360/5 | Ums-Ufs-Am-Am-Um-Af-Am-Am-Am-Am-Um-Gm-Cm-Uf-Am-Cf-Am-Am-Am-Am-Cms-Cms-Cm | 392/17 |
| | P92-si84-DV21 | Gms-Ums-Um-Um-Um-Gm-Uf-Am-Gf-Cf-Af-Um-Um-Um-Um-Uf-Af-Um-Um-Am-Am | 361/5 | Ums-Ufs-Am-Am-Um-Af-Am-Am-Am-Am-Um-Gm-Cm-Uf-Am-Cf-Am-Am-Am-Am-Cms-Cms-Cm | 392/17 |
| | P92-si84-DV22 | Gms-Ums-Um-Um-Um-Gm-Uf-Am-Gf-Cf-Af-Um-Um-Um-Um-Um-Am-Um-Um-Am-Am | 359/5 | Ums-Ufs-Am-Am-Um-Af-Am-Am-Am-Am-Um-Gm-Cm-Uf-Am-Cf-Am-Am-Am-Am-Cms-Cms-Cm | 392/17 |
| 3 | P92-si3-DV25 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gm-Am-Cm-Af-Um-Gm-Cm-Am-Gm-Gm-Am-Uf-Cm-Uf-Um-Gm-Gm-Um-Gms-Ams-Gm | 396/18 |
| | P92-si3-DV26 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gf-Af-Cm-Af-Um-Gm-Cm-Am-Gm-Gm-Am-Uf-Cm-Uf-Um-Gm-Gm-Um-Gms-Ams-Gm | 397/18 |
| | P92-si3-DV27 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gm-Af-Cf-Af-Um-Gm-Cm-Am-Gm-Gm-Am-Uf-Cm-Uf-Um-Gm-Gm-Um-Gms-Ams-Gm | 398/18 |
| | P92-si3-DV28 | Cms-Ams-Cm-Cm-Am-Am-Gf-Am-Uf-Cm-Cf-Um-Gm-Cm-Am-Um-Gf-Um-Cm-Um-Um | 363/6 | Ams-Afs-Gf-Af-Cm-Af-Um-Gm-Cm-Am-Gm-Gm-Am-Uf-Cm-Uf-Um-Gm-Gm-Um-Gms-Ams-Gm | 397/18 |

(continued)

| Basic sequence N O. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si3-DV29 | Cms-Ams-Cm-Cm-Am-Am-Gf-Am-Uf-Cm-Cf-Um-Gm-Cm-Am-Um-Gf-Um-Cm-Um-Um | 363/6 | Ams-Afs-Gm-Af-Cf-Af-Um-Gm-Cm-Am-Gm-Gm-Am-Uf-Cm-Uf-Um-Gm-Gm-Um-Gms-Ams-Gm | 398/18 |
| 8 | P92-si8-DV25 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Um-Am-Am-Af-Cm-Um-Cm-Cm-Am-Gm-Gm-Cf-Cm-Uf-Am-Um-Gm-Am-Gms-Gms-Gm | 399/19 |
| | P92-si8-DV26 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Uf-Af-Am-Af-Cm-Um-Cm-Cm-Am-Gm-Gm-Cf-Cm-Uf-Am-Um-Gm-Am-Gms-Gms-Gm | 400/19 |
| | P92-si8-DV27 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Um-Af-Af-Af-Cm-Um-Cm-Cm-Am-Gm-Gm-Cf-Cm-Uf-Am-Um-Gm-Am-Gms-Gms-Gm | 401/19 |
| | P92-si8-DV28 | Cms-Ums-Cm-Am-Um-Am-Gf-Gm-Cf-Cm-Uf-Gm-Gm-Am-Gm-Um-Uf-Um-Am-Um-Um | 365/7 | Ams-Afs-Uf-Af-Am-Af-Cm-Um-Cm-Cm-Am-Gm-Gm-Cf-Cm-Uf-Am-Um-Gm-Am-Gms-Gms-Gm | 400/19 |
| | P92-si8-DV29 | Cms-Ums-Cm-Am-Um-Am-Gf-Gm-Cf-Cm-Uf-Gm-Gm-Am-Gm-Um-Uf-Um-Am-Um-Um | 365/7 | Ams-Afs-Um-Af-Af-Af-Cm-Um-Cm-Cm-Am-Gm-Gm-Cf-Cm-Uf-Am-Um-Gm-Am-Gms-Gms-Gm | 401/19 |
| 9 | P92-si9-DV25 | Gms-Gms-Cm-Cm-Um-Gm-Gf-Am-Gf-Uf-Uf-Um-Am-Um-Um-Cm-Gf-Gm-Am-Am-Am | 366/8 | Ums-Ufs-Um-Cm-Cm-Gf-Am-Am-Um-Am-Am-Am-Cm-Uf-Cm-Cf-Am-Gm-Gm-Cm-Cms-Ums-Am | 402/20 |
| | P92-si9-DV26 | Gms-Gms-Cm-Cm-Um-Gm-Gf-Am-Gf-Uf-Uf-Um-Am-Um- | 366/8 | Ums-Ufs-Uf-Cf-Cm-Gf-Am-Am-Um-Am-Am-Am-Cm-Uf-Cm-Cf- | 403/20 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | | Um-Cm-Gf-Gm-Am-Am-Am | | Am-Gm-Gm-Cm-Cms-Ums-Am | |
| | P92-si9-DV27 | Gms-Gms-Cm-Cm-Um-Gm-Gf-Am-Gf-Uf-Uf-Um-Am-Um-Um-Cm-Gf-Gm-Am-Am-Am | 366/8 | Ums-Ufs-Um-Cf-Cf-Gf-Am-Am-Um-Am-Am-Am-Cm-Uf-Cm-Cf-Am-Gm-Gm-Cm-Cms-Ums-Am | 404/20 |
| | P92-si9-DV28 | Gms-Gms-Cm-Cm-Um-Gm-Gf-Am-Gf-Um-Uf-Um-Am-Um-Um-Cm-Gf-Gm-Am-Am-Am | 367/8 | Ums-Ufs-Uf-Cf-Cm-Gf-Am-Am-Um-Am-Am-Am-Cm-Uf-Cm-Cf-Am-Gm-Gm-Cm-Cms-Ums-Am | 403/20 |
| | P92-si9-DV29 | Gms-Gms-Cm-Cm-Um-Gm-Gf-Am-Gf-Um-Uf-Um-Am-Um-Um-Cm-Gf-Gm-Am-Am-Am | 367/8 | Ums-Ufs-Um-Cf-Cf-Gf-Am-Am-Um-Am-Am-Am-Cm-Uf-Cm-Cf-Am-Gm-Gm-Cm-Cms-Ums-Am | 404/20 |
| 21 | P92-si21-DV25 | Cms-Ams-Gm-Cm-Am-Cm-Cf-Um-Gf-Cf-Uf-Um-Um-Gm-Um-Gm-Uf-Cm-Am-Cm-Am | 368/9 | Ums-Gfs-Um-Gm-Am-Cf-Am-Cm-Am-Am-Am-Gm-Cm-Af-Gm-Gf-Um-Gm-Cm-Um-Gms-Cms-Am | 405/21 |
| | P92-si21-DV26 | Cms-Ams-Gm-Cm-Am-Cm-Cf-Um-Gf-Cf-Uf-Um-Um-Gm-Um-Gm-Uf-Cm-Am-Cm-Am | 368/9 | Ums-Gfs-Uf-Gf-Am-Cf-Am-Cm-Am-Am-Am-Gm-Cm-Af-Gm-Gf-Um-Gm-Cm-Um-Gms-Cms-Am | 406/21 |
| | P92-si21-DV27 | Cms-Ams-Gm-Cm-Am-Cm-Cf-Um-Gf-Cf-Uf-Um-Um-Gm-Um-Gm-Uf-Cm-Am-Cm-Am | 368/9 | Ums-Gfs-Um-Gf-Af-Cf-Am-Cm-Am-Am-Am-Gm-Cm-Af-Gm-Gf-Um-Gm-Cm-Um-Gms-Cms-Am | 407/21 |
| | P92-si21-DV28 | Cms-Ams-Gm-Cm-Am-Cm-Cf-Um-Gf-Cm-Uf-Um-Um-Gm-Um-Gm-Uf-Cm-Am-Cm-Am | 369/9 | Ums-Gfs-Uf-Gf-Am-Cf-Am-Cm-Am-Am-Am-Gm-Cm-Af-Gm-Gf-Um-Gm-Cm-Um-Gms-Cms-Am | 406/21 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si21-DV29 | Cms-Ams-Gm-Cm-Am-Cm-Cf-Um-Gf-Cm-Uf-Um-Um-Gm-Um-Gm-Uf-Cm-Am-Cm-Am | 369/9 | Ums-Gfs-Um-Gf-Af-Cf-Am-Cm-Am-Am-Am-Gm-Cm-Af-Gm-Gf-Um-Gm-Cm-Um-Gms-Cms-Am | 407/21 |
| 31 | P92-si31-DV25 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Um-Gm-Am-Cf-Um-Um-Um-Gm-Cm-Am-Um-Uf-Cm-Cf-Am-Gm-Am-Cm-Cms-Ums-Gm | 408/22 |
| | P92-si31-DV26 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Uf-Gf-Am-Cf-Um-Um-Um-Gm-Cm-Am-Um-Uf-Cm-Cf-Am-Gm-Am-Cm-Cms-Ums-Gm | 409/22 |
| | P92-si31-DV27 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Um-Gf-Af-Cf-Um-Um-Um-Gm-Cm-Am-Um-Uf-Cm-Cf-Am-Gm-Am-Cm-Cms-Ums-Gm | 410/22 |
| | P92-si31-DV28 | Gms-Gms-Um-Cm-Um-Gm-Gf-Am-Af-Um-Gf-Cm-Am-Am-Am-Gm-Uf-Cm-Am-Am-Gm | 371/10 | Cms-Ufs-Uf-Gf-Am-Cf-Um-Um-Um-Gm-Cm-Am-Um-Uf-Cm-Cf-Am-Gm-Am-Cm-Cms-Ums-Gm | 409/22 |
| | P92-si31-DV29 | Gms-Gms-Um-Cm-Um-Gm-Gf-Am-Af-Um-Gf-Cm-Am-Am-Am-Gm-Uf-Cm-Am-Am-Gm | 371/10 | Cms-Ufs-Um-Gf-Af-Cf-Um-Um-Um-Gm-Cm-Am-Um-Uf-Cm-Cf-Am-Gm-Am-Cm-Cms-Ums-Gm | 410/22 |
| 73 | P92-si73-DV25 | Gms-Cms-Gm-Gm-Am-Gm-Af-Um-Gf-Cf-Uf-Um-Cm-Um-Am-Am-Gf-Gm-Cm-Am-Um | 372/11 | Ams-Ufs-Gm-Cm-Cm-Uf-Um-Am-Gm-Am-Am-Gm-Cm-Af-Um-Cf-Um-Cm-Cm-Gm-Cms-Cms-Am | 411/23 |
| | P92-si73-DV26 | Gms-Cms-Gm-Gm-Am-Gm-Af-Um-Gf-Cf-Uf-Um-Cm-Um-Am-Am-Gf-Gm-Cm-Am-Um | 372/11 | Ams-Ufs-Gf-Cf-Cm-Uf-Um-Am-Gm-Am-Am-Gm-Cm-Af-Um-Cf-Um-Cm-Cm-Gm-Cms-Cms-Am | 412/23 |

(continued)

| Basic sequence NO. | Template-modified Sequence No. | Sense strand 5'-3' | Modified sequence SEQ ID NO:/ Corresponding basic sequence SEQ ID NO: | Antisense strand 5'-3' | Modified sequence SEQ ID NO:/ Corresponding basic sequence SEQ ID NO: |
|---|---|---|---|---|---|
| | P92-si73-DV27 | Gms-Cms-Gm-Gm-Am-Gm-Af-Um-Gf-Cf-Uf-Um-Cm-Um-Am-Am-Gf-Gm-Cm-Am-Um | 372/11 | Ams-Ufs-Gm-Cf-Cf-Uf-Um-Am-Gm-Am-Am-Gm-Cm-Af-Um-Cf-Um-Cm-Cm-Gm-Cms-Cms-Am | 413/23 |
| | P92-si73-DV28 | Gms-Cms-Gm-Gm-Am-Gm-Af-Um-Gf-Cm-Uf-Um-Cm-Um-Am-Am-Gf-Gm-Cm-Am-Um | 373/11 | Ams-Ufs-Gf-Cf-Cm-Uf-Um-Am-Gm-Am-Am-Gm-Cm-Af-Um-Cf-Um-Cm-Cm-Gm-Cms-Cms-Am | 412/23 |
| | P92-si73-DV29 | Gms-Cms-Gm-Gm-Am-Gm-Af-Um-Gf-Cm-Uf-Um-Cm-Um-Am-Am-Gf-Gm-Cm-Am-Um | 373/11 | Ams-Ufs-Gm-Cf-Cf-Uf-Um-Am-Gm-Am-Am-Gm-Cm-Af-Um-Cf-Um-Cm-Cm-Gm-Cms-Cms-Am | 413/23 |
| 83 | P92-si83-DV25 | Gms-Gms-Gm-Um-Um-Um-Uf-Gm-Uf-Af-Gf-Cm-Am-Um-Um-Um-Uf-Um-Am-Um-Um | 374/12 | Ams-Afs-Um-Am-Am-Af-Am-Am-Um-Gm-Cm-Um-Am-Cf-Am-Af-Am-Am-Cm-Cm-Cms-Ams-Gm | 414/24 |
| | P92-si83-DV26 | Gms-Gms-Gm-Um-Um-Um-Uf-Gm-Uf-Af-Gf-Cm-Am-Um-Um-Um-Uf-Um-Am-Um-Um | 374/12 | Ams-Afs-Uf-Af-Am-Af-Am-Am-Um-Gm-Cm-Um-Am-Cf-Am-Af-Am-Am-Cm-Cm-Cms-Ams-Gm | 415/24 |
| | P92-si83-DV27 | Gms-Gms-Gm-Um-Um-Um-Uf-Gm-Uf-Af-Gf-Cm-Am-Um-Um-Um-Uf-Um-Am-Um-Um | 374/12 | Ams-Afs-Um-Af-Af-Af-Am-Am-Um-Gm-Cm-Um-Am-Cf-Am-Af-Am-Am-Cm-Cm-Cms-Ams-Gm | 416/24 |
| | P92-si83-DV28 | Gms-Gms-Gm-Um-Um-Um-Uf-Gm-Uf-Am-Gf-Cm-Am-Um-Um-Um-Uf-Um-Am-Um-Um | 375/12 | Ams-Afs-Uf-Af-Am-Af-Am-Am-Um-Gm-Cm-Um-Am-Cf-Am-Af-Am-Am-Cm-Cm-Cms-Ams-Gm | 415/24 |
| | P92-si83-DV29 | Gms-Gms-Gm-Um-Um-Um-Uf-Gm-Uf-Am-Gf-Cm-Am-Um-Um-Um-Uf-Um-Am-Um-Um | 375/12 | Ams-Afs-Um-Af-Af-Af-Am-Am-Um-Gm-Cm-Um-Am-Cf-Am-Af-Am-Am-Cm-Cm-Cms-Ams-Gm | 416/24 |

Cell type: HEP3B cell line cells

Drug solvent: DNase/RNase-Free Water, Gibco DMEM (purchased from Thermo Fisher; catalog No.: 10569010)

**2. Experimental Methods**

The inhibition of the mRNA expression of the PCSK9 gene by the test samples was detected in the HEP3B cell line using qRT-PCR. Hep3B cells were provided by Shanghai WuXi AppTec New Drug Development Co., Ltd. (ATCC-tings-1618164).

**2.1 Cell culture**

The subcultured HEP3B cell line cells were used. The logarithmically growing cells were cultured with 10% fetal bovine serum RPMI 1640 medium (supplemented with 100 μL/mL of penicillin and streptomycin) in a cell culture incubator at 37°C containing 5% $CO_2$, and the medium was changed once a day. The cells were digested with 0.25% trypsin and centrifuged at 1000 r/min for 5 min. The supernatant was discarded, and fresh culture medium was added for subculture.

**2.2 Cell transfection**

Preparation of a transfection mixture: Lipofectamine RNAiMAX and Opti-MEM were mixed in a ratio of 1.5:98.5 and then mixed well by vortex.
Preparation of transfection reagent: 60 μL of siRNA solution diluted in Opti-MEM was added to 60 μL of the transfection mixture at a ratio of 1:1 (v/v), mixed well by vortex and left to stand at room temperature for 15 min to obtain lipid nanoparticles (LNPs), 12.5 μL of which was used for detection of encapsulation efficiency.
Transfection reagent for blank control group: 60 μL of the prepared transfection mixture was added to 60 μL of Opti-MEM, mixed well by vortex and left to stand at room temperature for 15 min.
The prepared transfection reagent was added to a 24-well cell culture plate (100 μL per well) to a final siRNA concentration of 0.05 nM per well. 500 μL of cell suspension ($6\times10^4$ cells per ml) was added and mixed well using the cross method. The plate was placed in a 37°C, 5% $CO_2$ cell culture incubator for 40 h.

**2.3 PCSK9 mRNA detection**

The steps were the same as those in "2.3 PCSK9 mRNA detection" in Example 2.

**2.4 Data Processing**

The PCSK9 mRNA expression rate (%) was calculated as follows:

Expression rate = (PCSK9 mRNA expression level/PCSK9 mRNA expression level in blank control group) × 100%;

$$\text{PCSK9 gene expression inhibition rate} = 1\text{-expression rate (\%)}.$$

**2.5 EC50 experiment**

In this experiment, the siRNA concentration for each sequence in the EC50 experiment started from 0.2 nM and subjected to 4-fold dilution to obatin a total of 8 concentration points (0.2 nM, 0.05 nM, 0.0125 nM, 3.13 pM, 0.78 pM, 0.2 pM, 0.05 pM and 0.01 pM). The inhibition rate of each sequence at each concentration was measured and plotted to calculate the EC50 concentration of each sequence.

**3. Experimental results**

After three repeated experiments, the inhibition rates against the PCSK9 gene by each modified sequence in Hep3B cells are shown in Tables 14-25.
The activity detection experiment results show that after modified with the modification template DV25-29 designed in the present disclosure, 12 candidate sequences (unmodified sequences si5, si51, si81, si82, si84, si3, si8, si9,

si21, si31, si73 and si83) exhibited a significant inhibitory effect on the PCSK9 gene, with inhibition rates above 70%, among which the inhibition rates of P92-si81-DV26, P92-si82-DV27 and P92-si82-DV29 reached 89.3%, 89.3% and 89.1%, respectively.

After the modification with the modification templates DV25-29 of the present disclosure, the inhibition rate against the PCSK9 gene expression was significantly improved compared with that of the alternately modified sequences. For example, sequence P92-si51-DV27 obtained by modifying basic sequence 51 with the template DV27 increased the inhibition rate by 29.1% compared to the alternately modified sequence, and sequence P92-si81-DV26 obtained by modifying basic sequence 81 with the template DV26 increased the inhibition rate by 26.9% compared to the alternately modified sequence.

Moreover, the activity varied greatly after the same siRNA sequence was modified with different modification templates. For example, the inhibition rate of the sequence of basic sequence 51 modified with modification template DV27 of the present disclosure was increased by 22.8% compared with that of the sequence modified with modification template DV31 of the present disclosure, and increased by 21.3% compared with that of the sequence modified with the disclosed Advanced ESC template DV21.

EC50 experiments show that the EC50 values of sequences modified with the modification templates designed in the present disclosure were between 0.0001 and 0.005 nM. For example, the EC50 values of P92-si81-DV27, P92-si84-DV26, and P92-si82-DV27 were 0.0003 nM, 0.0004 nM, and 0.0006 nM, respectively. These sequences can effectively inhibit the expression of the PCSK9 gene at low concentrations.

**(i) Inhibitory effect of template-modified basic sequences on the PCSK9 gene**

**(1) Basic sequence 5**

Table 14 Inhibition rate against PCSK9 gene by template-modified basic sequence 5

| Basic sequenc e NO. | Template-modified sequence No. | Inhibition rate (%) of template-modifie d sequence | Inhibition rate (%) of corresponding alternately-modified sequence | Increase (%) |
|---|---|---|---|---|
| 5 | P92-si5-DV25 | 81.8 | 65.4 | 16.4 |
| | P92-si5-DV26 | 84.9 | 65.4 | 19.5 |
| | P92-si5-DV27 | 82.0 | 65.4 | 16.6 |
| | P92-si5-DV28 | 80.1 | 65.4 | 14.7 |
| | P92-si5-DV29 | 80.7 | 65.4 | 15.3 |
| | P92-si5-DV30 | 65.3 | 65.4 | -0.1 |
| | P92-si5-DV31 | 62.4 | 65.4 | -3.0 |
| | P92-si5-DV21 | 68.2 | 65.4 | 2.8 |
| | P92-si5-DV22 | 69.1 | 65.4 | 3.7 |

**I. DV25-29 templates of the present disclosure**

Basic sequence 5 modified with the modification templates DV25-29 designed by the present disclosure exhibited an inhibition rate against the PCSK9 gene exceeding 80%. Compared with the alternate modification with 2'-position methoxy and 2'-position fluorine, the inhibition rate against the PCSK9 gene increased significantly. For example, after modification with templates DV26 and DV27, the inhibition rates of P92-si5-DV26 and P92-si5-DV27 increased by 19.5% and 16.6% respectively (FIG. 7).

**II. Other modification templates of the present disclosure**

P92-si5-DV30 and P92-si5-DV31 obtained by modifying basic sequence 5 with templates DV30 and DV31 had inhibition rates against the PCSK9 gene of 65.3% and 62.4% respectively, which were 0.1% and 3.0% lower than the corresponding alternately modified sequences, respectively.

P92-si5-DV26 and P92-si5-DV27 modified with templates DV26 and DV27 of the present disclosure increased the inhibition rate by 19.6% and 16.7% compared with P92-si5-DV30, and increased by 22.5% and 19.6% compared with P92-si5-DV31, indicating a significant improvement.

**III. Advanced ESC templates DV21 (fluorinated sites: positions 2, 6, 14 and 16 on the antisense strand and positions 7, 9, 10, 11, 16 and 17 on the sense strand) and DV22 (fluorinated sites: positions 2, 6, 14 and 16 on the antisense strand and positions 7, 9, 10 and 11 on the sense strand) disclosed in the prior art**

P92-si5-DV21 and P92-si5-DV22 obtained by modifying basic sequence 5 with templates DV21 and DV22 had inhibition rates against the PCSK9 gene of 68.2% and 69.1% respectively, which were 2.8% and 3.7% higher than the corresponding alternately modified sequences, respectively.

P92-si5-DV26 and P92-si5-DV27 modified with templates DV26 and DV27 of the present disclosure increased the inhibition rate by 16.7% and 13.8% compared with P92-si5-DV21, and increased by 15.8% and 12.9% compared with P92-si5-DV22, indicating a significant improvement.

It can be concluded that:

1) After modification on basic sequence 5 with the modification templates DV25-29 of the present disclosure, the inhibition effect on the PCSK9 gene was significantly improved compared with the alternately modified sequences. For example, the inhibition rate of the basic sequence 5 modified with DV26 was 19.5% higher than that of the alternately modified sequence.

2) The activity varied greatly after the same siRNA sequence was modified with different modification templates. For example, the inhibition rate of the sequences obtained by modifying basic sequence 5 with modification templates DV26 and DV27 of the present disclosure can be increased by up to 22.5% compared with the sequences modified with modification templates DV30 and DV31 of the present disclosure, indicating a significant improvement; and can be increased by up to 16.7% compared with the sequences modified with the disclosed Advanced ESC templates DV21 and DV22, indicating a significant improvement.

3) The activity of siRNA sequences modified with different templates varied greatly, up to 20%. It can be seen that it is uncertain which template modification can be used in siRNA sequences to achieve high activity.

**(2) Basic sequence 51**

Table 15 Inhibition rate against PCSK9 gene by template-modified basic sequence 51

| Basic sequence NO. | Template-modified sequence No. | Inhibition rate (%) of template-mod ified sequence | Inhibition rate (%) of corresponding alternately-modified sequence | Increase (%) |
|---|---|---|---|---|
| 51 | P92-si51-DV25 | 83.1 | 56.5 | 26.6 |
| | P92-si51-DV26 | 84.2 | 56.5 | 27.7 |
| | P92-si51-DV27 | 85.6 | 56.5 | 29.1 |
| | P92-si51-DV28 | 76.8 | 56.5 | 20.3 |
| | P92-si51-DV29 | 78.9 | 56.5 | 22.4 |
| | P92-si51-DV30 | 63.5 | 56.5 | 7.0 |
| | P92-si51-DV31 | 62.8 | 56.5 | 6.3 |
| | P92-si51-DV21 | 64.3 | 56.5 | 7.8 |
| | P92-si51-DV22 | 65.1 | 56.5 | 8.6 |

**I. DV25-29 templates of the present disclosure**

Basic sequence 51 modified with the modification templates DV25-29 designed by the present disclosure

exhibited an inhibition rate against the PCSK9 gene exceeding 75%. Compared with the alternate modification with 2'-position methoxy and 2'-position fluorine, the inhibition rate against the PCSK9 gene increased significantly. For example, after modification with templates DV26 and DV27, the inhibition rates of P92-si51-DV26 and P92-si51-DV27 increased by 27.7% and 29.1% respectively.

**II. Other modification templates of the present disclosure**

P92-si51-DV30 and P92-si51-DV31 obtained by modifying basic sequence 51 with templates DV30 and DV31 had inhibition rates against the PCSK9 gene of 63.5% and 62.8% respectively, which were only 7.0% and 6.3% higher than the corresponding alternately modified sequences, respectively.

P92-si51-DV26 and P92-si51-DV27 modified with templates DV26 and DV27 of the present disclosure increased the inhibition rate by 20.7% and 22.1% compared with P92-si51-DV30, and increased by 21.4% and 22.8% compared with P92-si51-DV31, indicating a significant improvement.

**III. Advanced ESC templates DV21 (fluorinated sites: positions 2, 6, 14 and 16 on the antisense strand and positions 7, 9, 10, 11, 16 and 17 on the sense strand) and DV22 (fluorinated sites: positions 2, 6, 14 and 16 on the antisense strand and positions 7, 9, 10 and 11 on the sense strand) disclosed in the prior art**

P92-si51-DV21 and P92-si51-DV22 obtained by modifying basic sequence 51 with templates DV21 and DV22 had inhibition rates against the PCSK9 gene of 64.3% and 65.1% respectively, which were 7.8% and 8.6% higher than the corresponding alternately modified sequences, respectively.

P92-si51-DV26 and P92-si51-DV27 modified with templates DV26 and DV27 of the present disclosure increased the inhibition rate by 19.9% and 21.3% compared with P92-si51-DV21, and increased by 19.1% and 20.5% compared with P92-si5-DV22, indicating a significant improvement.

It can be concluded that:

1) After modification on basic sequence 51 using the modification templates DV25-29 of the present disclosure, the inhibition effect on the PCSK9 gene was significantly improved compared with the alternately modified sequences. For example, the inhibition rate of the basic sequence 51 modified with DV27 was 29.1% higher than that of the alternately modified sequence.

2) The activity varied greatly after the same siRNA sequence was modified with different modification templates. For example, the inhibition rate of the sequences obtained by modifying basic sequence 51 with modification templates DV26 and DV27 of the present disclosure can be increased by up to 22.8% compared with the sequences modified with modification templates DV30 and DV31 of the present disclosure, indicating a significant improvement; and can be increased by up to 21.3% compared with the sequences modified with the disclosed Advanced ESC templates DV21 and DV22, indicating a significant improvement.

3) The activity of siRNA sequences modified with different templates varied greatly, up to 20% difference. It can be seen that it is uncertain which template modification can be used in siRNA sequences to achieve high activity.

**(3) Basic sequence 81**

Table 16 Inhibition rate against PCSK9 gene by template-modified basic sequence 81

| Basic sequence NO. | Template-modified sequence No. | Inhibition rate (%) of template-mod ified sequence | Inhibition rate (%) of corresponding alternately-modified sequence | Increase (%) |
|---|---|---|---|---|
| | P92-si81-DV25 | 85.9 | 62.4 | 23.5 |
| | P92-si81-DV26 | 89.3 | 62.4 | 26.9 |
| | P92-si81-DV27 | 87.1 | 62.4 | 24.7 |
| | P92-si81-DV28 | 84.8 | 62.4 | 22.4 |
| 81 | P92-si81-DV29 | 85.6 | 62.4 | 23.2 |

(continued)

| Basic sequence NO. | Template-modified sequence No. | Inhibition rate (%) of template-mod ified sequence | Inhibition rate (%) of corresponding alternately-modified sequence | Increase (%) |
|---|---|---|---|---|
| | P92-si81-DV30 | 71.6 | 62.4 | 9.2 |
| | P92-si81-DV31 | 73.5 | 62.4 | 11.1 |
| | P92-si81-DV21 | 78.3 | 62.4 | 15.9 |
| | P92-si81-DV22 | 75.1 | 62.4 | 12.7 |

**I. DV25-29 templates of the present disclosure**

Basic sequence 81 modified with the modification templates DV25-29 designed by the present disclosure exhibited an inhibition rate against the PCSK9 gene exceeding 80%. Compared with the alternate modification with 2'-position methoxy and 2'-position fluorine, the inhibition rate against the PCSK9 gene increased significantly. For example, after modification with templates DV26 and DV27, the inhibition rates of P92-si81-DV26 and P92-si81-DV27 increased by 26.9% and 24.7% respectively.

**II. Other modification templates of the present disclosure**

P92-si81-DV30 and P92-si81-DV31 obtained by modifying basic sequence 81 with templates DV30 and DV31 had inhibition rates against the PCSK9 gene of 71.6% and 73.5% respectively, which were 9.2% and 11.1% higher than that of the corresponding alternately modified sequences, respectively.

P92-si81-DV26 and P92-si81-DV27 modified with templates DV26 and DV27 of the present disclosure increased the inhibition rate by 17.7% and 15.5% compared with P92-si81-DV27, and increased by 15.8% and 13.6% compared with P92-si81-DV31, indicating a significant improvement.

**III. Advanced ESC templates DV21 (fluorinated sites: positions 2, 6, 14 and 16 on the antisense strand and positions 7, 9, 10, 11, 16 and 17 on the sense strand) and DV22 (fluorinated sites: positions 2, 6, 14 and 16 on the antisense strand and positions 7, 9, 10 and 11 on the sense strand) disclosed in the prior art**

P92-si81-DV21 and P92-si81-DV22 obtained by modifying basic sequence 81 with templates DV21 and DV22 had inhibition rates against the PCSK9 gene of 78.3% and 75.1% respectively, which were 15.9% and 12.7% higher than that of the corresponding alternately modified sequences, respectively.

P92-si81-DV26 and P92-si81-DV27 modified with templates DV26 and DV27 of the present disclosure increased the inhibition rate by 11.0% and 8.8% compared with P92-si81-DV21, and increased by 14.2% and 12.0% compared with P92-si81-DV22, indicating a significant improvement.

It can be concluded that:

1) After modification on basic sequence 81 with the modification templates DV25-29 of the present disclosure, the inhibition effect on the PCSK9 gene was significantly improved compared with the alternately modified sequences. For example, the inhibition rate of the basic sequence 81 modified with DV26 was 26.9% higher than that of the alternately modified sequence.

2) The activity varied greatly after the same siRNA sequence was modified with different modification templates. For example, the inhibition rate of the sequences obtained by modifying basic sequence 81 with modification templates DV26 and DV27 of the present disclosure can be increased by up to 17.7% compared with the sequences modified with modification templates DV30 and DV31 of the present disclosure, indicating a significant improvement; and can be increased by up to 14.2% compared with the sequences modified with the disclosed Advanced ESC templates DV21 and DV22, indicating a significant improvement.

3) The activity of siRNA sequences modified with different templates varied greatly, up to 20% difference. It can be seen that it is uncertain which template modification can be used in siRNA sequences to achieve high activity.

**(4) Basic sequence 82**

Table 17 Inhibition rate against PCSK9 gene by template-modified basic sequence 82

| Basic sequence NO. | Template-modified sequence No. | Inhibition rate (%) of template-modifie d sequence | Inhibition rate (%) of corresponding alternately-modified sequence | Increase (%) |
|---|---|---|---|---|
| 82 | P92-si82-DV25 | 83.8 | 69.7 | 14.1 |
| | P92-si82-DV26 | 84.4 | 69.7 | 14.7 |
| | P92-si82-DV27 | 89.3 | 69.7 | 19.6 |
| | P92-si82-DV28 | 86.7 | 69.7 | 17.0 |
| | P92-si82-DV29 | 89.1 | 69.7 | 19.4 |
| | P92-si82-DV30 | 70.2 | 69.7 | 0.5 |
| | P92-si82-DV31 | 69.7 | 69.7 | 0.0 |
| | P92-si82-DV21 | 76.5 | 69.7 | 6.8 |
| | P92-si82-DV22 | 75.4 | 69.7 | 5.7 |

**I. DV25-29 templates of the present disclosure**

Basic sequence 82 modified with the modification templates DV25-29 designed by the present disclosure exhibited an inhibition rate against the PCSK9 gene exceeding 80%. Compared with the alternate modification with 2'-position methoxy and 2'-position fluorine, the inhibition rate against the PCSK9 gene increased significantly. For example, after modification with templates DV27 and DV29, the inhibition rates of P92-si82-DV27 and P92-si82-DV29 increased by 19.6% and 19.4% respectively.

**II. Other modification templates of the present disclosure**

P92-si82-DV30 and P92-si82-DV31 obtained by modifying basic sequence 82 with templates DV30 and DV31 had inhibition rates against the PCSK9 gene of 70.2% and 69.7% respectively, which were comparable to the corresponding alternately modified sequences.
P92-si82-DV27 and P92-si82-DV29 modified with templates DV27 and DV29 of the present disclosure increased the inhibition rate by 19.1% and 18.9% compared with P92-si82-DV27, and increased by 19.6% and 19.4% compared with P92-si82-DV31, indicating a significant improvement.

**III. Advanced ESC templates DV21 (fluorinated sites: positions 2, 6, 14 and 16 on the antisense strand and positions 7, 9, 10, 11, 16 and 17 on the sense strand) and DV22 (fluorinated sites: positions 2, 6, 14 and 16 on the antisense strand and positions 7, 9, 10 and 11 on the sense strand) disclosed in the prior art**

P92-si82-DV21 and P92-si82-DV22 obtained by modifying basic sequence 82 with templates DV21 and DV22 had inhibition rates against the PCSK9 gene of 76.5% and 75.4% respectively, which were 6.8% and 5.7% higher than the corresponding alternately modified sequences, respectively.
P92-si82-DV27 and P92-si82-DV29 modified with templates DV27 and DV29 of the present disclosure increased the inhibition rate by 12.8% and 12.6% compared with P92-si82-DV21, and increased by 13.9% and 13.7% compared with P92-si82-DV22, indicating a significant improvement.
It can be concluded that:

1) After modification on basic sequence 82 using the modification templates DV25-29 of the present disclosure, the inhibition effect on the PCSK9 gene was significantly improved compared with the alternately modified sequences. For example, the inhibition rate of the basic sequence 82 modified with DV27 was 19.6% higher than that of the alternately modified sequence.

2) The activity varied greatly after the same siRNA sequence was modified with different modification templates. For example, the inhibition rate of the sequences obtained by modifying basic sequence 82 with modification templates DV27 and DV29 of the present disclosure can be increased by up to 19.6% compared with the sequences modified with modification templates DV30 and DV31 of the present disclosure, indicating a significant improvement; and can be increased by up to 13.9% compared with the sequences modified with the disclosed Advanced ESC templates DV21 and DV22, indicating a significant improvement.

3) The activity of siRNA sequences modified with different templates varied greatly, up to 20% difference. It can be seen that it is uncertain which template modification can be used in siRNA sequences to achieve high activity.

**(5) Basic sequence 84**

Table 18 Inhibition rate against PCSK9 gene by template-modified basic sequence 84

| Basic sequence NO. | Template-modified sequence No. | Inhibition rate (%) of template-modi fied sequence | Inhibition rate (%) of corresponding alternately-modi fied sequence | Increa se (%) |
|---|---|---|---|---|
| 84 | P92-si84-DV25 | 86.0 | 61.6 | 24.4 |
| | P92-si84-DV26 | 85.7 | 61.6 | 24.1 |
| | P92-si84-DV27 | 86.8 | 61.6 | 25.2 |
| | P92-si84-DV28 | 88.4 | 61.6 | 26.8 |
| | P92-si84-DV29 | 87.5 | 61.6 | 25.9 |
| | P92-si84-DV30 | 70.3 | 61.6 | 8.7 |
| | P92-si84-DV31 | 72.4 | 61.6 | 10.8 |
| | P92-si84-DV21 | 77.7 | 61.6 | 16.1 |
| | P92-si84-DV22 | 75.1 | 61.6 | 13.5 |

**I. DV25-29 templates of the present disclosure**

Basic sequence 84 modified with the modification templates DV25-29 designed by the present disclosure exhibited an inhibition rate against the PCSK9 gene exceeding 85%. Compared with the alternate modification with 2'-position methoxy and 2'-position fluorine, the inhibition rate against the PCSK9 gene increased significantly. For example, after modification with templates DV28 and DV29, the inhibition rates of P92-si84-DV28 and P92-si84-DV29 increased by 26.8% and 25.9% respectively.

**II. Other modification templates of the present disclosure**

P92-si84-DV30 and P92-si84-DV31 obtained by modifying basic sequence 84 with templates DV30 and DV31 had inhibition rates against the PCSK9 gene of 70.3% and 72.4% respectively, which were 8.7% and 10.8% higher than that of the corresponding alternately modified sequences.

P92-si84-DV28 and P92-si84-DV29 modified with templates DV28 and DV29 of the present disclosure increased the inhibition rate by 18.1% and 17.2% compared with P92-si84-DV30, and increased by 16.0% and 15.1% compared with P92-si84-DV31, indicating a significant improvement.

**III. Advanced ESC templates DV21 (fluorinated sites: positions 2, 6, 14 and 16 on the antisense strand and positions 7, 9, 10, 11, 16 and 17 on the sense strand) and DV22 (fluorinated sites: positions 2, 6, 14 and 16 on the antisense strand and positions 7, 9, 10 and 11 on the sense strand) disclosed in the prior art**

P92-si84-DV21 and P92-si84-DV22 obtained by modifying basic sequence 84 with templates DV21 and DV22 had inhibition rates against the PCSK9 gene of 77.7% and 75.1% respectively, which were 16.1% and 13.5% higher than that of the corresponding alternately modified sequences, respectively.

P92-si84-DV28 and P92-si84-DV29 modified with templates DV28 and DV29 of the present disclosure increased the inhibition rate by 10.7% and 9.8% compared with P92-si84-DV21, and increased by 13.3% and 12.4% compared with P92-si84-DV22, indicating a significant improvement.

It can be concluded that:

1) After modification on basic sequence 84 using the modification templates DV25-29 of the present disclosure, the inhibition effect on the PCSK9 gene was significantly improved compared with the alternately modified sequences. For example, the inhibition rate of the basic sequence 84 modified with DV28 was 26.8% higher than that of the alternately modified sequence.

2) The activity varied greatly after the same siRNA sequence was modified with different modification templates. For example, the inhibition rate of the sequences obtained by modifying basic sequence 84 with modification templates DV28 and DV29 of the present disclosure can be increased by up to 18.1% compared with the sequences modified with modification templates DV30 and DV31 of the present disclosure, indicating a significant improvement; and can be increased by up to 13.3% compared with the sequences modified with the disclosed Advanced ESC templates DV21 and DV22, indicating a significant improvement.

3) The activity of siRNA sequences modified with different templates varied greatly, up to 20% difference. It can be seen that it is uncertain which template modification can be used in siRNA sequences to achieve high activity.

**(6) Basic sequence 3**

Table 19 Inhibition rate against PCSK9 gene by template-modified basic sequence 3

| Basic sequence NO. | Template-modified sequence No. | Inhibition rate (%) of template-mod ified sequence | Inhibition rate (%) of corresponding alternately-modified sequence | Increase (%) |
|---|---|---|---|---|
| 3 | P92-si3-DV25 | 79.7 | 60.3 | 19.4 |
| | P92-si3-DV26 | 80.9 | 60.3 | 20.6 |
| | P92-si3-DV27 | 83.0 | 60.3 | 22.7 |
| | P92-si3-DV28 | 84.3 | 60.3 | 24.0 |
| | P92-si3-DV29 | 85.0 | 60.3 | 24.7 |

1) Basic sequence 3 modified with the modification templates DV25-29 designed by the present disclosure exhibited an inhibition rate against the PCSK9 gene exceeding 75%. Compared with the alternate modification with 2'-position methoxy and 2'-position fluorine, the inhibition rate against the PCSK9 gene increased significantly. For example, after modification with templates DV28 and DV29, the inhibition rates of P92-si3-DV28 and P92-si3-DV29 increased by 24.0% and 24.7% respectively.

2) It can be concluded that after modification on basic sequence 3 using the modification templates DV25-29 of the present disclosure, the inhibition effect on the PCSK9 gene was significantly improved compared with the alternately modified sequences.

**(7) Basic sequence 8**

Table 20 Inhibition rate against PCSK9 gene by template-modified basic sequence 8

| Basic sequenc e NO. | Template-modified sequence No. | Inhibition rate (%) of template-modifie d sequence | Inhibition rate (%) of corresponding alternately-modifie d sequence | Increas e (%) |
|---|---|---|---|---|
| 8 | P92-si8-DV25 | 79.8 | 61.9 | 17.9 |
| | P92-si8-DV26 | 80.6 | 61.9 | 18.7 |
| | P92-si8-DV27 | 81.6 | 61.9 | 19.7 |
| | P92-si8-DV28 | 82.1 | 61.9 | 20.2 |
| | P92-si8-DV29 | 79.5 | 61.9 | 17.6 |

1) Basic sequence 8 modified with the modification templates DV25-29 designed by the present disclosure exhibited an inhibition rate against the PCSK9 gene exceeding 75%. Compared with the alternate modification with 2'-position methoxy and 2'-position fluorine, the inhibition rate against the PCSK9 gene increased significantly. For example, after modification with templates DV27 and DV28, the inhibition rates of P92-si8-DV27 and P92-si8-DV28 increased by 19.7% and 20.2% respectively.

2) It can be concluded that after modification on basic sequence 8 using the modification templates DV25-29 of the present disclosure, the inhibition effect on the PCSK9 gene was significantly improved compared with the alternately modified sequences.

**(8) Basic sequence 9**

Table 21 Inhibition rate against PCSK9 gene by template-modified basic sequence 9

| Basic sequence NO. | Template-modified sequence No. | Inhibition rate (%) of template-mod ified sequence | Inhibition rate (%) of corresponding alternately-modified sequence | Increa se (%) |
|---|---|---|---|---|
| 9 | P92-si9-DV25 | 71.9 | 52.4 | 19.5 |
| | P92-si9-DV26 | 73.7 | 52.4 | 21.3 |
| | P92-si9-DV27 | 79.6 | 52.4 | 27.2 |
| | P92-si9-DV28 | 77.8 | 52.4 | 25.4 |
| | P92-si9-DV29 | 76.8 | 52.4 | 24.4 |

1) Basic sequence 9 modified with the modification templates DV25-29 designed by the present disclosure exhibited an inhibition rate against the PCSK9 gene exceeding 70%. Compared with the alternate modification with 2'-position methoxy and 2'-position fluorine, the inhibition rate against the PCSK9 gene increased significantly. For example, after modification with templates DV27 and DV28, the inhibition rates of P92-si9-DV27 and P92-si9-DV28 increased by 27.2% and 25.4% respectively.

2) It can be concluded that after modification on basic sequence 9 using the modification templates DV25-29 of the present disclosure, the inhibition effect on the PCSK9 gene was significantly improved compared with the alternately modified sequences.

**(9) Basic sequence 21**

Table 22 Inhibition rate against PCSK9 gene by template-modified basic sequence 21

| Basic sequenc e NO. | Template-modified sequence No. | Inhibition rate (%) of template-modifie d sequence | Inhibition rate (%) of corresponding alternately-modifie d sequence | Increas e (%) |
|---|---|---|---|---|
| 21 | P92-si21-DV25 | 74.0 | 49.6 | 24.4 |
| | P92-si21-DV26 | 76.1 | 49.6 | 26.5 |
| | P92-si21-DV27 | 71.1 | 49.6 | 21.5 |
| | P92-si21-DV28 | 71.5 | 49.6 | 21.9 |
| | P92-si21-DV29 | 72.0 | 49.6 | 22.4 |

1) Basic sequence 21 modified with the modification templates DV25-29 designed by the present disclosure exhibited an inhibition rate against the PCSK9 gene exceeding 70%. Compared with the alternate modification with 2'-position methoxy and 2'-position fluorine, the inhibition rate against the PCSK9 gene increased significantly. For example, after modification with templates DV25 and DV26, the inhibition rates of P92-si21-DV25 and P92-si21-DV26 increased by 24.4% and 26.5% respectively.

2) It can be concluded that after modification on basic sequence 21 using the modification templates DV25-29 of the present disclosure, the inhibition effect on the PCSK9 gene was significantly improved compared with the alternately modified sequences.

**(10) Basic sequence 31**

Table 23 Inhibition rate against PCSK9 gene by template-modified basic sequence 31

| Basic sequence NO. | Template-modified sequence No. | Inhibition rate (%) of template-mod ified sequence | Inhibition rate (%) of corresponding alternately-modifie d sequence | Increase (%) |
|---|---|---|---|---|
| 31 | P92-si31-DV25 | 79.2 | 56.3 | 22.9 |
| | P92-si31-DV26 | 78.9 | 56.3 | 22.6 |
| | P92-si31-DV27 | 71.2 | 56.3 | 14.9 |
| | P92-si31-DV28 | 70.6 | 56.3 | 14.3 |
| | P92-si31-DV29 | 70.0 | 56.3 | 13.7 |

1) Basic sequence 31 modified with the modification templates DV25-29 designed by the present disclosure exhibited an inhibition rate against the PCSK9 gene exceeding 70%. Compared with the alternate modification with 2'-position methoxy and 2'-position fluorine, the inhibition rate against the PCSK9 gene increased significantly. For example, after modification with templates DV25 and DV26, the inhibition rates of P92-si31-DV25 and P92-si31-DV26 increased by 22.9% and 22.6% respectively.

2) It can be concluded that after modification on basic sequence 31 using the modification templates DV25-29 of the present disclosure, the inhibition effect on the PCSK9 gene was significantly improved compared with the alternately modified sequences.

**(11) Basic sequence 73**

Table 24 Inhibition rate against PCSK9 gene by template-modified basic sequence 73

| Basic sequence NO. | Template-modified sequence No. | Inhibition rate (%) of template-modified sequence | Inhibition rate (%) of corresponding alternately-modified sequence | Increase (%) |
|---|---|---|---|---|
| 73 | P92-si73-DV25 | 70.1 | 49.4 | 20.7 |
| | P92-si73-DV26 | 71.8 | 49.4 | 22.4 |
| | P92-si73-DV27 | 77.6 | 49.4 | 28.2 |
| | P92-si73-DV28 | 80.0 | 49.4 | 30.6 |
| | P92-si73-DV29 | 82.1 | 49.4 | 32.7 |

1) Basic sequence 73 modified with the modification templates DV25-29 designed by the present disclosure exhibited an inhibition rate against the PCSK9 gene exceeding 70%. Compared with the alternate modification with 2'-position methoxy and 2'-position fluorine, the inhibition rate against the PCSK9 gene increased significantly. For example, after modification with templates DV28 and DV29, the inhibition rates of P92-si73-DV28 and P92-si73-DV29 increased by 30.6% and 32.7% respectively.

2) It can be concluded that after modification on basic sequence 73 using the modification templates DV25-29 of the present disclosure, the inhibition effect on the PCSK9 gene was significantly improved compared with the alternately modified sequences.

**(12) Basic sequence 83**

Table 25 Inhibition rate against PCSK9 gene by template-modified basic sequence 83

| Basic sequenc e NO. | Template-modified sequence No. | Inhibition rate (%) of template-modifie d sequence | Inhibition rate (%) of corresponding alternately-modifie d sequence | Increas e (%) |
|---|---|---|---|---|
| 83 | P92-si83-DV25 | 77.9 | 51.7 | 26.2 |
| | P92-si83-DV26 | 75.0 | 51.7 | 23.3 |
| | P92-si83-DV27 | 82.6 | 51.7 | 30.9 |
| | P92-si83-DV28 | 78.6 | 51.7 | 26.9 |
| | P92-si83-DV29 | 81.4 | 51.7 | 29.7 |

1) Basic sequence 83 modified with the modification templates DV25-29 designed by the present disclosure exhibited an inhibition rate against the PCSK9 gene exceeding 75%. Compared with the alternate modification with 2'-position methoxy and 2'-position fluorine, the inhibition rate against the PCSK9 gene increased significantly. For example, after modification with templates DV27 and DV29, the inhibition rates of P92-si83-DV27 and P92-si83-DV29 increased by 30.9% and 29.7% respectively.

2) It can be concluded that after modification on basic sequence 83 using the modification templates DV25-29 of the present disclosure, the inhibition effect on the PCSK9 gene was significantly improved compared with the alternately modified sequences.

**Summary:**

(1) The screened basic sequences 5, 51, 81, 82, 84, 3, 8, 9, 21, 31, 73 and 83, after being modified with the modification templates DV25-29 designed in the present disclosure, exhibited a significant inhibitory effect on PCSK9 gene

expression, with an inhibition rate above 70%, among which the inhibition rates of P92-si81-DV26, P92-si82-DV27 and P92-si82-DV29 reached 89.3%, 89.3% and 89.1%, respectively.

(2) The above 12 sequences modified with the modification templates DV25-29 of the present disclosure significantly improved the inhibition rate against the PCSK9 gene expression compared with the alternately modified sequences. For example, P92-si51-DV27 obtained by modifying basic sequence 51 with templates DV27 increased the inhibition rate by 29.1% compared to the alternately modified sequence, and P92-si81-DV26 obtained by modifying basic sequence 81 with templates DV26 increased the inhibition rate by 26.9% compared to the alternately modified sequence.

(3) The sequences modified on the same sequence with the modification templates DV25-29 of the present disclosure significantly improved the inhibition rate against the PCSK9 gene expression compared with using the modification templates disclosed in the prior art. For example, basic sequence 51 modified with the modification template DV27 of the present disclosure increased the inhibition rate by 21.3% compared with the disclosed Advanced ESC template DV21.

(4) The sequences modified on the same sequence with the modification templates DV25-29 of the present disclosure significantly improved the inhibition rate against the PCSK9 gene expression compared with using other modification templates DV30 and DV31 of the present disclosure. For example, basic sequence 51 modified with the modification template DV27 of the present disclosure increased the inhibition rate by 22.8% compared with the modification template DV31 of the present disclosure.

(5) The activity of sequences modified with different template varied greatly. For example, the sequence obtained by modifying basic sequence 5 with DV26 increased the inhibition rate by 22.5% compared with the sequence modified with DV31; the sequence obtained by modifying basic sequence 51 with DV27 increased the inhibition rate by 20.5% compared with the sequence modified with DV22. It can be concluded that it is uncertain which modification template should be used to modify the siRNA sequence to achieve high activity.

**(ii) EC50 experiment**

Some template-modified sequences were selected for the EC50 experiment, including P92-si5-DV26, P92-si51-DV25, P92-si81-DV27, P92-si82-DV27, P92-si84-DV26, P92-si3-DV28, P92-si8-DV29, P92-si9-DV28, P92-si21-DV25, P92-si31-DV27, P92-si73-DV28 and P92-si83-DV26, a total of 12 sequences, in order to determine the EC50 concentration of each sequence. The experimental results are shown in Table 26.

Table 26 EC50 experimental results of each modified sequence

| Sequence No. | Sequence EC50 (nM) |
|---|---|
| P92-si5-DV26 | 0.0010 |
| P92-si51-DV25 | 0.0027 |
| P92-si81-DV27 | 0.0003 |
| P92-si82-DV27 | 0.0006 |
| P92-si84-DV26 | 0.0004 |
| P92-si3-DV28 | 0.0022 |
| P92-si8-DV29 | 0.0039 |
| P92-si9-DV28 | 0.0047 |
| P92-si21-DV25 | 0.0048 |
| P92-si31-DV27 | 0.0035 |
| P92-si73-DV28 | 0.0044 |
| P92-si83-DV26 | 0.0025 |

As can be seen from Table 26, the EC50 values of these 12 sequences were between 0.0001 nM and 0.005 nM, among which the EC50 values of P92-si81-DV27, P92-si84-DV26 and P92-si82-DV27 were 0.0003 nM, 0.0004 nM and

0.0006 nM, respectively. These results demonstrate that the sequences can effectively inhibit PCSK9 gene expression at low concentrations.

**Example 5: Comparison with the inhibitory effect of the sequences disclosed in the prior art on the PCSK9 gene**

In this example, the inhibition efficiency of the PCSK9 gene was compared between the unmodified sequences disclosed in the prior art that were identical or similar to the basic sequences 3, 5, 8, 9, 31, 81, 82, 83 and 84 of the present disclosure, and the alternately modified sequences and the sequences modified with the templates DV25-29 of the present disclosure.

**1. Experimental Materials**

Test samples:

(1) Sequences disclosed in the prior art

Table 27 Sequences disclosed in the prior art

| Sequence No. | Position | Sense strand | **Sequence** ID NO. | Antisense strand | **Sequence** ID NO. | Source |
|---|---|---|---|---|---|---|
| si81 | 3546 | CUUUUGUAACUU GAAGAUAUU | 3 | AAUAUCUUCAAGUU ACAAAAGCA | 15 | CN11599 2138 A |
| si84 | 3574 | GUUUUGUAGCAU UUUUAUUAA | 5 | UUAAUAAAAAUGC UACAAAACCC | 17 | CN11599 2138 A |
| si3 | 614 | CACCAAGAUCCUG CAUGUCUU | 6 | AAGACAUGCAGGAU CUUGGUGAG | 18 | Page 104, WO20140 89313 A1 |
| si8 | 1083 | CUCAUAGGCCUGG AGUUUAUU | 7 | AAUAAACUCCAGGC CUAUGAGGG | 19 | Page 91, CN10822 0295 B |
| si9 | 1089 | GGCCUGGAGUUU AUUCGGAAA | 8 | UUUCCGAAUAAACU CCAGGCCUA | 20 | Page 106, WO20140 89313 A1 |
| si83 | 3572 | GGGUUUUGUAGC AUUUUUAUU | 12 | AAUAAAAAUGCUAC AAAACCCAG | 24 | Page 16, CN11599 2138 A |
| 5P | 618 | AAGAUCCUGCAUG UCUUCCAU | 1 | AUGGAAGACAUGCA GGAUCUU | 417 | WO2023/ 017004 Al |
| 82P | 3557 | GAAGAUAUUUAU UCUGGGUUU | 4 | AAACCCAGAAUAAA UAUCUUC | 418 | WO2023/ 017004 Al |
| 31P | 2103 | GGUCUGGAAUGC AAAGUCAAG | 10 | CUUGACUUUGCAUU CCAGACC | 419 | Page 25, CN11506 6498 A |
| Note: The sequences disclosed in the above patent applications were all unmodified sequence RNA. | | | | | | |

(2) siRNA sequences modified alternately with 2'-OMe and 2'-F and terminally thio-modified in Example 2: P92-si5, P92-si81, P92-si82, P92-si84, P92-si3, P92-si8, P92-si9, P92-si31 and P92-si83;
(3) siRNA sequences modified with the modification templates DV25-29. The specific sequences are shown in Table 28.

Table 28 Sequences modified with modification templates DV25-29

| Bas ic seq uen ce NO . | Templat e-modifi ed Sequenc e No. | Sense strand sequence 5'-3' | Modifie d sequence SEQ ID NO:/Cor respondi ng basic sequence SEQ ID NO: | Antisense strand sequence 5'-3' | Modifie d sequenc e SEQ ID NO:/Co rrespon ding basic sequenc e SEQ ID NO: |
|---|---|---|---|---|---|
| 5 | P92-si5-DV25 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |
| | P92-si5-DV26 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gf-Gf-Am-Af-Gm-Am-Cm-Am-Um-Gm-Cm-Af-Gm-Gf-Am-Um-Cm-Um-Ums-Gms-Gm | 377/13 |
| | P92-si5-DV27 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gf-Af-Af-Gm-Am-Cm-Am-Um-Gm-Cm-Af-Gm-Gf-Am-Um-Cm-Um-Ums-Gms-Gm | 378/13 |
| | P92-si5-DV28 | Ams-Ams-Gm-Am-Um-Cm-Cf-Um-Gf-Cm-Af-Um-Gm-Um-Cm-Um-Uf-Cm-Cm-Am-Um | 338/1 | Ams-Ufs-Gf-Gf-Am-Af-Gm-Am-Cm-Am-Um-Gm-Cm-Af-Gm-Gf-Am-Um-Cm-Um-Ums-Gms-Gm | 377/13 |
| | P92-si5-DV29 | Ams-Ams-Gm-Am-Um-Cm-Cf-Um-Gf-Cm-Af-Um-Gm-Um-Cm-Um-Uf-Cm-Cm-Am-Um | 338/1 | Ams-Ufs-Gm-Gf-Af-Af-Gm-Am-Cm-Am-Um-Gm-Cm-Af-Gm-Gf-Am-Um-Cm-Um-Ums-Gms-Gm | 378/13 |

(continued)

| Basic sequence NO. | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 81 | P92-si81-DV25 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Am-Um-Cf-Um-Um-Cm-Am-Am-Gm-Um-Uf-Am-Cf-Am-Am-Am-Am-Gms-Cms-Am | 384/15 |
| | P92-si81-DV26 | Cms-Ums-Um-Um-Um-Gm-Uf-Am-Af-Cf-Uf-Um-Gm-Am-Am-Gm-Af-Um-Am-Um-Um | 347/3 | Ams-Afs-Uf-Af-Um-Cf-Um-Um-Cm-Am-Am-Gm-Um-Uf-Am-Cf-Am-Am-Am-Am-Gms-Cms-Am | 385/15 |
| | P92-si81-DV27 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Af-Uf-Cf-Um-Um-Cm-Am-Am-Gm-Um-Uf-Am-Cf-Am-Am-Am-Am-Gms-Cms-Am | 386/15 |
| | P92-si81-DV28 | Cms-Ums-Um-Um-Um-Gm-Uf-Am-Af-Cm-Uf-Um-Gm-Am-Am-Gm-Af-Um-Am-Um-Um | 348/3 | Ams-Afs-Uf-Af-Um-Cf-Um-Um-Cm-Am-Am-Gm-Um-Uf-Am-Cf-Am-Am-Am-Am-Gms-Cms-Am | 385/15 |
| | P92-si81-DV29 | Cms-Ums-Um-Um-Um-Gm-Uf-Am-Af-Cm-Uf-Um-Gm-Am-Am-Gm-Af-Um-Am-Um-Um | 348/3 | Ams-Afs-Um-Af-Uf-Cf-Um-Um-Cm-Am-Am-Gm-Um-Uf-Am-Cf-Am-Am-Am-Am-Gms-Cms-Am | 386/15 |

(continued)

| Basic sequence NO. | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 82 | P92-si82-DV25 | Gms-Ams-Am-Gm-Am-Um-Af-Um-Uf-Uf-Af-Um-Um-Cm-Um-Gm-Gf-Gm-Um-Um-Um | 352/4 | Ams-Afs-Am-Cm-Cm-Cf-Am-Gm-Am-Am-Um-Am-Am-Af-Um-Af-Um-Cm-Um-Um-Cms-Ams-Am | 388/16 |
| | P92-si82-DV26 | Gms-Ams-Am-Gm-Am-Um-Af-Um-Uf-Uf-Af-Um-Um-Cm-Um-Gm-Gf-Gm-Um-Um-Um | 352/4 | Ams-Afs-Af-Cf-Cm-Cf-Am-Gm-Am-Am-Um-Am-Am-Af-Um-Af-Um-Cm-Um-Um-Cms-Ams-Am | 389/16 |
| | P92-si82-DV27 | Gms-Ams-Am-Gm-Am-Um-Af-Um-Uf-Uf-Af-Um-Um-Cm-Um-Gm-Gf-Gm-Um-Um-Um | 352/4 | Ams-Afs-Am-Cf-Cf-Cf-Am-Gm-Am-Am-Um-Am-Am-Af-Um-Af-Um-Cm-Um-Um-Cms-Ams-Am | 390/16 |
| | P92-si82-DV28 | Gms-Ams-Am-Gm-Am-Um-Af-Um-Uf-Um-Af-Um-Um-Cm-Um-Gm-Gf-Gm-Um-Um-Um | 353/4 | Ams-Afs-Af-Cf-Cm-Cf-Am-Gm-Am-Am-Um-Am-Am-Af-Um-Af-Um-Cm-Um-Um-Cms-Ams-Am | 389/16 |
| | P92-si82-DV29 | Gms-Ams-Am-Gm-Am-Um-Af-Um-Uf-Um-Af-Um-Um-Cm-Um-Gm-Gf-Gm-Um-Um-Um | 353/4 | Ams-Afs-Am-Cf-Cf-Cf-Am-Gm-Am-Am-Um-Am-Am-Af-Um-Af-Um-Cm-Um-Um-Cms-Ams-Am | 390/16 |

(continued)

| Basic sequence NO . | Templat e-modifi ed Sequenc e No. | Sense strand sequence 5'-3' | Modifie d sequence SEQ ID NO:/Cor respondi ng basic sequence SEQ ID NO: | Antisense strand sequence 5'-3' | Modifie d sequenc e SEQ ID NO:/Co rrespon ding basic sequenc e SEQ ID NO: |
|---|---|---|---|---|---|
| 84 | P92-si84 -DV25 | Gms-Ums-Um-Um-Um-Gm-Uf-Am-Gf-Cf-Af-Um-Um-Um-Um-Um-Af-Um-Um-Am-Am | 357/5 | Ums-Ufs-Am-Am-Um-Af-Am-Am-Am-Am-Um-Gm-Cm-Uf-Am-Cf-Am-Am-Am-Am-Cms-Cms-Cm | 392/17 |
| | P92-si84 -DV26 | Gms-Ums-Um-Um-Um-Gm-Uf-Am-Gf-Cf-Af-Um-Um-Um-Um-Um-Af-Um-Um-Am-Am | 357/5 | Ums-Ufs-Af-Af-Um-Af-Am-Am-Am-Am-Um-Gm-Cm-Uf-Am-Cf-Am-Am-Am-Am-Cms-Cms-Cm | 393/17 |
| | P92-si84 -DV27 | Gms-Ums-Um-Um-Um-Gm-Uf-Am-Gf-Cf-Af-Um-Um-Um-Um-Um-Af-Um-Um-Am-Am | 357/5 | Ums-Ufs-Am-Af-Uf-Af-Am-Am-Am-Am-Um-Gm-Cm-Uf-Am-Cf-Am-Am-Am-Am-Cms-Cms-Cm | 394/17 |
| | P92-si84 -DV28 | Gms-Ums-Um-Um-Um-Gm-Uf-Am-Gf-Cm-Af-Um-Um-Um-Um-Um-Af-Um-Um-Am-Am | 358/5 | Ums-Ufs-Af-Af-Um-Af-Am-Am-Am-Am-Um-Gm-Cm-Uf-Am-Cf-Am-Am-Am-Am-Cms-Cms-Cm | 393/17 |
| | P92-si84 -DV29 | Gms-Ums-Um-Um-Um-Gm-Uf-Am-Gf-Cm-Af-Um-Um-Um-Um-Um-Af-Um-Um-Am-Am | 358/5 | Ums-Ufs-Am-Af-Uf-Af-Am-Am-Am-Am-Um-Gm-Cm-Uf-Am-Cf-Am-Am-Am-Am-Cms-Cms-Cm | 394/17 |

(continued)

| Basic sequence NO . | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 3 | P92-si3-DV25 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gm-Am-Cm-Af-Um-Gm-Cm-Am-Gm-Gm-Am-Uf-Cm-Uf-Um-Gm-Gm-Um-Gms-Ams-Gm | 396/18 |
| | P92-si3-DV26 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gf-Af-Cm-Af-Um-Gm-Cm-Am-Gm-Gm-Am-Uf-Cm-Uf-Um-Gm-Gm-Um-Gms-Ams-Gm | 397/18 |
| | P92-si3-DV27 | Cms-Ams-Cm-Cm-Am-Am-Gf-Am-Uf-Cf-Cf-Um-Gm-Cm-Am-Um-Gf-Um-Cm-Um-Um | 362/6 | Ams-Afs-Gm-Af-Cf-Af-Um-Gm-Cm-Am-Gm-Gm-Am-Uf-Cm-Uf-Um-Gm-Gm-Um-Gms-Ams-Gm | 398/18 |
| | P92-si3-DV28 | Cms-Ams-Cm-Cm-Am-Am-Gf-Am-Uf-Cm-Cf-Um-Gm-Cm-Am-Um-Gf-Um-Cm-Um-Um | 363/6 | Ams-Afs-Gf-Af-Cm-Af-Um-Gm-Cm-Am-Gm-Gm-Am-Uf-Cm-Uf-Um-Gm-Gm-Um-Gms-Ams-Gm | 397/18 |
| | P92-si3-DV29 | Cms-Ams-Cm-Cm-Am-Am-Gf-Am-Uf-Cm-Cf-Um-Gm-Cm-Am-Um-Gf-Um-Cm-Um-Um | 363/6 | Ams-Afs-Gm-Af-Cf-Af-Um-Gm-Cm-Am-Gm-Gm-Am-Uf-Cm-Uf-Um-Gm-Gm-Um-Gms-Ams-Gm | 398/18 |

(continued)

| Basic sequence NO . | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 8 | P92-si8-DV25 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Um-Am-Am-Af-Cm-Um-Cm-Cm-Am-Gm-Gm-Cf-Cm-Uf-Am-Um-Gm-Am-Gms-Gms-Gm | 399/19 |
| | P92-si8-DV26 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Uf-Af-Am-Af-Cm-Um-Cm-Cm-Am-Gm-Gm-Cf-Cm-Uf-Am-Um-Gm-Am-Gms-Gms-Gm | 400/19 |
| | P92-si8-DV27 | Cms-Ums-Cm-Am-Um-Am-Gf-Gm-Cf-Cf-Uf-Gm-Gm-Am-Gm-Um-Uf-Um-Am-Um-Um | 364/7 | Ams-Afs-Um-Af-Af-Af-Cm-Um-Cm-Cm-Am-Gm-Gm-Cf-Cm-Uf-Am-Um-Gm-Am-Gms-Gms-Gm | 401/19 |
| | P92-si8-DV28 | Cms-Ums-Cm-Am-Um-Am-Gf-Gm-Cf-Cm-Uf-Gm-Gm-Am-Gm-Um-Uf-Um-Am-Um-Um | 365/7 | Ams-Afs-Uf-Af-Am-Af-Cm-Um-Cm-Cm-Am-Gm-Gm-Cf-Cm-Uf-Am-Um-Gm-Am-Gms-Gms-Gm | 400/19 |
| | P92-si8-DV29 | Cms-Ums-Cm-Am-Um-Am-Gf-Gm-Cf-Cm-Uf-Gm-Gm-Am-Gm-Um-Uf-Um-Am-Um-Um | 365/7 | Ams-Afs-Um-Af-Af-Af-Cm-Um-Cm-Cm-Am-Gm-Gm-Cf-Cm-Uf-Am-Um-Gm-Am-Gms-Gms-Gm | 401/19 |

(continued)

| Basic sequence NO . | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 9 | P92-si9-DV25 | Gms-Gms-Cm-Cm-Um-Gm-Gf-Am-Gf-Uf-Uf-Um-Am-Um-Um-Cm-Gf-Gm-Am-Am-Am | 366/8 | Ums-Ufs-Um-Cm-Cm-Gf-Am-Am-Um-Am-Am-Am-Cm-Uf-Cm-Cf-Am-Gm-Gm-Cm-Cms-Ums-Am | 402/20 |
| | P92-si9-DV26 | Gms-Gms-Cm-Cm-Um-Gm-Gf-Am-Gf-Uf-Uf-Um-Am-Um-Um-Cm-Gf-Gm-Am-Am-Am | 366/8 | Ums-Ufs-Uf-Cf-Cm-Gf-Am-Am-Um-Am-Am-Am-Cm-Uf-Cm-Cf-Am-Gm-Gm-Cm-Cms-Ums-Am | 403/20 |
| | P92-si9-DV27 | Gms-Gms-Cm-Cm-Um-Gm-Gf-Am-Gf-Uf-Uf-Um-Am-Um-Um-Cm-Gf-Gm-Am-Am-Am | 366/8 | Ums-Ufs-Um-Cf-Cf-Gf-Am-Am-Um-Am-Am-Am-Cm-Uf-Cm-Cf-Am-Gm-Gm-Cm-Cms-Ums-Am | 404/20 |
| | P92-si9-DV28 | Gms-Gms-Cm-Cm-Um-Gm-Gf-Am-Gf-Um-Uf-Um-Am-Um-Um-Cm-Gf-Gm-Am-Am-Am | 367/8 | Ums-Ufs-Uf-Cf-Cm-Gf-Am-Am-Um-Am-Am-Am-Cm-Uf-Cm-Cf-Am-Gm-Gm-Cm-Cms-Ums-Am | 403/20 |
| | P92-si9-DV29 | Gms-Gms-Cm-Cm-Um-Gm-Gf-Am-Gf-Um-Uf-Um-Am-Um-Um-Cm-Gf-Gm-Am-Am-Am | 367/8 | Ums-Ufs-Um-Cf-Cf-Gf-Am-Am-Um-Am-Am-Am-Cm-Uf-Cm-Cf-Am-Gm-Gm-Cm-Cms-Ums-Am | 404/20 |

(continued)

| Basic sequence NO. | Template-modified Sequence No. | Sense strand sequence 5'-3' | Modified sequence SEQ ID NO:/Corresponding basic sequence SEQ ID NO: | Antisense strand sequence 5'-3' | Modified sequence SEQ ID NO:/Corresponding basic sequence SEQ ID NO: |
|---|---|---|---|---|---|
| 31 | P92-si31-DV25 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Um-Gm-Am-Cf-Um-Um-Um-Gm-Cm-Am-Um-Uf-Cm-Cf-Am-Gm-Am-Cm-Cms-Ums-Gm | 408/22 |
| | P92-si31-DV26 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Uf-Gf-Am-Cf-Um-Um-Um-Gm-Cm-Am-Um-Uf-Cm-Cf-Am-Gm-Am-Cm-Cms-Ums-Gm | 409/22 |
| | P92-si31-DV27 | Gms-Gms-Um-Cm-Um-Gm-Gf-Am-Af-Uf-Gf-Cm-Am-Am-Am-Gm-Uf-Cm-Am-Am-Gm | 370/10 | Cms-Ufs-Um-Gf-Af-Cf-Um-Um-Um-Gm-Cm-Am-Um-Uf-Cm-Cf-Am-Gm-Am-Cm-Cms-Ums-Gm | 410/22 |
| | P92-si31-DV28 | Gms-Gms-Um-Cm-Um-Gm-Gf-Am-Af-Um-Gf-Cm-Am-Am-Am-Gm-Uf-Cm-Am-Am-Gm | 371/10 | Cms-Ufs-Uf-Gf-Am-Cf-Um-Um-Um-Gm-Cm-Am-Um-Uf-Cm-Cf-Am-Gm-Am-Cm-Cms-Ums-Gm | 409/22 |
| | P92-si31-DV29 | Gms-Gms-Um-Cm-Um-Gm-Gf-Am-Af-Um-Gf-Cm-Am-Am-Am-Gm-Uf-Cm-Am-Am-Gm | 371/10 | Cms-Ufs-Um-Gf-Af-Cf-Um-Um-Um-Gm-Cm-Am-Um-Uf-Cm-Cf-Am-Gm-Am-Cm-Cms-Ums-Gm | 410/22 |

(continued)

| Basic sequence NO . | Templat e-modifi ed Sequenc e No. | Sense strand sequence 5'-3' | Modifie d sequence SEQ ID NO:/Cor respondi ng basic sequence SEQ ID NO: | Antisense strand sequence 5'-3' | Modifie d sequenc e SEQ ID NO:/Co rrespon ding basic sequenc e SEQ ID NO: |
|---|---|---|---|---|---|
| 83 | P92-si83 -DV25 | Gms-Gms-Gm-Um-Um-Um-Uf-Gm-Uf-Af-Gf-Cm-Am-Um-Um-Um-Uf-Um-Am-Um-Um | 374/12 | Ams-Afs-Um-Am-Am-Af-Am-Am-Um-Gm-Cm-Um-Am-Cf-Am-Af-Am-Am-Cm-Cm-Cms-Ams-Gm | 414/24 |
| | P92-si83 -DV26 | Gms-Gms-Gm-Um-Um-Um-Uf-Gm-Uf-Af-Gf-Cm-Am-Um-Um-Um-Uf-Um-Am-Um-Um | 374/12 | Ams-Afs-Uf-Af-Am-Af-Am-Am-Um-Gm-Cm-Um-Am-Cf-Am-Af-Am-Am-Cm-Cm-Cms-Ams-Gm | 415/24 |
| | P92-si83 -DV27 | Gms-Gms-Gm-Um-Um-Um-Uf-Gm-Uf-Af-Gf-Cm-Am-Um-Um-Um-Uf-Um-Am-Um-Um | 374/12 | Ams-Afs-Um-Af-Af-Af-Am-Am-Um-Gm-Cm-Um-Am-Cf-Am-Af-Am-Am-Cm-Cm-Cms-Ams-Gm | 416/24 |
| | P92-si83 -DV28 | Gms-Gms-Gm-Um-Um-Um-Uf-Gm-Uf-Am-Gf-Cm-Am-Um-Um-Um-Uf-Um-Am-Um-Um | 375/12 | Ams-Afs-Uf-Af-Am-Af-Am-Am-Um-Gm-Cm-Um-Am-Cf-Am-Af-Am-Am-Cm-Cm-Cms-Ams-Gm | 415/24 |
| | P92-si83 -DV29 | Gms-Gms-Gm-Um-Um-Um-Uf-Gm-Uf-Am-Gf-Cm-Am-Um-Um-Um-Uf-Um-Am-Um-Um | 375/12 | Ams-Afs-Um-Af-Af-Af-Am-Am-Um-Gm-Cm-Um-Am-Cf-Am-Af-Am-Am-Cm-Cm-Cms-Ams-Gm | 416/24 |

Cell type: Hep3B cells, provided by Shanghai WuXi AppTec New Drug Development Co., Ltd. (ATCC-tings-1618164).

Hep3B cells were cultured in EMEM medium (ATCC-30-2003) containing 10% fetal bovine serum (FBS, ExCell Bio-FSP500), 1% penicillin-streptomycin (HyClone-SV30010), 1% non-essential amino acid solution (Gibco-11140-050), and 1% GlutaMAX supplement (Gibco-35050-061).

Drug solvent: DNase/RNase-free water (Nuclease-free water) , Gibco DMEM.

**2. Experimental Methods**

The inhibition of the mRNA expression of the PCSK9 gene by the test samples was detected in the HEP3B cell line using qRT-PCR.

**2.1 Cell culture**

The subcultured HEP3B cell line cells were used. The logarithmically growing cells were cultured with 10% fetal bovine serum RPMI 1640 medium (supplemented with 100 μL/mL of penicillin and streptomycin) in a cell culture incubator at 37°C containing 5% $CO_2$, and the medium was changed once a day. The cells were digested with 0.25% trypsin and centrifuged at 1000 r/min for 5 min. The supernatant was discarded, and fresh culture medium was added for subculture.

**2.2 Cell transfection**

2 μL of siRNA (1 μg/μL) was added to 18 μL of DNase/RNase-free water according to a ratio of 1:0.06 (wt/wt) and mixed well. Then 22 μL of prepared LNP was added, mixed well and left to stand at room temperature for 15 min. 12.5 μL of the mixture was used for detection of encapsulation efficiency. The final transfection volume per well was = (1 μg theoretical sample volume/siRNA purity)/drug loading concentration, and the final siRNA concentration was 0.05 nM.

Negative control group: 22 μL of prepared LNP was added to 20 μL of DNase/RNase-free water, mixed well, and left to stand at room temperature for 15 min.

After mixing using the cross method, the plate was placed in a 37°C, 5% $CO_2$ cell culture incubator for 40 h.

**2.3 PCSK9 mRNA detection**

1) RNA extraction

The steps were the same as "1) RNA extraction" in "2.3 PCSK9 mRNA detection" in Example 2.

2) RNA concentration detection

The steps were the same as those in "2) RNA concentration detection" in "2.3 PCSK9 mRNA detection" in Example 2.

3) PCSK9 mRNA quantitative detection

In a 15 mL centrifuge tube, the remaining components except primers and template were added in 7.5 × 48 = 360 portions, marked as A.

1.5 mL EP tubes were labeled. 77 μL of A and 420 ng of total RNA were added to each tube and mixed well for later use.

The upstream and downstream primers of the internal reference gene GAPDH and the target gene PCSK9 were mixed well for later use.

11 μL of B + 1 μL of C were added to each well of a PCR plate, which was covered with a sealing film, centrifuged at 3000 rpm for 1 min, and transferred to the instrument.

*This step was performed on ice to maintain low temperature conditions.

The plate was placed into the qPCR instrument and subjected to the following program:

Reverse transcription: 55°C, 15 min;

Pre-denaturation: 95°C, 30 sec;

Cyclic reaction: 95°C, 10 sec; 60°C, 35 sec; 40 cycles;

Melting curve: 95°C, 15 sec; 60°C, 60 sec; 95°C, 15 sec.

The running time was about 2 h. The experimental results were analyzed and 2-ΔΔCt was calculated.

**2.4 Data processing**

The PCSK9 mRNA expression rate (%) was calculated as follows:

Expression rate = (PCSK9 mRNA expression level/PCSK9 mRNA expression level in blank control group) × 100%;

$$\text{PCSK9 gene expression inhibition rate} = 1\text{-expression rate } (\%).$$

**3. Experimental Results**

The specific experimental results are shown in Table 29 and Table 30.

Experimental results show that the alternately modified sequences and sequences modified with specific modification templates in the present disclosure significantly improve PCSK9 inhibitory activity compared with the same or similar unmodified sequences disclosed in the prior art. For example, the inhibition rate of the unmodified sequence si84 was 38.6%. The inhibition rate after alternate modification was 61.6%, which was 23.0% higher than the unmodified sequence si84, and the inhibition rate after modification with the modification template DV26 of the present disclosure reached 86.8%, which was 48.2% higher than the unmodified sequence.

Sequence 31P disclosed in the prior art differs from sequence si31 of the present disclosure only in 2 missing bases UG at the 3'-end of the antisense chain, and the remaining bases are identical. However, the inhibition rate of the unmodified sequence si31 of the present disclosure was 10.4% higher than 31P, the inhibition rate of the alternately modified sequence was 59.5%, which was 21.2% higher than 31P, and the inhibition rate after modification with the modification template DV26 of the present disclosure reached 80.2%, which was 41.9% higher than 31P.

**(1) Compared with the identical unmodified sequences disclosed in the prior art, the alternately modified sequences and the sequences modified with the specific modification templates of the present disclosure significantly improved the inhibition rate against PCSK9 gene, which can be increased by up to 40%.**

Compared with the identical sequences disclosed in the prior art, the alternately modified sequences and the sequences modified using specific modification templates in the present disclosure significantly improved the inhibition rate against PCSK9 gene. For example, the inhibition rate of the unmodified sequence si84 was 38.6%. The inhibition rate after alternate modification was 61.6%, which was 23.0% higher than the unmodified sequence si84, and the inhibition rate after modification with the modification template DV26 of the present disclosure reached 86.8%, which was 48.2% higher than the unmodified sequence.

Table 29 Inhibition rate against the PCSK9 gene by the identical sequences of the prior art

| Sequences disclosed in the prior art | | Alternately modified sequence | | | Template-modified sequence | | |
|---|---|---|---|---|---|---|---|
| Nam e | Inhibitio n rate (%) | Sequenc e No. | Inhibitio n rate (%) | Increase (%) compare d with sequence s disclosed in the prior art | Sequence No. | Inhibitio n rate (%) | Increase (%) compare d with sequence s disclosed in the prior art |
| si81 | 50.0 | P92-si81 | 60.4 | 10.4 | P92-si81-DV2 5 | 85.5 | 35.5 |
| | | | | | P92-si81-DV2 6 | 87.7 | 37.7 |
| | | | | | P92-si81-DV2 7 | 86.7 | 36.7 |
| | | | | | P92-si81-DV2 8 | 84.6 | 34.6 |
| | | | | | P92-si81-DV2 9 | 84.0 | 34.0 |
| si84 | 38.6 | P92-si84 | 61.6 | 23.0 | P92-si84-DV2 5 | 84.9 | 46.3 |
| | | | | | P92-si84-DV2 6 | 86.8 | 48.2 |
| | | | | | P92-si84-DV2 7 | 84.5 | 45.9 |
| | | | | | P92-si84-DV2 8 | 86.2 | 47.6 |
| | | | | | P92-si84-DV2 9 | 86.4 | 47.8 |
| si3 | 51.7 | P92-si3 | 61.7 | 10.0 | P92-si3-DV25 | 77.4 | 25.7 |
| | | | | | P92-si3-DV26 | 78.5 | 26.8 |
| | | | | | P92-si3-DV27 | 84.2 | 32.5 |
| | | | | | P92-si3-DV28 | 82.5 | 30.8 |
| | | | | | P92-si3-DV29 | 85.2 | 33.5 |
| si8 | 55.2 | P92-si8 | 62.4 | 7.2 | P92-si8-DV25 | 77.2 | 22.0 |
| | | | | | P92-si8-DV26 | 78.3 | 23.1 |
| | | | | | P92-si8-DV27 | 72.0 | 16.8 |
| | | | | | P92-si8-DV28 | 73.1 | 17.9 |
| | | | | | P92-si8-DV29 | 74.8 | 19.6 |

(continued)

| Sequences disclosed in the prior art | | Alternately modified sequence | | | Template-modified sequence | | |
|---|---|---|---|---|---|---|---|
| Nam e | Inhibitio n rate (%) | Sequenc e No. | Inhibitio n rate (%) | Increase (%) compare d with sequence s disclosed in the prior art | Sequence No. | Inhibitio n rate (%) | Increase (%) compare d with sequence s disclosed in the prior art |
| si9 | 41.5 | P92-si9 | 54.7 | 13.2 | P92-si9-DV25 | 71.2 | 29.7 |
| | | | | | P92-si9-DV26 | 70.8 | 29.3 |
| | | | | | P92-si9-DV27 | 80.1 | 38.6 |
| | | | | | P92-si9-DV28 | 76.5 | 35.0 |
| | | | | | P92-si9-DV29 | 75.0 | 33.5 |
| si83 | 35.2 | P92-si83 | 51.5 | 16.3 | P92-si83-DV2 5 | 78.6 | 43.4 |
| | | | | | P92-si83-DV2 6 | 77.3 | 42.1 |
| | | | | | P92-si83-DV2 7 | 81.4 | 46.2 |
| | | | | | P92-si83-DV2 8 | 79.2 | 44.0 |
| | | | | | P92-si83-DV2 9 | 83.4 | 48.2 |

The unmodified sequences si81, si84, si3, si8, si9 and si83 disclosed in the prior art in Table 29 were identical to the sequences of the present disclosure. After alternate modification and modification with the modification templates of the present disclosure, the inhibitory effect on PCSK9 was significantly improved. For example, the inhibition rate of the unmodified sequence si84 was 38.6%. After alternate modification, the inhibition rate of P92-si84 was 61.6%, which was 23.0% higher than the unmodified sequence si84. After modification with the modification templates DV25-29 of the present disclosure, the inhibition rates of P92-si84-DV25, P92-si84-DV26, P92-si84-DV27, P92-si84-DV28 and P92-si84-DV29 reached 84.9%, 86.8%, 84.5%, 86.2% and 86.4% respectively, which were all 40% higher than the unmodified sequence si84.

**(2) Compared with the unmodified sequences disclosed in the prior art with only a slight difference, the unmodified sequences, alternately modified sequences and sequences modified with specific modification templates of the present disclosure significantly improved the inhibition rate against PCSK9 gene, which can be increased by up to 40%.**

Compared with the sequences disclosed in the prior art with only a slight difference, the unmodified sequences, alternately modified sequences and sequences modified with specific modification templates of the present disclosure significantly improved the inhibition rate against PCSK9 gene. For example, the inhibition rate of the unmodified sequence 31P was 38.3%. The inhibition rate of the similar unmodified sequence si31 of the present disclosure was 48.7%, which was 10.4% higher than 31P. The inhibition rate of the alternately modified sequence was 59.5%, which was 21.2% higher than 31P. After modification with the modification template DV26 of the present disclosure, the inhibition rate reached 80.2%, which was 41.9% higher than 31P.

Table 30 Inhibition rate against the PCSK9 gene by sequences of the present disclosure similar to those in the prior art

| Sequences disclosed in the prior art | | Unmodified sequence of the present disclosure | | | Alternately modified sequence of the present disclosure | | | Template-modified sequence of the present disclosure | | |
|---|---|---|---|---|---|---|---|---|---|---|
| Na me | Inhi bitio n rate (%) | Se qu enc e No. | Inhibi tion rate (%) | Increase (%) compare d with sequence s disclosed in the prior art | Sequ ence No. | Inhib ition rate (%) | Increas e (%) compar ed with sequenc es disclose d in the prior art | Sequenc e No. | Inhib ition rate (%) | Increas e (%) compar ed with sequenc es disclose d in the prior art |
| 5P | 41.8 | si5 | 50.1 | 8.3 | P92-s i5 | 63.8 | 22.0 | P92-si5-DV25 | 81.2 | 39.4 |
| | | | | | | | | P92-si5-DV26 | 81.5 | 39.7 |
| | | | | | | | | P92-si5-DV27 | 82.6 | 40.8 |
| | | | | | | | | P92-si5-DV28 | 77.0 | 35.2 |
| | | | | | | | | P92-si5-DV29 | 76.1 | 34.3 |
| 82P | 46.7 | si8 2 | 53.8 | 7.1 | P92-s i82 | 68.3 | 21.6 | P92-si82 -DV25 | 82.1 | 35.4 |
| | | | | | | | | P92-si82 -DV26 | 84.2 | 37.5 |
| | | | | | | | | P92-si82 -DV27 | 88.0 | 41.3 |
| | | | | | | | | P92-si82 -DV28 | 86.7 | 40.0 |
| | | | | | | | | P92-si82 -DV29 | 89.5 | 42.8 |
| 31P | 38.3 | si3 1 | 48.7 | 10.4 | P92-s i31 | 59.5 | 21.2 | P92-si31 -DV25 | 79.6 | 41.3 |
| | | | | | | | | P92-si31 -DV26 | 80.2 | 41.9 |
| | | | | | | | | P92-si31 -DV27 | 70.0 | 31.7 |
| | | | | | | | | P92-si31 -DV28 | 73.9 | 35.6 |
| | | | | | | | | P92-si31 -DV29 | 70.8 | 32.5 |

**I. Sequence Comparison**

The sequences 5P, 31P and 82P disclosed in the prior art in Table 30 are very similar to the sequences si5, si31 and si82 of the present disclosure, with only slight differences. The specific comparison is shown in the table below:

Table 31 Comparison of sequences disclosed in the prior art and sequences of the present disclosure

| Name | Sense strand | Basic sequence SEQ ID NO: | Antisense strand | Basic sequence SEQ ID NO: | Source |
|---|---|---|---|---|---|
| 5P | AAGAUCCUGCAUGUCUUCCAU | 1 | AUGGAAGACAUGCAGGAUCUU | 417 | WO2023/01700 4 Al |
| si5 | AAGAUCCUGCAUGUCUUCCAU | 1 | AUGGAAGACAUGCAGGAUCUUGG | 13 | Designed in the present disclosure |
| 31P | GGUCUGGAAUGCAAAGUCAAG | 10 | CUUGACUUUGCAUUCCAGACC | 419 | Page 25, CN115066498 A |
| si31 | GGUCUGGAAUGCAAAGUCAAG | 10 | CUUGACUUUGCAUUCCAGACCUG | 22 | Designed in the present disclosure |
| 82P | GAAGAUAUUUAUUCUGGGUUU | 4 | AAACCCAGAAUAAAUAUCUUC | 418 | WO2023/01700 4 A1 |
| si82 | GAAGAUAUUUAUUCUGGGUUU | 4 | AAACCCAGAAUAAAUAUCUUCAA | 16 | Designed in the present disclosure |

It can be seen from Table 31 that sequence 5P disclosed in the prior art differs from sequence si5 of the present disclosure only in 2 missing bases GG at the 3'-end of the antisense chain; sequence 31P differs from sequence si31 of the present disclosure only in 2 missing bases UG at the 3'-end of the antisense; sequence 82P differs from sequence si82 of the present disclosure only in 2 missing bases AA at the 3'-end of the antisense; and the remaining bases are identical.

**II. Activity Comparison**

**1) The unmodified sequences of the present disclosure significantly improved the activity compared with the similar unmodified sequences disclosed in the prior art. For example, the unmodified sequence si31 of the present disclosure improved the inhibition rate by 10.4% compared with 31P with a similar structure.**

The inhibition rate against PCSK9 gene of the unmodified sequence si5 in the present disclosure was 50.1%, which was 8.3% higher than the similar sequence 5P (inhibition rate of 41.8%) disclosed in the prior art, indicating a significant improvement.

The inhibition rate against PCSK9 gene of the unmodified sequence si82 in the present disclosure was 53.8%, which was 7.1% higher than the similar sequence 82P (inhibition rate of 46.7%) disclosed in the prior art, indicating a significant improvement.

The inhibition rate against PCSK9 gene of the unmodified sequence si31 in the present disclosure was 48.7%, which was 10.4% higher than the similar sequence 31P (inhibition rate of 38.3%) disclosed in the prior art, indicating a significant improvement.

**2) The alternately modified sequences of the present disclosure significantly improved the activity compared with the similar unmodified sequences disclosed in the prior art. For example, the alternately modified sequence P92-si5 of the present disclosure improved the inhibition rate by 22.0% compared with sequence 5P.**

After alternate modification on the basic sequence 5 of the present disclosure, the inhibition rate against PCSK9 gene by P92-si5 was 63.8%, which was 22.0% higher than the unmodified sequence 5P disclosed in the prior art, indicating a significant improvement.

After alternate modification on the basic sequence 82 of the present disclosure, the inhibition rate against PCSK9 gene by P92-si82 was 68.3%, which was 21.6% higher than the unmodified sequence 82P disclosed in the prior art,

indicating a significant improvement.

After alternate modification on the basic sequence 31 of the present disclosure, the inhibition rate against PCSK9 gene by P92-si31 was 59.5%, which was 21.2% higher than the unmodified sequence 31P disclosed in the prior art, indicating a significant improvement.

**3) The sequences modified with the modification templates of the present disclosure significantly improved the activity compared with the similar unmodified sequences disclosed in the prior art. For example, the alternately modified sequence P92-si82-DV29 of the present disclosure improved the inhibition rate by 42.8% compared with sequence 82P.**

After modification on basic sequence 5 with modification templates 25-29 of the present disclosure, the inhibition rate can be increased by more than 30% compared with the unmodified sequence 5P disclosed in the prior art. For example, there was an increase in 40.8% by P92-si5-DV27, indicating a significant improvement.

After modification on basic sequence 82 with modification templates 25-29 of the present disclosure, the inhibition rate can be increased by more than 30% compared with the unmodified sequence 82P disclosed in the prior art. For example, there was an increase in 42.8% by P92-si82-DV29, indicating a significant improvement.

After modification on basic sequence 31 with modification templates 25-29 of the present disclosure, the inhibition rate can be increased by more than 30% compared with the unmodified sequence 31P disclosed in the prior art. For example, there was an increase in 41.9% by P92-si31-DV26, indicating a significant improvement.

It can be concluded that compared with the similar unmodified sequences disclosed in the prior art, the unmodified sequences, the alternately modified sequences and the template-modified sequences of the present disclosure can significantly enhance the inhibitory activity against PCSK9, and the inhibition rate can be increased by up to 40%..

**Summary:**

(1) Compared with the identical sequences disclosed in the prior art, the alternately modified sequences and the sequences modified using specific modification templates in the present disclosure significantly improved the inhibition rate against PCSK9 gene. For example, the inhibition rate of the unmodified sequence si84 was 38.6%. The inhibition rate after alternate modification was 61.6%, which was 23.0% higher than the unmodified sequence si84, and the inhibition rate after modification with the modification template DV26 of the present disclosure reached 86.8%, which was 48.2% higher than the unmodified sequence.

(2) Compared with similar sequences disclosed in the prior art, the unmodified sequences, alternately modified sequences and modified sequences using specific modification templates in the present disclosure significantly improve the inhibitory effect on the PCSK9 gene. For example, the unmodified sequence si31 of the present disclosure improved the inhibition rate by 10.4% compared with 31P with a similar sequence disclosed in the prior art. The alternately modified sequence P92-si5 of the present disclosure improved the inhibition rate by 22.0% compared with sequence 5P with a sequence disclosed in the prior art similar to sequence si5. The sequence P92-si82-DV29 modified on sequence si82 of the present disclosure with the modification template DV29 of the present disclosure improved the inhibition rate by 42.8% compared with the unmodified sequence 82P with a sequence disclosed in the prior art similar to sequence si82.

**Example 6: Inhibitory effects on PCSK9 and off-target genes by specific sequences designed to prevent off-target genes**

In the practical application of siRNA, there are a large number of cases where the expression of non-target mRNA that is only partially complementary to the guide strand (antisense strand) is inhibited. Alnylam's research shows that hepatotoxicity of n-acetylgalactosamine (GalNAc)-conjugated siRNA is mainly attributed to off-target effects resulting in gene suppression of the wrong target through a microRNA (miRNA)-like recognition mechanism.

To solve this problem, in the latest fifth-generation template design, Alnylam has used a glycol nucleic acid (GNA) modification at position 7 of the siRNA antisense strand to disrupt the seed region of the antisense strand, thereby significantly reducing off-target effects and alleviating hepatotoxicity. Such modifications can affect the way siRNA binds to non-designed targets through seed region recognition and inhibit off-target effects.

Natural 5'-end phosphorylation or simple direct 5'-end phosphorylation may be dephosphorylated in cells. Direct 5'-end phosphorylated oligonucleotide chains can be dephosphorylated by 90% after circulating in the blood for 2 hours and completely disappear after 24 hours. 5'-end phosphorylation design (5'-E-VP) uses E-vinyl phosphonate to replace the bridging oxygen, which has the best phosphorylation effect and high stability.

In summary, in order to reduce the off-target effect of the sequence and examine the effect of 5'-end phosphorylation on the off-target effect, the basic sequences 5, 51, 81 and 84 modified with DV25-29 were used in this example, and 5'-E-VP modification and GNA anti-off-target modification at position 7 were added to the 5'-end of the antisense strand.

Moreover, comparison was conducted between the sequences P92-si3, P92-si8, P92-si21, P92-si31, P92-si73 and P92-si83 modified alternately with 2'-methoxy and 2'-fluoro, and sequences P92-si3+, P92-si8+, P92-si21+, P92-si31+, P92-si73+ and P92-si83+, which were the above sequences but contained GNA anti-off-target modification at position 7 of the antisense chain, in order to detect off-target effects.

Experimental results show that at a concentration of 10 nM, the sequences with the off-target design had a significant inhibitory effect on the target gene PCSK9, and the inhibitory effect on off-target genes was significantly reduced. It demonstrates that the use of anti-off-target design will not affect the inhibitory effect of the alternately modified and template-modified sequences of the present disclosure on the PCSK9 gene, but can also significantly inhibit the off-target effect.

**1. Experimental Materials**

**1) Test samples:**

Alternately modified sequences: sequences P92-si3, P92-si8, P92-si9, P92-si21, P92-si31, P92-si73 and P92-si83 with alternate modification.

Sequences with alternate modification and anti-off-target design: P92-si3+, P92-si8+, P92-si9+, P92-si21+, P92-si31+, P92-si73+ and P92-si83+ (Table 32).

Sequences obtained by modifying basic sequences 5, 84, 81 and 51 with template modification, with template modification and 5'-E-VP modification, with template modification and anti-off-target design, and with template modification, 5'-E-VP modification and anti-off-target design (Table 33).

2) Sequence synthesis:

**I. Alternately modified sequences and template-modified sequences**

The synthesis method refers to Example 1.

**II. siRNA sequences containing 5'-E-VP**

Referring to Example 1, the siRNA sequence was synthesized. When synthesizing the base at the 5'-end of the antisense strand (the last base), a monomer containing a phosphonate group at the 5'-end was used, for example, vinyl-(E)-phosphonate-A-OMe phosphoramidite monomer (Formula 9), vinyl-(E)-phosphonate-U-OMe phosphoramidite monomer (Formula 10), which have the following structures:

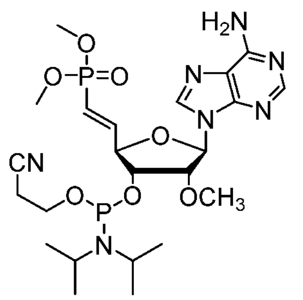

Formula 9

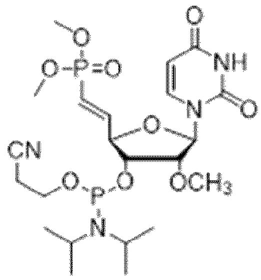

Formula 10

**II. siRNA sequences with anti-off-target design**

Referring to Example 1, the siRNA sequence was synthesized. When synthesizing the 7th base at the 5'-end of the antisense strand, GNA monomers were used to synthesize the sequence, which have the following structures:

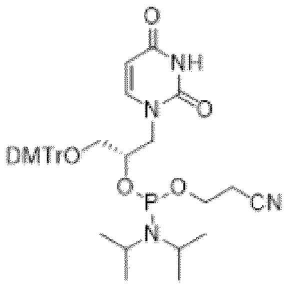

(*S*)-GNA-U

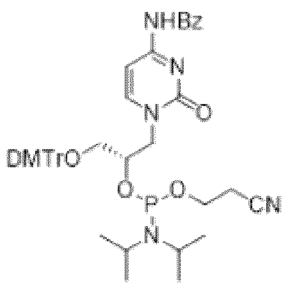

(*S*)-GNA-C

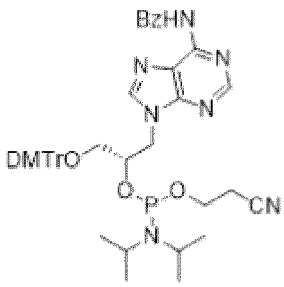

(*S*)-GNA-A

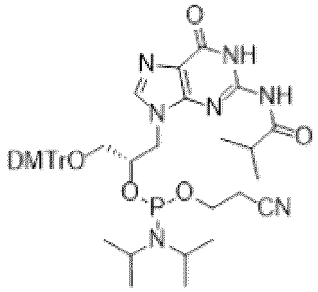

(*S*)-GNA-G

Table 32 Sequences with alternative modification and anti-off-target modification

| Sequence No. | Sense strand 5'-3' | Modified sequence SEQ ID NO:/Corr esponding basic sequence SEQ ID NO: | Antisense strand 5'-3' | Modified sequence SEQ ID NO:/Corr esponding basic sequence SEQ ID NO: |
|---|---|---|---|---|
| P92-si3+ | Cfs-Ams-Cf-Cm-Af-Am-Gf-Am-Uf-Cm-Cf-Um-Gf-Cm-Af-Um-Gf-Um-Cf-Um-Uf | 174/6 | Ams-Afs-Gm-Af-Cm-Af-Ugna-Gf-Cm-Af-Gm-Gf-Am-Uf-Cm-Uf-Um-Gf-Gm-Uf-Gms-Afs-Gm | 420/18 |
| P92-si8+ | Cfs-Ums-Cf-Am-Uf-Am-Gf-Gm-Cf-Cm-Uf-Gm-Gf-Am-Gf-Um-Uf-Um-Af-Um-Uf | 175/7 | Ams-Afs-Um-Af-Am-Af-Cgna-Uf-Cm-Cf-Am-Gf-Gm-Cf-Cm-Uf-Am-Uf-Gm-Af-Gms-Gfs-Gm | 421/19 |
| P92-si9+ | Gfs-Gms-Cf-Cm-Uf-Gm-Gf-Am-Gf-Um-Uf-Um-Af-Um-Uf-Cm-Gf-Gm-Af-Am-Af | 176/8 | Ums-Ufs-Um-Cf-Cm-Gf-Agna-Af-Um-Af-Am-Af-Cm-Uf-Cm-Cf-Am-Gf-Gm-Cf-Cms-Ufs-Am | 422/20 |
| P92-si21+ | Cfs-Ams-Gf-Cm-Af-Cm-Cf-Um-Gf-Cm-Uf-Um-Uf-Gm-Uf-Gm-Uf-Cm-Af-Cm-Af | 177/9 | Ums-Gfs-Um-Gf-Am-Cf-Agna-Cf-Am-Af-Am-Gf-Cm-Af-Gm-Gf-Um-Gf-Cm-Uf-Gms-Cfs-Am | 423/21 |
| P92-si31+ | Gfs-Gms-Uf-Cm-Uf-Gm-Gf-Am-Af-Um-Gf-Cm-Af-Am-Af-Gm-Uf-Cm-Af-Am-Gf | 178/10 | Cms-Ufs-Um-Gf-Am-Cf-Ugna-Uf-Um-Gf-Cm-Af-Um-Uf-Cm-Cf-Am-Gf-Am-Cf-Cms-Ufs-Gm | 424/22 |
| P92-si73+ | Gfs-Cms-Gf-Gm-Af-Gm-Af-Um-Gf-Cm-Uf-Um-Cf-Um-Af-Am-Gf-Gm-Cf-Am-Uf | 179/11 | Ams-Ufs-Gm-Cf-Cm-Uf-Ugna-Af-Gm-Af-Am-Gf-Cm-Af-Um-Cf-Um-Cf-Cm-Gf-Cms-Cfs-Am | 425/23 |
| P92-si83+ | Gfs-Gms-Gf-Um-Uf-Um-Uf-Gm-Uf-Am-Gf-Cm-Af-Um-Uf-Um-Uf-Um-Af-Um-Uf | 180/12 | Ams-Afs-Um-Af-Am-Af-Agna-Af-Um-Gf-Cm-Uf-Am-Cf-Am-Af-Am-Af-Cm-Cf-Cms-Afs-Gm | 426/24 |

The above sequences all adopted the scheme of alternate modification of 2'-methoxy (2'-OMe) and 2'-fluoro (2'-F), wherein:

(1) the nucleotides in the sense strand were modified with 2'-F in odd-numbered positions and with 2'-OMe in even-numbered positions;

(2) the nucleotides in the antisense strand were modified with 2'-OMe in odd-numbered positions and with 2'-F in even-numbered positions;

(3) the 5'-end of the sense strand and both the 5'- and 3'-ends of the antisense strand were each modified with two thio-modifications;

(4) the 7th base of the antisense strand (5'-3' direction) was modified with GNA.

Table 33 Sequences with template modification, 5'-E-VP modification, and anti-off-target design

| Sequ ence No. | Sense strand 5'-3' | Modifie d sequen ce SEQ ID NO:/Co rrespon ding basic sequen ce SEQ ID NO: | Antisense strand 5'-3' | Modifie d sequen ce SEQ ID NO:/Co rrespon ding basic sequen ce SEQ ID NO: |
|---|---|---|---|---|
| D5-DV2 5 | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gm-Am-Af-Gm-Am-Cm-Am-Um-Gm-Cm-Af-Gm-Gf-Am-Um-Cm-Um-Ums-Gms-Gm | 376/13 |
| D5-DV2 5+ | Ams-Ams-Gm-Am-Um-Cm-Cf-Um-Gf-Cf-Af-Um-Gm-Um-Cm-Um-Uf-Cm-Cm-Am-Um | 337/1 | Ams-Ufs-Gm-Gm-Am-Af-Ggna-Am-Cm-Am-Um-Gm-Cm-Af-Gm-Gf-Am-Um-Cm-Um-Ums-Gms-Gm | 427/13 |
| D5-DV2 5P | Ams-Ams-Gm-Am-Um-Cm-Cf-Um-Gf-Cf-Af-Um-Gm-Um-Cm-Um-Uf-Cm-Cm-Am-Um | 337/1 | AmsEVP-Ufs-Gm-Gm-Am-Af-Gm-Am-Cm-Am-Um-Gm-Cm-Af-Gm-Gf-Am-Um-Cm-Um-Ums-Gms-Gm | 428/13 |
| D5-DV2 5+P | Ams-Ams-Gm-Am-Um-Cm-Cf-Um-Gf-Cf-Af-Um-Gm-Um-Cm-Um-Uf-Cm-Cm-Am-Um | 337/1 | AmsEVP-Ufs-Gm-Gm-Am-Af-Ggna-Am-Cm-Am-Um-Gm-Cm-Af-Gm-Gf-Am-Um-Cm-Um-Ums-Gms-Gm | 429/13 |
| D51-DV2 6 | Ums-Gms-Gm-Am-Gm-Gm-Cf-Um-Uf-Af-Gf-Cm-Um-Um-Um-Cm-Uf-Gm-Gm-Am-Um | 342/2 | Ams-Ufs-Cf-Cf-Am-Gf-Am-Am-Am-Gm-Cm-Um-Am-Af-Gm-Cf-Cm-Um-Cm-Cm-Ams-Ums-Um | 381/14 |
| D51-DV2 6+ | Ums-Gms-Gm-Am-Gm-Gm-Cf-Um-Uf-Af-Gf-Cm-Um-Um-Um-Cm-Uf-Gm-Gm-Am-Um | 342/2 | Ams-Ufs-Cf-Cf-Am-Gf-Agna-Am-Am-Gm-Cm-Um-Am-Af-Gm-Cf-Cm-Um-Cm-Cm-Ams-Ums-Um | 430/14 |
| D51-DV2 6P | Ums-Gms-Gm-Am-Gm-Gm-Cf-Um-Uf-Af-Gf-Cm-Um-Um-Um-Cm-Uf-Gm-Gm-Am-Um | 342/2 | AmsEVP-Ufs-Cf-Cf-Am-Gf-Am-Am-Am-Gm-Cm-Um-Am-Af-Gm-Cf-Cm-Um-Cm-Cm-Ams-Ums-Um | 431/14 |
| D51-DV2 6+P | Ums-Gms-Gm-Am-Gm-Gm-Cf-Um-Uf-Af-Gf-Cm-Um-Um-Um-Cm-Uf-Gm-Gm-Am-Um | 342/2 | AmsEVP-Ufs-Cf-Cf-Am-Gf-Agna-Am-Am-Gm-Cm-Um-Am-Af-Gm-Cf-Cm-Um-Cm-Cm-Ams-Ums-Um | 432/14 |
| D81-DV2 5 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Am-Um-Cf-Um-Um-Cm-Am-Am-Gm-Um-Uf-Am-Cf-Am-Am-Am-Am-Gms-Cms-Am | 384/15 |

(continued)

| Sequ ence No. | Sense strand 5'-3' | Modifie d sequen ce SEQ ID NO:/Co rrespon ding basic sequen ce SEQ ID NO: | Antisense strand 5'-3' | Modifie d sequen ce SEQ ID NO:/Co rrespon ding basic sequen ce SEQ ID NO: |
|---|---|---|---|---|
| D81-DV2 5+ | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Am-Um-Cf-Ugna-Um-Cm-Am-Am-Gm-Um-Uf-Am-Cf-Am-Am-Am-Am-Gms-Cms-Am | 433/15 |
| D81-DV2 5P | Cms-Ums-Um-Um-Um-Gm-Uf-Am-Af-Cf-Uf-Um-Gm-Am-Am-Gm-Af-Um-Am-Um-Um | 347/3 | AmsEVP-Afs-Um-Am-Um-Cf-Um-Um-Cm-Am-Am-Gm-Um-Uf-Am-Cf-Am-Am-Am-Am-Gms-Cms-Am | 434/15 |
| D81-DV2 5+P | Cms-Ums-Um-Um-Um-Gm-Uf-Am-Af-Cf-Uf-Um-Gm-Am-Am-Gm-Af-Um-Am-Um-Um | 347/3 | AmsEVP-Afs-Um-Am-Um-Cf-Ugna-Um-Cm-Am-Am-Gm-Um-Uf-Am-Cf-Am-Am-Am-Am-Gms-Cms-Am | 435/15 |
| D81-DV2 7 | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Af-Uf-Cf-Um-Um-Cm-Am-Am-Gm-Um-Uf-Am-Cf-Am-Am-Am-Am-Gms-Cms-Am | 386/15 |
| D81-DV2 7+ | Cms-Ums-Um-Um-Um-Gm-Uf-Am-Af-Cf-Uf-Um-Gm-Am-Am-Gm-Af-Um-Am-Um-Um | 347/3 | Ams-Afs-Um-Af-Uf-Cf-Ugna-Um-Cm-Am-Am-Gm-Um-Uf-Am-Cf-Am-Am-Am-Am-Gms-Cms-Am | 436/15 |
| D81-DV2 7P | Cms-Ums-Um-Um-Um-Gm-Uf-Am-Af-Cf-Uf-Um-Gm-Am-Am-Gm-Af-Um-Am-Um-Um | 347/3 | AmsEVP-Afs-Um-Af-Uf-Cf-Um-Um-Cm-Am-Am-Gm-Um-Uf-Am-Cf-Am-Am-Am-Am-Gms-Cms-Am | 437/15 |
| D81-DV2 7+P | Cms-Ums-Um-Um-Um-Gm-Uf-Am-Af-Cf-Uf-Um-Gm-Am-Am-Gm-Af-Um-Am-Um-Um | 347/3 | AmsEVP-Afs-Um-Af-Uf-Cf-Ugna-Um-Cm-Am-Am-Gm-Um-Uf-Am-Cf-Am-Am-Am-Am-Gms-Cms-Am | 438/15 |
| D81-DV2 8 | Cms-Ums-Um-Um-Um-Gm-Uf-Am-Af-Cm-Uf-Um-Gm-Am-Am-Gm-Af-Um-Am-Um-Um | 348/3 | Ams-Afs-Uf-Af-Um-Cf-Um-Um-Cm-Am-Am-Gm-Um-Uf-Am-Cf-Am-Am-Am-Am-Gms-Cms-Am | 385/15 |
| D81-DV2 8+ | Cms-Ums-Um-Um-Um-Gm-Uf-Am-Af-Cm-Uf-Um-Gm-Am-Am-Gm-Af-Um-Am-Um-Um | 348/3 | Ams-Afs-Uf-Af-Um-Cf-Ugna-Um-Cm-Am-Am-Gm-Um-Uf-Am-Cf-Am-Am-Am-Am-Gms-Cms-Am | 439/15 |

(continued)

| Sequ ence No. | Sense strand 5'-3' | Modifie d sequen ce SEQ ID NO:/Co rrespon ding basic sequen ce SEQ ID NO: | Antisense strand 5'-3' | Modifie d sequen ce SEQ ID NO:/Co rrespon ding basic sequen ce SEQ ID NO: |
|---|---|---|---|---|
| D81-DV2 8P | Cms-Ums-Um-Um-Um-Gm-Uf-Am-Af-Cm-Uf-Um-Gm-Am-Am-Gm-Af-Um-Am-Um-Um | 348/3 | AmsEVP-Afs-Uf-Af-Um-Cf-Um-Um-Cm-Am-Am-Gm-Um-Uf-Am-Cf-Am-Am-Am-Am-Gms-Cms-Am | 440/15 |
| D81-DV2 8+P | Cms-Ums-Um-Um-Um-Gm-Uf-Am-Af-Cm-Uf-Um-Gm-Am-Am-Gm-Af-Um-Am-Um-Um | 348/3 | AmsEVP-Afs-Uf-Af-Um-Cf-Ugna-Um-Cm-Am-Am-Gm-Um-Uf-Am-Cf-Am-Am-Am-Am-Gms-Cms-Am | 441/15 |
| D84-DV2 6 | Gms-Ums-Um-Um-Um-Gm-Uf-Am-Gf-Cf-Af-Um-Um-Um-Um-Um-Af-Um-Um-Am-Am | 357/5 | Ums-Ufs-Af-Af-Um-Af-Am-Am-Am-Am-Um-Gm-Cm-Uf-Am-Cf-Am-Am-Am-Am-Cms-Cms-Cm | 393/17 |
| D84-DV2 6+ | Gms-Ums-Um-Um-Um-Gm-Uf-Am-Gf-Cf-Af-Um-Um-Um-Um-Um-Af-Um-Um-Am-Am | 357/5 | Ums-Ufs-Af-Af-Um-Af-Agna-Am-Am-Am-Um-Gm-Cm-Uf-Am-Cf-Am-Am-Am-Am-Cms-Cms-Cm | 442/17 |
| D84-DV2 6P | Gms-Ums-Um-Um-Um-Gm-Uf-Am-Gf-Cf-Af-Um-Um-Um-Um-Um-Af-Um-Um-Am-Am | 357/5 | UmsEVP-Ufs-Af-Af-Um-Af-Am-Am-Am-Am-Um-Gm-Cm-Uf-Am-Cf-Am-Am-Am-Am-Cms-Cms-Cm | 443/17 |
| D84-DV2 6+P | Gms-Ums-Um-Um-Um-Gm-Uf-Am-Gf-Cf-Af-Um-Um-Um-Um-Um-Af-Um-Um-Am-Am | 357/5 | UmsEVP-Ufs-Af-Af-Um-Af-Agna-Am-Am-Am-Um-Gm-Cm-Uf-Am-Cf-Am-Am-Am-Am-Cms-Cms-Cm | 444/17 |
| D84-DV2 7 | Gms-Ums-Um-Um-Um-Gm-Uf-Am-Gf-Cf-Af-Um-Um-Um-Um-Um-Af-Um-Um-Am-Am | 357/5 | Ums-Ufs-Am-Af-Uf-Af-Am-Am-Am-Am-Um-Gm-Cm-Uf-Am-Cf-Am-Am-Am-Am-Cms-Cms-Cm | 394/17 |
| D84-DV2 7+ | Gms-Ums-Um-Um-Um-Gm-Uf-Am-Gf-Cf-Af-Um-Um-Um-Um-Um-Af-Um-Um-Am-Am | 357/5 | Ums-Ufs-Am-Af-Uf-Af-Agna-Am-Am-Am-Um-Gm-Cm-Uf-Am-Cf-Am-Am-Am-Am-Cms-Cms-Cm | 445/17 |
| D84-DV2 7P | Gms-Ums-Um-Um-Um-Gm-Uf-Am-Gf-Cf-Af-Um-Um-Um-Um-Um-Af-Um-Um-Am-Am | 357/5 | UmsEVP-Ufs-Am-Af-Uf-Af-Am-Am-Am-Am-Um-Gm-Cm-Uf-Am-Cf-Am-Am-Am-Am-Cms-Cms-Cm | 446/17 |

(continued)

| Sequ ence No. | Sense strand 5'-3' | Modifie d sequen ce SEQ ID NO:/Co rrespon ding basic sequen ce SEQ ID NO: | Antisense strand 5'-3' | Modifie d sequen ce SEQ ID NO:/Co rrespon ding basic sequen ce SEQ ID NO: |
|---|---|---|---|---|
| D84-DV2 7+P | Gms-Ums-Um-Um-Um-Gm-Uf-Am-Gf-Cf-Af-Um-Um-Um-Um-Um-Af-Um-Um-Am-Am | 357/5 | UmsEVP-Ufs-Am-Af-Uf-Af-Agna-Am-Am-Am-Um-Gm-Cm-Uf-Am-Cf-Am-Am-Am-Am-Cms-Cms-Cm | 447/17 |

Cell type: Hep3B cells, provided by Shanghai WuXi AppTec New Drug Development Co., Ltd. (ATCC-tings-1618164).
Hep3B cells were cultured in EMEM medium (ATCC-30-2003) containing 10% fetal bovine serum (FBS, ExCell Bio-FSP500), 1% penicillin-streptomycin (HyClone-SV30010), 1% non-essential amino acid solution (Gibco-11140-050), and 1% GlutaMAX supplement (Gibco-35050-061).
Drug solvent: DNase/RNase-free water, Gibco DMEM.

**2. Experimental Methods**

**1) Compound dilution**

For off-target experiments, the test samples were diluted to a concentration of 10 nM and tested in triplicate wells.

Digestion and count of Hep3B cells:

Hep3B cells with a density of 80% were taken out, rinsed with Dulbecco's phosphate buffered saline (DPBS), and digested with 0.05% trypsin for 3-10 min. After the cells were collected, they were counted with Countstar Rigel S2 and the cell density was adjusted to $2\times10^5$ /mL.

**2) Preparation of transfection reagent**

RNAiMAX transfection reagent was prepared. The appropriate volume prepared in a 15 mL centrifuge tube at a ratio of RNAiMAX: Opti-MEM = 3:97, mixed well by vortex for 15 sec, and incubated at room temperature for 15 min. 40 μL RNAiMAX Opti-MEM was added to each well at the corresponding position, and 40 μL of the diluted compound of the corresponding concentration was added to the corresponding well of the dilution plate, mixed well and incubated for 15 min.

**3) Cell plating**

Hep3B cells ($2\times10^4$ cells/well) were plated into 96-well cell plates. The siRNA compounds were mixed with RNAiMAX Opti-MEM transfection reagent and added to the cells in each well. A control group without siRNA compounds containing RNAiMAX Opti-MEM was also set up.
Anti-off-target experiment: The mixture of compounds and RNAiMAX Opti-MEM was taken out from the dilution plate and added to a 96-well cell culture plate (20 μL/well). Then 100 μL/well cells were added to the 96-well plate, and the final volume per well was 120 μL. After plating, the plate was placed in a 5% $CO_2$, 37°C incubator for 24 h.

**4) RNA extraction and reverse transcription**

24 h after transfection, the medium was removed and the cells were collected for RNA extraction. Total RNA was extracted using RNeasy® 96 Kit (QIAGEN-74182) according to the kit instructions. Subsequently, cDNA was synthesized using FastKing RT Kit (With gDNase) (TIANGEN-KR116-02) according to the instructions.

**5) RT-qPCR**

The target cDNA was detected by qPCR, and GAPDH cDNA was detected in parallel as an internal control. 8 μL of the prepared qPCR reaction solution and 2 μL of sample cDNA were added to 384 wells. The TaqMan qPCR reaction program included: heating at 95°C for 10 min, and then entering the cycle mode of heating at 95°C for 15 s and then 60°C for 1 min, for a total of 40 cycles. The SYBR qPCR reaction program included: heating at 50°C for 2 min, heating at 95°C for 10 min, and then entering the cycle mode of heating at 95°C for 15 sec and then 60°C for 1 min, for a total of 40 cycles; and the final melting curve was heated at 95°C for 15 sec, 60°C for 1 min, and 95°C for 15 sec.

6) **Data analysis**

The RNA expression level of the target gene in the sample was calculated based on the Ct value of each sample using the ΔΔCt relative quantitative method. The relative expression of the target gene was expressed as 2-ΔΔCT.
The calculation formulas are as follows:

$$\Delta CT = \text{average Ct value of target gene - average Ct value of internal reference gene;}$$

$$\Delta\Delta CT = \Delta CT\ (\text{medication group}) - \Delta CT\ (\text{RNAiMAX control group});$$

$$\text{Relative expression level of target gene mRNA} = 2\text{-}\Delta\Delta CT$$

Inhibition rate = (1 - relative expression level of sample / average expression level of RNAiMAX control) × 100%

**3. Experimental Results**

(1) Different modified sequences exhibited a significant inhibitory effect on the PCSK9 gene. For example, the inhibition rate of alternately modified P92-si3 reached 75.6%, and the inhibition rate of sequence D84-DV27P with template modification and 5'-E-VP modification was as high as 94.4%.

The inhibition rates of all tested samples on the PCSK9 gene are shown in Tables 34-36.

All alternately modified sequences exhibited a significant inhibitory effect on PCSK9 at a concentration of 10 nM. For example, the inhibition rate of the alternately modified sequence P92-si3 reached 75.6%.

**I. The alternately modified sequence exhibited a significant inhibitory effect on the PCSK9 gene, with the inhibition rate exceeding 60%, among which P92-si3 reached 75.6%.**

Table 34 Inhibition rate against the PCSK9 gene by alternately modified sequences

| Basic sequence NO. | Alternately modified sequence No. | Inhibition rate (%) |
|---|---|---|
| 3 | P92-si3 | 75.6 |
| 8 | P92-si8 | 74.0 |
| 9 | P92-si9 | 69.7 |
| 21 | P92-si21 | 67.3 |
| 31 | P92-si31 | 72.3 |
| 51 | P92-si51 | 70.1 |
| 73 | P92-si73 | 69.9 |
| 83 | P92-si83 | 66.5 |

As can be seen from the table above, at a concentration of 10 nM, the alternately modified sequences P92-si3, P92-si8, P92-si9, P92-si21, P92-si31, P92-si51, P92-si73 and P92-si83 all exhibited a significant inhibitory effect on the PCSK9 gene, with the inhibition rate exceeding 60%, among which P92-si3 had the highest inhibition rate, reaching 75.6%.

**II. The template-modified sequences with anti-off-target design or/and 5'-E-VP modification exhibited a significant inhibitory effect on the PCSK9 gene. For example, the inhibition rate of sequence D84-DV27P with template modification and 5'-E-VP modification was as high as 94.4%.**

Table 35 Inhibition rate against the PCSK9 gene by template-modified sequences and anti-off-target design or/and 5'-E-VP modifications thereof

| Basic sequence NO. | Alternately modified sequence No. | Modification method | Inhibition rate (%) |
|---|---|---|---|
| 5 | D5-DV25 | Template modification | 87.2 |
| | D5-DV25+ | Template modification and anti-off-target design | 82.2 |
| | D5-DV25P | Template modification and 5'-E-VP | 87.9 |
| | D5-DV25+P | Template modification, anti-off-target design and 5'-E-VP | 82.7 |
| 51 | D51-DV26 | Template modification | 90.7 |
| | D51-DV26+ | Template modification and anti-off-target design | 87.2 |
| | D51-DV26P | Template modification and 5'-E-VP | 91.8 |
| | D51-DV26+P | Template modification, anti-off-target design and 5'-E-VP | 84.0 |
| 81 | D81-DV25 | Template modification | 92.6 |
| | D81-DV25+ | Template modification and anti-off-target design | 88.1 |
| | D81-DV25P | Template modification and 5'-E-VP | 93.3 |
| | D81-DV25+P | Template modification, anti-off-target design and 5'-E-VP | 87.5 |
| 81 | D81-DV27 | Template modification | 93.0 |
| | D81-DV27+ | Template modification and anti-off-target design | 86.5 |
| | D81-DV27P | Template modification and 5'-E-VP | 93.7 |
| | D81-DV27+P | Template modification, anti-off-target design and 5'-E-VP | 87.8 |
| 81 | D81-DV28 | Template modification | 90.7 |
| | D81-DV28+ | Template modification and anti-off-target design | 89.2 |
| | D81-DV28P | Template modification and 5'-E-VP | 91.6 |
| | D81-DV28+P | Template modification, anti-off-target design and 5'-E-VP | 87.8 |
| 84 | D84-DV26 | Template modification | 92.0 |
| | D84-DV26+ | Template modification and anti-off-target design | 90.1 |
| | D84-DV26P | Template modification and 5'-E-VP | 93.1 |
| | D84-DV26+P | Template modification, anti-off-target design and 5'-E-VP | 89.8 |
| 84 | D84-DV27 | Template modification | 91.5 |
| | D84-DV27+ | Template modification and anti-off-target design | 89.2 |
| | D84-DV27P | Template modification and 5'-E-VP | 94.4 |
| | D84-DV27+P | Template modification, anti-off-target design and 5'-E-VP | 90.4 |

1) After the basic sequences 5, 51, 81 and 84 were modified with the modification templates of the present disclosure, they exhibited a significant inhibitory effect on the PCSK9 gene, with an inhibition rate exceeding 90%. Moreover, the inhibitory effect was further enhanced compared to alternately modified sequences. For example, after the basic sequence 51 was alternately modified, the inhibition rate of P92-si51 was 70.1% (see Table 34). After modification with template DV26, the inhibition rate by D51-DV26 reached 90.7%, which was 20.6% higher than the alternately modified sequence, indicating a significantly improvement (FIG. 8).

2) After template modification and then anti-off-target design, the sequences had a significant inhibitory activity on PCSK9 gene expression. For example, after the basic sequence 84 was modified with template DV26 and then with anti-off-target design, the inhibition rate by D84-DV26+ was as high as 90.1%.

3) After template modification and then 5'-E-VP modification, the inhibitory effect on the PCSK9 gene can be

enhanced. For example, after the basic sequence 84 was modified with template DV27, the inhibition rate by D84-DV27 was 91.5%. After further 5'-E-VP modification, the inhibition rate of D84-DV27P was 94.4%, which was increased by 2.9%.

4) After template modification, anti-off-target design and 5'-E-VP modification, the inhibitory activity on the PCSK9 gene could reach more than 90%. For example, after the basic sequence 84 was modified with template DV27, anti-off-target and 5'-E-VP modification, the inhibition rate of D84-DV27+P could reach 90.4%.

It can be concluded that modification with modification templates DV25-29 of the present disclosure can further enhance the inhibitory effect compared to the alternately modified sequences. Moreover, modification with modification templates DV25-29 of the present disclosure and further anti-off-target design or/and 5'-E-VP modification achieved a significant inhibitory effect on PCSK9 gene expression, with an inhibition rate exceeding 90%.

**(2) Modification with any one or more of the various modification methods of the present disclosure and further anti-off-target design achieved a significant anti-off-target effect on off-target genes and reduced the inhibitory effect on off-target genes.**

Experimental results show that:

1) The alternately modified or template-modified sequences of the present disclosure with an anti-off-target design can reduce the inhibitory effect on off-target genes, indicating a significant anti-off-target effect;

2) The template-modified sequences with both an anti-off-target design and 5'-E-VP modification significantly reduced the inhibition rate of off-target genes, indicating a significant anti-off-target effect.

**I. The alternately modified sequences of the present disclosure with an anti-off-target design exhibited a significant anti-off-target effect and can reduce the inhibition rate of off-target genes by 20%.**

Table 36 Inhibition rate of off-target genes by alternate modification sequences and anti-off-target modifications thereof

| Basic sequence NO. | Sequence No. | Off-target gene | Average inhibition rate (%) | Reduction (%) in inhibition rate compared with no anti-off-target design |
|---|---|---|---|---|
| 3 | P92-si3 | AARSD1 | 43.5 | - |
| | P92-si3+ | | 23.5 | 20.0 |
| 3 | P92-si3 | ACAP2 | 56.0 | - |
| | P92-si3+ | | 35.5 | 20.5 |
| 8 | P92-si8 | AIFM2 | 17.3 | - |
| | P92-si8+ | | 10.1 | 7.2 |
| 31 | P92-si31 | ERG | 16.8 | - |
| | P92-si31+ | | 8.4 | 8.4 |
| 9 | P92-si9 | MACF1 | 24.9 | - |
| | P92-si9+ | | 20.1 | 4.8 |
| 8 | P92-si8 | RETREG2 | 14.1 | - |
| | P92-si8+ | | 8.2 | 5.9 |

As can be seen from Table 36, after the alternately modified sequences of the present disclosure adopted an anti-off-target design, the inhibition rate of off-target genes was reduced, indicating an anti-off-target effect. For example, the alternately modified sequence P92-si3+ with an anti-off-target design reduced the inhibition rate of the off-target gene AARSD1 by 20.0% and reduced the inhibition rate of the off-target gene ACAP2 by 20.5% compared with sequence P92-si3 with no anti-off-target design, indicating a significant reduction.

**II. The sequences modified with the modification templates of the present disclosure with an anti-off-target design exhibited a significant anti-off-target effect and can reduce the inhibition rate of off-target genes by**

**up to 73.6%.**

Table 37 Inhibition rate of off-target genes by template-modified sequences and anti-off-target or/and 5'-E-VP modifications thereof

| Basic sequence NO. | Sequence No. | Off-target gene | Inhibition rate of off-target gene (%) | Reduction (%) in inhibition rate compared with no anti-off-target design |
|---|---|---|---|---|
| 5 | D5-DV25 | DTWD1 | 26.4 | 0 |
| | D5-DV25+ | | 15.5 | 10.9 |
| | D5-DV25P | | 40 | -13.6 |
| | D5-DV25+P | | 18.7 | 7.7 |
| 51 | D51-DV26 | NEPRO | 37.8 | 0 |
| | D51-DV26+ | | 11.9 | 25.9 |
| | D51-DV26P | | 18.1 | 19.7 |
| | D51-DV26+P | | -4.8 | 42.6 |
| 51 | D51-DV26 | KIF1B | 35.9 | 0 |
| | D51-DV26+ | | 13.1 | 22.8 |
| | D51-DV26P | | 24.8 | 11.1 |
| | D51-DV26+P | | -5.7 | 41.6 |
| 81 | D81-DV25 | DSE | 56.2 | 0 |
| | D81-DV25+ | | 31.9 | 24.2 |
| | D81-DV25P | | 45.1 | 11.0 |
| | D81-DV25+P | | 51.0 | 5.1 |
| | D81-DV27 | | 44.5 | 0 |
| | D81-DV27+ | | 23.7 | 20.8 |
| | D81-DV27P | | 43.7 | 0.7 |
| | D81-DV27+P | | 29.6 | 14.9 |
| 81 | D81-DV25 | PCYOX1 | 64.1 | 0 |
| | D81-DV25+ | | 10.0 | 54.1 |
| | D81-DV25P | | 47.1 | 17.0 |
| | D81-DV25+P | | 34.2 | 29.9 |
| | D81-DV27 | | 54.4 | 0 |
| | D81-DV27+ | | 17.0 | 37.4 |
| | D81-DV27P | | 58.0 | -3.6 |
| | D81-DV27+P | | 23.3 | 31.1 |
| 84 | D84-DV26 | MXD1 | 69.3 | 0 |
| | D84-DV26+ | | 29.4 | 39.9 |

(continued)

| Basic sequence NO. | Sequence No. | Off-target gene | Inhibition rate of off-target gene (%) | Reduction (%) in inhibition rate compared with no anti-off-target design |
|---|---|---|---|---|
| | D84-DV26P | | 59.3 | 10.0 |
| | D84-DV26+P | | -4.3 | 73.6 |
| | D84-DV27 | | 44.6 | 0 |
| | D84-DV27+ | | 28.1 | 16.5 |
| | D84-DV27P | | 49.1 | -4.5 |
| | D84-DV27+P | | 37.2 | 7.4 |

**1) Only anti-off-target design (indicated by "+" in the sequence number)**

After the template-modified sequences were modified with an anti-off-target design, the inhibitory effect on off-target genes was significantly reduced. For example, D84-DV26+ reduced the inhibition rate of the MXD1 gene by 39.9%, D81-DV25+ reduced the inhibition rate of the PCYOX1 gene by 54.1%, D51-DV26+ reduced the inhibition rate of the NEPRO gene by 25.9%, and D5-DV25+ reduced the inhibition rate of the DTWD1 gene by 10.9%.
It shows that after the basic sequences 5, 51, 81 and 84 were modified with the modification templates of the present disclosure and further an anti-off-target design, a significant anti-off-target effect was achieved.

**2) Both anti-off-target design and 5’-E-VP modification (indicated by "+P" in the sequence number)**

Sequences with both template modification and 5’-E-VP modification significantly reduced the inhibition rate of off-target genes, with a significant anti-off-target effect. For example, D84-DV26+P reduced the inhibition rate of the off-target gene MXD1 by 73.6%, D81-DV27+P reduced the inhibition rate of the off-target gene PCYOX1 by 31.1%, and D51-DV26+P reduced the inhibition rate of the off-target gene KIF1B by 41.6%, indicating a significant reduction.
It shows that after the basic sequences 5, 51, 81 and 84 were modified with the modification templates of the present disclosure and further an anti-off-target design and 5’-E-VP modification, a significant anti-off-target effect was achieved.

**Summary:**

1. Different modified sequences exhibited a significant inhibitory effect on the PCSK9 gene. For example, the inhibition rate of alternately modified P92-si3 reached 75.6%, and the inhibition rate of sequence D84-DV27P with template modification and 5’-E-VP modification was as high as 94.4%.

    (1) The alternately modified sequence exhibited a significant inhibitory effect on the PCSK9 gene, with the inhibition rate exceeding 60%, among which P92-si3 reached 75.6%.

    (2) The template-modified sequences with anti-off-target design or/and 5’-E-VP modification exhibited a significant inhibitory effect on the PCSK9 gene. For example, the inhibition rate of sequence D84-DV27P with template modification and 5’-E-VP modification was as high as 94.4%.

2. Modification with any one or more of the various modification methods of the present disclosure and further anti-off-target design achieved a significant anti-off-target effect on off-target genes and reduced the inhibitory effect on off-target genes.

    (1) The alternately modified sequences of the present disclosure with an anti-off-target design exhibited a significant anti-off-target effect and can reduce the inhibition rate of off-target genes by 20%.

    (2) The sequences modified with the modification templates of the present disclosure with an anti-off-target design exhibited a significant anti-off-target effect and can reduce the inhibition rate of off-target genes by up to

73.6%.

**Example 7: Effects of sequences modified with the modification templates of the present disclosure on PCSK9, low-density lipoprotein cholesterol (LDL-C) and total cholesterol (TC) in mouse serum**

This example exemplarily selected some sequences, including basic sequences 5, 51, 81, 82 and 84, which were modified, for example, with only template modification, with both template modification and an anti-off-target design, with both template modification and 5'-E-VP modification, or with template modification, 5'-E-VP modification and anti-off-target design simultaneously. Transgenic mice expressing the human PCSK9 gene were used to detect the inhibitory effects of each of the above sequences on PCSK9, low-density lipoprotein cholesterol (LDL-C) and total cholesterol (TC) in serum at different time points by ELISA.

**1. Experimental Materials**

**Test drugs:**

Each of the sequences in Table 38, including sequences with only template modification, with both template modification and an anti-off-target design, with both template modification and 5'-E-VP modification, or with template modification, 5'-E-VP modification and anti-off-target design simultaneously, which conjugated with GalNAc ligand G4, G5, G6 or G7 at the 3'-end of the sense strand.

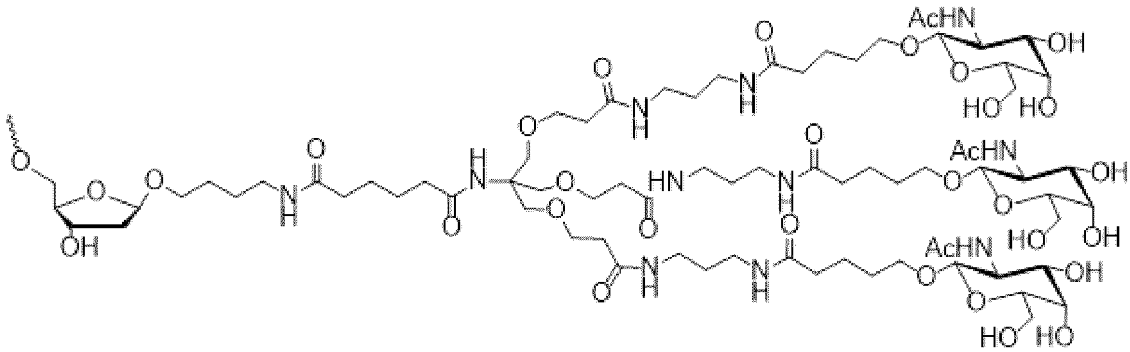

G4

,

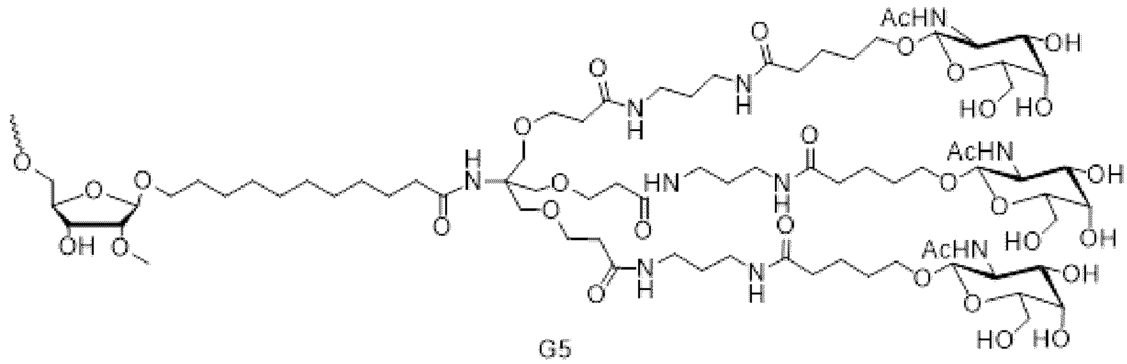

G5

,

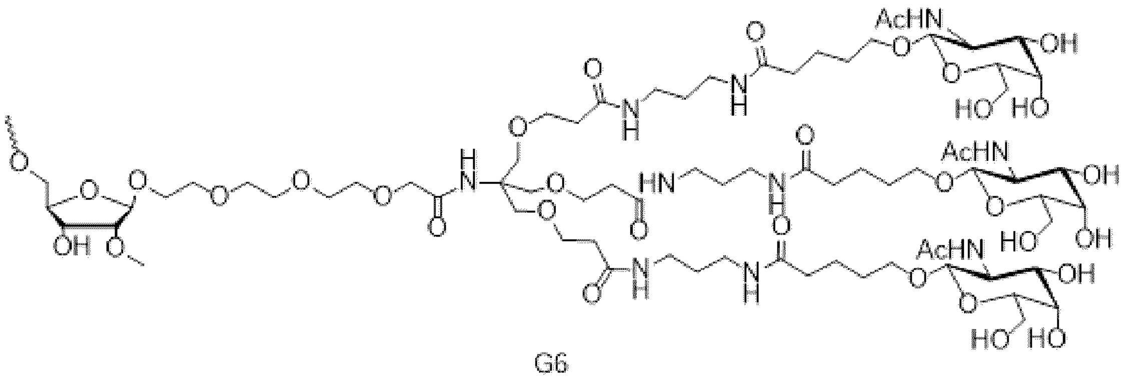

G6

,

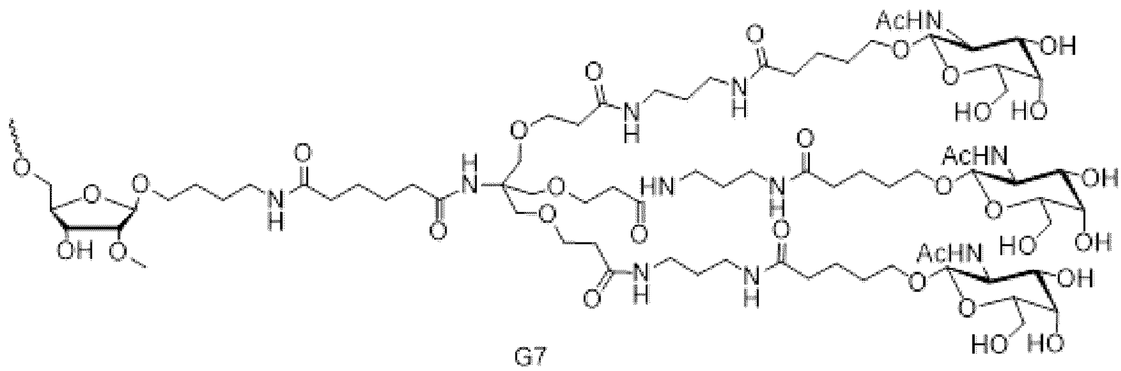

G7

.

The method for conjugating the oligonucleotide with the ligand G4, G5, G6 or G7 was the same as the preparation method of conjugate 4, conjugate 5, conjugate 6 and conjugate 7 in Example 3 of patent application CN116854754A, i.e., conjugating YK-GAL-304, YK-GAL-305, YK-GAL-306 and YK-GAL-307 with the oligonucleotides. The synthesis method of YK-GAL-304, YK-GAL-305, YK-GAL-306 and YK-GAL-307 was the same as Example 1 of this patent application.

The oligonucleotide and the ligand formed a conjugate as shown below:

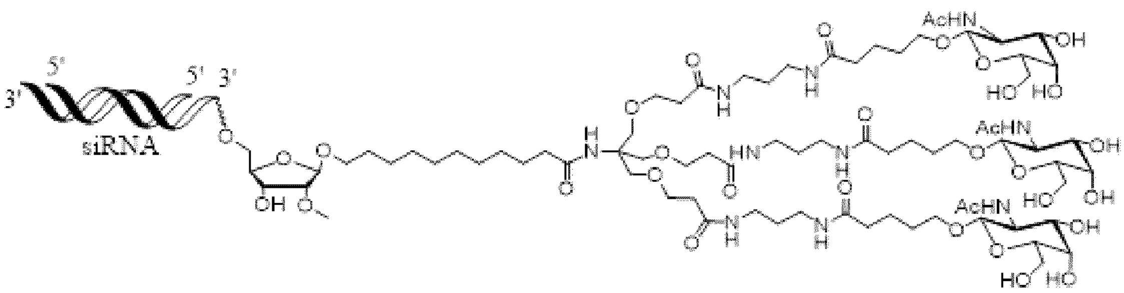

,

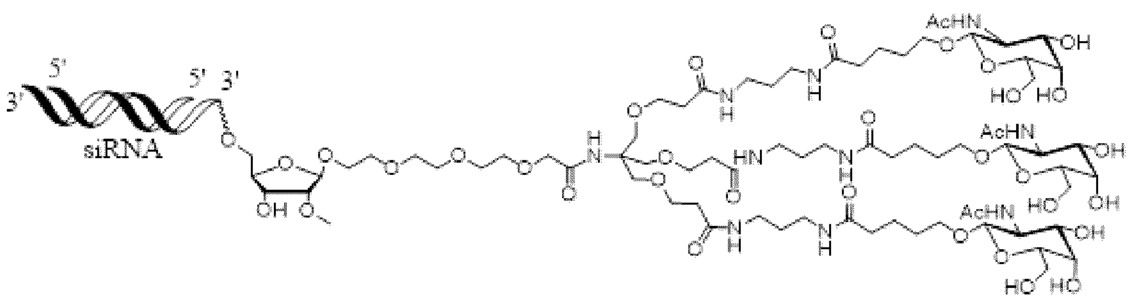

,

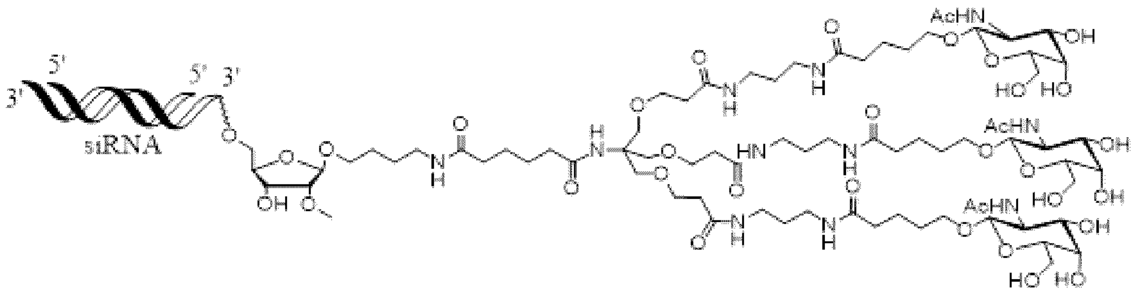

The specific sequences of each sequence are shown in Table 38. G4, G5, G6 and G7 in the sequence number indicate that the sequence was conjugated with the GalNAc ligand G4, G5, G6 or G7.

Table 38 Sequences in animal experiments

| Seque nce No. | Sense strand 5'-3' | Modi fied seque nceS EQ ID NO:/ Corre spond ing basic seque nce SEQ ID NO: | Antisense strand 5'-3' | Modi fied seque nceS EQ ID NO:/ Corre spond ing basic seque nce SEQ ID NO: |
|---|---|---|---|---|
| D5-D V25+ G4 | Ams-Ams-Gm-Am-Um-Cm-Cf-Um-Gf-Cf-Af-Um-Gm-Um-Cm-Um-Uf-Cm-Cm-Am-Um-G4 | 337/1 | Ams-Ufs-Gm-Gm-Am-Af-Ggna-Am-Cm-Am-Um-Gm-Cm-Af-Gm-Gf-Am-Um-Cm-Um-Ums-Gms-Gm | 427/1 3 |
| D5-D V25+ G7 | Ams-Ams-Gm-Am-Um-Cm-Cf-Um-Gf-Cf-Af-Um-Gm-Um-Cm-Um-Uf-Cm-Cm-Am-Um-G7 | 337/1 | Ams-Ufs-Gm-Gm-Am-Af-Ggna-Am-Cm-Am-Um-Gm-Cm-Af-Gm-Gf-Am-Um-Cm-Um-Ums-Gms-Gm | 427/1 3 |
| D5-D V25P G4 | Ams-Ams-Gm-Am-Um-Cm-Cf-Um-Gf-Cf-Af-Um-Gm-Um-Cm-Um-Uf-Cm-Cm-Am-Um-G4 | 337/1 | AmsEVP-Ufs-Gm-Gm-Am-Af-Gm-Am-Cm-Am-Um-Gm-Cm-Af-Gm-Gf-Am-Um-Cm-Um-Ums-Gms-Gm | 428/1 3 |
| D5-D V25P G5 | Ams-Ams-Gm-Am-Um-Cm-Cf-Um-Gf-Cf-Af-Um-Gm-Um-Cm-Um-Uf-Cm-Cm-Am-Um-G5 | 337/1 | AmsEVP-Ufs-Gm-Gm-Am-Af-Gm-Am-Cm-Am-Um-Gm-Cm-Af-Gm-Gf-Am-Um-Cm-Um-Ums-Gms-Gm | 428/1 3 |
| D5-D V25P G7 | Ams-Ams-Gm-Am-Um-Cm-Cf-Um-Gf-Cf-Af-Um-Gm-Um-Cm-Um-Uf-Cm-Cm-Am-Um-G7 | 337/1 | AmsEVP-Ufs-Gm-Gm-Am-Af-Gm-Am-Cm-Am-Um-Gm-Cm-Af-Gm-Gf-Am-Um-Cm-Um-Ums-Gms-Gm | 428/1 3 |
| D51- DV26 G4 | Ums-Gms-Gm-Am-Gm-Gm-Cf-Um-Uf-Af-Gf-Cm-Um-Um-Um-Cm-Uf-Gm-Gm-Am-Um-G4 | 342/2 | Ams-Ufs-Cf-Cf-Am-Gf-Am-Am-Am-Gm-Cm-Um-Am-Af-Gm-Cf-Cm-Um-Cm-Cm-Ams-Ums-Um | 381/1 4 |
| D51-DV26 G6 | Ums-Gms-Gm-Am-Gm-Gm-Cf-Um-Uf-Af-Gf-Cm-Um-Um-Um-Cm-Uf-Gm-Gm-Am-Um-G6 | 342/2 | Ams-Ufs-Cf-Cf-Am-Gf-Am-Am-Am-Gm-Cm-Um-Am-Af-Gm-Cf-Cm-Um-Cm-Cm-Ams-Ums-Um | 381/1 4 |
| D51-DV26 +G5 | Ums-Gms-Gm-Am-Gm-Gm-Cf-Um-Uf-Af-Gf-Cm-Um-Um-Um-Cm-Uf-Gm-Gm-Am-Um-G5 | 342/2 | Ams-Ufs-Cf-Cf-Am-Gf-Agna-Am-Am-Gm-Cm-Um-Am-Af-Gm-Cf-Cm-Um-Cm-Cm-Ams-Ums-Um | 430/1 4 |
| D51-DV26 +G7 | Ums-Gms-Gm-Am-Gm-Gm-Cf-Um-Uf-Af-Gf-Cm-Um-Um-Um-Cm-Uf-Gm-Gm-Am-Um-G7 | 342/2 | Ams-Ufs-Cf-Cf-Am-Gf-Agna-Am-Am-Gm-Cm-Um-Am-Af-Gm-Cf-Cm-Um-Cm-Cm-Ams-Ums-Um | 430/1 4 |

(continued)

| Seque nce No. | Sense strand 5'-3' | Modi fied seque nceS EQ ID NO:/ Corre spond ing basic seque nce SEQ ID NO: | Antisense strand 5'-3' | Modi fied seque nceS EQ ID NO:/ Corre spond ing basic seque nce SEQ ID NO: |
|---|---|---|---|---|
| D51-DV26 PG6 | Ums-Gms-Gm-Am-Gm-Gm-Cf-Um-Uf-Af-Gf-Cm-Um-Um-Um-Cm-Uf-Gm-Gm-Am-Um-G6 | 342/2 | AmsEVP-Ufs-Cf-Cf-Am-Gf-Am-Am-Am-Gm-Cm-Um-Am-Af-Gm-Cf-Cm-Um-Cm-Cm-Ams-Ums-Um | 431/1 4 |
| D51-DV26 PG7 | Ums-Gms-Gm-Am-Gm-Gm-Cf-Um-Uf-Af-Gf-Cm-Um-Um-Um-Cm-Uf-Gm-Gm-Am-Um-G7 | 342/2 | AmsEVP-Ufs-Cf-Cf-Am-Gf-Am-Am-Am-Gm-Cm-Um-Am-Af-Gm-Cf-Cm-Um-Cm-Cm-Ams-Ums-Um | 431/1 4 |
| D81-DV25 G4 | Cms-Ums-Um-Um-Um-Gm-Uf-Am-Af-Cf-Uf-Um-Gm-Am-Am-Gm-Af-Um-Am-Um-Um-G4 | 347/3 | Ams-Afs-Um-Am-Um-Cf-Um-Um-Cm-Am-Am-Gm-Um-Uf-Am-Cf-Am-Am-Am-Am-Gms-Cms-Am | 384/1 5 |
| D81-DV25 G7 | Cms-Ums-Um-Um-Um-Gm-Uf-Am-Af-Cf-Uf-Um-Gm-Am-Am-Gm-Af-Um-Am-Um-Um-G7 | 347/3 | Ams-Afs-Um-Am-Um-Cf-Um-Um-Cm-Am-Am-Gm-Um-Uf-Am-Cf-Am-Am-Am-Am-Gms-Cms-Am | 384/1 5 |
| D81-DV27 PG4 | Cms-Ums-Um-Um-Um-Gm-Uf-Am-Af-Cf-Uf-Um-Gm-Am-Am-Gm-Af-Um-Am-Um-Um-G4 | 347/3 | AmsEVP-Afs-Um-Af-Uf-Cf-Um-Um-Cm-Am-Am-Gm-Um-Uf-Am-Cf-Am-Am-Am-Am-Gms-Cms-Am | 437/1 5 |
| D81-DV27 +PG5 | Cms-Ums-Um-Um-Um-Gm-Uf-Am-Af-Cf-Uf-Um-Gm-Am-Am-Gm-Af-Um-Am-Um-Um-G5 | 347/3 | AmsEVP-Afs-Um-Af-Uf-Cf-Ugna-Um-Cm-Am-Am-Gm-Um-Uf-Am-Cf-Am-Am-Am-Am-Gms-Cms-Am | 438/1 5 |
| D81-DV27 +PG7 | Cms-Ums-Um-Um-Um-Gm-Uf-Am-Af-Cf-Uf-Um-Gm-Am-Am-Gm-Af-Um-Am-Um-Um-G7 | 347/3 | AmsEVP-Afs-Um-Af-Uf-Cf-Ugna-Um-Cm-Am-Am-Gm-Um-Uf-Am-Cf-Am-Am-Am-Am-Gms-Cms-Am | 438/1 5 |
| D81-DV28 G6 | Cms-Ums-Um-Um-Um-Gm-Uf-Am-Af-Cm-Uf-Um-Gm-Am-Am-Gm-Af-Um-Am-Um-Um-G6 | 348/3 | Ams-Afs-Uf-Af-Um-Cf-Um-Um-Cm-Am-Am-Gm-Um-Uf-Am-Cf-Am-Am-Am-Am-Gms-Cms-Am | 385/1 5 |
| D82-DV27 PG5 | Gms-Ams-Am-Gm-Am-Um-Af-Um-Uf-Uf-Af-Um-Um-Cm-Um-Gm-Gf-Gm-Um-Um-Um-G5 | 352/4 | AmsEVP-Afs-Am-Cf-Cf-Cf-Am-Gm-Am-Am-Um-Am-Am-Af-Um-Af-Um-Cm-Um-Um-Cms-Ams-Am | 448/1 6 |

(continued)

| Seque nce No. | Sense strand 5'-3' | Modi fied seque nceS EQ ID NO:/ Corre spond ing basic seque nce SEQ ID NO: | Antisense strand 5'-3' | Modi fied seque nceS EQ ID NO:/ Corre spond ing basic seque nce SEQ ID NO: |
|---|---|---|---|---|
| D82-DV27 PG7 | Gms-Ams-Am-Gm-Am-Um-Af-Um-Uf-Uf-Af-Um-Um-Cm-Um-Gm-Gf-Gm-Um-Um-Um-G7 | 352/4 | AmsEVP-Afs-Am-Cf-Cf-Cf-Am-Gm-Am-Am-Um-Am-Am-Af-Um-Af-Um-Cm-Um-Um-Cms-Ams-Am | 448/1 6 |
| D82-DV29 G4 | Gms-Ams-Am-Gm-Am-Um-Af-Um-Uf-Um-Af-Um-Um-Cm-Um-Gm-Gf-Gm-Um-Um-Um-G4 | 353/4 | Ams-Afs-Am-Cf-Cf-Cf-Am-Gm-Am-Am-Um-Am-Am-Af-Um-Af-Um-Cm-Um-Um-Cms-Ams-Am | 390/1 6 |
| D82-DV29 G7 | Gms-Ams-Am-Gm-Am-Um-Af-Um-Uf-Um-Af-Um-Um-Cm-Um-Gm-Gf-Gm-Um-Um-Um-G7 | 353/4 | Ams-Afs-Am-Cf-Cf-Cf-Am-Gm-Am-Am-Um-Am-Am-Af-Um-Af-Um-Cm-Um-Um-Cms-Ams-Am | 390/1 6 |
| D82-DV29 PG5 | Gms-Ams-Am-Gm-Am-Um-Af-Um-Uf-Um-Af-Um-Um-Cm-Um-Gm-Gf-Gm-Um-Um-Um-G5 | 353/4 | AmsEVP-Afs-Am-Cf-Cf-Cf-Am-Gm-Am-Am-Um-Am-Am-Af-Um-Af-Um-Cm-Um-Um-Cms-Ams-Am | 448/1 6 |
| D82-DV29 PG6 | Gms-Ams-Am-Gm-Am-Um-Af-Um-Uf-Um-Af-Um-Um-Cm-Um-Gm-Gf-Gm-Um-Um-Um-G6 | 353/4 | AmsEVP-Afs-Am-Cf-Cf-Cf-Am-Gm-Am-Am-Um-Am-Am-Af-Um-Af-Um-Cm-Um-Um-Cms-Ams-Am | 448/1 6 |
| D82- DV29 PG7 | Gms-Ams-Am-Gm-Am-Um-Af-Um-Uf-Um-Af-Um-Um-Cm-Um-Gm-Gf-Gm-Um-Um-Um-G7 | 353/4 | AmsEVP-Afs-Am-Cf-Cf-Cf-Am-Gm-Am-Am-Um-Am-Am-Af-Um-Af-Um-Cm-Um-Um-Cms-Ams-Am | 448/1 6 |
| D84-DV26 G4 | Gms-Ums-Um-Um-Um-Gm-Uf-Am-Gf-Cf-Af-Um-Um-Um-Um-Um-Af-Um-Um-Am-Am-G4 | 357/5 | Ums-Ufs-Af-Af-Um-Af-Am-Am-Am-Am-Um-Gm-Cm-Uf-Am-Cf-Am-Am-Am-Am-Cms-Cms-Cm | 393/1 7 |
| D84-DV26 G6 | Gms-Ums-Um-Um-Um-Gm-Uf-Am-Gf-Cf-Af-Um-Um-Um-Um-Um-Af-Um-Um-Am-Am-G6 | 357/5 | Ums-Ufs-Af-Af-Um-Af-Am-Am-Am-Am-Um-Gm-Cm-Uf-Am-Cf-Am-Am-Am-Am-Cms-Cms-Cm | 393/1 7 |
| D84-DV27 PG4 | Gms-Ums-Um-Um-Um-Gm-Uf-Am-Gf-Cf-Af-Um-Um-Um-Um-Um-Af-Um-Um-Am-Am-G4 | 357/5 | UmsEVP-Ufs-Am-Af-Uf-Af-Am-Am-Am-Am-Um-Gm-Cm-Uf-Am-Cf-Am-Am-Am-Am-Cms-Cms-Cm | 446/1 7 |

(continued)

| Seque nce No. | Sense strand 5'-3' | Modi fied seque nceS EQ ID NO:/ Corre spond ing basic seque nce SEQ ID NO: | Antisense strand 5'-3' | Modi fied seque nceS EQ ID NO:/ Corre spond ing basic seque nce SEQ ID NO: |
|---|---|---|---|---|
| D84-DV27 PG6 | Gms-Ums-Um-Um-Um-Gm-Uf-Am-Gf-Cf-Af-Um-Um-Um-Um-Um-Af-Um-Um-Am-Am-G6 | 357/5 | UmsEVP-Ufs-Am-Af-Uf-Af-Am-Am-Am-Am-Um-Gm-Cm-Uf-Am-Cf-Am-Am-Am-Am-Cms-Cms-Cm | 446/1 7 |
| D84-DV27 +PG4 | Gms-Ums-Um-Um-Um-Gm-Uf-Am-Gf-Cf-Af-Um-Um-Um-Um-Um-Af-Um-Um-Am-Am-G4 | 357/5 | UmsEVP-Ufs-Am-Af-Uf-Af-Agna-Am-Am-Am-Um-Gm-Cm-Uf-Am-Cf-Am-Am-Am-Am-Cms-Cms-Cm | 447/1 7 |
| D84-DV27 +PG5 | Gms-Ums-Um-Um-Um-Gm-Uf-Am-Gf-Cf-Af-Um-Um-Um-Um-Um-Af-Um-Um-Am-Am-G5 | 357/5 | UmsEVP-Ufs-Am-Af-Uf-Af-Agna-Am-Am-Am-Um-Gm-Cm-Uf-Am-Cf-Am-Am-Am-Am-Cms-Cms-Cm | 447/1 7 |
| D84-DV27 +PG7 | Gms-Ums-Um-Um-Um-Gm-Uf-Am-Gf-Cf-Af-Um-Um-Um-Um-Um-Af-Um-Um-Am-Am-G7 | 357/5 | UmsEVP-Ufs-Am-Af-Uf-Af-Agna-Am-Am-Am-Um-Gm-Cm-Uf-Am-Cf-Am-Am-Am-Am-Cms-Cms-Cm | 447/1 7 |

**Preparation of test drug:**

Drug solvent: PBS buffer

Preparation conditions: Sterile environment

Labeling method: The prepared drug formulations were labeled, and the outer packaging was marked with the topic number, name, concentration, amount, preparation date, preparer, and storage conditions;

Storage conditions: The samples were prepared immediately before use and the remaining samples were stored at -80°C.

**Experimental Animal Information:**

Species/Strain: hPCSK9 transgenic mice

Rating: SPF

Gender: Male

Quantity: 214

Age: 4-7 weeks

Weight: 18-20g

Source: Jiangsu GemPharmatech Co., Ltd.

Production license number: SCXK (Su) 2018-0008

**Experimental Animal Ethics Review (IACUC):**

The experimental animals were received and then kept at Tianjin Yugen Medtech Co., Ltd., with the use license number: SYXK (Jinbin) 2019-0002. This project had been reviewed by the Experimental Animal Ethics Committee of Tianjin Yugen Medtech Co., Ltd., and the experimental process was carried out in strict accordance with IACUC requirements to ensure animal welfare.

**Feeding and management:**

Feeding conditions: The experimental animals were received and kept by Tianjin Yugen Medtech Co., Ltd., with the license number SYXK (Jinbin) 2019-0002. They were kept in a feeding cage with the specifications of length × width × height = 29.0 cm × 18.5 cm × 13.0 cm; the temperature range was set at 20-26°C, the humidity range was set at 40%-70%, the air change frequency was not less than 15 times of fresh air/hour, and the artificial lighting was alternating between 12 hours of light and 12 hours of dark.

The breeding environment conditions were based on the National Standard of the People's Republic of China GB14925-2010, and the environment was controlled by a modular air-conditioning unit.

Animals were allowed to eat and drink freely. The drinking water bottles and the drinking water in the bottles should be changed at least twice a week. After use, the drinking water bottles were sterilized under high pressure in a pulsating vacuum sterilizer and then reused.

Animal cages and bedding should be replaced at least once a week. All animal cages and bedding should be sterilized under high pressure in a pulsating vacuum sterilizer before being used in a barrier environment. Animal cages should be cleaned, disinfected and wiped at least once a week.

The animal breeding and observation room is cleaned and disinfected every day, including the flat racks, floors, tables, etc.

The disinfectants used in the barrier environment included: 6.67% bromo-geraminum solution, 0.5% 84 disinfectant, 75% disinfectant, and 0.08% Bestaquam. The four disinfectants should be used in turn and cannot be mixed.

Experimental animal feed: SPF rat maintenance feed, produced by SPF (Beijing) biotechnology Co. Ltd., with the animal feed production license number of SCXK (Beijing) 2019-0010, issued by Beijing Municipal Science and Technology Commission.

Feed testing: Each batch of feed had a quality certificate, and our company conducted microbiological testing once a quarter. The feed supplier provided a recent third-party feed testing report every six months. Feed nutrient testing referred to the National Standard of the People's Republic of China GB14924.3-2010, and pollutant index testing referred to the National Standard of the People's Republic of China GB14924.2-2001.

Drinking water for experimental animals: Drinking water: sterile water prepared by the filtration system, directly filled in drinking bottles.

Drinking water testing: Our company conducted microbiological testing once a quarter and sent water to a third-party testing agency for water quality testing once a year. Drinking water testing referred to the National Standard of the People's Republic of China GB5749-2006.

Animal bedding: Corn cob bedding: produced by SPF (Beijing) Biotechnology Co., Ltd., with the animal bedding production license number of SCXK (Beijing) 2019-0004, issued by Beijing Municipal Science and Technology Commission.

Bedding testing: Our company conducted microbiological testing once a quarter, and the bedding supplier provided at least one third-party bedding testing report every six months. Bedding testing referred to the National Standard of the People's Republic of China GB14924.2-2001.

## 2. Experimental Methods

### Dosage design and grouping

Definition of experimental date: The day on which the animals were administered with the drug solvent or the test drug was defined as day 0.

Grouping and administration: After the experimental animals were adaptively fed, they were randomly divided into a negative control group and a test drug group according to the serum PSCK9 protein content, with 5 animals in each group. The drug was administered by a single subcutaneous injection, with an amount of 6 mg/kg, a volume of 1 mL/kg, and a concentration of 6 mg/mL. The day of administration was recorded as day 0.

Animals were individually identified by ear tags. Cages were identified by hanging cage cards. Laboratory identification signs were hung at the door of the laboratory. Grouping information is detailed in the table below:

Table 39

| Group | Drug | Amount (mg/kg) | Administration route/ frequency | Cycle (days) | Number of animals |
|---|---|---|---|---|---|
| Negative control group | drug solvent | / | / | 35 | 5 |
| Test drug group | siRNA | 6 | s.c./once | 35 | 5 |

### Detection Indicator

### (1) General observation

The subjects were observed once a day from one week before administration to the end of the experiment.

Observation content: The animal's death or dying, mental state, behavioral activities, feces characteristics, feed and water supply, etc. were observed beside the cage.

Test animals: all animals in the negative control group and the test drug group.

### (2) Expression of PCSK9 protein in serum

Detection time: before administration on day-3 (day-3), 1 week after administration on day 7 (1 w), 2 weeks after administration on day 14 (2 w), 3 weeks after administration on day 21 (3 w), 4 weeks after administration on day 28 (4 w), and 5 weeks after administration on day 35 (5 w).

PCSK9 protein level detection method: ELISA kit was used for detection; serum samples were prevented from repeated freezing and thawing.

Test animals: all animals in the negative control group and the test drug group.

**(3) Blood lipid detection**

At detection times of 3 days before administration (day-3), day 7 after administration (1 week, 1 w), day 14 after administration (2 weeks, 2 w), day 21 after administration (3 weeks, 3 w), day 28 after administration (4 weeks, 4 w), and day 35 after administration (5 weeks, 5 w), about 200 μL of blood was collected from the inner canthus. The whole blood sample was temporarily stored in an ice box, then centrifuged at 2-8°C and about 3000 g for 10 min, divided into 2 tubes (one of which was about 60 μL, and the remaining serum was stored in the other tube), and stored at-70 to -86°C for blood lipid detection.

**(4) Data processing and statistical analysis**

The experimental data were expressed as mean ± standard deviation (Mean ± SD) and analyzed using GraphPad Prism 8.3 analysis software. The LSD test was used to test for homogeneity of variance, and the Dunnett T3 test was used for unequal variance. $P < 0.05$ was considered statistically significant.

**3. Experimental Results**

Specific experimental results are shown in Tables 40-42.

It can be seen that these sequences can continuously and significantly inhibit PCSK9 protein in serum, and significantly reduce low-density lipoprotein cholesterol (LDL-C) and total cholesterol (TC) levels in serum. For example, in the D81-DV25G7 group, the inhibition rates of PCSK9 protein in serum reached 83.1%, 82.2% and 70.3% on days 7, 14, and 28, respectively; in the D82-DV27PG5 group, the serum LDL-C levels were reduced by 32.6%, 35.8% and 21.7% on days 7, 14, and 28, respectively; in the D82-DV29PG7 group, the serum TC levels were reduced by 32.2%, 26.8% and 19.6% on days 7, 14, and 28, respectively.

**(1) The sequences of the present disclosure, such as the basic sequences 5, 51, 81, 82 and 84 with different modifications exhibited a significant inhibitory effect on the expression of PCSK9 protein in serum. For example, the inhibition rates by D81-DV25G7 reached 83.1%, 82.2%, and 70.3% on days 7, 14, and 28, respectively.**

Table 40 Inhibition rates of PCSK9 protein in serum by different sequences

| Basic sequence NO. | Sequence No. | Inhibition rate (%) | | |
|---|---|---|---|---|
| | | Day 7 | Day 14 | Day 28 |
| 5 | D5-DV25PG4 | 78.1 | 70.5 | 53.8 |
| | D5-DV25PG5 | 77.0 | 64.7 | 58.4 |
| 51 | D51-DV26PG6 | 78.2 | 82.6 | 68.2 |
| | D51-DV26+G5 | 74.7 | 68.0 | 59.3 |
| | D51-DV26+G7 | 70.6 | 75.3 | 58.1 |
| 81 | D81-DV25G4 | 80.2 | 81.6 | 63.8 |
| | D81-DV25G7 | 83.1 | 82.2 | 70.3 |
| 82 | D82-DV27PG5 | 88.2 | 87.4 | 69.2 |
| | D82-DV27PG7 | 81.9 | 86.3 | 72.6 |
| | D82-DV29G4 | 74.2 | 81.6 | 72.6 |
| | D82-DV29PG5 | 83.8 | 90.0 | 76.4 |

(continued)

| Basic sequence NO. | Sequence No. | Inhibition rate (%) | | |
|---|---|---|---|---|
| | | Day 7 | Day 14 | Day 28 |
| 84 | D84-DV26G4 | 81.7 | 77.4 | 65.8 |
| | D84-DV26G6 | 75.6 | 79.5 | 56.3 |
| | D84-DV27+PG5 | 84.0 | 76.1 | 63.4 |
| | D84-DV27+PG7 | 78.9 | 77.6 | 60.8 |

As can be seen from Table 40, the conjugates formed by conjugating each sequence with a GalNAc compound exhibited a significant inhibitory effect on PCSK9 protein expression in serum. For example, the inhibition rates by D81-DV25G7 reached 83.1%, 82.2%, and 70.3% on days 7, 14, and 28, respectively (FIG. 9).

It shows that the basic sequences designed in the present disclosure, such as 5, 51, 81, 82 and 84, after modification with the modification templates of the present disclosure, or with further 5'-E-VP modification or/and an anti-off-target design and conjugation with the GalNAc compound, can be efficiently delivered to animal liver and significantly inhibit PCSK9 gene expression.

**(2) The sequences of the present disclosure, such as the basic sequences 5, 51, 81, 82 and 84 with different modifications can significantly reduce low-density lipoprotein cholesterol (LDL-C) levels in serum. For example, in the D82-DV27PG5 group, the LDL-C levels decreased by 32.6%, 35.8%, and 21.7% on days 7, 14, and 28, respectively.**

Table 41 Reduction of low-density lipoprotein cholesterol (LDL-C) levels in serum by different sequences

| Basic sequence NO. | Sequence No. | Reduction (%) | | |
|---|---|---|---|---|
| | | Day 7 | Day 14 | Day 28 |
| 5 | D5-DV25PG5 | 21.2 | 16.9 | 15.4 |
| | D5-DV25PG7 | 20.7 | 18.7 | 19.2 |
| 51 | D51-DV26G4 | 19.7 | 21.9 | 17.7 |
| | D51-DV26G6 | 24.5 | 18.4 | 16.8 |
| 81 | D81-DV27PG4 | 25.2 | 25.3 | 17.3 |
| | D81-DV27+PG5 | 25.2 | 18.3 | 18.2 |
| | D81-DV27+PG7 | 21.9 | 17.9 | 16.8 |
| 82 | D82-DV27PG5 | 32.6 | 35.8 | 21.7 |
| | D82-DV29G4 | 29.3 | 34.1 | 22.5 |
| | D82-DV29G7 | 30.6 | 32.9 | 23.7 |
| | D82-DV29PG5 | 30.1 | 31.2 | 22.8 |
| 84 | D84-DV27PG4 | 30.1 | 26.1 | 20.7 |
| | D84-DV27PG6 | 28.9 | 29.6 | 18.5 |

As can be seen from Table 41, serum LDL-C was significantly reduced in each experimental group. For example, in the D82-DV27PG5 group, the LDL-C levels decreased by 32.6%, 35.8%, and 21.7% on days 7, 14, and 28, respectively (FIG. 10).

It shows that the basic sequences designed in the present disclosure, such as 5, 51, 81, 82 and 84, after modification with the modification templates of the present disclosure, or with further 5'-E-VP modification or/and an anti-off-target design and conjugation with the GalNAc compound, can be efficiently delivered to animal liver and significantly reduce low-density lipoprotein cholesterol (LDL-C) levels in serum.

**(3) The sequences of the present disclosure, such as the basic sequences 5, 51, 81, 82 and 84 with different modifications, can significantly reduce total cholesterol (TC) levels in serum. For example, in the D82-DV29PG7 group, the TC levels decreased by 32.2%, 26.8%, and 19.6% on days 7, 14, and 28, respectively.**

Table 42 Reduction of total cholesterol (TC) levels in serum by different sequences

| Basic sequence NO. | Sequence No. | Reduction (%) | | |
|---|---|---|---|---|
| | | Day 7 | Day 14 | Day 28 |
| 5 | D5-DV27+G4 | 21.5 | 20.8 | 13.2 |
| | D5-DV27+G7 | 19.7 | 18.7 | 9.2 |
| 51 | D51-DV26PG6 | 24.1 | 18.3 | 11.9 |
| | D51-DV26PG7 | 19.7 | 20.3 | 10.7 |
| 81 | D81-DV25G7 | 21.7 | 22.2 | 13.1 |
| | D81-DV27+PG5 | 21.1 | 16.1 | 15.6 |
| | D81-DV27+PG7 | 17.9 | 21.6 | 13.8 |
| | D81-DV28G6 | 22.9 | 19.1 | 13.4 |
| 82 | D82-DV29PG5 | 31.4 | 27.0 | 18.5 |
| | D82-DV29PG6 | 28.9 | 29.6 | 17.1 |
| | D82-DV29PG7 | 32.2 | 26.8 | 19.6 |
| 84 | D84-DV26G6 | 28.9 | 29.6 | 18.5 |
| | D84-DV27+PG4 | 28.4 | 25.7 | 14.9 |
| | D84-DV27+PG5 | 22.8 | 22.9 | 16.5 |

As can be seen from Table 42, total cholesterol (TC) in serum was significantly reduced in each experimental group. For example, in the D82-DV29PG7 group, the TC levels decreased by 32.2%, 26.8%, and 19.6% on days 7, 14, and 28, respectively (FIG. 11).

It shows that the basic sequences designed in the present disclosure, such as 5, 51, 81, 82 and 84, after modification with the modification templates of the present disclosure, or with further 5'-E-VP modification or/and an anti-off-target design and conjugation with the GalNAc compound, can be efficiently delivered to animal liver and significantly reduce total cholesterol (TC) levels in serum.

**Summary:**

The basic sequences designed in the present disclosure, such as 5, 51, 81, 82 and 84, after modification with the modification templates of the present disclosure, or with further 5'-E-VP modification or/and an anti-off-target design and conjugation with the GalNAc compound, can be efficiently delivered to animal liver, and can continuously and significantly inhibit the expression of PCSK9 protein in serum and reduce low-density lipoprotein cholesterol (LDL-C) levels in serum and total cholesterol (TC) levels in serum.

For example, the inhibition rate of PCSK9 protein expression in the serum by the D81-DV25G7 group reached 83.1%, 82.2%, and 70.3% on days 7, 14, and 28, respectively; in the D82-DV27PG5 group, the low-density lipoprotein cholesterol (LDL-C) levels in serum decreased by 32.6%, 35.8%, and 21.7% on days 7, 14, and 28, respectively; in the D82-DV29PG7 group, the total cholesterol (TC) levels in serum decreased by 32.2%, 26.8%, and 19.6% on days 7, 14, and 28, respectively

**Conclusion:**

The present disclosure designed a series of siRNAs based on the PCSK9 mRNA sequence, which were alternately modified and modified using a specific set of modification templates, and some of the sequences were modified with an anti-off-target and 5'-E-VP modifications. The results demonstrate:

(1) Unmodified sequences, including basic sequences 5, 51, 81, 82, 84, 3, 8, 9, 21, 31, 73 and 83, all exhibited a significant inhibitory effect on PCSK9, with the highest inhibition rate exceeding 60%.

(2) The inhibition rate of sequences with multiple modifications reached up to 90% or more. Moreover, the modified sequence conjugated with a GalNAc compound can be efficiently delivered to the animal liver, markedly inhibiting PCSK9 gene expression, substantially reducing low-density lipoprotein cholesterol (LDL-C) levels in serum, and

significantly reducing total cholesterol (TC) levels in serum.

The details are as follows:

**1. The alternately modified sequence of the present disclosure had a significant inhibitory effect on the PCSK9 gene**

(1) Among the 84 designed sequences, 12 sequences exhibited significant inhibitory effects on the PCSK9 gene, with inhibition rates exceeding 50%, including P92-si5, P92-si51, P92-si81, P92-si82, P92-si84, P92-si3, P92-si8, P92-si9, P92-si21, P92-si31, P92-si73 and P92-si83, among which the inhibition rate of P92-si5, P92-si81, P92-si82, P92-si3, P92-si8 and P92-si31 exceeded 70%.

(2) The inhibition rate of 26 sequences on the PCSK9 gene was 30%-50%. For example, the inhibition rates of P92-si6 and P92-si7 were 38.3% and 35.7%, respectively.

(3) The inhibition rate of 46 sequences on the PCSK9 gene was less than 30%. For example, the inhibition rates of P92-si1 and P92-si20 were only 9.2% and 6.0%, respectively.

(4) siRNAs with similar sequences had very different activities. For example, the inhibition rate of P92-si5 was 61.8% higher than that of P92-si4, indicating a significant improvement.

**2. The unmodified sequence of the present disclosure had a significant inhibitory effect on the PCSK9 gene**

(1) 12 unmodified sequences exhibited a significant inhibitory effect on the PCSK9 gene, with inhibition rates exceeding 50%, including si5, si51, si81, si82, si84, si3, si8, si9, si21, si31, si73 and si83, among which the inhibition rate of si5, si81, si82, si3 and si8 exceeded 60%.

(2) The inhibition rates of other unmodified sequences on the PCSK9 gene were less than 40%. For example, the inhibition rates of si52 and si74 were only 0.5% and 2.2%, respectively.

(3) siRNAs with similar sequences had very different activities. For example, the inhibition rate of basic sequence 5 was 51.2% higher than that of basic sequence 4, indicating a significant improvement.

(4) Different sequences exhibited disparate effects on activity after alternate modifications. Some inhibition rates demonstrated a notable enhancement, exemplified by basic sequence 5, whose alternately modified sequence exhibited an elevated inhibition rate of 12.4% relative to its unmodified sequence. Conversely, some inhibition rates exhibited a less pronounced alteration, as observed in basic sequences 4, 22, and 52, where the inhibition rates of alternately modified and unmodified sequences exhibited minimal deviation.

**3. The sequence modified with the modification templates of the present disclosure had a significant inhibitory effect on the PCSK9 gene.**

(1) The basic sequences 5, 51, 81, 82, 84, 3, 8, 9, 21, 31, 73 and 83, after being modified with the modification templates DV25-29 designed in the present disclosure, exhibited a significant inhibitory effect on PCSK9 gene expression, with an inhibition rate above 70%, among which the inhibition rates of P92-si81-DV26, P92-si82-DV27 and P92-si82-DV29 reached 89.3%, 89.3% and 89.1%, respectively.

(2) The above 12 sequences modified with the modification templates DV25-29 of the present disclosure significantly improved the inhibition rate against the PCSK9 gene expression compared with the alternately modified sequences. For example, P92-si51-DV27 obtained by modifying basic sequence 51 with templates DV27 increased the inhibition rate by 29.1% compared to the alternately modified sequence, and P92-si81-DV26 obtained by modifying basic sequence 81 with templates DV26 increased the inhibition rate by 26.9% compared to the alternately modified sequence.

(3) The sequences modified on the same sequence with the modification templates DV25-29 of the present disclosure significantly improved the inhibition rate against the PCSK9 gene expression compared with using the modification templates disclosed in the prior art. For example, basic sequence 51 modified with the modification template DV27 of the present disclosure increased the inhibition rate by 21.3% compared with the disclosed

Advanced ESC template DV21.

(4) The sequences modified on the same sequence with the modification templates DV25-29 of the present disclosure significantly improved the inhibition rate against the PCSK9 gene expression compared with using other modification templates DV30 and DV31 of the present disclosure. For example, basic sequence 51 modified with the modification template DV27 of the present disclosure increased the inhibition rate by 22.8% compared with the modification template DV31 of the present disclosure.

(5) The EC50 values of the above 12 sequences were between 0.0001 nM and 0.005 nM, among which the EC50 values of P92-si81-DV27, P92-si84-DV26 and P92-si82-DV27 were 0.0003 nM, 0.0004 nM and 0.0006 nM, respectively. These results demonstrate that the sequences can effectively inhibit PCSK9 gene expression at low concentrations.

**4. Each sequence modified by alternate modifications and modification templates of the present disclosure has significantly improved inhibitory activity against PCSK9 compared with the unmodified sequences disclosed in the prior art with identical sequences or minimal differences.**

(1) Compared with the identical unmodified sequences disclosed in the prior art, the alternately modified sequences and the sequences modified with the specific modification templates of the present disclosure significantly improved the inhibition rate against PCSK9 gene, which can be increased by up to 40%.

(2) Compared with the unmodified sequences disclosed in the prior art with minimal differences, the unmodified sequences, alternately modified sequences and sequences modified with specific modification templates of the present disclosure significantly improved the inhibition rate against PCSK9 gene, which can be increased by up to 40%.

I. The unmodified sequences of the present disclosure significantly improved the activity compared with the similar unmodified sequences disclosed in the prior art. For example, the unmodified sequence si31 of the present disclosure improved the inhibition rate by 10.4% compared with 31P with a similar structure.

II. The alternately modified sequences of the present disclosure significantly improved the activity compared with the similar unmodified sequences disclosed in the prior art. For example, the alternately modified sequence P92-si5 of the present disclosure improved the inhibition rate by 22.0% compared with sequence 5P.

**III**. The sequences modified with the modification templates of the present disclosure significantly improved the activity compared with the similar unmodified sequences disclosed in the prior art. For example, the alternately modified sequence P92-si82-DV29 of the present disclosure improved the inhibition rate by 42.8% compared with sequence 82P.

**5. The alternately modified sequences and template-modified sequences of the present disclosure with a specific anti-off-target design exhibited a significant inhibitory effect on the PCSK9 gene and significantly reduced the inhibitory effect on off-target genes.**

(1) Different modified sequences exhibited a significant inhibitory effect on the PCSK9 gene. For example, the inhibition rate of alternately modified P92-si3 reached 75.6%, and the inhibition rate of sequence D84-DV27P with template modification and 5'-E-VP modification was as high as 94.4%.

I. The alternately modified sequence exhibited a significant inhibitory effect on the PCSK9 gene, with the inhibition rate exceeding 60%, among which P92-si3 reached 75.6%.

II. The template-modified sequences with anti-off-target design or/and 5'-E-VP modification exhibited a significant inhibitory effect on the PCSK9 gene. For example, the inhibition rate of sequence D84-DV27P with template modification and 5'-E-VP modification was as high as 94.4%.

(2) Modification with any one or more of the various modification methods of the present disclosure and further anti-off-target design achieved a significant anti-off-target effect on off-target genes and reduced the inhibitory effect on off-target genes.

I. The alternately modified sequences of the present disclosure with an anti-off-target design exhibited a significant anti-off-target effect and can reduce the inhibition rate of off-target genes by 20%.

II. The sequences modified with the modification templates of the present disclosure with an anti-off-target design exhibited a significant anti-off-target effect and can reduce the inhibition rate of off-target genes by up to 73.6%.

**6. Sequences modified with the modification templates of the present disclosure significantly inhibited PCSK9 expression in mouse serum and significantly reduced low-density lipoprotein cholesterol (LDL-C) and total cholesterol (TC) levels.**

(1) The basic sequences 5, 51, 81, 82 and 84 of the present disclosure with different modifications exhibited a significant inhibitory effect on the expression of PCSK9 protein in serum. For example, the inhibition rates by D81-DV25G7 reached 83.1%, 82.2%, and 70.3% on days 7, 14, and 28, respectively.

(2) The basic sequences 5, 51, 81, 82 and 84 of the present disclosure with different modifications can significantly reduce low-density lipoprotein cholesterol (LDL-C) levels in serum. For example, in the D82-DV27PG5 group, the LDL-C levels decreased by 32.6%, 35.8%, and 21.7% on days 7, 14, and 28, respectively.

(3) The basic sequences 5, 51, 81, 82 and 84 of the present disclosure with different modifications, can significantly reduce total cholesterol (TC) levels in serum. For example, in the D82-DV29PG7 group, the TC levels decreased by 32.2%, 26.8%, and 19.6% on days 7, 14, and 28, respectively.

## Claims

**1.** A double-stranded RNAi agent for reducing PCSK9 expression, comprising an oligonucleotide duplex selected from any one of the following paired sense strand and antisense strand:

(1) the sense strand has a sequence set forth in SEQ ID NO: 1 or a fragment thereof, or a modified sequence of the sequence or the fragment thereof; and the antisense strand has a sequence set forth in SEQ ID NO: 13 or a fragment thereof, or a modified sequence of the sequence or the fragment thereof;
(2) the sense strand has a sequence set forth in SEQ ID NO: 2 or a fragment thereof, or a modified sequence of the sequence or the fragment thereof; and the antisense strand has a sequence set forth in SEQ ID NO: 14 or a fragment thereof, or a modified sequence of the sequence or the fragment thereof;
(3) the sense strand has a sequence set forth in SEQ ID NO: 3 or a fragment thereof, or a modified sequence of the sequence or the fragment thereof; and the antisense strand has a sequence set forth in SEQ ID NO: 15 or a fragment thereof, or a modified sequence of the sequence or the fragment thereof;
(4) the sense strand has a sequence set forth in SEQ ID NO: 4 or a fragment thereof, or a modified sequence of the sequence or the fragment thereof; and the antisense strand has a sequence set forth in SEQ ID NO: 16 or a fragment thereof, or a modified sequence of the sequence or the fragment thereof;
(5) the sense strand has a sequence set forth in SEQ ID NO: 5 or a fragment thereof, or a modified sequence of the sequence or the fragment thereof; and the antisense strand has a sequence set forth in SEQ ID NO: 17 or a fragment thereof, or a modified sequence of the sequence or the fragment thereof;
(6) the sense strand has a sequence set forth in SEQ ID NO: 6 or a fragment thereof, or a modified sequence of the sequence or the fragment thereof; and the antisense strand has a sequence set forth in SEQ ID NO: 18 or a fragment thereof, or a modified sequence of the sequence or the fragment thereof;
(7) the sense strand has a sequence set forth in SEQ ID NO: 7 or a fragment thereof, or a modified sequence of the sequence or the fragment thereof; and the antisense strand has a sequence set forth in SEQ ID NO: 19 or a fragment thereof, or a modified sequence of the sequence or the fragment thereof;
(8) the sense strand has a sequence set forth in SEQ ID NO: 8 or a fragment thereof, or a modified sequence of the sequence or the fragment thereof; and the antisense strand has a sequence set forth in SEQ ID NO: 20 or a fragment thereof, or a modified sequence of the sequence or the fragment thereof;
(9) the sense strand has a sequence set forth in SEQ ID NO: 9 or a fragment thereof, or a modified sequence of the sequence or the fragment thereof; and the antisense strand has a sequence set forth in SEQ ID NO: 21 or a fragment thereof, or a modified sequence of the sequence or the fragment thereof;
(10) the sense strand has a sequence set forth in SEQ ID NO: 10 or a fragment thereof, or a modified sequence of the sequence or the fragment thereof; and the antisense strand has a sequence set forth in SEQ ID NO: 22 or a fragment thereof, or a modified sequence of the sequence or the fragment thereof;

(11) the sense strand has a sequence set forth in SEQ ID NO: 11 or a fragment thereof, or a modified sequence of the sequence or the fragment thereof; and the antisense strand has a sequence set forth in SEQ ID NO: 23 or a fragment thereof, or a modified sequence of the sequence or the fragment thereof; or
(12) the sense strand has a sequence set forth in SEQ ID NO: 12 or a fragment thereof, or a modified sequence of the sequence or the fragment thereof; and the antisense strand has a sequence set forth in SEQ ID NO: 24 or a fragment thereof, or a modified sequence of the sequence or the fragment thereof.

**2.** The double-stranded RNAi agent according to claim 1, wherein all nucleotides in the sense strand and the antisense strand are modified nucleotides.

**3.** The double-stranded RNAi agent according to claim 1 or 2, which is an RNAi agent for inhibiting PCSK9 gene expression.

**4.** The double-stranded RNAi agent according to claim 1 or 2, wherein the sense strand differs from any one of SEQ ID NOs. 1 to 12 by 1 to 3 nucleotides.

**5.** The double-stranded RNAi agent according to claim 1 or 2, wherein the antisense strand differs from any one of SEQ ID NOs. 13 to 24 by 1 to 3 nucleotides.

**6.** The double-stranded RNAi agent according to claim 1 or 2, wherein at least one modified nucleotide is selected from the group consisting of: a deoxy-nucleotide, a 3'-end deoxy-thymine (dT) nucleotide, a 2'-O-methyl modified nucleotide, a 2'-fluoro modified nucleotide, a 2'-deoxy-modified nucleotide, a locked nucleotide, a non-locked nucleotide, a conformationally restricted nucleotide, a constrained ethyl nucleotide, an abasic nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising phosphorothioate, a nucleotide comprising methylphosphonate, a nucleotide comprising 5'-phosphate, and a nucleotide comprising 5'-phosphate mimic.

**7.** The double-stranded RNAi agent according to claim 1 or 2, wherein at least one strand comprises a 3' overhang of at least 1 nucleotide.

**8.** The double-stranded RNAi agent according to claim 1 or 2, wherein at least one strand comprises a 3' overhang of at least 2 nucleotides.

**9.** The double-stranded RNAi agent according to claim 1 or 2, wherein the double-stranded region is from 15 to 30 nucleotide pairs in length.

**10.** The double-stranded RNAi agent according to claim 1 or 2, wherein the double-stranded region is from 17 to 25 nucleotide pairs in length.

**11.** The double-stranded RNAi agent according to claim 1 or 2, wherein the double-stranded region is from 19 to 23 nucleotide pairs in length.

**12.** The double-stranded RNAi agent according to claim 1 or 2, wherein the double-stranded region is 21 nucleotide pairs in length.

**13.** The double-stranded RNAi agent according to claim 1 or 2, wherein each strand has 15 to 30 nucleotides.

**14.** The double-stranded RNAi agent according to claim 1 or 2, wherein each strand has 19 to 25 nucleotides.

**15.** The double-stranded RNAi agent according to claim 1 or 2, wherein the sense strand has 21 nucleotides and the antisense strand has 23 nucleotides.

**16.** The double-stranded RNAi agent according to claim 1 or 2, wherein all nucleotides in the sense strand and the antisense strand are modified with a chemical modification on the 2' position of the ribose of the nucleotide.

**17.** The double-stranded RNAi agent according to claim 1 or 2, wherein the chemical modification on the 2’ position of the ribose of the nucleotide is selected from the group consisting of 2’-methoxy, 2’-methoxyethyl, 2’-fluoro, 2’-benzyloxy, 2’-methylcarbonylamino, 2’-pyridylmethoxy, and a combination thereof.

**18.** The double-stranded RNAi agent according to claim 1 or 2, wherein the chemical modification on the 2’ position of the ribose of the nucleotide is selected from a combination of 2’-methoxy and 2’-fluoro.

**19.** The double-stranded RNAi agent according to claim 1 or 2, wherein the chemical modification on the 2’ position of the ribose of the nucleotide is alternate 2’-methoxy and 2’-fluoro.

**20.** The double-stranded RNAi agent according to claim 1 or 2, wherein for the chemical modification on the 2’ position of the ribose of the nucleotide, the nucleotides in the sense strand are modified with 2’-fluoro in odd-numbered positions and with 2’-methoxy in even-numbered positions, and the nucleotides in the antisense strand are modified with 2’-methoxy in odd-numbered positions and with 2’-fluoro in even-numbered positions.

**21.** The double-stranded RNAi agent according to claim 1 or 2, wherein nucleotide monomers are connected by a 3’,5’-phosphodiester bond.

**22.** The double-stranded RNAi agent according to claim 1 or 2, wherein nucleotide monomers are connected by a 3’,5’-phosphodiester bond with thio-modification.

**23.** The double-stranded RNAi agent according to claim 1 or 2, further comprising the following modifications:

the antisense strand is modified with modifications selected from the group consisting of A, Band C:

| No. | **Antisense strand (AS) 5’-3’** | | | | | | | | | | | | | | | | | | | | | | |
|---|---|---|---|---|---|---|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** |
| **A** | 2’-OMe+PS | **2’-F+PS** | 2’-OMe | 2’-OMe | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |
| **B** | 2’-OMe+PS | **2’-F+PS** | **2’-F** | **2’-F** | 2’-OMe | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |
| **C** | 2’-OMe+PS | **2’-F+PS** | 2’-OMe | **2’-F** | **2’-F** | **2’-F** | 2’-OMe | 2’-OMe | 2’-OMe | 2’-OMe | 2’-OMe | 2’-OMe | 2’-OMe | **2’-F** | 2’-OMe | **2’-F** | 2’-OMe | 2’-OMe | 2’-OMe | 2’-OMe | 2’-OMe+PS | 2’-OMe+PS | 2’-OMe |

the sense strand is modified with modifications selected from the group consisting of a and b:

| No. | **Sense strand (SS) 5’-3’** | | | | | | | | | | | | | | | | | | | | |
|---|---|---|---|---|---|---|---|---|---|---|---|---|---|---|---|---|---|---|---|---|---|
| | **1** | **2** | **3** | **4** | **5** | **6** | **7** | **8** | **9** | **10** | **11** | **12** | **13** | **14** | **15** | **16** | **17** | **18** | **19** | **20** | **21** |
| **a** | 2’-OMe+PS | 2’-OMe+PS | 2’-OMe | 2’-OMe | 2’-OMe | 2’-OMe | **2’-F** | 2’-OMe | **2’-F** | **2’-F** | **2’-F** | 2’-OMe | 2’-OMe | 2’-OMe | 2’-OMe | 2’-OMe | **2’-F** | 2’-OMe | 2’-OMe | 2’-OMe | 2’-OMe |
| **b** | 2’-OMe+PS | 2’-OMe+PS | 2’-OMe | 2’-OMe | 2’-OMe | 2’-OMe | **2’-F** | 2’-OMe | **2’-F** | 2’-OMe | **2’-F** | 2’-OMe | 2’-OMe | 2’-OMe | 2’-OMe | 2’-OMe | **2’-F** | 2’-OMe | 2’-OMe | 2’-OMe | 2’-OMe |

in the above tables, 2'-OMe represents 2'-methoxy, 2'-F represents 2'-fluoro, and PS represents a thiophosphate backbone;

wherein the antisense strand is modified with modification A, and the sense strand is modified with modification a; the antisense strand is modified with modification B, and the sense strand is modified with modification a; the antisense strand is modified with modification C, and the sense strand is modified with modification a; the antisense strand is modified with modification B, and the sense strand is modified with modification b; or the antisense strand is modified with modification C, and the sense strand is modified with modification b.

**24.** The double-stranded RNAi agent according to claim 1 or 2, wherein the antisense strand is modified with a modification group in the 2 to 8 positions from the 5'-end, wherein the modification group is selected from the group consisting of UNA, GNA, and DNA, wherein UNA and GNA have the structures of:

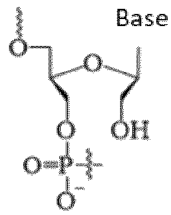

Unlocked Nucleic Acids (UNA)

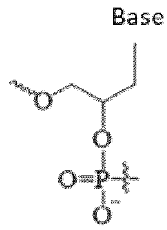

Glycol Nucleic Acids (GNA)

;

wherein the base is selected from the group consisting of adenine, guanine, cytosine, thymine and uracil.

**25.** The double-stranded RNAi agent according to claim 1 or 2, wherein the 5' carbon atom of the glycoside of the 5'-end nucleotide in the modified antisense strand is phosphorylated by a 5' phosphorylation group selected from the group consisting of 5'-(E)-vinyl phosphonate (5'-E-VP), 5'-methyl phosphonate (5'-MP), 5'-C-methyl phosphate, 5'-thiophosphate (5'-PS), and 5'-phosphate (5'-P), with structures of:

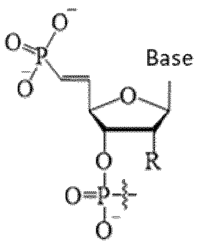

5'-(E)-vinyl phosphonate (5'-E-VP)

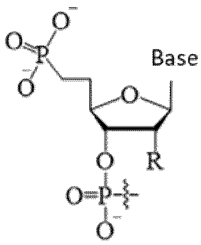

5'-methyl phosphonate (5'-MP)

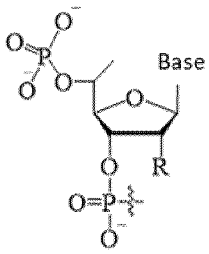

5'-C-methyl phosphate

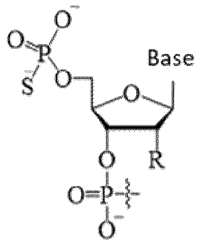

5'-thiophosphate (5'-PS)

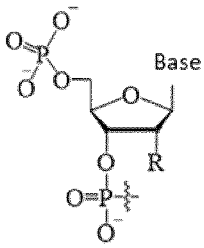

5'-thiophosphate (5'-P)

wherein, R is selected from the group consisting of hydrogen, hydroxyl, amine, $C_{1-4}$ alkyl, aryl, $C_{1-4}$ alkoxy, $C_{1-4}$ alkylcarbonylamino, and halogen; and

the base is selected from the group consisting of adenine, guanine, cytosine, thymine and uracil.

**26.** The double-stranded RNAi agent according to claim 1 or 2, wherein a 3',5'-phosphodiester bond connecting nucleotide monomers at an end of the strand has thio-modification and form a chirality pure 3',5'-thiophosphodiester bond, wherein the 5'-ends of the sense strand and the antisense strand comprise 1-3 thio-modifications as linkages, and the 3'-end of the antisense strand comprises 1-3 thio-modifications as linkages.

**27.** A conjugate for reducing PCSK9 expression, comprising the double-stranded RNAi agent according to any one of claims 1 to 26, and a ligand conjugated thereto.

**28.** The conjugate according to claim 27, wherein the ligand is conjugated to the 3'-end or the 5'-end of the sense strand of the oligonucleotide.

**29.** The conjugate according to claim 27 or 28, wherein the ligand is one or more GalNAc derivatives attached via a

divalent or trivalent branched linker.

**30.** The conjugate according to claim 27 or 28, wherein the ligand is:

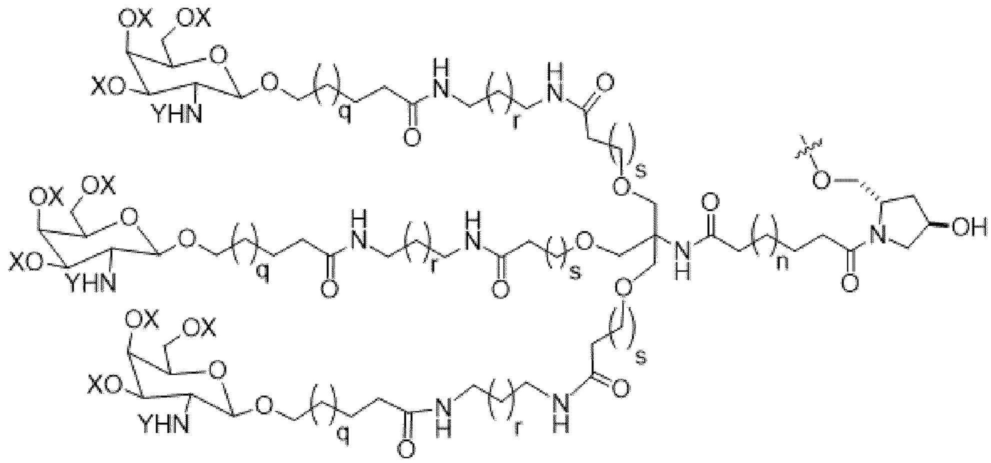

,

wherein X is hydrogen or a hydroxyl protecting group or H, wherein the hydroxyl protecting group is selected from the group consisting of acetyl, benzoyl and isobutyryl; Y is an amine protecting group or H, wherein the amine protecting group is selected from the group consisting of formyl, acetyl, propionyl, n-butyryl and isobutyryl; n is an integer selected from 0 to 20; and q, r and s are independently integers selected from 1 to 7.

**31.** The conjugate according to claim 30, wherein the ligand is:

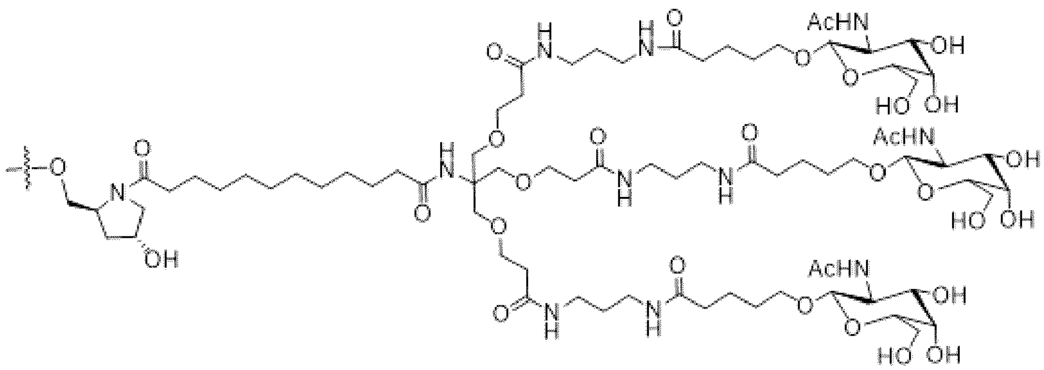

.

**32.** The conjugate according to claim 27 or 28, wherein the ligand is:

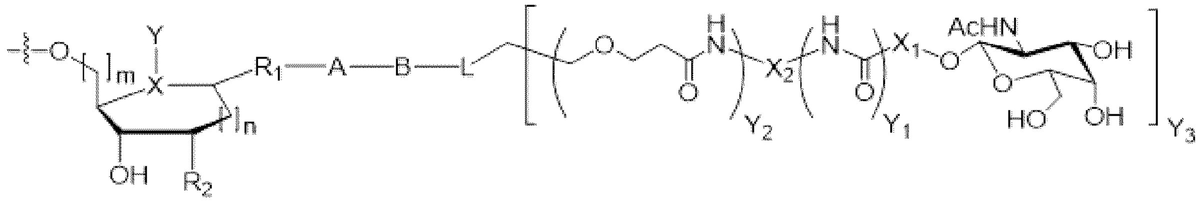

,

wherein, X is selected from the group consisting of oxygen, nitrogen and sulfur;
Y is alkyl or aryl;
$R_1$ is oxygen or sulfur;
$R_2$ is selected from the group consisting of hydrogen, amine, $C_{1-4}$ alkyl, aryl, $C_{1-4}$ alkoxy and halogen;
A is selected from the group consisting of $-(CH_2)_a-$, $-(CH_2CH_2O)_b-$, $-((CH_2)_cNHCO)_d-$, and $-((CH_2)_cCONH)_d-$, wherein a is an integer selected from 1-15, b is an integer selected from 1-7, c is an integer selected from 1-7, and d is an integer selected from 1-5;

B is $-(CH_2)_e-$, wherein e is an integer selected from 0 to 7;
L is-CONH- or -NHCO-;
$X_1$ is $-(CH_2)_f-$ or $-(CH_2CH_2O)_fCH_2-$, wherein f is an integer selected from 1 to 5;
$X_2$ is $-(CH_2)_g-$, wherein g is an integer selected from 1 to 6;
$Y_1$ is 0 or 1;
$Y_2$ is 0, 1 or 2;
$Y_3$ is 1, 2 or 3;
m is an integer selected from 0 to 4; and
n is an integer selected from 0 to 4.

**33.** The conjugate according to claim 32, wherein the ligand is G4, G5, G6 or G7:

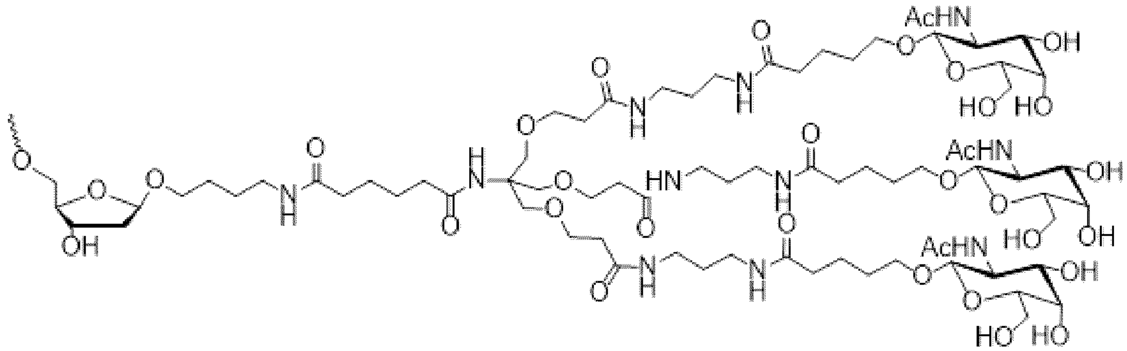

G4

,

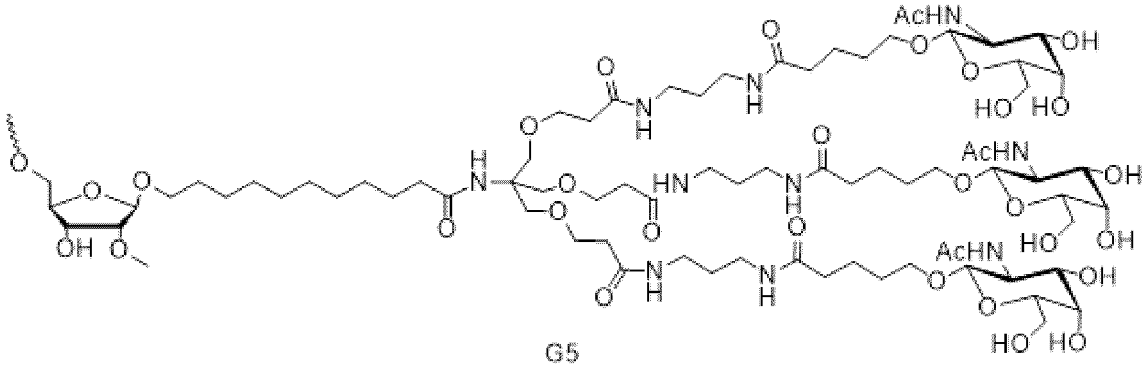

G5

,

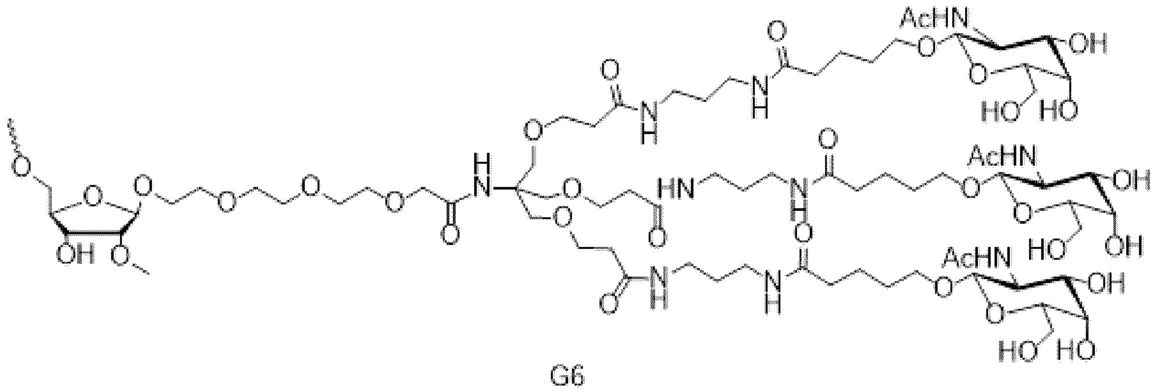

G6

,

or

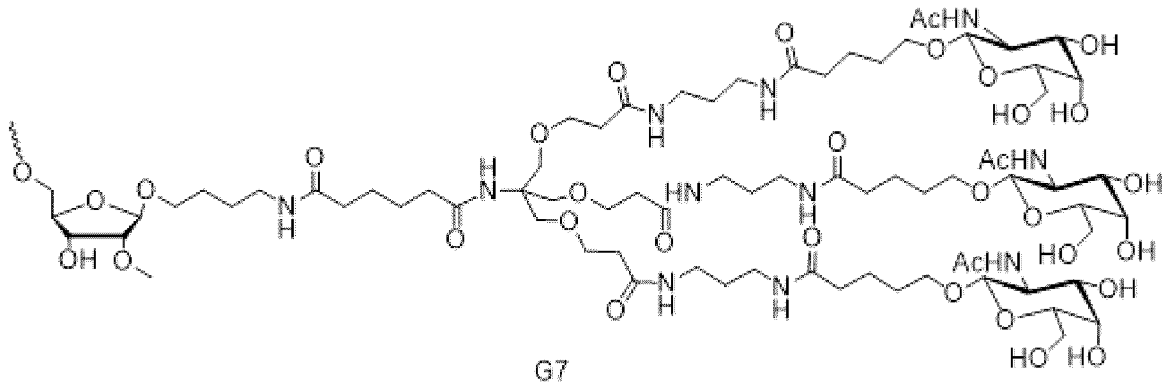

G7

.

**34.** The conjugate according to claim 27 or 28, which has a structure selected from the group consisting of:

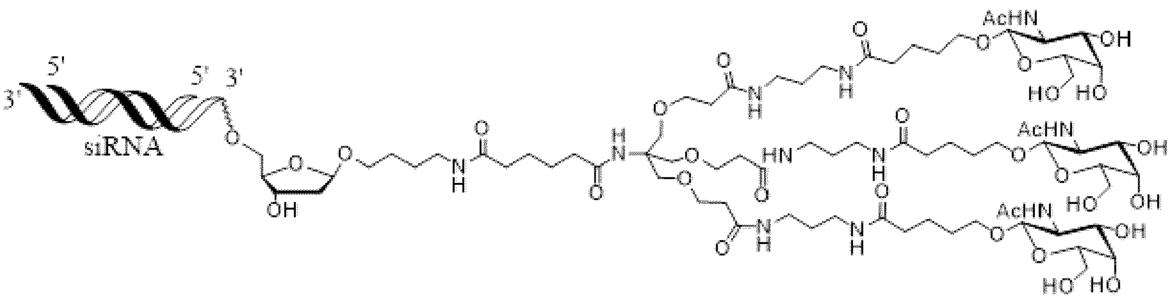

,

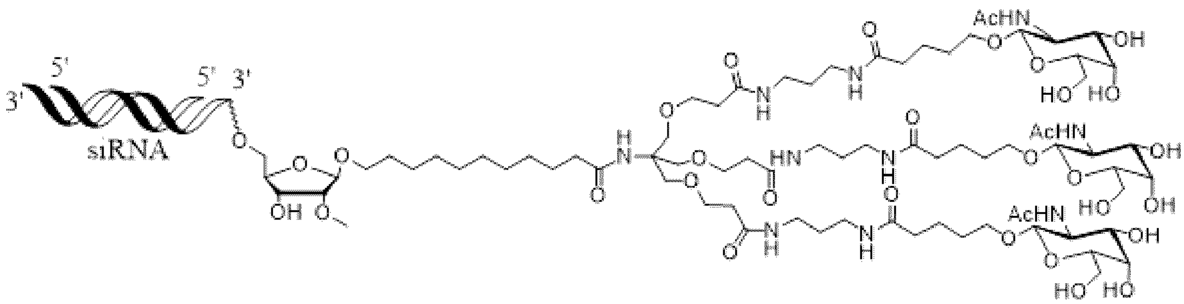

,

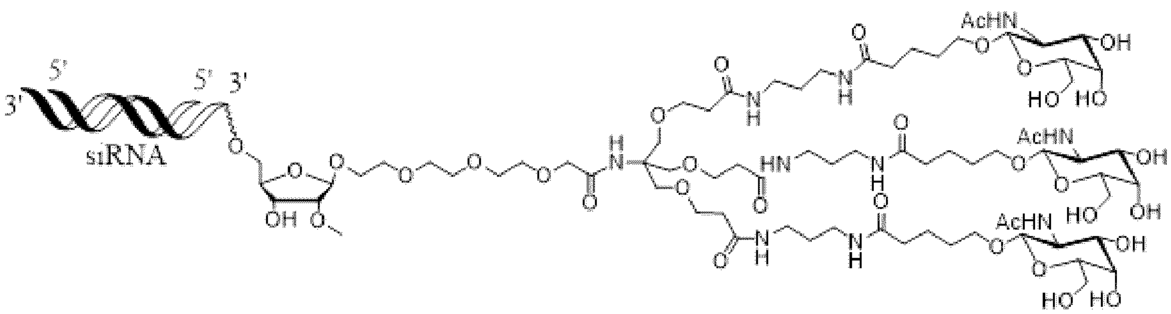

,

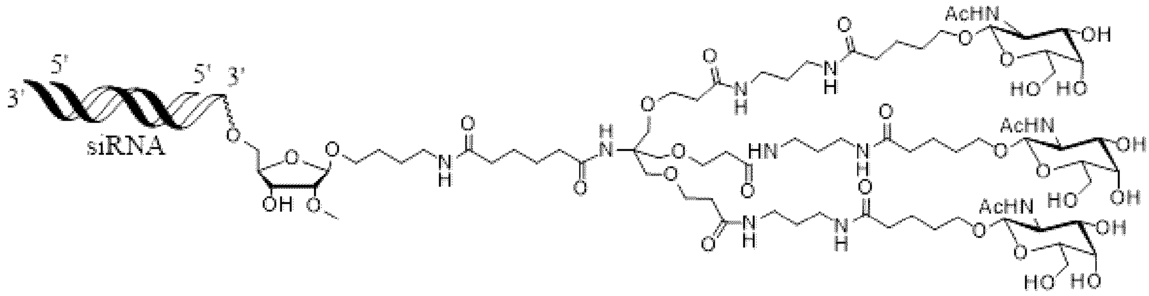

,

or

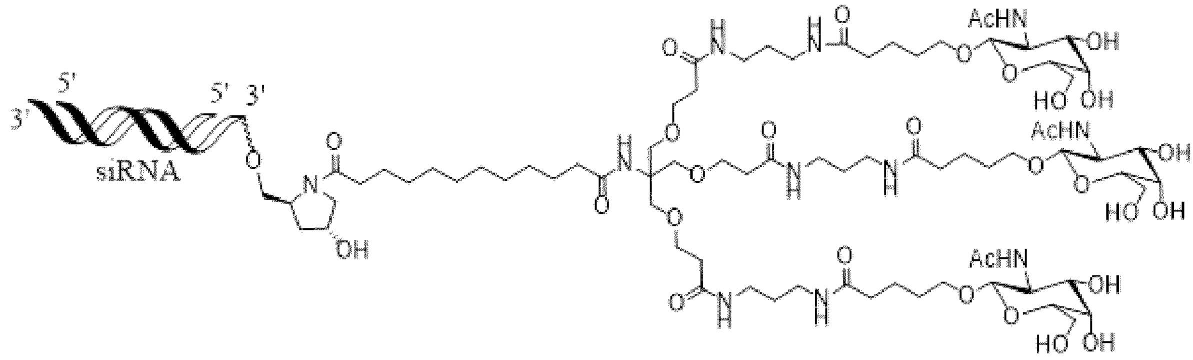

.

**35.** The conjugate according to claim 27 or 28, wherein the double-stranded RNAi agent comprises an oligonucleotide duplex consisting of paired sense strand and antisense strand, wherein

(1) the sense strand has a sequence set forth in SEQ ID NO: 337; and the antisense strand has a sequence set forth in SEQ ID NO: 427;
(2) the sense strand has a sequence set forth in SEQ ID NO: 337; and the antisense strand has a sequence set forth in SEQ ID NO: 428;
(3) the sense strand has a sequence set forth in SEQ ID NO: 342; and the antisense strand has a sequence set forth in SEQ ID NO: 381;
(4) the sense strand has a sequence set forth in SEQ ID NO: 342; and the antisense strand has a sequence set forth in SEQ ID NO: 430;
(5) the sense strand has a sequence set forth in SEQ ID NO: 342; and the antisense strand has a sequence set forth in SEQ ID NO: 431;
(6) the sense strand has a sequence set forth in SEQ ID NO: 347; and the antisense strand has a sequence set forth in SEQ ID NO: 384;
(7) the sense strand has a sequence set forth in SEQ ID NO: 347; and the antisense strand has a sequence set forth in SEQ ID NO: 437;
(8) the sense strand has a sequence set forth in SEQ ID NO: 347; and the antisense strand has a sequence set forth in SEQ ID NO: 438;
(9) the sense strand has a sequence set forth in SEQ ID NO: 348; and the antisense strand has a sequence set forth in SEQ ID NO: 385;
(10) the sense strand has a sequence set forth in SEQ ID NO: 352; and the antisense strand has a sequence set forth in SEQ ID NO: 448;
(11) the sense strand has a sequence set forth in SEQ ID NO: 353; and the antisense strand has a sequence set forth in SEQ ID NO: 390;
(12) the sense strand has a sequence set forth in SEQ ID NO: 353; and the antisense strand has a sequence set forth in SEQ ID NO: 448;
(14) the sense strand has a sequence set forth in SEQ ID NO: 357; and the antisense strand has a sequence set forth in SEQ ID NO: 393;
(15) the sense strand has a sequence set forth in SEQ ID NO: 357; and the antisense strand has a sequence set forth in SEQ ID NO: 446; or
(16) the sense strand has a sequence set forth in SEQ ID NO: 357; and the antisense strand has a sequence set

forth in SEQ ID NO: 447;

wherein, the oligonucleotide duplex is conjugated with ligand G4, G5, G6 or G7.

**36.** The conjugate according to claim 27 or 28, wherein the double-stranded RNAi agent comprises an oligonucleotide duplex consisting of paired sense strand and antisense strand, wherein

(1) the sense strand has a sequence set forth in SEQ ID NO: 352; and the antisense strand has a sequence set forth in SEQ ID NO: 448;
(2) the sense strand has a sequence set forth in SEQ ID NO: 353; and the antisense strand has a sequence set forth in SEQ ID NO: 390; or
(3) the sense strand has a sequence set forth in SEQ ID NO: 353; and the antisense strand has a sequence set forth in SEQ ID NO: 448;

wherein, the oligonucleotide duplex is conjugated with ligand G4, G5, G6 or G7.

**37.** The conjugate according to claim 27 or 28, wherein the double-stranded RNAi agent comprises an oligonucleotide duplex consisting of paired sense strand and antisense strand, wherein the sense strand has a sequence set forth in SEQ ID NO: 353, the antisense strand has a sequence set forth in SEQ ID NO: 448, and the oligonucleotide duplex is conjugated with ligand G5.

**38.** A pharmaceutical composition, comprising the double-stranded RNAi agent according to any one of claims 1 to 26 or the conjugate according to any one of claims 27 to 37, and a pharmaceutically acceptable carrier.

**39.** Use of the double-stranded RNAi agent according to any one of claims 1 to 26, the conjugate according to any one of claims 27 to 37, or the pharmaceutical composition according to claim 38 in the manufacture of a medicament for treating a PCSK9-related disease.

**40.** The use according to claim 39, wherein the PCSK9-related disease is selected from the group consisting of hypercholesterolemia, atherosclerosis, dyslipidemia, and cardiovascular and cerebrovascular diseases.

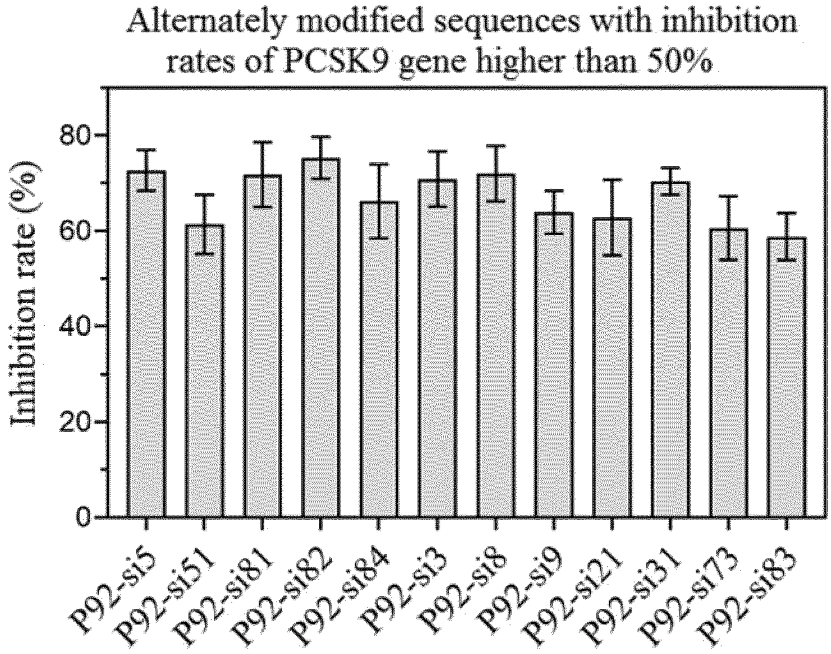
Alternately modified sequences with inhibition
rates of PCSK9 gene higher than 50%
Inhibition rate (%)
80
60
40
20
0
P92-si5
P92-si51
P92-si81
P92-si82
P92-si84
P92-si3
P92-si8
P92-si9
P92-si21
P92-si31
P92-si73
P92-si83

**FIG. 1**

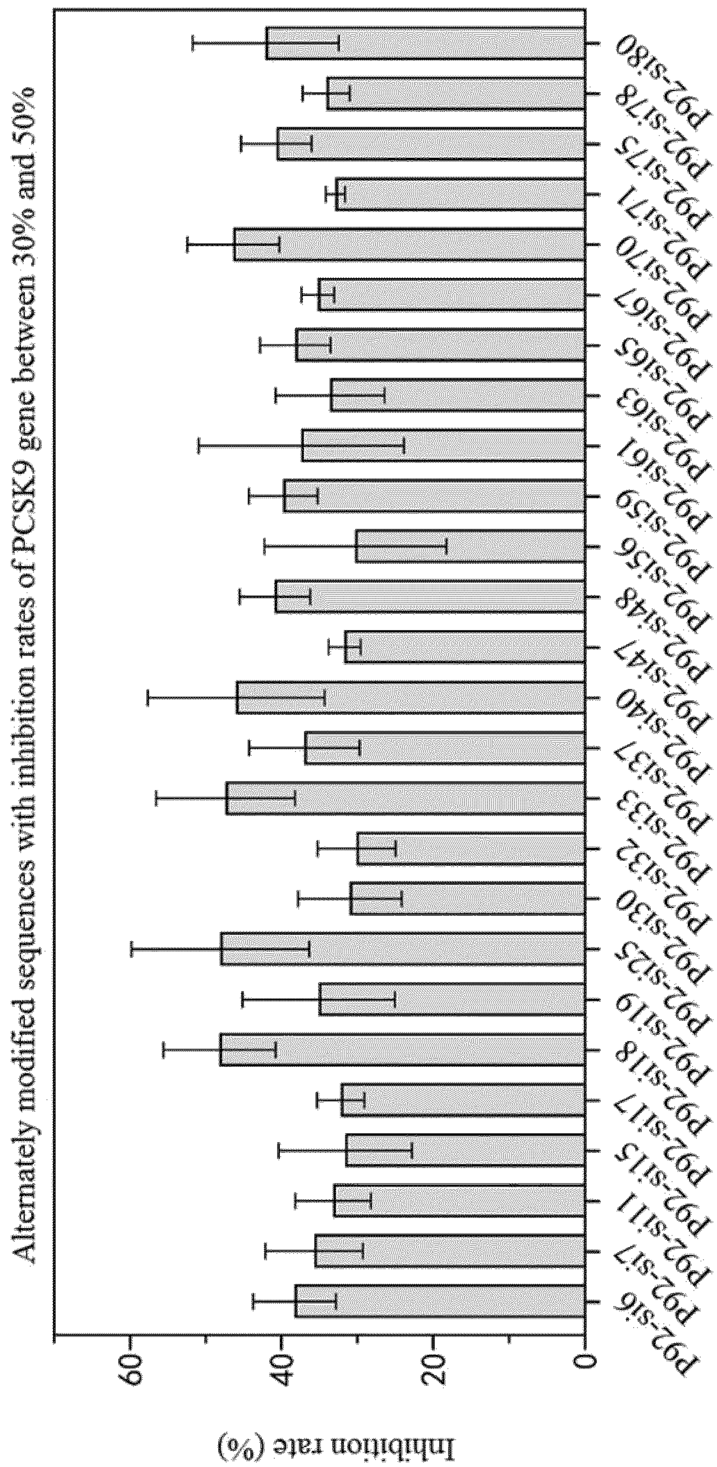
Alternately modified sequences with inhibition rates of PCSK9 gene between 30% and 50%
Inhibition rate (%)
0
20
40
60
P92-si6
P92-si7
P92-si11
P92-si15
P92-si17
P92-si18
P92-si19
P92-si25
P92-si30
P92-si32
P92-si33
P92-si37
P92-si40
P92-si47
P92-si48
P92-si56
P92-si59
P92-si61
P92-si63
P92-si65
P92-si67
P92-si70
P92-si71
P92-si75
P92-si78
P92-si80

FIG. 2

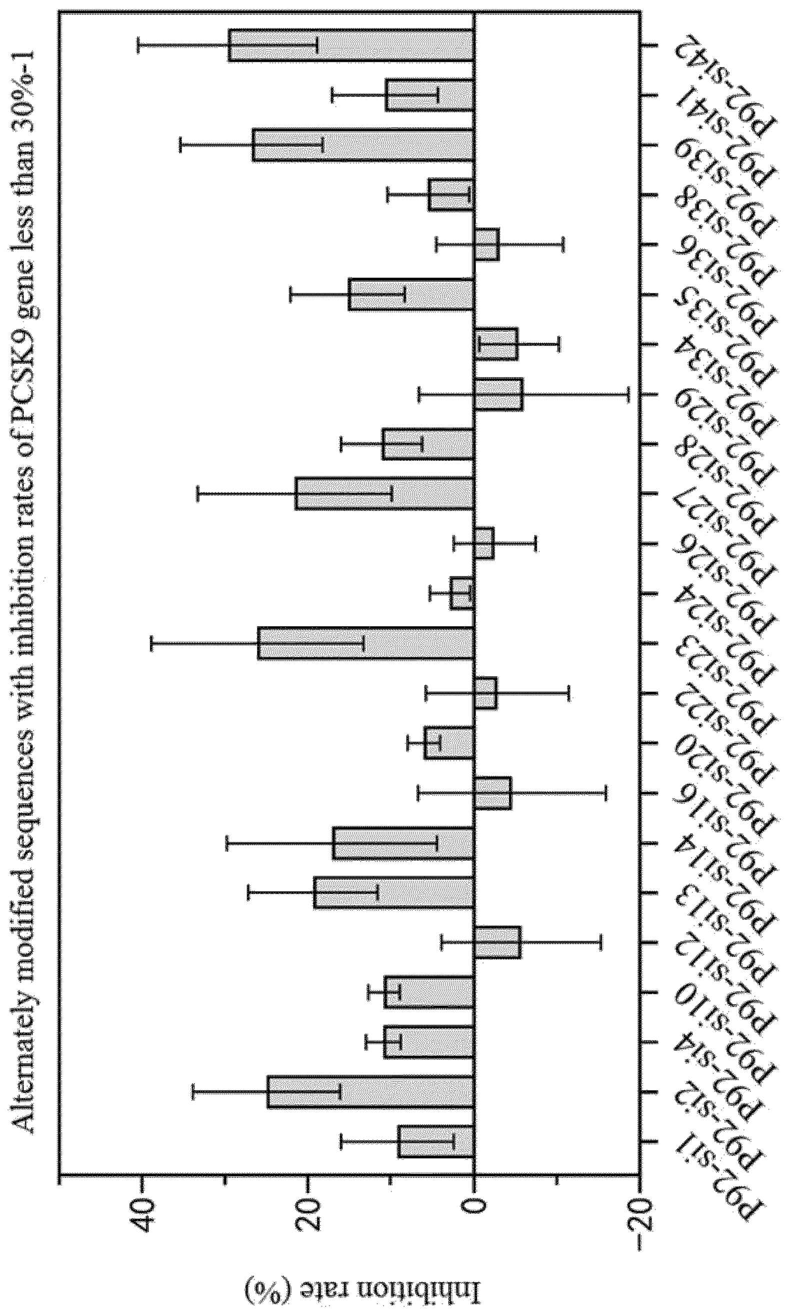
Alternately modified sequences with inhibition rates of PCSK9 gene less than 30%-1
Inhibition rate (%)
40
20
0
−20
p92-si1
p92-si2
p92-si4
p92-si10
p92-si12
p92-si13
p92-si14
p92-si16
p92-si20
p92-si22
p92-si23
p92-si24
p92-si26
p92-si27
p92-si28
p92-si29
p92-si34
p92-si35
p92-si36
p92-si38
p92-si39
p92-si41
p92-si42

FIG. 3

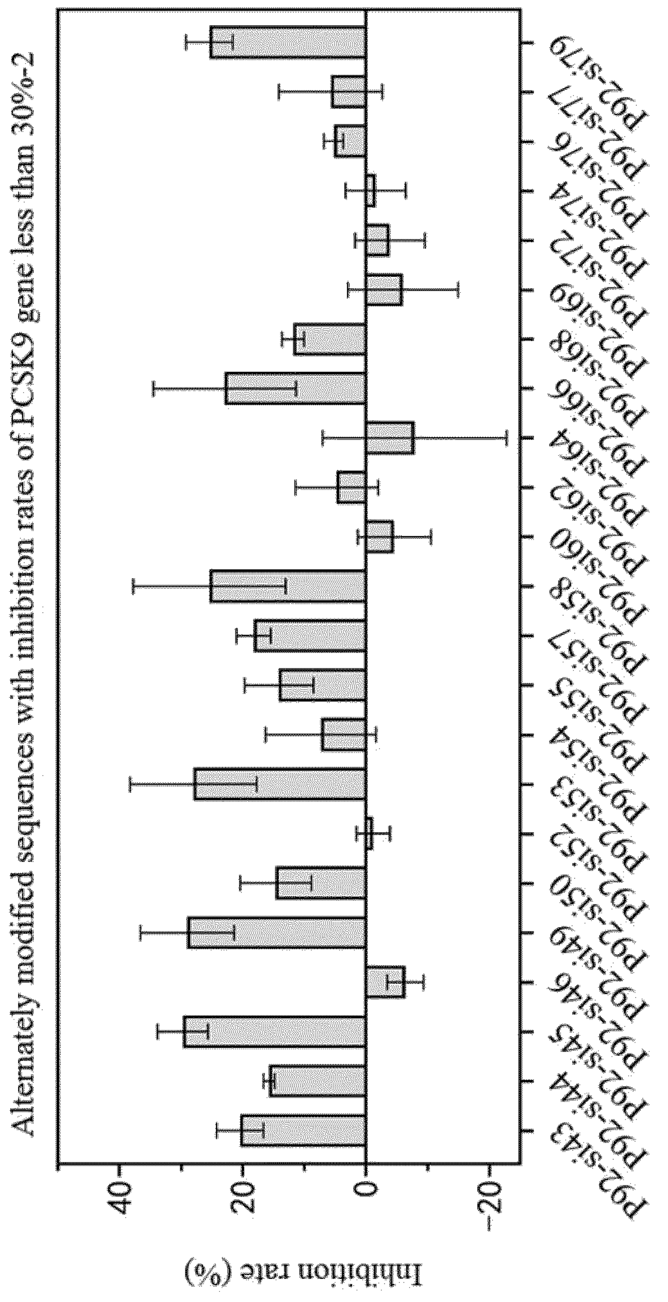
Alternately modified sequences with inhibition rates of PCSK9 gene less than 30%-2
Inhibition rate (%)
40
20
0
-20
P92-si43
P92-si44
P92-si45
P92-si46
P92-si49
P92-si50
P92-si52
P92-si53
P92-si54
P92-si55
P92-si57
P92-si58
P92-si60
P92-si62
P92-si64
P92-si66
P92-si68
P92-si69
P92-si72
P92-si74
P92-si76
P92-si77
P92-si79

FIG. 4

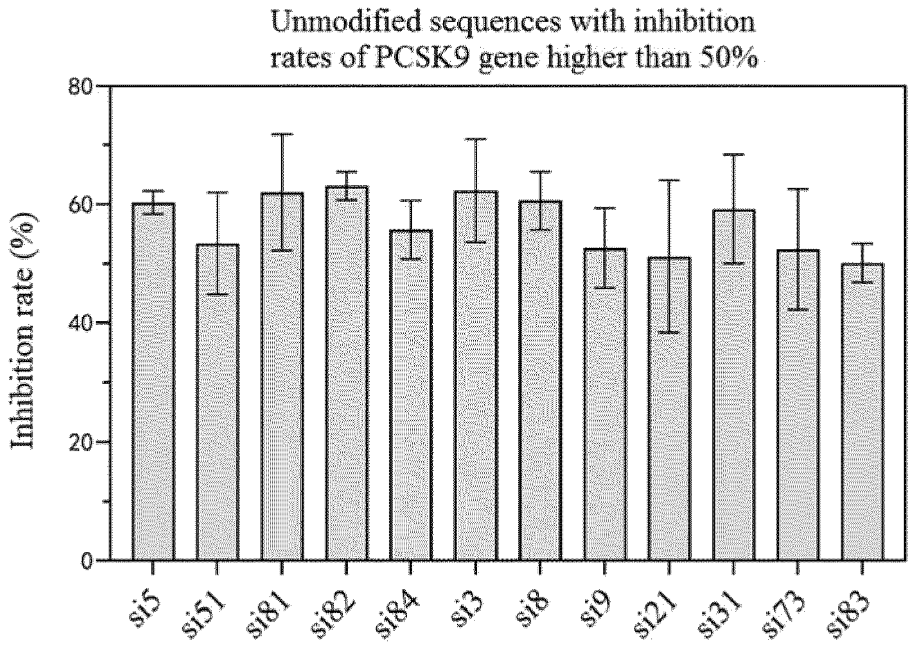
Unmodified sequences with inhibition
rates of PCSK9 gene higher than 50%
Inhibition rate (%)
80
60
40
20
0
si5
si51
si81
si82
si84
si3
si8
si9
si21
si31
si73
si83

**FIG. 5**

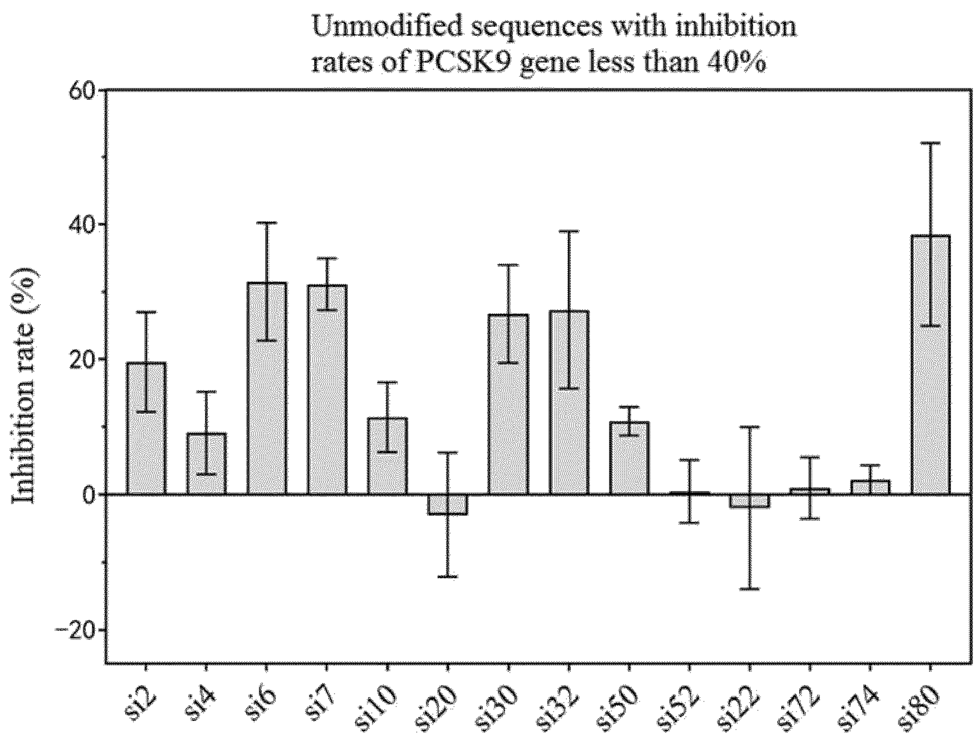
Unmodified sequences with inhibition
rates of PCSK9 gene less than 40%
Inhibition rate (%)
60
40
20
0
-20
si2
si4
si6
si7
si10
si20
si30
si32
si50
si52
si22
si72
si74
si80

**FIG. 6**

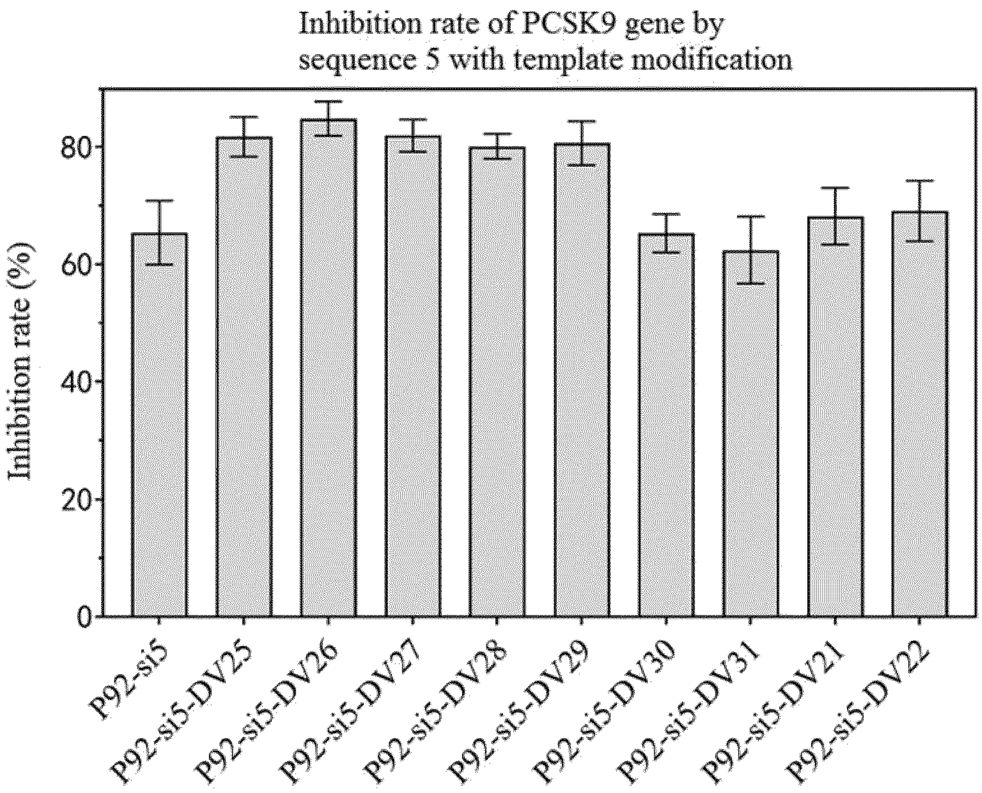
Inhibition rate of PCSK9 gene by
sequence 5 with template modification
Inhibition rate (%)
0
20
40
60
80
P92-si5
P92-si5-DV25
P92-si5-DV26
P92-si5-DV27
P92-si5-DV28
P92-si5-DV29
P92-si5-DV30
P92-si5-DV31
P92-si5-DV21
P92-si5-DV22

**FIG. 7**

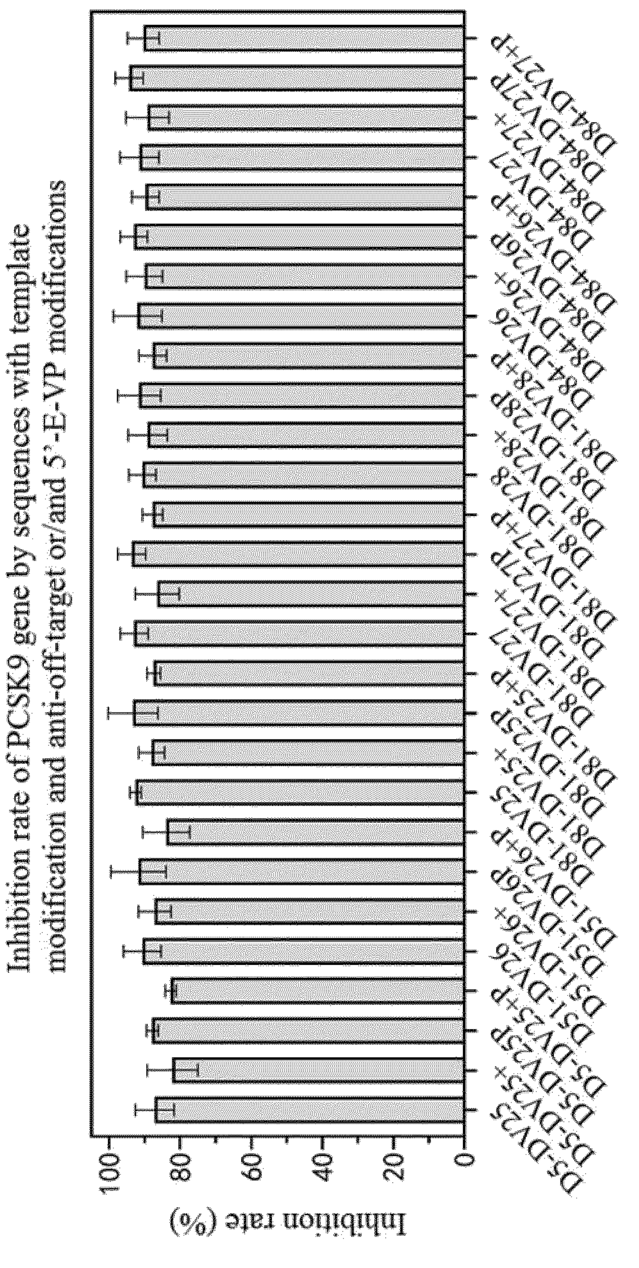

Inhibition rate of PCSK9 gene by sequences with template modification and anti-off-target or/and 5'-E-VP modifications
Inhibition rate (%)
0
20
40
60
80
100
D5-DV25
D5-DV25×
D5-DV25P
D5-DV25+P
D51-DV26
D51-DV26×
D51-DV26P
D51-DV26+P
D81-DV25
D81-DV25×
D81-DV25P
D81-DV25+P
D81-DV27
D81-DV27×
D81-DV27P
D81-DV27+P
D81-DV28
D81-DV28×
D81-DV28P
D81-DV28+P
D84-DV26
D84-DV26×
D84-DV26P
D84-DV26+P
D84-DV27
D84-DV27×
D84-DV27P
D84-DV27+P


FIG. 8

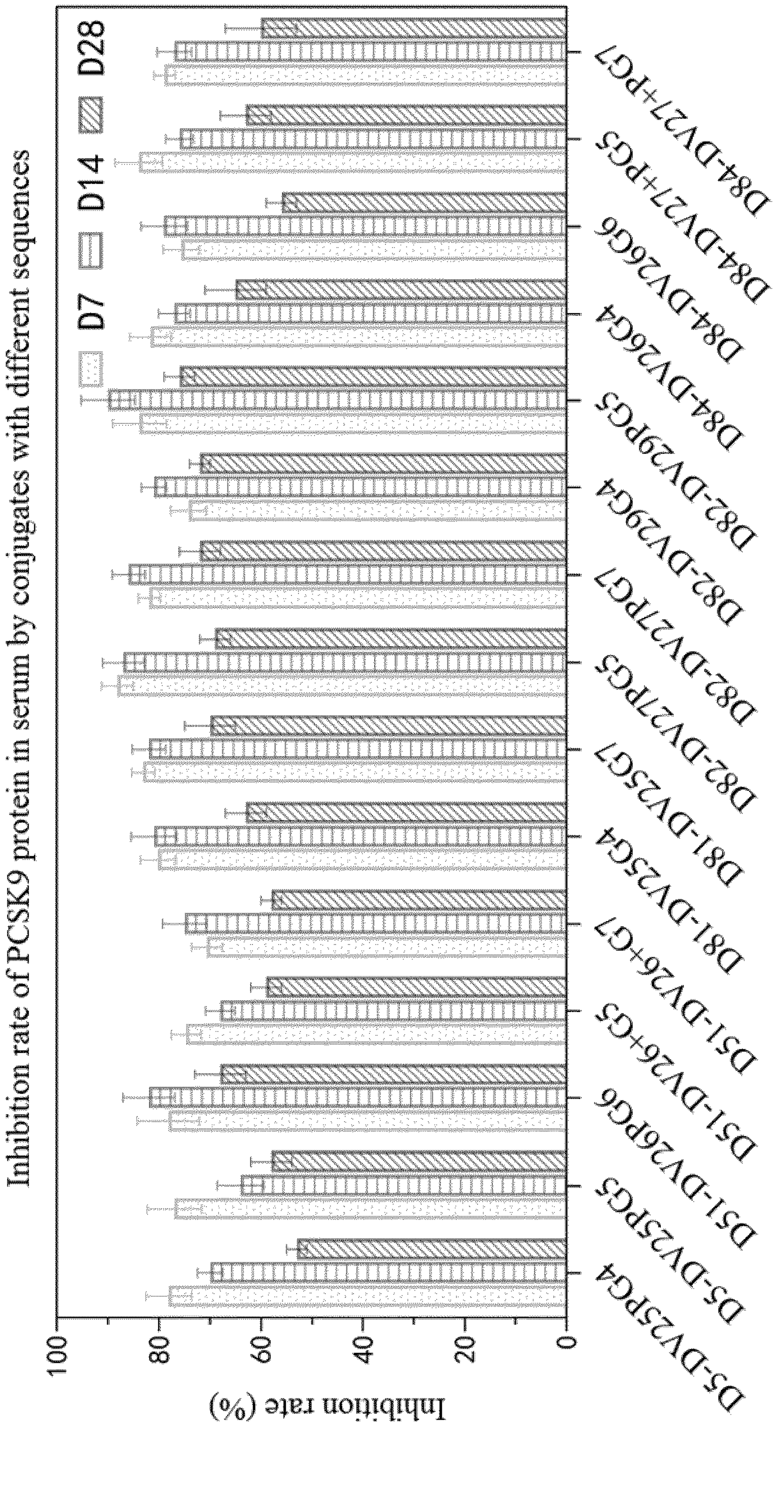

Inhibition rate of PCSK9 protein in serum by conjugates with different sequences
D7
D14
D28
Inhibition rate (%)
100
80
60
40
20
0
D5-DV25PG4
D5-DV25PG5
D51-DV26PG6
D51-DV26+G5
D51-DV26+G7
D81-DV25G4
D81-DV25G7
D82-DV27PG5
D82-DV27PG7
D82-DV29G4
D82-DV29PG5
D84-DV26G4
D84-DV26G6
D84-DV27+PG5
D84-DV27+PG7


FIG. 9

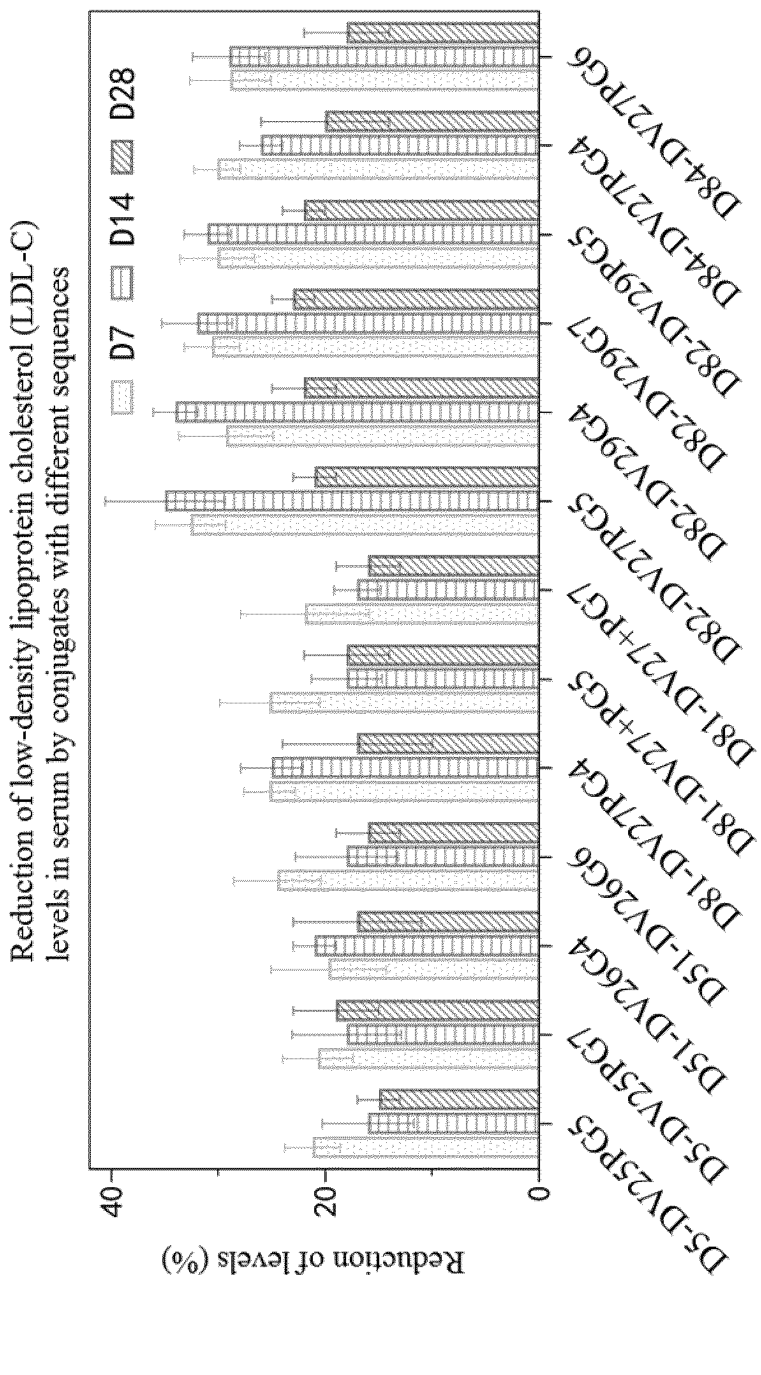
Reduction of low-density lipoprotein cholesterol (LDL-C)
levels in serum by conjugates with different sequences
D7
D14
D28
Reduction of levels (%)
40
20
0
D5-DV25PG5
D5-DV25PG7
D51-DV26G4
D51-DV26G6
D81-DV27PG4
D81-DV27+PG5
D81-DV27+PG7
D82-DV27PG5
D82-DV29G4
D82-DV29G7
D84-DV29PG5
D84-DV27PG4
D84-DV27PG6

FIG. 10

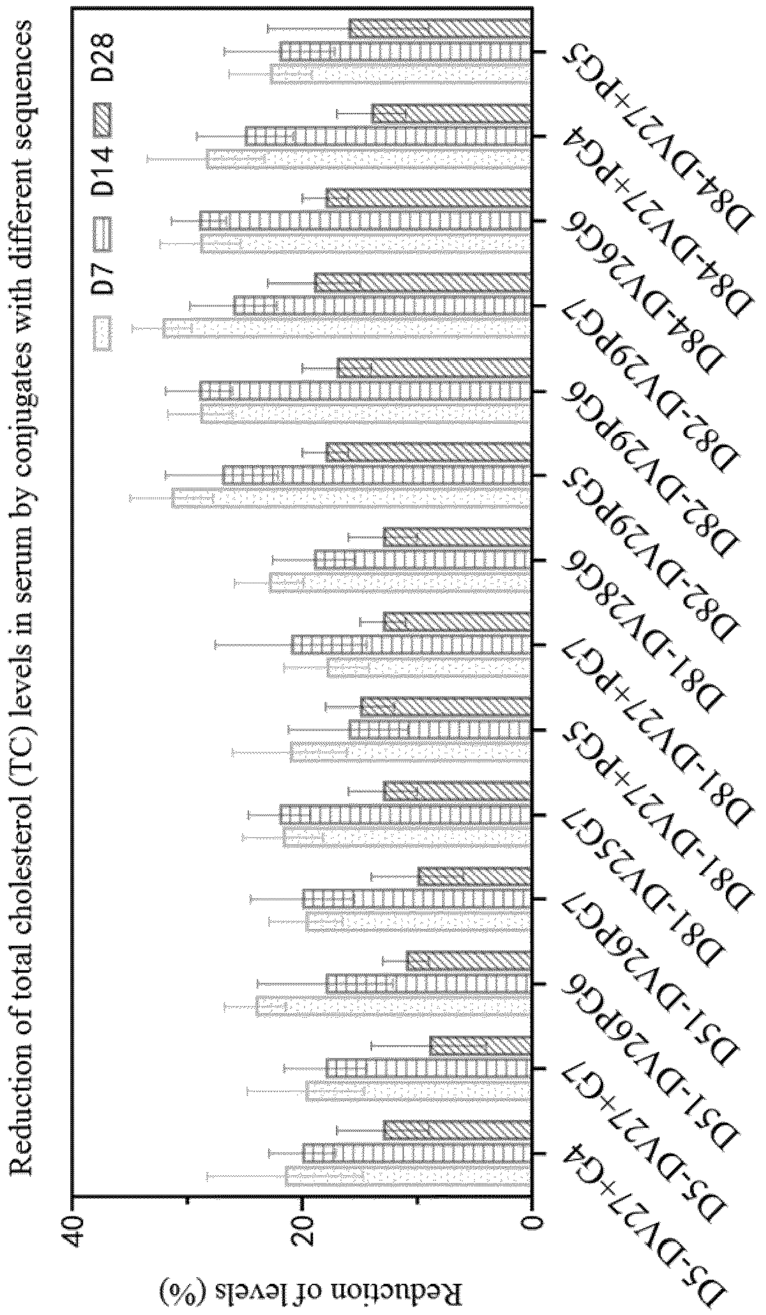
Reduction of total cholesterol (TC) levels in serum by conjugates with different sequences
D7
D14
D28
Reduction of levels (%)
0
20
40
D5-DV27+G4
D5-DV27+G7
D51-DV26PG6
D51-DV26PG7
D81-DV25G7
D81-DV27+PG5
D81-DV27+PG7
D81-DV28G6
D82-DV29PG5
D82-DV29PG6
D82-DV29PG7
D84-DV26G6
D84-DV27+PG4
D84-DV27+PG5

FIG. 11

## INTERNATIONAL SEARCH REPORT

International application No.
**PCT/CN2024/082133**

**A. CLASSIFICATION OF SUBJECT MATTER**

C12N15/113(2010.01)i; A61K31/713(2006.01)i; A61P3/06(2006.01)i; A61P9/10(2006.01)i; A61P9/00(2006.01)i

According to International Patent Classification (IPC) or to both national classification and IPC

**B. FIELDS SEARCHED**

Minimum documentation searched (classification system followed by classification symbols)

IPC: C12N; A61K; A61P

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, Wanfang, 百度, Baidu, bing, PUBMED, ISI Web of Knowledge, NCBI GenBank, EBI-EMBL, STN, 读秀, DUXIU: SEQ ID Nos: 1 and 13, PCSK9, siRNA, RNAi, 正义链, 反义链, 修饰, 配体, 缀合, 乙酰半乳糖胺, 高胆固醇血症, 动脉粥样硬化, 血脂异常, 心脑血管疾病, Sense strand, antisense strand, modification, ligand, conjugation, acetylgalactosamine, hypercholesterolemia, atherosclerosis, dyslipidemia, cardiocerebrovascular disease.

**C. DOCUMENTS CONSIDERED TO BE RELEVANT**

| Category* | Citation of document, with indication, where appropriate, of the relevant passages | Relevant to claim No. |
|---|---|---|
| PX | CN 117210468 A (BEIJING YOUCARE KECHUANG PHARMACEUTICAL TECHNOLOGY CO., LTD.) 12 December 2023 (2023-12-12)<br>claims 1-40 | 1-35, 38-40 |
| X | CN 109957565 A (GUANGZHOU RIBOBIO CO., LTD.) 02 July 2019 (2019-07-02)<br>claims 1-8 and 10, description, paragraphs 97 and 154-157, and sequence listing, sequences 25-26 | 1-23, 25-35, 38-40 |
| Y | CN 109957565 A (GUANGZHOU RIBOBIO CO., LTD.) 02 July 2019 (2019-07-02)<br>claims 1-8 and 10, description, paragraphs 97 and 154-157, and sequence listing, sequences 25-26 | 24, 27-35, 38-40 |
| Y | CN 115677810 A (SUZHOU RIBO LIFE SCIENCE CO., LTD. et al.) 03 February 2023 (2023-02-03)<br>claims 1-37, and description, paragraphs 85 and 104-106 | 24, 27-35, 38-40 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 July 2024** | **23 July 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2024/082133**

**C. DOCUMENTS CONSIDERED TO BE RELEVANT**

| Category* | Citation of document, with indication, where appropriate, of the relevant passages | Relevant to claim No. |
|---|---|---|
| A | WO 2023049294 A2 (SIRIUS THERAPEUTICS, INC.) 30 March 2023 (2023-03-30)<br>entire document | 1-35, 38-40 |
| A | 孟凡华等 (MENG, Fanhua et al.). "一种新型的治疗血脂异常的干扰小RNA药物 (Inclisiran, A New Small Interfering RNA Drug for Treatment of Dyslipidemia)"<br>*心血管病学进展 (Advances in Cardiovascular Diseases),*<br>Vol. 42, No. (2), 28 February 2021 (2021-02-28),<br>pp. 167-170 | 1-35, 38-40 |
| A | ZHANG, Y. et al. "Inclisiran: A New Generation of Lipid-Lowering siRNA Therapeutic"<br>*Frontiers in Pharmacology,* 13 October 2023 (2023-10-13),<br>pp. 1-9 | 1-35, 38-40 |

Form PCT/ISA/210 (second sheet) (July 2022)

**INTERNATIONAL SEARCH REPORT**

International application No.
**PCT/CN2024/082133**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT

International application No.
PCT/CN2024/082133

**Box No. III Observations where unity of invention is lacking (Continuation of item 3 of first sheet)**

This International Searching Authority found multiple inventions in this international application, as follows:

The international application has 12 claimed inventions:

I: claims 1-35 (in part) and 38-40 (in part) relate to a double-stranded RNAi agent for reducing the expression of PCSK9 and a conjugate thereof, wherein the double-stranded RNAi agent contains an oligonucleotide duplex formed by pairing a sense strand and an antisense strand, the sense strand has a sequence as shown in SEQ ID NO. 1 or a fragment thereof, or a modification sequence of the sequence or the fragment thereof, and the antisense strand has a sequence as shown in SEQ ID NO. 13 or a fragment thereof, or a modification sequence of the sequence or the fragment thereof; and a pharmaceutical composition of the double-stranded RNAi agent and the conjugate thereof for treating PCSK9-related diseases and the use thereof.

II: claims 1-35 (in part) and 38-40 (in part) relate to a double-stranded RNAi agent for reducing the expression of PCSK9 and a conjugate thereof, wherein the double-stranded RNAi agent contains an oligonucleotide duplex formed by pairing a sense strand and an antisense strand, the sense strand has a sequence as shown in SEQ ID NO. 2 or a fragment thereof, or a modification sequence of the sequence or the fragment thereof, and the antisense strand has a sequence as shown in SEQ ID NO. 14 or a fragment thereof, or a modification sequence of the sequence or the fragment thereof; and a pharmaceutical composition of the double-stranded RNAi agent and the conjugate thereof for treating PCSK9-related diseases and the use thereof.

III: claims 1-35 (in part) and 38-40 (in part) relate to a double-stranded RNAi agent for reducing the expression of PCSK9 and a conjugate thereof, wherein the double-stranded RNAi agent contains an oligonucleotide duplex formed by pairing a sense strand and an antisense strand, the sense strand has a sequence as shown in SEQ ID NO. 3 or a fragment thereof, or a modification sequence of the sequence or the fragment thereof, and the antisense strand has a sequence as shown in SEQ ID NO. 15 or a fragment thereof, or a modification sequence of the sequence or the fragment thereof; and a pharmaceutical composition of the double-stranded RNAi agent and the conjugate thereof for treating PCSK9-related diseases and the use thereof.

IV: claims 1-35 (in part), 36-37 and 38-40 (in part) relate to a double-stranded RNAi agent for reducing the expression of PCSK9 and a conjugate thereof, wherein the double-stranded RNAi agent contains an oligonucleotide duplex formed by pairing a sense strand and an antisense strand, the sense strand has a sequence as shown in SEQ ID NO. 4 or a fragment thereof, or a modification sequence of the sequence or the fragment thereof, and the antisense strand has a sequence as shown in SEQ ID NO. 16 or a fragment thereof, or a modification sequence of the sequence or the fragment thereof; and a pharmaceutical composition of the double-stranded RNAi agent and the conjugate thereof for treating PCSK9-related diseases and the use thereof.

V: claims 1-35 (in part) and 38-40 (in part) relate to a double-stranded RNAi agent for reducing the expression of PCSK9 and a conjugate thereof, wherein the double-stranded RNAi agent contains an oligonucleotide duplex formed by pairing a sense strand and an antisense strand, the sense strand has a sequence as shown in SEQ ID NO. 5 or a fragment thereof, or a modification sequence of the sequence or the fragment thereof, and the antisense strand has a sequence as shown in SEQ ID NO. 17 or a fragment thereof, or a modification sequence of the sequence or the fragment thereof; and a pharmaceutical composition of the double-stranded RNAi agent and the conjugate thereof for treating PCSK9-related diseases and the use thereof.

VI-XII: claims 1-35 (in part) and 38-40 (in part) relate to double-stranded RNAi agents for reducing the expression of PCSK9 and conjugates thereof, wherein the double-stranded RNAi agents contain oligonucleotide duplexes formed by pairing sense strands and antisense strands, the sense strands have sequences as shown in SEQ ID NOs. 6-12 or fragments thereof, or modification sequences of the sequences or the fragments thereof, respectively, and the antisense strands have sequences as shown in SEQ ID NOs. 18-24 or fragments thereof, or modification sequences of the sequences or the fragments thereof, respectively; and pharmaceutical compositions of the double-stranded RNAi agents and the conjugates thereof for treating PCSK9-related diseases and the uses thereof.

The same or corresponding technical features of inventions I-XII described above are: a double-stranded RNAi agent for reducing the expression of PCSK9 and a conjugate thereof, and the two forming a pharmaceutical composition. However, the same or corresponding technical features described above are disclosed in D1 (CN 109957565 A, publication date: 02 July 2019): an siRNA molecule for inhibiting the gene expression of PCSK9, the siRNA molecule comprising a sense strand and an antisense strand complementary to each other to form a double-stranded region, wherein the sense strand and/or the antisense strand comprise 15-27 nucleotides or are composed of 15-27 nucleotides, the length of the double-stranded region is 15-25 bp, and at least one nucleotide in the siRNA molecule is modified; the siRNA molecule is further modified by a ligand, the ligand modification involves performing one or more ligand modifications on the 3' end, the 5' end and/or the middle of the sequence

**INTERNATIONAL SEARCH REPORT**

International application No.
**PCT/CN2024/082133**

**Box No. III Observations where unity of invention is lacking (Continuation of item 3 of first sheet)**

of the siRNA molecule, and the ligand is selected from the following: cholesterol, biotin, vitamin, a galactose derivative or analogue, a lactose derivative or analogue, an N-acetylgalactosamine derivative or analogue, and an N-acetylglucosamine derivative or analogue (equivalent to a conjugate of an siRNA); and the siRNA or ligand-modified siRNA and other pharmaceutically acceptable components are combined to form a pharmaceutical composition for treating diseases mediated by a PCSK9 gene, such as hypercholesterolemia, atherosclerosis cardiovascular diseases and dyslipidemia (see D1, claims 1-8 and 10, and description, paragraph 97). It can be seen that the same or corresponding technical features described above do not belong to special technical features as defined in PCT Rule 13.2. Therefore, inventions I-XII described above lack unity of invention (PCT Rule 13.1).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-35 (in part), 38-40 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/082133**

| Patent document cited in search report | Publication date (day/month/year) | Patent family member(s) | Publication date (day/month/year) |
|---|---|---|---|
| CN 117210468 A | 12 December 2023 | None | |
| CN 109957565 A | 02 July 2019 | None | |
| CN 115677810 A | 03 February 2023 | None | |
| WO 2023049294 A2 | 30 March 2023 | WO 2023049294 A3 | 16 November 2023 |
| | | CA 3232743 A1 | 30 March 2023 |
| | | AU 2022349448 A1 | 11 April 2024 |
| | | TW 202321444 A | 01 June 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311463375
- US 8349809 B
- US 8513207 B
- CN 115784921 B
- CN 115677518 B
- CN 115745820 B
- CN 108368028 B
- CN 110520409 A
- CN 102625696 B
- US 10125369 B2
- CN 115992138 A
- WO 2014089313 A1
- CN 108220295 B
- WO 2023017004 A1
- CN 115066498 A
- CN 116854754 A